# EUROPEAN PATENT APPLICATION

(11) **EP 4 219 761 A1**
(43) Date of publication of application: **02.08.2023**
(21) Application number: 23158699.1
(22) Date of filing: 14.03.2014
(51) Int. Cl.: C12Q 1/6883

(54) **BIOMARKERS FOR DIAGNOSIS OF LUNG DISEASES AND METHODS OF USE THEREOF**

(30) Priority: 15.03.2013 US 201361799754 P
(62) Divisional of application: 14764225.0
(71) Applicant: Veracyte, Inc., South San Francisco, CA 94080 (US)
(72) Inventor: WILDE, Jonathan, South San Francisco, 94080 (US); VELICHKO, Sharlene, South San Francisco, 94080 (US); BARBACIORU, Catalin, South San Francisco, 94080 (US); DIGGANS, James, South San Francisco, 94080 (US); KENNEDY, Giulia, South San Francisco, 94080 (US)
(74) Representative: Cabinet Becker et Associés

(57) **Abstract**

The present disclosure provides methods for diagnosis of interstitial lung diseases (ILDs). The present disclosure provides methods for differential diagnosis of idiopathic pulmonary fibrosis from other ILDs. Compositions and kits useful in carrying out a subject method are also provided.

## Description

### CROSS-REFERENCE

This application claims the benefit of U.S. Provisional Patent Application No. 61/799,754, filed March 15, 2013, which application is incorporated herein by reference in its entirety.

### INTRODUCTION

Interstitial Lung Disease (ILD), also known as diffuse parenchymal lung disease (DPLD), represent a variety of disorders that lead to diffuse remodeling, architectural damage to normal lung tissue and inflammation that lead to progressive loss of lung function. In addition to the inflammation and fibrosis that is often seen in the lung parenchyma in ILD, the airways and the vasculature may also be prominently affected. The most prominent forms of ILD are IPF and pulmonary sarcoidosis. Some clinical findings are common to the ILDs: exertional dyspnea or cough; bilateral diffuse interstitial infiltrates on chest radiographs; physiological and gas exchange abnormalities including a decreased carbon monoxide diffusion capacity (DLCO) and an abnormal alveolar-arteriolar PO₂ difference; and histopathologic abnormalities of the pulmonary parenchyma that are characterized by varying degrees of inflammation, fibrosis and remodeling. The incidence of ILD is estimated to be 31.5 per 100,000/year in males and 26.1 per 100,000/year in females and the clinical prognosis of these diseases range from mild illness to respiratory failure and death. The standard therapies for ILD include corticosteroids and immunosuppressive agents but current treatments are variably effective depending on the specific disease entity being treated.

Idiopathic pulmonary fibrosis (IPF) is a chronic, progressive fibrotic disorder of the lower respiratory tract. In contrast to other ILDs, there are currently no effective treatments for IPF. Increasing fibrosis leads to decreasing lung function and patients usually die of respiratory failure or other complications within three years of biopsy-confirmed diagnosis. While high resolution computed tomography (HRCT) has aided significantly in the diagnosis of interstitial lung diseases (ILD) the classical usual interstitial pneumonia (UIP) pattern observed in IPF is shared by many other ILDs, and a comprehensive clinical and occupational history is essential to rule out treatable diseases. The current diagnostic paradigm for diagnosing ILDs is costly, time consuming, and often leaves a significant proportion of patients languishing with under- or over-treatment and the morbid consequences of such.

There is a need in the art for methods of diagnosing ILDs.

### SUMMARY

The present disclosure provides methods for diagnosis of interstitial lung diseases (ILDs). The present disclosure provides methods for differential diagnosis of idiopathic pulmonary fibrosis from other ILDs. Compositions and kits useful in carrying out a subject method are also provided.

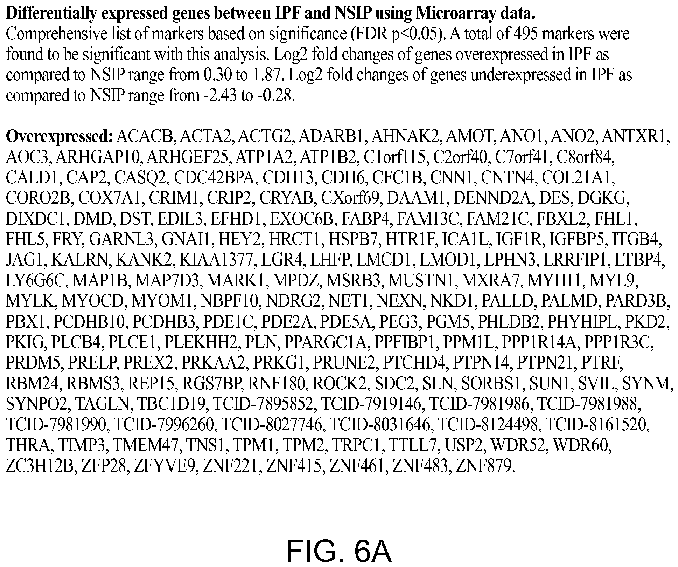

The present disclosure provides methods for evaluating a lung tissue, the method comprising: a) determining an expression level of a gene product of a gene set forth in any of Figures 6A-27E, 33A-B, or 34A-B, or in any of Tables 3, 5, 8, and 9, or Example 3, in a lung tissue sample obtained from a patient, generating an expression level value; and b) classifying the lung tissue sample as an interstitial lung disease (ILD) tissue sample by comparing the expression level value to a reference expression level value.

The present disclosure provides methods of diagnosing an interstitial lung disease (ILD) in a patient, the method comprising: a) determining an expression level of a gene product of a gene set forth in any one of Figures 6A-27E, 33A-B, or 34A-B, or in any of Tables 3, 5, 8, and 9, or in Example 3, in a lung tissue sample obtained from a patient suspected of having an ILD; and b) providing a diagnosis of an ILD based on comparison of the expression level value to a reference expression level value.

The present disclosure provides methods of diagnosing an interstitial lung disease (ILD) in patient, the method comprising: a) assaying, in a tissue sample obtained from a patient suspected of having an ILD, an expression level of a gene product of a gene set forth in any one of Figures 6A-27E, 33A-B, or 34A-B, or in any of Tables 3, 5, 8, and 9, or in Example 3, generating an expression level value; b) identifying the patient as having an ILD when the expression level value differs significantly from a reference gene expression level value; and c) outputting a report indicating that the patient has an ILD based on said identifying to facilitate a treatment decision by a clinician.

The present disclosure provides methods of diagnosing an interstitial lung disease (ILD), the method comprising: a) assaying, in a tissue sample obtained from a patient suspected of having an ILD, an expression level of a gene product of a gene set forth in any one of Figures 6A-27E, 33A-B, or 34A-B, or in any of Tables 3, 5, 8, and 9, or in Example 3, generating an expression level value; and b) inputting the expression level value into a computer programmed to execute an algorithm that compares the expression level value to expression level value for a reference expression level value and determines whether the expression level value differs significantly from a reference expression level value, said inputting generating a result from execution of the algorithm; and c) generating a report providing the result.

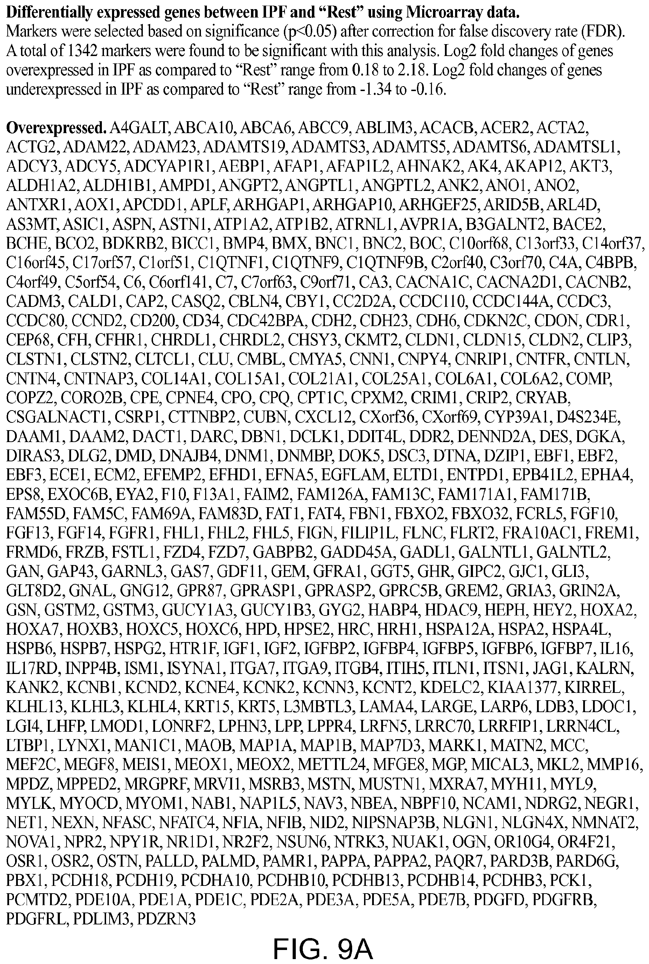

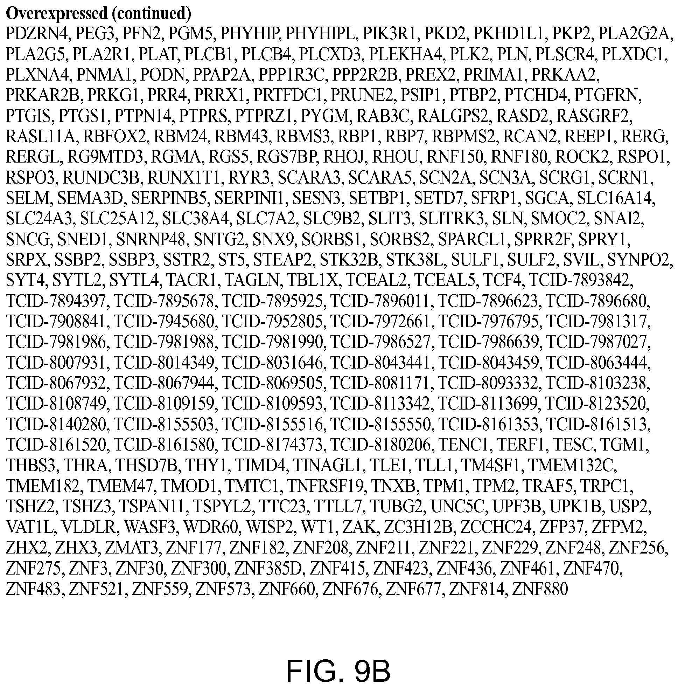

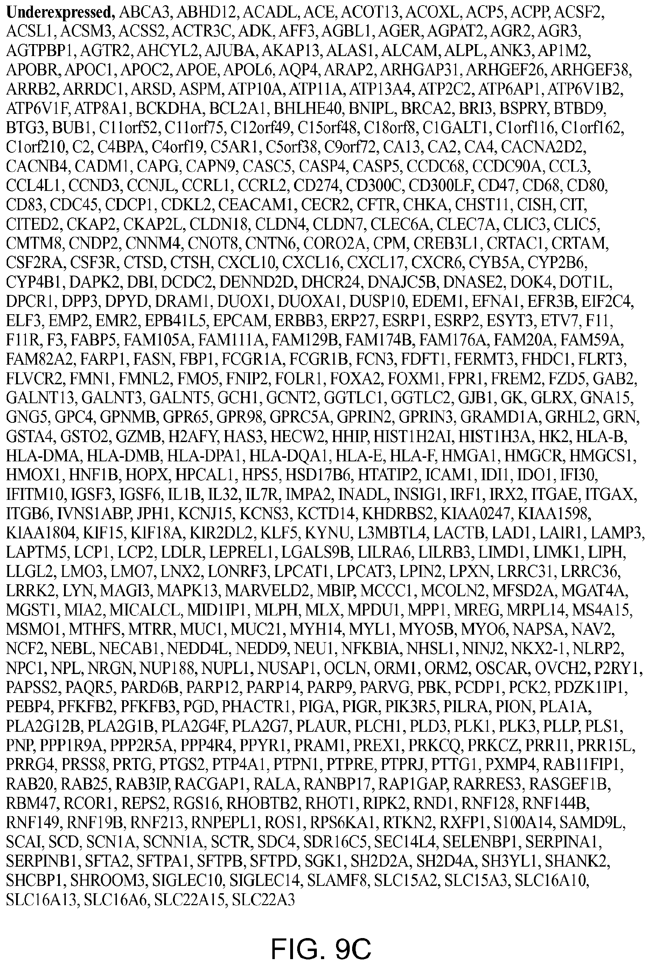

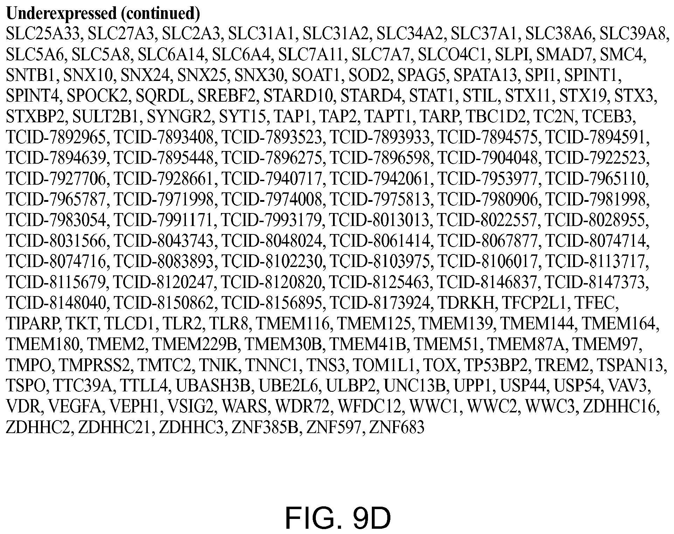

The present disclosure provides methods of diagnosing idiopathic pulmonary fibrosis (IPF) in a patient, the method comprising: a) assaying, in a tissue sample obtained from a patient suspected of having an interstitial lung disease, an expression level of a gene product of a gene set forth in any one of Figures 9A-9D, 10A-10G, 19A-22C, and 33A-B, or Table 4, generating an expression level value; b) identifying the patient as having IPF when the expression level value differs significantly from a reference expression level value.

In related embodiments, the ILD is idiopathic pulmonary fibrosis, sarcoidosis, Hamman-Rich syndrome, antisynthetase syndrome, silicosis, asbestosis, berylliosis, hypersensitivity pneumonitis, or non-specific interstitial pneumonia. In further related embodiments, the ILD is associated with a connective tissue disease selected from systemic sclerosis, polymyositis, systemic lupus erythematosus, or rheumatoid arthritis. In further embodiments, the ILD is drug induced or results from a viral infection, a bacterial infection, or tuberculosis.

In some embodiments, the methods of the present disclosure, the diagnosis provides for diagnosis of an ILD versus lack of an ILD. In some embodiments, the methods of the present disclosure, the diagnosis provides for diagnosis of a type of ILD (e.g., IPF, hypersensitivity pneumonia, NSIP, and the like). In some embodiments, the methods of the present disclosure, the diagnosis provides for differentiation between types of ILDs (e.g., between idiopathic pulmonary fibrosis (IPF) and an ILD other than IPF (e.g., IPF versus non-specific interstitial pneumonia; IPF versus hypersensitivity pneumonia, etc.).

In some embodiments, the gene used in the methods is selected from 1) ASPM; 2) BUB1; 3) PTTG1; 4) SHCBP1; 5) NUSAP1; 6) MKI67; 7) HJURP; 8) CDCA3; 9) PLK1; 10) PRR11; 11) BRCA2; 12) ORM1; 13) CCNB2; 14) SMC4; 15) HM13; 16) DMD; 17) FHL1; 18) ORM2; 19) NDUFC2-KCTD14; 20) NCAPH; 21) TTLL7; 22) DEPDC1B; 23) CNTN4; 24) PRKAA2; 25) PRKCQ; 26) CDC42BPA; 27) PARD3B; 28) SCTR; 29) CSF3R; and 30) MPDZ.

In some embodiments, the methods include creating a report summarizing said diagnosis. In some embodiments, the methods include providing a recommendation for treatment of the ILD.

In some embodiments, the gene product is mRNA. In related embodiments, the method includes assaying by determining a level of mRNA using a microarray, serial analysis of gene expression, blotting, reverse transcription-polymerase chain reaction, sequencing, or quantitative polymerase chain reaction. In some embodiments, the expression level is normalized relative to the expression level of an RNA transcript of at least one reference gene.

In some embodiments, the methods include obtaining a normalized expression of a gene product of a gene in a sample and comparing the normalized expression level to gene expression data for at least two different sets of biomarkers, the gene expression data for each set of biomarkers comprising one or more reference gene expression levels correlated with the presence of one or more tissue types, wherein said expression level is compared to gene expression data for said at least two sets of biomarkers sequentially. In related embodiments, the sequential comparison ends with comparing said expression level to gene expression data for a final set of biomarkers by analyzing said expression level using a main classifier, said main classifier obtained from gene expression data from one or more sets of biomarkers.

In some embodiments, methods involve analysis of a gene set forth in any one or more of Figures 6A-27E, Figures 33A-B, Figures 34A-B, Table 3, Table 5, Table 8, and/or Table 9.

The methods of the present disclosure include methods of modifying therapy of a patient, the method comprising: diagnosing an interstitial lung disease (ILD) in the patient, according to the methods of the present disclosure, and modifying therapy in the patient according to said diagnosing.

The methods of the present disclosure include methods of modifying therapy of a patient, the method comprising: diagnosing an interstitial lung disease (ILD) in the patient, according to the methods of the present disclosure, and treating the individual for the ILD. For example, if diagnosing indicates that the individual has idiopathic pulmonary fibrosis, said treating step comprises administering to the individual an effective amount of pirfenidone, prednisone, azathioprine, or N-acetylcysteine.

The present disclosure provides arrays comprising a plurality of nucleic acids, each of which hybridizes to a gene differentially expressed in a cell present in a tissue sample obtained from an individual being tested for an interstitial lung disease. The present disclosure provides kits for analyzing a lung tissue sample, the kit comprising an array of the present disclosure and a reagent for analyzing an expression level of a gene product.

### BRIEF DESCRIPTION OF THE DRAWINGS

Figure 1 depicts classification error rate of IPF vs. NSIP using a 30-gene signature.
Figure 2 depicts classification of IPF vs. NSIP using a 30-gene signature.
Figure 3 depicts classification error rate of IPF vs. Normal using a 50-gene signature.
Figure 4 depicts classification of IPF vs. Normal using a 50-gene signature.
Figure 5 provides a general schematic of a computerized system for use in the methods of the present disclosure.
Figures 6A and 6B provide genes differentially expressed between IPF and NSIP using microarray data.
Figure 7 provides genes differentially expressed between IPF and NSIP using RNA-Seq.
Figure 8 provides genes differentially expressed between IPF and NSIP using RNA-Seq.
Figures 9A-27E provide lists of differentially expressed genes that are suitable biomarkers.
Figures 28A-E depict accuracy of ILD classifiers.
Figures 29A-C depict classifier comparisons.
Figure 30 depicts IPF expression signal hypothesis.
Figure 31 depicts classification error rate of IPF vs. NSIP using a 9-gene signature.
Figure 32 depicts classification error rate of IPF vs. NSIP using a 15-gene signature.
Figures 33A-B are tables listing biomarkers useful for IPF vs. Rest/non-IPF classification.
Figures 34A-B are tables listing biomarkers useful for IPF vs. ILD inflammation.
Figure 35 provides ENSEMBL identifiers and corresponding gene symbols.

### DEFINITIONS

"Interstitial lung disease" or "ILD" (also known as diffuse parenchymal lung disease (DPLD)) as used herein refers to a group of lung diseases affecting the interstitium (the tissue and space around the air sacs of the lungs). ILD can be classified according to a suspected or known cause, or can be idiopathic. For example, ILD can be classified as caused by inhaled substances (inorganic or organic), drug induced (e.g., antibiotics, chemotherapeutic drugs, antiarrhythmic agents, statins), associated with connective tissue disease (e.g., systemic sclerosis, polymyositis, dermatomyositis, systemic lupus erythematous, rheumatoid arthritis), associated with pulmonary infection (e.g., atypical pneumonia, Pneumocystis pneumonia (PCP), tuberculosis, Chlamydia trachomatis, Respiratory Syncytial Virus), associated with a malignancy (e.g., Lymphangitic carcinomatosis), or can be idiopathic (e.g., sarcoidosis, idiopathic pulmonary fibrosis, Hamman-Rich syndrome, antisynthetase syndrome).

"ILD Inflammation" as used herein refers to an analytical grouping of inflammatory ILD subtypes characterized by underlying inflammation. These subtypes can be used collectively as a comparator against IPF and/or any other non-inflammation lung disease subtype. "ILD inflammation" can include HP, NSIP, sarcoidosis, and/or organizing pneumonia.

"Idiopathic interstitial pneumonia" or "IIP" (also referred to as noninfectious pneumonia" refers to a class of ILDs which includes, for example, desquamative interstitial pneumonia, nonspecific interstitial pneumonia, lymphoid interstitial pneumonia , cryptogenic organizing pneumonia, and idiopathic pulmonary fibrosis.

"Idiopathic pulmonary fibrosis" or "IPF" as used herein refers to a chronic, progressive form of lung disease characterized by fibrosis of the supporting framework (interstitium) of the lungs. By definition, the term is used when the cause of the pulmonary fibrosis is unknown ("idiopathic"). Microscopically, lung tissue from patients having IPF shows a characteristic set of histologic/pathologic features known as usual interstitial pneumonia (UIP), which is a pathologic counterpart of IPF.

"Nonspecific interstitial pneumonia" or "NSIP" is a form of idiopathic interstitial pneumonia generally characterized by a cellular pattern defined by chronic inflammatory cells with collagen deposition that is consistent or patchy, and a fibrosing pattern defined by a diffuse patchy fibrosis. In contrast to UIP, there is no honeycomb appearance nor fibroblast foci that characterize usual interstitial pneumonia.

"Hypersensitivity pneumonitis" or "HP" refers to also called extrinsic allergic alveolitis, EAA) refers to an inflammation of the alveoli within the lung caused by an exaggerted immune response and hypersensitivity to as a result of an inhaled antigen (e.g., organic dust).

"Pulmonary sarcoidosis" or "PS" refers to a syndrome involving abnormal collections of chronic inflammatory cells (granulomas) that can form as nodules. The inflammatory process for HP generally involves the alveoli, small bronchi, and small blood vessels. In acute and subacute cases of HP, physical examination usually reveals dry rales.

The term "microarray" refers to an ordered arrangement of hybridizable array elements, preferably polynucleotide probes, on a substrate.

The term "polynucleotide," when used in singular or plural, generally refers to any polyribonucleotide or polydeoxribonucleotide, which may be unmodified RNA or DNA or modified RNA or DNA. Thus, for instance, polynucleotides as defined herein include, without limitation, single- and double-stranded DNA, DNA including single- and double-stranded regions, single- and double-stranded RNA, and RNA including single- and double-stranded regions, hybrid molecules comprising DNA and RNA that may be single-stranded or, more typically, double-stranded or include single- and double-stranded regions. In addition, the term "polynucleotide" as used herein refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide. The term "polynucleotide" can also include DNAs (e.g., cDNAs) and RNAs that contain one or more modified bases (e.g., to provide a detectable signal, such as a fluorophore). Thus, DNAs or RNAs with backbones modified for stability or for other reasons are "polynucleotides" as that term is intended herein. Moreover, DNAs or RNAs comprising unusual bases, such as inosine, or modified bases, such as tritiated bases, are included within the term "polynucleotides" as defined herein. In general, the term "polynucleotide" embraces all chemically, enzymatically and/or metabolically modified forms of unmodified polynucleotides, as well as the chemical forms of DNA and RNA characteristic of viruses and cells, including simple and complex cells.

The term "oligonucleotide" refers to a relatively short polynucleotide (e.g., 100, 50, 20 or fewer nucleotides) including, without limitation, single-stranded deoxyribonucleotides, single- or double-stranded ribonucleotides, RNA:DNA hybrids and double-stranded DNAs. Oligonucleotides, such as single-stranded DNA probe oligonucleotides, are often synthesized by chemical methods, for example using automated oligonucleotide synthesizers that are commercially available. However, oligonucleotides can be made by a variety of other methods, including *in vitro* recombinant DNA-mediated techniques and by expression of DNAs in cells and organisms.

The terms "gene product" or "expression product" are used herein interchangeably to refer to the RNA transcription products (RNA transcript) of a gene, including mRNA, and the polypeptide translation product of such RNA transcripts. A gene product can be, for example, a polynucleotide gene expression product (e.g., an unspliced RNA, an mRNA, a splice variant mRNA, a microRNA, a fragmented RNA, and the like) or a protein expression product (e.g., a mature polypeptide, a post-translationally modified polypeptide, a splice variant polypeptide, and the like).

The term "normalized expression level" as applied to a gene expression product refers to a level of the gene product normalized relative to one or more reference (or control) gene expression products.

A "reference expression level value" as applied to a gene expression product refers to an expression level value for one or more reference (or control) gene expression products. A "reference normalized expression level value" as applied to a gene expression product refers to a normalized expression level value for one or more reference (or control) gene expression products.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to re-anneal when complementary strands are present in an environment below their melting temperature. The higher the degree of desired homology between the probe and hybridizable sequence, the higher the relative temperature that can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, (Wiley Interscience, 1995).

"Stringent conditions" or "high stringency conditions", as defined herein, typically: (1) employ low ionic strength solutions and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1 % polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately stringent conditions" may be identified as described by Sambrook et al., Molecular Cloning: A Laboratory Manual (Cold Spring Harbor Press, 1989), and include the use of washing solution and hybridization conditions (e.g., temperature, ionic strength and %SDS) less stringent that those described above. An example of moderately stringent condition is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

"Sensitivity" as used herein refers to the proportion of true positives of the total number tested who actually have the target disorder (i.e., the proportion of patients with the target disorder who have a positive test result). "Specificity" as used herein refers to the proportion of true negatives of all the patients tested who actually do not have the target disorder (i.e., the proportion of patients without the target disorder who have a negative test result).

In the context of the present invention, reference to "at least one," "at least two," "at least five," etc. of the genes listed in any particular gene set means any one or any and all combinations of the genes listed.

The terms "splicing" and "RNA splicing" are used interchangeably and refer to RNA processing that removes introns and joins exons to produce mature mRNA with continuous coding sequence that moves into the cytoplasm of a eukaryotic cell.

The term "exon" refers to any segment of an interrupted gene that is represented in a mature RNA product (B. Lewin,Genes IV(Cell Press, 1990)). In theory the term "intron" refers to any segment of DNA that is transcribed but removed from within the transcript by splicing together the exons on either side of it. Operationally, exon sequences occur in the mRNA sequence of a gene as defined by Ref. SEQ ID numbers. Operationally, intron sequences are the intervening sequences within the genomic DNA of a gene, bracketed by exon sequences and usually having GT and AG splice consensus sequences at their 5' and 3' boundaries.

A "computer-based system" refers to a system of hardware, software, and data storage medium used to analyze information. The minimum hardware of a patient computer-based system comprises a central processing unit (CPU), and hardware for data input, data output (e.g., display), and data storage. An ordinarily skilled artisan can readily appreciate that any currently available computer-based systems and/or components thereof are suitable for use in connection with the methods of the present disclosure. The data storage medium may comprise any manufacture comprising a recording of the present information as described above, or a memory access device that can access such a manufacture.

To "record" data, programming or other information on a computer readable medium refers to a process for storing information, using any such methods as known in the art. Any convenient data storage structure may be chosen, based on the means used to access the stored information. A variety of data processor programs and formats can be used for storage, e.g. word processing text file, database format, etc.

A "processor" or "computing means" references any hardware and/or software combination that will perform the functions required of it. For example, a suitable processor may be a programmable digital microprocessor such as available in the form of an electronic controller, mainframe, server or personal computer (desktop or portable). Where the processor is programmable, suitable programming can be communicated from a remote location to the processor, or previously saved in a computer program product (such as a portable or fixed computer readable storage medium, whether magnetic, optical or solid state device based). For example, a magnetic medium or optical disk may carry the programming, and can be read by a suitable reader communicating with each processor at its corresponding station.

Before the present invention is further described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described. All publications mentioned herein are incorporated herein by reference to disclose and describe the methods and/or materials in connection with which the publications are cited.

It must be noted that as used herein and in the appended claims, the singular forms "a," "an," and "the" include plural referents unless the context clearly dictates otherwise. Thus, for example, reference to "a gene product" includes a plurality of such gene products and reference to "the algorithm" includes reference to one or more algorithms and equivalents thereof known to those skilled in the art, and so forth. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments pertaining to the invention are specifically embraced by the present invention and are disclosed herein just as if each and every combination was individually and explicitly disclosed. In addition, all sub-combinations of the various embodiments and elements thereof are also specifically embraced by the present invention and are disclosed herein just as if each and every such sub-combination was individually and explicitly disclosed herein.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### DETAILED DESCRIPTION

The present disclosure provides methods for diagnosis of interstitial lung diseases (ILDs). The present disclosure provides methods for differential diagnosis of idiopathic pulmonary fibrosis from other ILDs. Compositions and kits useful in carrying out a subject method are also provided.

### METHODS FOR EVALUATING A LUNG TISSUE SAMPLE

The present disclose provides methods for evaluating a lung tissue, where the methods generally involve: a) determining an expression level of a gene product of a gene set forth in any one of Figures 6A-27E, 33A-B, or 34A-B, or any of Tables 3, 4, 8, and 9, or set out in Example 3, in a lung tissue sample obtained from a patient, generating an expression level value; and b) classifying the lung tissue sample as an ILD tissue sample by comparing the expression level value (e.g., a normalized expression level value) to gene expression data based on a population study comprising ILD tissue.

### METHODS FOR DIAGNOSING AN INTERSTITIAL LUNG DISEASE

The present disclosure provides methods for diagnosis of interstitial lung diseases (ILDs). The methods generally involve determining an expression level (e.g., a normalized expression level) of a gene product of a gene set forth in any one of Figures 6A-27E, 33A-B, or 34A-B, or any of Tables 3, 4, 8, and 9, or set out in Example 3, in a lung tissue sample obtained from a patient, generating an expression level value; and classifying the lung tissue sample as an interstitial lung disease (ILD) tissue sample by comparing the expression level value (e.g., a normalized expression level value) to gene expression data based on a population study comprising ILD tissue.

"ILD" refers to a group of lung diseases affecting the interstitium (the tissue and space around the air sacs of the lungs). Lung tissues affected by ILD include alveolar epithelium, pulmonary capillary endothelium, basement membrane, perivascular, and perilymphatic lung tissues. The ILDs can be classified into seven main groups: iatrogenic or drug-induced; occupational or environmental; granulomatous diseases including pulmonary sarcoidosis collagen-vascular disease; unique entities such as alveolar proteinosis, Langerhans cell granulomatosis, and lymphangioleiomyomatosis; idiopathic interstitial pneumonias including idiopathic pulmonary fibrosis (IPF); and inherited disorders such as tuberous sclerosis, neurofibromatosis, metabolic storage disorders and Hermansky-Pudlak syndrome.

ILDs include, but are not limited to, idiopathic pulmonary fibrosis; lymphangioleiomyomatosis; nonspecific interstitial pneumonia; cryptogenic organizing pneumonia; acute interstitial pneumonia; respiratory bronchiolitis-associated interstitial lung disease; desquamative interstitial pneumonia; lymphocytic interstitial pneumonia; and pulmonary sarcoidosis. ILDs can be associated with a connective tissue disease selected from systemic sclerosis, polymyositis, systemic lupus erythematosus, or rheumatoid arthritis. ILDs can also be drug induced, e. g., induced by antibiotics, chemotherapeutic agents, antiarrhythmia agents, statins, and the like. ILDs can also result from a viral infection, a bacterial infection, or tuberculosis.

In carrying out a subject diagnostic method, expression levels of a gene expression product ("biomarker"), or a set (or "panel") of biomarkers (e.g., a "set of genes"), can be assayed. Examples of biomarkers for use in the methods of the present disclosure include the gene products of the genes of Figures 6A-27E, 33A-B, or 34A-B, and Tables 3, 4, 8, and 9 and Example 3.

The present methods and compositions contemplate the use of "biomarker panels" for purposes of identification, classification, diagnosis, or to otherwise characterize a biological sample. The methods and compositions may also use groups of biomarker panels, referred to herein as "classification panels," examples of which can be found in each of Figures 6A-27E, 33A-B, or 34A-B, and Tables 3, 4, 8, and 9. The pattern of levels of gene expression of biomarkers in a panel (or "signature") can be determined and then used to evaluate the signature of the same panel of biomarkers in a biological sample, such as by a measure of similarity between the sample signature and the reference signature. In some embodiments, the method involves measuring (or obtaining) the levels of two or more gene expression products that are within a biomarker panel and/or within a classification panel. For example, in some embodiments, a biomarker panel or a classification panel may contain at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 or more different biomarkers. In some embodiments, a biomarker panel or a classification panel contains no more than 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 different biomarkers. In some embodiments, a classification panel contains at least 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 different biomarker panels. In other embodiments, a classification panel contains no more than 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 different biomarker panels.

In some embodiments, the present disclosure provides a method of identifying, classifying, or diagnosing an ILD comprising the steps of: obtaining an expression level for one or more gene expression products of a biological sample; and identifying the biological sample as lacking an indication of the ILD assayed when the gene expression level in the biological sample indicates the absence of the ILD assayed. In some embodiments, the present invention provides a method of identifying, classifying, or diagnosing ILD comprising the steps of: obtaining an expression level for one or more gene expression products of a biological sample; and identifying the biological sample as affected with the ILD assayed when the gene expression level in the biological sample is indicative of the ILD assayed. For example, this can be accomplished by correlating the patterns of gene expression levels, as defined in classification panels described herein, with the gene expression level in the sample, in order to identify (or rule out) the presence of the presence of an ILD in the biological sample.

In some embodiments, the present disclosure provides a method of identifying, classifying, or diagnosing an ILD to provide a specificity and a sensitivity that each are at least 50%, or 70%, using the subject methods described herein, wherein the gene expression product levels are compared between the biological sample and a biomarker panel, or between the biological sample and a classification panel; and identifying the biological sample as affected, or unaffected, by the ILD being assayed based on the comparison of gene expression profiles. In some embodiments, the specificity of the present method is at least 50%, 60%, 70%, 75%, 80%, 85%, 86%, 87%, 88%, 89%, 90%, 91%, 92%, 93%, 94%, 95%, 96%, 97%, 98%, or 99%. In some embodiments, the sensitivity of the present method is at least 50%, 60%, 70%, 75%, 80%, 85%, 86%, 87%, 88%, 89%, 90%, 91%, 92%, 93%, 94%, 95%, 96%, 97%, 98%, or 99%. In some embodiments, the specificity is at least 50% and the sensitivity of the present method is at least 50%. In some embodiments, the specificity of the present method is at least 70% and the sensitivity of the present method is at least 70%. In some embodiments, the specificity is at least 50%, and the sensitivity is at least 70%.

In some embodiments, the nominal specificity is greater than or equal to 50%. In some embodiments, the nominal specificity is greater than or equal to 70%. In some embodiments, the nominal negative predictive value (NPV) is greater than or equal to 95%. In some embodiments, the NPV is at least 90%, 91%, 92%, 93%, 94%, 95%, 95.5%, 96%, 96.5%, 97%, 97.5%, 98%, 98.5%, 99%, 99.5% (e.g., 90%, 91%, 92%, 93%, 94%, 95%, 95.5%, 96%, 96.5%, 97%, 97.5%, 98%, 98.5%, 99%, 99.5%, or 100%) and the specificity (or positive predictive value (PPV)) is at least 30%, 35%, 40%, 50%, 60%, 70%, 80%, 90%, 95%, 95.5%, 96%, 96.5%, 97%, 97.5%, 98%, 98.5%, 99%, or 99.5% (e.g., 30%, 35%, 40%, 50%, 60%, 70%, 80%, 90%, 95%, 95.5%, 96%, 96.5%, 97%, 97.5%, 98%, 98.5%, 99%, 99.5%, or 100%). In some cases the NPV is at least 95%, and the specificity is at least 50%. In some cases the NPV is at least 95% and the specificity is at least 70%.

In some embodiments, there is a specific (or range of) difference in gene expression between subtypes or sets of samples being compared to one another. In some examples, the gene expression of some similar subtypes is merged to form a super-class that is then compared to another subtype, or another super-class, or the set of all other subtypes. In some embodiments, the difference in gene expression level is at least 5%, 10%, 15%, 20%, 25%, 30%, 35%, 40%, 45% or 50% or more. In some embodiments, the difference in gene expression level is at least 2, 3, 4, 5, 6, 7, 8, 9, 10 fold or more.

In some embodiments, the biological sample is identified as having an ILD (e.g., IPF, NSIP, and the like) with an accuracy of at least 50%, 60%, 70%, 75%, 80%, 85%, 90%, 95%, 99% or more. In some embodiments, the biological sample is identified as having an ILD with an accuracy of greater than 50%, 60%, 70%, 75%, 80%, 85%, 90%, 95%, 99% or more. In some embodiments, the accuracy is calculated using a trained algorithm. In some embodiments, the biological sample is identified as ILD-affected (e.g., affected with a selected ILD) with a sensitivity of greater than 50% or 70%. In some embodiments, the biological sample is identified as ILD-affected (e.g., affected with a selected ILD) with a specificity of greater than 50% or 70%. In some embodiments, the biological sample is identified as ILD-affected (e.g., affected with a selected ILD) with a sensitivity of greater than 50% and a specificity of greater than 70%.

In some embodiments, method uses a panel of biomarkers (e.g., biomarker panel, classification panel, classifier) such that the method has a specificity of greater than 50%, 70%, 75%, 80%, 85%, 86%, 87%, 88%, 89%, 90%, 91%, 92%, 93%, 94%, 95%, 96%, 97%, 98%, 99%, or 99.5%, and a sensitivity of greater than 50%, 70%, 75%, 80%, 85%, 86%, 87%, 88%, 89%, 90%, 91%, 92%, 93%, 94%, 95%, 96%, 97%, 98%, 99%, or 99.5%. In some embodiments, the method uses a panel of biomarkers (e.g., biomarker panel, classification panel, classifier) such that the method has a positive predictive value of at least 95%, 95.5%, 96%, 96.5%, 97%, 97.5%, 98%, 98.5%, 99%, 99.5% or more; and/or a negative predictive value of at least 95%, 95.5%, 96%, 96.5%, 97%, 97.5%, 98%, 98.5%, 99%, 99.5% or more. In some embodiments, the method uses a panel of biomarkers (e.g., biomarker panel, classification panel, classifier) such that the method has a specificity or sensitivity of greater than 50%, 70%, 75%, 80%, 85%, 86%, 87%, 88%, 89%, 90%, 91%, 92%, 93%, 94%, 95%, 96%, 97%, 98%, 99%, or 99.5%, and a positive predictive value or negative predictive value of at least 95%, 95.5%, 96%, 96.5%, 97%, 97.5%, 98%, 98.5%, 99%, 99.5% or more. In some embodiments, the method uses a panel of biomarkers (e.g., biomarker panel, classification panel, classifier) such that the method has a negative predictive value of at least 95%, 95.5%, 96%, 96.5%, 97%, 97.5%, 98%, 98.5%, 99%, 99.5% or more.

In some embodiments, the present disclosure provides gene expression products corresponding to biomarkers selected from those set out in Figures 6A-27E, 33A-B, or 34A-B, and Tables 3, 4, 8, and 9, and Example 3. The methods and compositions provided herein can include gene expression products corresponding to any or all of the biomarkers selected from Figures 6A-27E, 33A-B, and 34A-B, and Tables 3, 4, 8, and 9, and Example 3, as well as any subset thereof, in any combination. For example, the methods may use gene expression products corresponding to at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 or more of the biomarkers provided in Figures 6A-27E, 33A-B, and 34A-B, and Tables 3, 4, 8, and 9, and Example 3. In some cases, certain biomarkers may be excluded or substituted with other biomarkers, for example with biomarkers that exhibit a similar expression level profile with respect to a particular tissue type or sub-type.

Marker panels can be chosen to accommodate adequate separation of ILD affected from ILD unaffected and/or to provide differentiation of patients affected with a first ILD from patients affected with a second ILD different from the first ILD. Training of this multi-dimensional classifier, i.e., algorithm, can be performed on numerous biological samples, such as at least 50, 100, 200, 300, 400, 500, 600, 700, 800, 900, 1000, 1500, 2000, 2500, 3000, 3500, or 4000 biological samples (e.g., lung tissue samples). In some embodiments, many training/test sets are used to develop the preliminary algorithm. The overall algorithm error rate may be shown as a function of gene number for ILD vs. non-ILD (or first ILD vs. second ILD) samples. In some embodiments, other performance metric may be used, such as a performance metric that is a function of gene number for ILD vs. non-ILD (or first ILD vs. second ILD). Such performance metric may be obtained using cross-validation, or other methods known in the art. All results may be obtained using a support vector machine model or other classification methods which are trained and tested in a cross-validated mode on the samples.

The methods of the present disclosure can facilitate a diagnosis of an ILD by comparison of an expression level of a gene product of one or more genes in any of Figures 6A-27E, 33A-B, and 34A-B, or any of Tables 3, 4, 8, and 9, or set out in Example 3, and determining whether such expression level differs significantly from a reference expression level (e.g., an expression level of a gene product of the same gene in a tissue of a known indication (e.g., unaffected or affected).

In any one of Figures 6A-27E, 33A-B, and 34A-B, or any one of Tables 3, 4, 8, and 9, or set out in Example 3, a negative logFC value for a given gene indicates that an expression level of a gene product of the given gene is lower in a tissue of a first disease indication (e.g., IPF) as compared to a reference expression level (e.g., an expression level of a gene product of the given gene in a tissue of a second indication, e.g., NSIP). A reference expression level can be a gene expression level in a second disease indication, or can be a gene expression level in normal (non-diseased) tissue. Thus, a negative logFC value for a given gene indicates a decrease in expression is correlated with the presence of the first disease indication.

Conversely, a positive logFC value for a given gene indicates that an expression level of a gene product of the given gene is greater in a tissue of a first disease indication (e.g., IPF) as compared to a reference expression level (e.g., an expression level of a gene product of the given gene in a tissue of a second indication, e.g., NSIP). A reference expression level can be a gene expression level in a second disease indication, or can be a gene expression level in normal (non-diseased) tissue. Thus, a negative logFC value for a given gene indicates a decrease in expression is correlated with the presence of the first disease indication.

In one embodiment, expression levels of a given gene product(s) can be compared to a reference expression level(s) arrived at from a population study involving analyzing gene expression levels in lung tissue samples from multiple individuals.

Methods of the present disclosure comprising use of a reference expression level value (e.g., a reference normalized expression level value) encompass use a reference expression level value representing an expression level (e.g., a normalized expression level) of one or more reference (or control) genes. A reference expression level value that represents an expression level of more than one reference (or control) genes can be provided by application of an algorithm to reference expression level values (e.g., reference normalized expression level values) so as to provide a score, where the score represents a threshold score (also referred to as a "threshold score" or "cutoff" value) indicative of a diagnosis (e.g., a test score above a threshold score indicates a diagnosis of an ILD or a differential diagnosis of an ILD (e.g., IPF vs. NISP).

In one embodiment, LogFC values can be used a threshold level of an increase or decrease of test gene expression levels as compared to a reference gene expression level to assist in a diagnosis of an ILD based on a selected comparison, e.g., IPF vs. NSIP, etc. Nonlimiting examples of such LogFC values are provided in Figures 6A-27E, 33A-B, and 34A-B, and Tables 3, 4, 8, and 9.

Genes with desired LogFC values can be selected as features in training a classification algorithm. For example, LogFC values above 1.2 or less than -1.2 may be used to identify genes whose signals are used by various algorithms to achieve classification.

### Diagnosing hypersensitivity pneumonitis

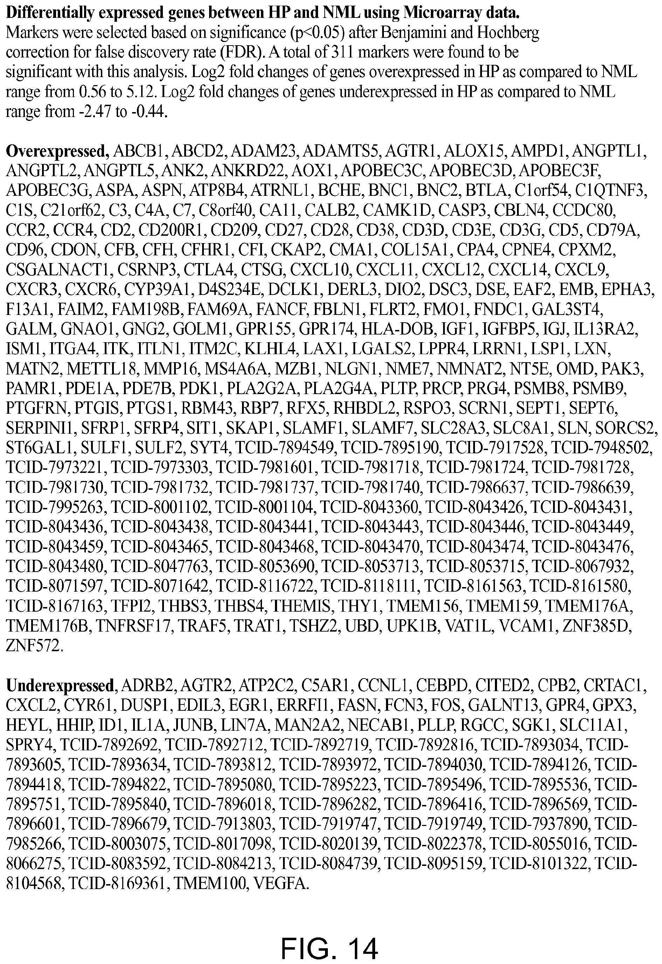

The present disclosure provides a method of diagnosing an ILD, where the ILD is hypersensitivity pneumonitis (HP). In some cases, the methods involve determining, in a lung tissue sample from a subject, an expression level of a gene product of a gene listed in Figures 14-16 (e.g., a normalized gene expression level). For example, a diagnosis of HP is indicated where an expression level of a gene product of one or more genes in Figures 14-16 (e.g., a normalized gene expression level) differs significantly from a threshold gene expression level value for the gene product(s).

In some cases, a subject method involves determining, in a lung tissue sample from a subject, an expression level (e.g., a normalized gene expression level) of a gene product of one or more genes selected from the genes in any of Figures 14-16, where the one or more genes can be a set of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 or more genes selected from the genes set forth in any of Figures 14-16.

### Diagnosing non-specific interstitial pneumonia

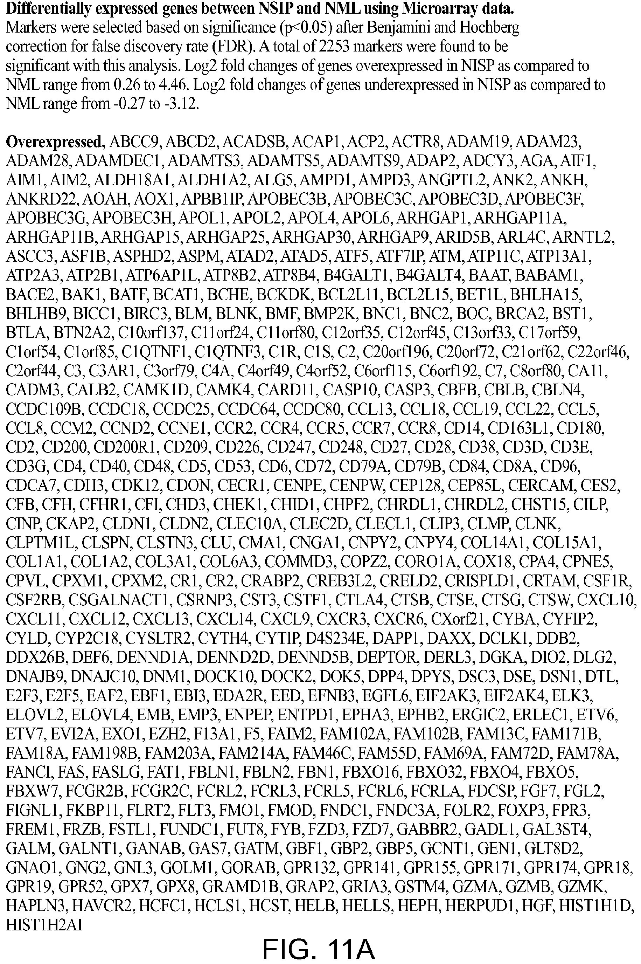

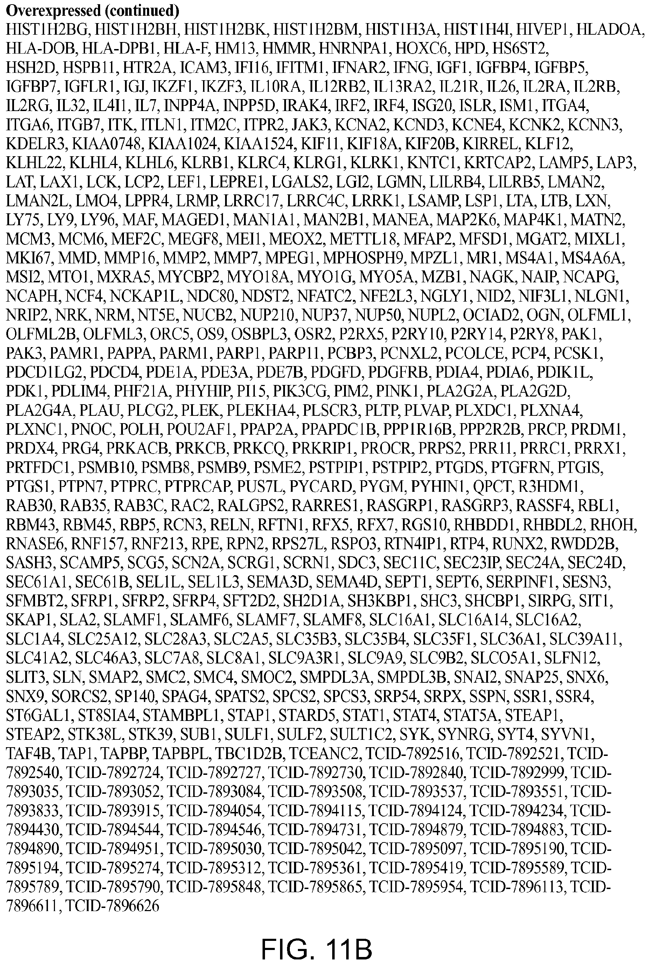

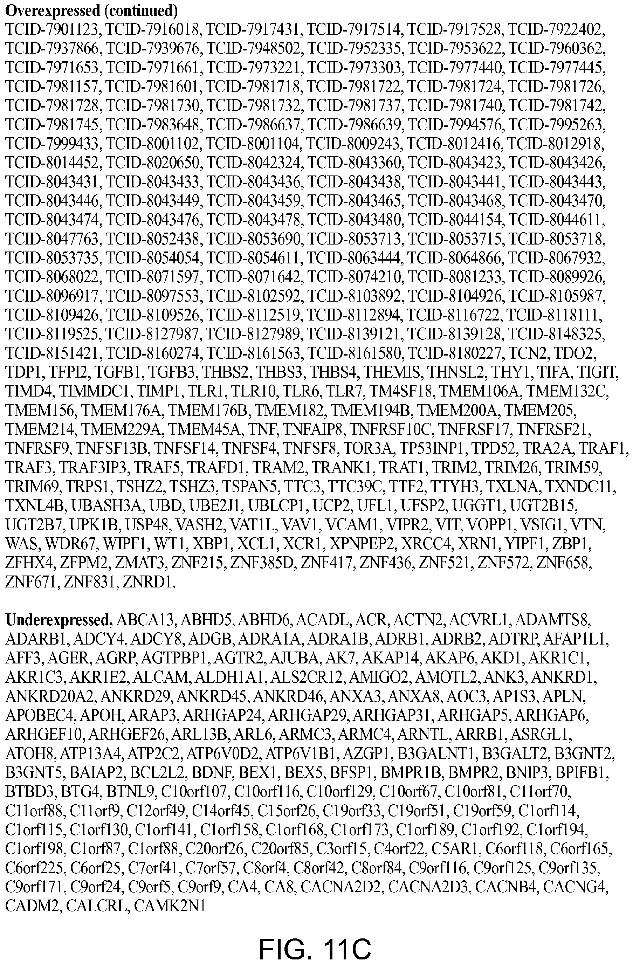

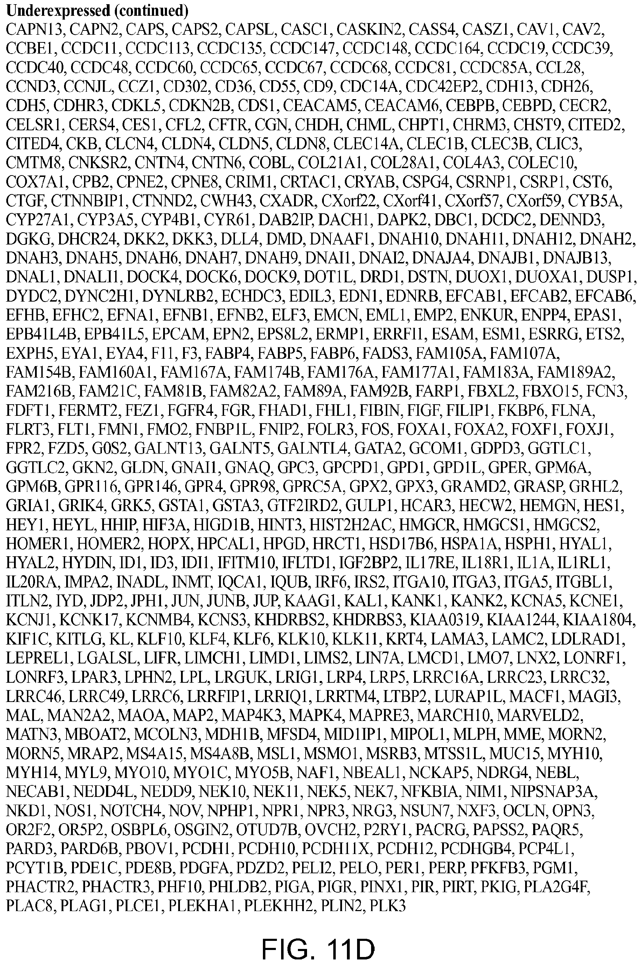

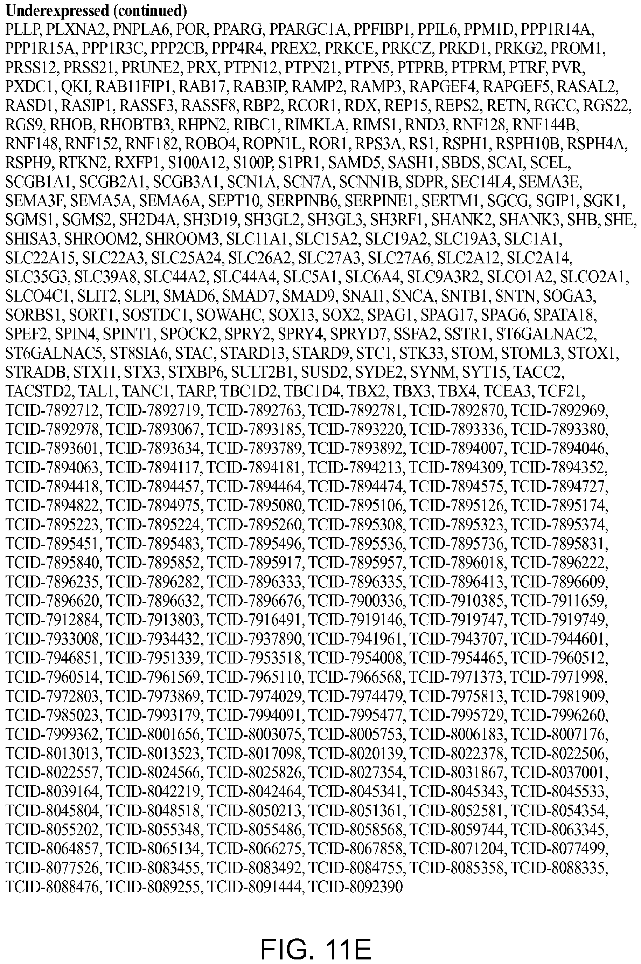

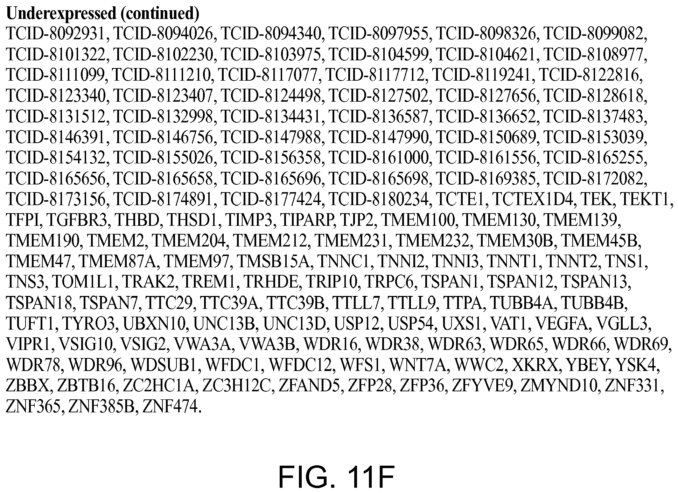

The present disclosure provides a method of diagnosing an ILD, where the ILD is non-specific interstitial pneumonia (NSIP). In some cases, the methods involve determining, in a lung tissue sample from a subject, an expression level of a gene product (e.g., a normalized gene expression level) of one or more genes in any one of Figures 11A-11F, 12, 25, and 26A-26E. For example, a diagnosis of NSIP is indicated where an expression level (e.g., a normalized expression level) of a gene product of a gene in any one of Figures 11A-11F, 12, 25, and 26A-26E differs significantly from a threshold gene expression level value for the gene product(s).

In some cases, a subject method involves determining, in a lung tissue sample from a subject, an expression level (e.g., a normalized expression level) of a gene product of one or more genes of any one of Figures 11A-11F, 12, 25, and 26A-26E, where the one or more genes can be or a set of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 or more genes selected from the genes listed in any one of any one of Figures 11A-11F, 12, 25, and 26A-26E.

### Diagnosing idiopathic pulmonary fibrosis

The present disclosure provides a method of diagnosing an ILD, where the ILD is IPF. In some cases, the methods involve determining, in a lung tissue sample from a subject, an expression level (e.g., a normalized expression level) of a gene product of one or more genes of any of Figures 9A-9D, 10A-10G, 19A-22C, and 33A-B, or Table 4. For example, a diagnosis of IPF is indicated where an expression level (e.g., a normalized expression level) of a gene product of one or more genes of any of Figures 9A-9D, 10A-10G, 19A-22C, and 33A-B, or Table 4 differs significantly from a threshold gene expression level value for the gene product(s).

In some cases, a subject method involves determining, in a lung tissue sample from a subject, an expression level (e.g., a normalized expression level) of a gene product of one or more genes of Figures 9A-9D, 10A-10G, 19A-22C, and 33, or Table 4, where the one more genes can be a set of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 or more genes selected from the genes of any of Figures 9A-9D, 10A-10G, 19A-22C, and 33, or Table 4.

In some cases, a subject method involves determining, in a lung tissue sample from a subject, an expression level (e.g., a normalized expression level) of a gene product of one or more genes of Table 4, where the one or more genes can be a set of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 or more genes selected from the genes in Table 4. For example, in some cases, a subject method involves determining, in a lung tissue sample from a subject, an expression level (e.g., a normalized expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or more genes) selected from: 1) THY1; 2) GALNT13; 3) CFH; 4) CRTAC1; 5) CHD3; 6) TSHZ2; 7) ENSG00000123119; 8) ISM1; 9) ENSG00000236972; 10) PDE7B; 11) SULF1; 12) FABP5; 13) FABP5; 14) PTGFRN; 15) IGFBP5; 16) CFHR1; 17) MS4A15; 18) FAM13C; 19) STC1; 20) RCAN2; 21) LG12; 22) CCND2; 23) DCLK1; 24) C22orf46; 25) IGF1; 26) CSF3R; 27) PPP4R4; 28) CEBPD; 29) NMNAT2; 30) EPB41L5; 31) ZNF385D; 32) FCN3; 33) CNTN6; 34) DGKA; 35) CSGALNACT1; 36) SYT15; 37) STX11; 38) ITSN1; 39) TMEM100; 40) EGFLAM; 41) C13orf15; 42) ENSG00000182010; 43) ERRFI1; 44) RGS16; 45) SLN; 46) ENSG00000146374; 47) TCID 8066275 ; 48) CSCL2; 49) ITLN1; and 50) PDE2A.

### Differential diagnosis of idiopathic pulmonary fibrosis vs. non-specific interstitial pneumonia

The present disclosure provides a method for differential diagnosis of IPF versus NSIP. The methods generally involve determining, in a lung tissue sample from a subject, a normalized expression level of a gene product of a gene set forth in any one of Figures 6A-8 and Tables 3 and 8.

In some cases, a subject method involves determining, in a lung tissue sample from a subject, an expression level (e.g., a normalized expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 or more genes) selected from the genes set forth in Figures 6A-8, Tables 3 and 8, and Example 3.

In some cases, a subject method involves determining, in a lung tissue sample from a subject, an expression level (e.g., a normalized gene expression level) of a gene product of one or more genes (e.g., a set of 2, 3, 4, 5, 6, 7, 8, 9, 10, 11, 12, 13, 14, or 15 genes) of the following gene set: CDKL2, CEACAM1, DST, EDIL3, HLA-F, KIF18A, LMOD1, MSRB3, MYH11, MYLK, PPP1R3C, PTCHD4, PTTG1, TMEM47, and TTLL7.

In some cases, a subject method involves determining, in a lung tissue sample from a subject, an expression level (e.g., a normalized gene expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29, or 30 more genes) of the following gene set: 1) ASPM; 2) BUB1; 3) PTTG1; 4) SHCBP1; 5) NUSAP1; 6) MKI67; 7) HJURP; 8) CDCA3; 9) PLK1; 10) PRR11; 11) BRCA2; 12) ORM1; 13) CCNB2; 14) SMC4; 15) HM13; 16) DMD; 17) FHL1; 18) ORM2; 19) NDUFC2-KCTD14; 20) NCAPH; 21) TTLL7; 22) DEPDC1B; 23) CNTN4; 24) PRKAA2; 25) PRKCQ; 26) CDC42BPA; 27) PARD3B; 28) SCTR; 29) CSF3R; and 30) MPDZ.

In some cases, a subject method involves determining, in a lung tissue sample from a subject, an expression level (e.g., a normalized gene expression level) of an ASPM gene product. In some of these embodiments, the method can further comprise determining an expression level (e.g., a normalized expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: 2) BUB1; 3) PTTG1; 4) SHCBP1; 5) NUSAP1; 6) MKI67; 7) HJURP; 8) CDCA3; 9) PLK1; 10) PRR11; 11) BRCA2; 12) ORM1; 13) CCNB2; 14) SMC4; 15) HM13; 16) DMD; 17) FHL1; 18) ORM2; 19) NDUFC2-KCTD14; 20) NCAPH; 21) TTLL7; 22) DEPDC1B; 23) CNTN4; 24) PRKAA2; 25) PRKCQ; 26) CDC42BPA; 27) PARD3B; 28) SCTR; 29) CSF3R; and 30) MPDZ.

In some cases, a subject method involves determining, in a lung tissue sample from a subject, an expression level (e.g., a normalized gene expression level) of a BUB1 gene product. In some of these embodiments, the method can further comprise determining an expression level (e.g., a normalized expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: 1) ASPM; 3) PTTG1; 4) SHCBP1; 5) NUSAP1; 6) MKI67; 7) HJURP; 8) CDCA3; 9) PLK1; 10) PRR11; 11) BRCA2; 12) ORM1; 13) CCNB2; 14) SMC4; 15) HM13; 16) DMD; 17) FHL1; 18) ORM2; 19) NDUFC2-KCTD14; 20) NCAPH; 21) TTLL7; 22) DEPDC1B; 23) CNTN4; 24) PRKAA2; 25) PRKCQ; 26) CDC42BPA; 27) PARD3B; 28) SCTR; 29) CSF3R; and 30) MPDZ.

In some cases, a subject method involves determining, in a lung tissue sample from a subject, an expression level (e.g., a normalized gene expression level) of a PTTG1 gene product. In some of these embodiments, the method can further comprise determining am expression level (e.g., a normalized level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: 1) ASPM; 2) BUB1; 4) SHCBP1; 5) NUSAP1; 6) MKI67; 7) HJURP; 8) CDCA3; 9) PLK1; 10) PRR11; 11) BRCA2; 12) ORM1; 13) CCNB2; 14) SMC4; 15) HM13; 16) DMD; 17) FHL1; 18) ORM2; 19) NDUFC2-KCTD14; 20) NCAPH; 21) TTLL7; 22) DEPDC1B; 23) CNTN4; 24) PRKAA2; 25) PRKCQ; 26) CDC42BPA; 27) PARD3B; 28) SCTR; 29) CSF3R; and 30) MPDZ.

In some cases, a subject method involves determining, in a lung tissue sample from a subject, an expression level (e.g., a normalized expression level) of a SHCBP1 gene product. In some of these embodiments, the method can further comprise determining an expression level (e.g., a normalized level) of a gene product of one or more genes, (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: 1) ASPM; 2) BUB1; 3) PTTG1; 5) NUSAP1; 6) MKI67; 7) HJURP; 8) CDCA3; 9) PLK1; 10) PRR11; 11) BRCA2; 12) ORM1; 13) CCNB2; 14) SMC4; 15) HM13; 16) DMD; 17) FHL1; 18) ORM2; 19) NDUFC2-KCTD14; 20) NCAPH; 21) TTLL7; 22) DEPDC1B; 23) CNTN4; 24) PRKAA2; 25) PRKCQ; 26) CDC42BPA; 27) PARD3B; 28) SCTR; 29) CSF3R; and 30) MPDZ.

In some cases, a subject method involves determining, in a lung tissue sample from a subject, an expression level (e.g., a normalized expression level) of a NUSAP1 gene product. In some of these embodiments, the method can further comprise determining an expression level (e.g., a normalized expression level) of a gene product of one or more of (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: 1) ASPM; 2) BUB1; 3) PTTG1; 4) SHCBP1; 6) MKI67; 7) HJURP; 8) CDCA3; 9) PLK1; 10) PRR11; 11) BRCA2; 12) ORM1; 13) CCNB2; 14) SMC4; 15) HM13; 16) DMD; 17) FHL1; 18) ORM2; 19) NDUFC2-KCTD14; 20) NCAPH; 21) TTLL7; 22) DEPDC1B; 23) CNTN4; 24) PRKAA2; 25) PRKCQ; 26) CDC42BPA; 27) PARD3B; 28) SCTR; 29) CSF3R; and 30) MPDZ.

In some cases, a subject method involves determining, in a lung tissue sample from a subject, an expression level (e.g., a normalized expression level) of a MKI67 gene product. In some of these embodiments, the method can further comprise determining an expression level (e.g., a normalized expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: 1) ASPM; 2) BUB1; 3) PTTG1; 4) SHCBP1; 5) NUSAP1; 7) HJURP; 8) CDCA3; 9) PLK1; 10) PRR11; 11) BRCA2; 12) ORM1; 13) CCNB2; 14) SMC4; 15) HM13; 16) DMD; 17) FHL1; 18) ORM2; 19) NDUFC2-KCTD14; 20) NCAPH; 21) TTLL7; 22) DEPDC1B; 23) CNTN4; 24) PRKAA2; 25) PRKCQ; 26) CDC42BPA; 27) PARD3B; 28) SCTR; 29) CSF3R; and 30) MPDZ.

In some cases, a subject method involves determining, in a lung tissue sample from a subject, an expression level (e.g., a normalized expression level) of an HJURP gene product. In some of these embodiments, the method can further comprise determining a an expression level (e.g., a normalized expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: 1) ASPM; 2) BUB1; 3) PTTG1; 4) SHCBP1; 5) NUSAP1; 6) MKI67; 8) CDCA3; 9) PLK1; 10) PRR11; 11) BRCA2; 12) ORM1; 13) CCNB2; 14) SMC4; 15) HM13; 16) DMD; 17) FHL1; 18) ORM2; 19) NDUFC2-KCTD14; 20) NCAPH; 21) TTLL7; 22) DEPDC1B; 23) CNTN4; 24) PRKAA2; 25) PRKCQ; 26) CDC42BPA; 27) PARD3B; 28) SCTR; 29) CSF3R; and 30) MPDZ.

In some cases, a subject method involves determining, in a lung tissue sample from a subject, an expression level (e.g., a normalized expression level) of a CDCA3 gene product. In some of these embodiments, the method can further comprise determining a an expression level (e.g., a normalized expression level) of one or more gene products (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: 1) ASPM; 2) BUB1; 3) PTTG1; 4) SHCBP1; 5) NUSAP1; 6) MKI67; 7) HJURP; 9) PLK1; 10) PRR11; 11) BRCA2; 12) ORM1; 13) CCNB2; 14) SMC4; 15) HM13; 16) DMD; 17) FHL1; 18) ORM2; 19) NDUFC2-KCTD14; 20) NCAPH; 21) TTLL7; 22) DEPDC1B; 23) CNTN4; 24) PRKAA2; 25) PRKCQ; 26) CDC42BPA; 27) PARD3B; 28) SCTR; 29) CSF3R; and 30) MPDZ.

In some cases, a subject method involves determining, in a lung tissue sample from a subject, an expression level (e.g., a normalized expression level) of a PKL1 gene product. In some of these embodiments, the method can further comprise determining an expression level (e.g., a normalized expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: 1) ASPM; 2) BUB1; 3) PTTG1; 4) SHCBP1; 5) NUSAP1; 6) MKI67; 7) HJURP; 8) CDCA3; 10) PRR11; 11) BRCA2; 12) ORM1; 13) CCNB2; 14) SMC4; 15) HM13; 16) DMD; 17) FHL1; 18) ORM2; 19) NDUFC2-KCTD14; 20) NCAPH; 21) TTLL7; 22) DEPDC1B; 23) CNTN4; 24) PRKAA2; 25) PRKCQ; 26) CDC42BPA; 27) PARD3B; 28) SCTR; 29) CSF3R; and 30) MPDZ.

In some cases, a subject method involves determining, in a lung tissue sample from a subject, an expression level (e.g., a normalized expression level) of a PRR11 gene product. In some of these embodiments, the method can further comprise determining an expression level (e.g., a normalized expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: 1) ASPM; 2) BUB1; 3) PTTG1; 4) SHCBP1; 5) NUSAP1; 6) MKI67; 7) HJURP; 8) CDCA3; 9) PLK1; 11) BRCA2; 12) ORM1; 13) CCNB2; 14) SMC4; 15) HM13; 16) DMD; 17) FHL1; 18) ORM2; 19) NDUFC2-KCTD14; 20) NCAPH; 21) TTLL7; 22) DEPDC1B; 23) CNTN4; 24) PRKAA2; 25) PRKCQ; 26) CDC42BPA; 27) PARD3B; 28) SCTR; 29) CSF3R; and 30) MPDZ.

In some cases, a subject method involves determining, in a lung tissue sample from a subject, an expression level (e.g., a normalized expression level) of a BRCA2 gene product. In some of these embodiments, the method can further comprise determining an expression level (e.g., a normalized expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: 1) ASPM; 2) BUB1; 3) PTTG1; 4) SHCBP1; 5) NUSAP1; 6) MKI67; 7) HJURP; 8) CDCA3; 9) PLK1; 10) PRR11; 12) ORM1; 13) CCNB2; 14) SMC4; 15) HM13; 16) DMD; 17) FHL1; 18) ORM2; 19) NDUFC2-KCTD14; 20) NCAPH; 21) TTLL7; 22) DEPDC1B; 23) CNTN4; 24) PRKAA2; 25) PRKCQ; 26) CDC42BPA; 27) PARD3B; 28) SCTR; 29) CSF3R; and 30) MPDZ.

In some cases, a subject method involves determining, in a lung tissue sample from a subject, an expression level (e.g., a normalized expression level) of an ORM1 gene product. In some of these embodiments, the method can further comprise determining a an expression level (e.g., a normalized expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: 1) ASPM; 2) BUB1; 3) PTTG1; 4) SHCBP1; 5) NUSAP1; 6) MKI67; 7) HJURP; 8) CDCA3; 9) PLK1; 10) PRR11; 11) BRCA2; 13) CCNB2; 14) SMC4; 15) HM13; 16) DMD; 17) FHL1; 18) ORM2; 19) NDUFC2-KCTD14; 20) NCAPH; 21) TTLL7; 22) DEPDC1B; 23) CNTN4; 24) PRKAA2; 25) PRKCQ; 26) CDC42BPA; 27) PARD3B; 28) SCTR; 29) CSF3R; and 30) MPDZ.

In some cases, a subject method involves determining, in a lung tissue sample from a subject, an expression level (e.g., a normalized expression level) of a CCNB2 gene product. In some of these embodiments, the method can further comprise determining an expression level (e.g., a normalized expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: 1) ASPM; 2) BUB1; 3) PTTG1; 4) SHCBP1; 5) NUSAP1; 6) MKI67; 7) HJURP; 8) CDCA3; 9) PLK1; 10) PRR11; 11) BRCA2; 12) ORM1; 14) SMC4; 15) HM13; 16) DMD; 17) FHL1; 18) ORM2; 19) NDUFC2-KCTD14; 20) NCAPH; 21) TTLL7; 22) DEPDC1B; 23) CNTN4; 24) PRKAA2; 25) PRKCQ; 26) CDC42BPA; 27) PARD3B; 28) SCTR; 29) CSF3R; and 30) MPDZ.

In some cases, a subject method involves determining, in a lung tissue sample from a subject, an expression level (e.g., a normalized expression level) of an SMC4 gene product. In some of these embodiments, the method can further comprise determining an expression level (e.g., a normalized expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: 1) ASPM; 2) BUB1; 3) PTTG1; 4) SHCBP1; 5) NUSAP1; 6) MKI67; 7) HJURP; 8) CDCA3; 9) PLK1; 10) PRR11; 11) BRCA2; 12) ORM1; 13) CCNB2; 15) HM13; 16) DMD; 17) FHL1; 18) ORM2; 19) NDUFC2-KCTD14; 20) NCAPH; 21) TTLL7; 22) DEPDC1B; 23) CNTN4; 24) PRKAA2; 25) PRKCQ; 26) CDC42BPA; 27) PARD3B; 28) SCTR; 29) CSF3R; and 30) MPDZ.

In some cases, a subject method involves determining, in a lung tissue sample from a subject, an expression level (e.g., a normalized expression level) of an HM13 gene product. In some of these embodiments, the method can further comprise determining an expression level (e.g., a normalized expression level) of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: 1) ASPM; 2) BUB1; 3) PTTG1; 4) SHCBP1; 5) NUSAP1; 6) MKI67; 7) HJURP; 8) CDCA3; 9) PLK1; 10) PRR11; 11) BRCA2; 12) ORM1; 13) CCNB2; 14) SMC4; 16) DMD; 17) FHL1; 18) ORM2; 19) NDUFC2-KCTD14; 20) NCAPH; 21) TTLL7; 22) DEPDC1B; 23) CNTN4; 24) PRKAA2; 25) PRKCQ; 26) CDC42BPA; 27) PARD3B; 28) SCTR; 29) CSF3R; and 30) MPDZ.

In some cases, a subject method involves determining, in a lung tissue sample from a subject, an expression level (e.g., a normalized expression level) of a DMD gene product. In some of these embodiments, the method can further comprise determining an expression level (e.g., a normalized expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: 1) ASPM; 2) BUB1; 3) PTTG1; 4) SHCBP1; 5) NUSAP1; 6) MKI67; 7) HJURP; 8) CDCA3; 9) PLK1; 10) PRR11; 11) BRCA2; 12) ORM1; 13) CCNB2; 14) SMC4; 15) HM13; 16) DMD; 17) FHL1; 18) ORM2; 19) NDUFC2-KCTD14; 20) NCAPH; 21) TTLL7; 22) DEPDC1B; 23) CNTN4; 24) PRKAA2; 25) PRKCQ; 26) CDC42BPA; 27) PARD3B; 28) SCTR; 29) CSF3R; and 30) MPDZ.

In some cases, a subject method involves determining, in a lung tissue sample from a subject, an expression level (e.g., a normalized expression level) of an FHL1 gene product. In some of these embodiments, the method can further comprise determining an expression level (e.g., a normalized expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: 1) ASPM; 2) BUB1; 3) PTTG1; 4) SHCBP1; 5) NUSAP1; 6) MKI67; 7) HJURP; 8) CDCA3; 9) PLK1; 10) PRR11; 11) BRCA2; 12) ORM1; 13) CCNB2; 14) SMC4; 15) HM13; 16) DMD; 18) ORM2; 19) NDUFC2-KCTD14; 20) NCAPH; 21) TTLL7; 22) DEPDC1B; 23) CNTN4; 24) PRKAA2; 25) PRKCQ; 26) CDC42BPA; 27) PARD3B; 28) SCTR; 29) CSF3R; and 30) MPDZ.

In some cases, a subject method involves determining, in a lung tissue sample from a subject, an expression level (e.g., a normalized expression level) of an ORM2 gene product. In some of these embodiments, the method can further comprise determining an expression level (e.g., a normalized expression level) of a gene production of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: 1) ASPM; 2) BUB1; 3) PTTG1; 4) SHCBP1; 5) NUSAP1; 6) MKI67; 7) HJURP; 8) CDCA3; 9) PLK1; 10) PRR11; 11) BRCA2; 12) ORM1; 13) CCNB2; 14) SMC4; 15) HM13; 16) DMD; 17) FHL1; 19) NDUFC2-KCTD14; 20) NCAPH; 21) TTLL7; 22) DEPDC1B; 23) CNTN4; 24) PRKAA2; 25) PRKCQ; 26) CDC42BPA; 27) PARD3B; 28) SCTR; 29) CSF3R; and 30) MPDZ.

In some cases, a subject method involves determining, in a lung tissue sample from a subject, an expression level (e.g., a normalized expression level) of a NDUFC2-KCTD14 gene product. In some of these embodiments, the method can further comprise determining an expression level (e.g., a normalized expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: 1) ASPM; 2) BUB1; 3) PTTG1; 4) SHCBP1; 5) NUSAP1; 6) MKI67; 7) HJURP; 8) CDCA3; 9) PLK1; 10) PRR11; 11) BRCA2; 12) ORM1; 13) CCNB2; 14) SMC4; 15) HM13; 16) DMD; 17) FHL1; 18) ORM2; 20) NCAPH; 21) TTLL7; 22) DEPDC1B; 23) CNTN4; 24) PRKAA2; 25) PRKCQ; 26) CDC42BPA; 27) PARD3B; 28) SCTR; 29) CSF3R; and 30) MPDZ.

In some cases, a subject method involves determining, in a lung tissue sample from a subject, an expression level (e.g., a normalized expression level) of an NCAPH gene product. In some of these embodiments, the method can further comprise determining a n expression level (e.g., a normalized expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: 1) ASPM; 2) BUB1; 3) PTTG1; 4) SHCBP1; 5) NUSAP1; 6) MKI67; 7) HJURP; 8) CDCA3; 9) PLK1; 10) PRR11; 11) BRCA2; 12) ORM1; 13) CCNB2; 14) SMC4; 15) HM13; 16) DMD; 17) FHL1; 18) ORM2; 19) NDUFC2-KCTD14; 21) TTLL7; 22) DEPDC1B; 23) CNTN4; 24) PRKAA2; 25) PRKCQ; 26) CDC42BPA; 27) PARD3B; 28) SCTR; 29) CSF3R; and 30) MPDZ.

In some cases, a subject method involves determining, in a lung tissue sample from a subject, an expression level (e.g., a normalized expression level) of a TTLL7 gene product. In some of these embodiments, the method can further comprise determining an expression level (e.g., a normalized expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: 1) ASPM; 2) BUB1; 3) PTTG1; 4) SHCBP1; 5) NUSAP1; 6) MKI67; 7) HJURP; 8) CDCA3; 9) PLK1; 10) PRR11; 11) BRCA2; 12) ORM1; 13) CCNB2; 14) SMC4; 15) HM13; 16) DMD; 17) FHL1; 18) ORM2; 19) NDUFC2-KCTD14; 20) NCAPH; 22) DEPDC1B; 23) CNTN4; 24) PRKAA2; 25) PRKCQ; 26) CDC42BPA; 27) PARD3B; 28) SCTR; 29) CSF3R; and 30) MPDZ.

In some cases, a subject method involves determining, in a lung tissue sample from a subject, an expression level (e.g., a normalized expression level) of a DEPDC1B gene product. In some of these embodiments, the method can further comprise determining an expression level (e.g., a normalized expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: 1) ASPM; 2) BUB1; 3) PTTG1; 4) SHCBP1; 5) NUSAP1; 6) MKI67; 7) HJURP; 8) CDCA3; 9) PLK1; 10) PRR11; 11) BRCA2; 12) ORM1; 13) CCNB2; 14) SMC4; 15) HM13; 16) DMD; 17) FHL1; 18) ORM2; 19) NDUFC2-KCTD14; 20) NCAPH; 21) TTLL7; 23) CNTN4; 24) PRKAA2; 25) PRKCQ; 26) CDC42BPA; 27) PARD3B; 28) SCTR; 29) CSF3R; and 30) MPDZ.

In some cases, a subject method involves determining, in a lung tissue sample from a subject, an expression level (e.g., a normalized expression level) of a CNTN4 gene product. In some of these embodiments, the method can further comprise determining an expression level (e.g., a normalized expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: 1) ASPM; 2) BUB1; 3) PTTG1; 4) SHCBP1; 5) NUSAP1; 6) MKI67; 7) HJURP; 8) CDCA3; 9) PLK1; 10) PRR11; 11) BRCA2; 12) ORM1; 13) CCNB2; 14) SMC4; 15) HM13; 16) DMD; 17) FHL1; 18) ORM2; 19) NDUFC2-KCTD14; 20) NCAPH; 21) TTLL7; 22) DEPDC1B; 24) PRKAA2; 25) PRKCQ; 26) CDC42BPA; 27) PARD3B; 28) SCTR; 29) CSF3R; and 30) MPDZ.

In some cases, a subject method involves determining, in a lung tissue sample from a subject, an expression level (e.g., a normalized expression level) of a PRKAA2 gene product. In some of these embodiments, the method can further comprise determining an expression level (e.g., a normalized expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: 1) ASPM; 2) BUB1; 3) PTTG1; 4) SHCBP1; 5) NUSAP1; 6) MKI67; 7) HJURP; 8) CDCA3; 9) PLK1; 10) PRR11; 11) BRCA2; 12) ORM1; 13) CCNB2; 14) SMC4; 15) HM13; 16) DMD; 17) FHL1; 18) ORM2; 19) NDUFC2-KCTD14; 20) NCAPH; 21) TTLL7; 22) DEPDC1B; 23) CNTN4; 25) PRKCQ; 26) CDC42BPA; 27) PARD3B; 28) SCTR; 29) CSF3R; and 30) MPDZ.

In some cases, a subject method involves determining, in a lung tissue sample from a subject, an expression level (e.g., a normalized expression level) of a PRKCQ gene product. In some of these embodiments, the method can further comprise determining an expression level (e.g., a normalized expression level) of a gene product one or more genes(e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: 1) ASPM; 2) BUB1; 3) PTTG1; 4) SHCBP1; 5) NUSAP1; 6) MKI67; 7) HJURP; 8) CDCA3; 9) PLK1; 10) PRR11; 11) BRCA2; 12) ORM1; 13) CCNB2; 14) SMC4; 15) HM13; 16) DMD; 17) FHL1; 18) ORM2; 19) NDUFC2-KCTD14; 20) NCAPH; 21) TTLL7; 22) DEPDC1B; 23) CNTN4; 24) PRKAA2; 26) CDC42BPA; 27) PARD3B; 28) SCTR; 29) CSF3R; and 30) MPDZ.

In some cases, a subject method involves determining, in a lung tissue sample from a subject, an expression level (e.g., a normalized expression level) of a CDC42BPA gene product. In some of these embodiments, the method can further comprise determining an expression level (e.g., a normalized expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: 1) ASPM; 2) BUB1; 3) PTTG1; 4) SHCBP1; 5) NUSAP1; 6) MKI67; 7) HJURP; 8) CDCA3; 9) PLK1; 10) PRR11; 11) BRCA2; 12) ORM1; 13) CCNB2; 14) SMC4; 15) HM13; 16) DMD; 17) FHL1; 18) ORM2; 19) NDUFC2-KCTD14; 20) NCAPH; 21) TTLL7; 22) DEPDC1B; 23) CNTN4; 24) PRKAA2; 25) PRKCQ; 27) PARD3B; 28) SCTR; 29) CSF3R; and 30) MPDZ.

In some cases, a subject method involves determining, in a lung tissue sample from a subject, an expression level (e.g., a normalized expression level) of a PARD3B gene product. In some of these embodiments, the method can further comprise determining an expression level (e.g., a normalized expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: 1) ASPM; 2) BUB1; 3) PTTG1; 4) SHCBP1; 5) NUSAP1; 6) MKI67; 7) HJURP; 8) CDCA3; 9) PLK1; 10) PRR11; 11) BRCA2; 12) ORM1; 13) CCNB2; 14) SMC4; 15) HM13; 16) DMD; 17) FHL1; 18) ORM2; 19) NDUFC2-KCTD14; 20) NCAPH; 21) TTLL7; 22) DEPDC1B; 23) CNTN4; 24) PRKAA2; 25) PRKCQ; 26) CDC42BPA; 28) SCTR; 29) CSF3R; and 30) MPDZ.

In some cases, a subject method involves determining, in a lung tissue sample from a subject, an expression level (e.g., a normalized expression level) of an SCTR gene product. In some of these embodiments, the method can further comprise determining an expression level (e.g., a normalized expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: 1) ASPM; 2) BUB1; 3) PTTG1; 4) SHCBP1; 5) NUSAP1; 6) MKI67; 7) HJURP; 8) CDCA3; 9) PLK1; 10) PRR11; 11) BRCA2; 12) ORM1; 13) CCNB2; 14) SMC4; 15) HM13; 16) DMD; 17) FHL1; 18) ORM2; 19) NDUFC2-KCTD14; 20) NCAPH; 21) TTLL7; 22) DEPDC1B; 23) CNTN4; 24) PRKAA2; 25) PRKCQ; 26) CDC42BPA; 27) PARD3B; 29) CSF3R; and 30) MPDZ.

In some cases, a subject method involves determining, in a lung tissue sample from a subject, an expression level (e.g., a normalized expression level) of a CSF3R gene product. In some of these embodiments, the method can further comprise determining an expression level (e.g., a normalized expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: 1) ASPM; 2) BUB1; 3) PTTG1; 4) SHCBP1; 5) NUSAP1; 6) MKI67; 7) HJURP; 8) CDCA3; 9) PLK1; 10) PRR11; 11) BRCA2; 12) ORM1; 13) CCNB2; 14) SMC4; 15) HM13; 16) DMD; 17) FHL1; 18) ORM2; 19) NDUFC2-KCTD14; 20) NCAPH; 21) TTLL7; 22) DEPDC1B; 23) CNTN4; 24) PRKAA2; 25) PRKCQ; 26) CDC42BPA; 27) PARD3B; 28) SCTR; and 30) MPDZ.

In some cases, a subject method involves determining, in a lung tissue sample from a subject, an expression level (e.g., a normalized expression level) of an MPDZ gene product. In some of these embodiments, the method can further comprise determining an expression level (e.g., a normalized expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: 1) ASPM; 2) BUB1; 3) PTTG1; 4) SHCBP1; 5) NUSAP1; 6) MKI67; 7) HJURP; 8) CDCA3; 9) PLK1; 10) PRR11; 11) BRCA2; 12) ORM1; 13) CCNB2; 14) SMC4; 15) HM13; 16) DMD; 17) FHL1; 18) ORM2; 19) NDUFC2-KCTD14; 20) NCAPH; 21) TTLL7; 22) DEPDC1B; 23) CNTN4; 24) PRKAA2; 25) PRKCQ; 26) CDC42BPA; 27) PARD3B; 28) SCTR; and 29) CSF3R.

In some cases, a subject method involves determining, in a lung tissue sample from a subject, an expression level (e.g., a normalized expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: ACTA2, ACTG2, AHNAK, AHNAK2, AKT3, ANO2, AOC3, ARHGEF25, ATP1A2, CALD1, CAMK2N1, CD109, CDH13, CNKSR2, CNN1, COL21A1, COPZ2, COX7A1, CRIM1, CRYAB, CSRP1, DENND2A, DES, DGKG, DIXDC1, DMD, DNAJB4, DPYSL3, DST, DTNA, EDIL3, EOGT, EPB41L2, FGF14, FHL1, FMO2, FMO3, GARNL3, HOXA-AS2, HOXA4, HOXA6, HPSE2, LAMA4, LARP6, LHFP, LMCD1, LMOD1, LRRFIP1, MAP1B, MARK1, MCAM, MFAP4, MPDZ, MSRB3, MXRA7, MYH10, MYH11, MYL9, MYLK, MYOCD, NEXN, PALLD, PCDHB10, PCDHGC5, PDE1C, PDE5A, PDLIM3, PEAK1, PGM5, PGM5P2, PHLDB2, PKD2, PKIG, PLCB4, PLCE1, PLN, PPP1R3C, PREX2, PRKAA2, PRKAB2, PRKG1, PRUNE2, PTCHD4, PTPRB, REEP1, RERG, SBF2, SORBS1, SYNM, SYNPO2, TAGLN, TIMP2, TIMP3, TMEM47, TMOD1, TPM1, TPM2, TRPC1, TTLL7, UACA, ZC3H12B.

In some cases, a subject method involves determining, in a lung tissue sample from a subject, an expression level (e.g., a normalized expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29 or more genes) of the following gene set: ABCA3, ACADSB, ACOXL, ADAR, APOL6, ARHGEF38, ARPC5L, ASPM, ATP11A, ATP2C2, ATP8A1, BUB1, C18orf8, C2, CASC5, CCL4, CCL4L1, CCNA2, CCNB1, CCNB2, CCR5, CD38, CDC45, CDK1, CDKL2, CEACAM1, CFTR, CISH, CLINT1, CNDP2, CRTAC1, CSF3R, CXCL10, CXCL11, CXCL9, CXCR6, DGKD, DLGAP5, DRAM1, DUSP16, EDEM1, EFNA1, EPSTI1, ESRP2, ETV7, EZH2, FHDC1, FOXM1, FZD5, GALNT3, GBP1, GBP5, GPR98, GRAMD1A, GUCD1, HAS3, HERC6, HIST1H2BI, HIST1H2BM, HIST1H3E, HIST1H3J, HLA-A, HLA-B, HLA-C, HLA-DOA, HLA-F, HLA-H, HMMR, IFI35, IRF1, IRF9, KCNJ15, KCNQ3, KCTD14, KIF11, KIF14, KIF18A, KIF4A, LARP4B, LGALS3BP, LNX2, MAP3K13, MAPK13, MARK2, METTL7B, MKI67, MTUS1, NPC1, NUSAP1, OAS2, ORM1, ORM2, PARP14, PARP9, PLAGL2, PRKCQ, PSMB10, PSMB8, PSMB9, PSME1, PSME2, PTTG1, PXMP4, RABGAP1L, RAD51AP1, RAP1GAP, RBM47, RCOR1, RFX5, RGS16, RNF145, RNF213, SCTR, SDC4, SDR16C5, SEMA4A, SFTPD, SLC16A13, SLC22A3, SMC4, SNX30, SQSTM1, SREBF2, STAT1, STIL, TAP1, TAPBP, TCID-7896714, TCF19, TKT, TLR2, TMED6, TMEM164, TMEM41B, TOP1, TOP2A, TOX, TRAFD1, TRIM26, TYMP, UBD, UBE2S, USP44, VDR, WARS, WWC1, YARS, ZDHHC16, ZNF385B.

### Differential diagnosis of idiopathic pulmonary fibrosis vs. hypersensitivity pneumonitis

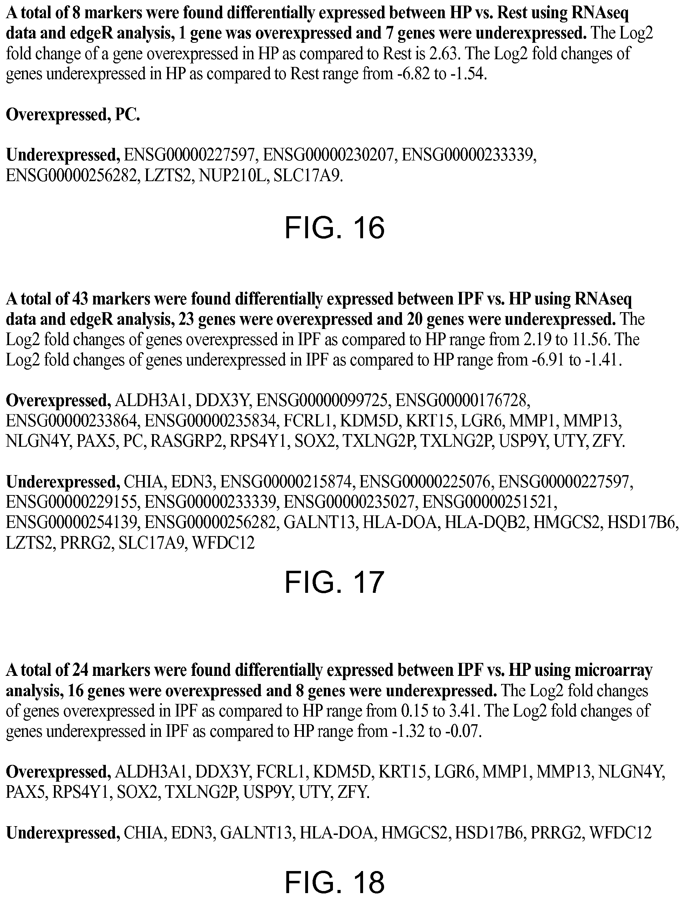

The present disclosure provides a method for differential diagnosis of IPF versus hypersensitivity pneumonitis (HP). The methods generally involve determining, in a lung tissue sample from a subject, a normalized expression level of a gene product of a gene set forth in any one of Figures 17 and 18, Table 9, and Example 3.

In some cases, a subject method involves determining, in a lung tissue sample from a subject, an expression level (e.g., a normalized expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 or more genes) selected from the genes set forth in Figures 17 and 18, Table 9, and Example 3.

In some cases, a subject method involves determining, in a lung tissue sample from a subject, an expression level (e.g., a normalized gene expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29, or 30 more genes) of the following gene set: ALDH3A1, DDX3Y, ENSG00000099725, ENSG00000176728, ENSG00000233864, ENSG00000235834, FCRL1, KDM5D, KRT15, LGR6, MMP1, MMP13, NLGN4Y, PAX5, PC, RASGRP2, RPS4Y1, SOX2, TXLNG2P, TXLNG2P, USP9Y; CHIA, EDN3, ENSG00000215874, ENSG00000225076, ENSG00000227597, ENSG00000229155, ENSG00000233339, ENSG00000235027, ENSG00000251521, ENSG00000254139, ENSG00000256282, GALNT13, HLA-DOA, HLA-DQB2, HMGCS2, HSD17B6, LZTS2, PRRG2, SLC17A9, and WFDC12.

In some cases, a subject method involves determining, in a lung tissue sample from a subject, an expression level (e.g., a normalized gene expression level) of a gene product of one or more genes (e.g., a set of 2, 3, 4, 5, 6, 7, 8, 9, 10, 11, 12, 13, 14, 15, 16, 17, 18, 19, 20, 21, 22, 23, or 24 genes) of the following gene set: ALDH3A1, DDX3Y, FCRL1, KDM5D, KRT15, LGR6, MMP1, MMP13, NLGN4Y, PAX5, RPS4Y1, SOX2, TXLNG2P, USP9Y, UTY, ZFY, CHIA, EDN3, GALNT13, HLA-DOA, HMGCS2, HSD17B6, PRRG2, WFDC12.

In some cases, a subject method involves determining, in a lung tissue sample from a subject, an expression level (e.g., a normalized gene expression level) of a gene product of one or more genes (e.g., a set of 2, 3, 4, 5, 6, 7, 8, 9, 10, 11, 12, 13, 14, 15, 16, 17, 18, 19, 20, 21, 22, 23, or 24 genes) of the following gene set: ACTA2, ACTG2, ARHGEF25, CACNA1C, CALD1, CNN1, COL21A1, DST, EDIL3, GPC6, HERC2P2, HSPA2, KALRN, LINC00328, LMOD1, MAP1B, MEIS1, MEOX2, MYH11, MYLK, MYOCD, NBPF3, NEXN, OSMR, PAPPA, PCDHB18, PDE3A, PLXNA4, PRUNE2, PTCHD4, PTGFRN, SCRG1, SLC25A27, SLIT3, SOGA1, SORBS1, SYNPO2, TAGLN, TCID-7906969, TPM1, TPM2, VCAN, ZNF154.

In some cases, a subject method involves determining, in a lung tissue sample from a subject, an expression level (e.g., a normalized gene expression level) of a gene product of one or more genes (e.g., a set of 2, 3, 4, 5, 6, 7, 8, 9, 10, 11, 12, 13, 14, 15, 16, 17, 18, 19, 20, 21, 22, 23, or 24 genes) of the following gene set: ADAMDEC1, ANKRD22, APOBEC3C, APOBEC3F, APOBEC3G, APOBEC3H, APOL1, APOL3, APOL4, APOL6, ASCL2, BATF, BATF2, BST2, BTN2A2, BTN3A1, BTN3A2, BTN3A3, C17orf49, CAMK4, CASP1, CASP4, CCL4, CCL4L1, CCL5, CCR5, CD2, CD226, CD244, CD247, CD274, CD3D, CD3E, CD3G, CD40, CD40LG, CD5, CD6, CD72, CD74, CD8A, CD96, CIITA, CLEC6A, CMTM1, CORO1A, CRTAM, CST7, CTLA4, CXCL10, CXCL11, CXCL9, CXCR3, CXCR6, DDX60, DNASE2, EBI3, EIF4E3, EPSTI1, ETV7, FAM26F, FAM78A, FASLG, FGL2, FYB, GATA3, GBP1, GBP2, GBP3, GBP4, GBP5, GBP7, GCH1, GPR171, GPR174, GPR68, GRAMD1B, GZMA, GZMB, GZMH, HCP5, HCST, HIST1H2BI, HLA-A, HLA-B, HLA-C, HLA-DMA, HLA-DMB, HLA-DOA, HLA-DPA1, HLA-DPB1, HLA-DPB2, HLA-E, HLA-F, HLA-G, HLA-H, HLA-J, HLA-L, ICOS, IDOl, IFI27, IFI27L2, IFI35, IFNG, IL12RB2, IL23A, IL26, IL27RA, IL2RB, IL2RG, IL32, IL6R, IRF1, IRF9, ITGA4, ITGAE, ITGAL, ITGB2, ITGB7, ITK, JAKMIP1, KIAA1324, KIF18A, KLRC2, KLRK1, L3MBTL4, LAP3, LAT, LCK, LCP2, LGALS2, LGR5, MIA2, NAGK, NFATC2, PAQR8, PARP12, PARP14, PARP9, PDCD1LG2, PLA2G12B, PLXNC1, PRKCQ, PSMB10, PSMB8, PSMB9, PSME1, PSME2, PTPN22, PTPRJ, PTTG1, PYCARD, PYHIN1, RABGAP1L, RARRES3, RASGRP1, RFX5, RGS10, RNF167, RNF19B, RNF213, RTP4, RUNX3, SAMD9L, SEMA4D, SEPT1, SH2D1A, SH2D2A, SKAP1, SLA2, SLAMF8, SLC35D2, STAT1, STAT4, TAP1, TAP2, TAPBP, TARP, TCID-7896691, TCID-7896701, TCID-7896702, TCID-7896719, TCID-7896720, TCID-7917530, TCID-7948502, TCID-8117739, TCID-8163000, TDRKH, THEMIS, TLR3, TLR8, TNF, TRAFD1, TRAT1, TRAV8-3, TRDV2, TRERF1, TUBA4A, UBASH3A, UBD, UBE2L6, VAMP5, WARS, XCL1, ZAP70, ZNF683, ZNF831.

### Differential diagnosis of idiopathic pulmonary fibrosis vs. interstitial lung disease inflammation

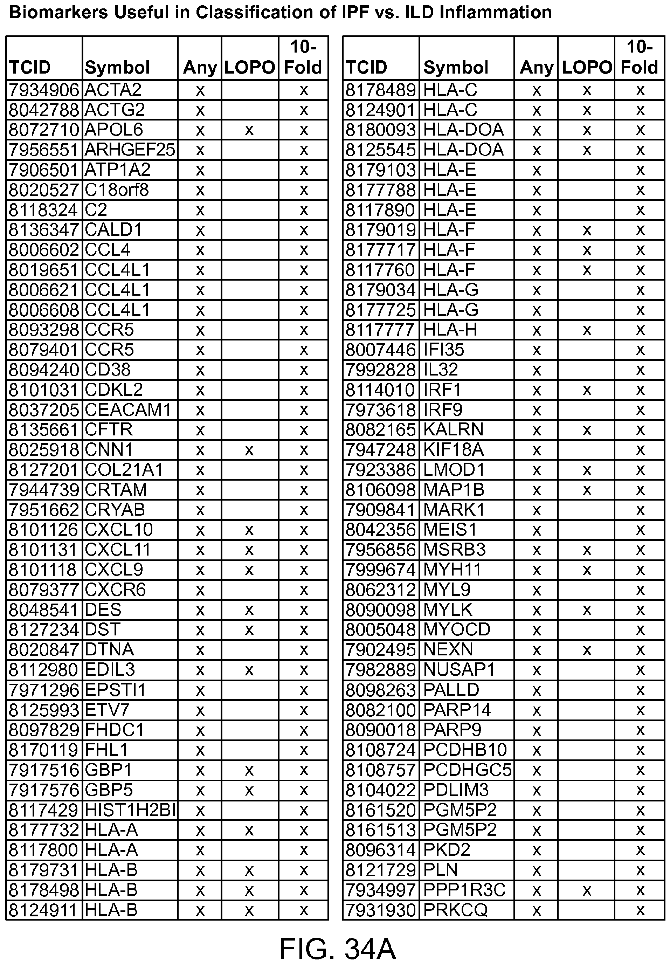

The present disclosure provides a method for differential diagnosis of IPF versus interstitial lung diseases inflammation (ILD inflammation; e.g., inflammation subtypes HP, NSIP, sarcoidosis, and organizing pneumonia (OP)). The methods generally involve determining, in a lung tissue sample from a subject, a normalized expression level of a gene product of a gene set forth in Figures 34A-B.

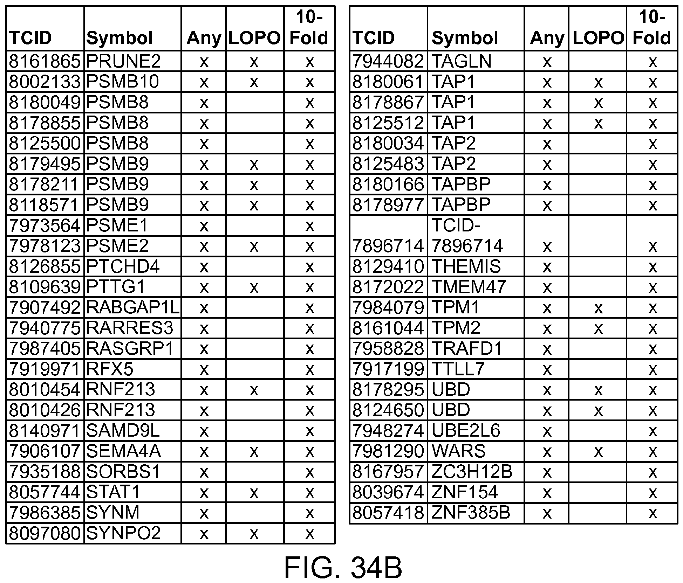

In some cases, a subject method involves determining, in a lung tissue sample from a subject, an expression level (e.g., a normalized expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 or more genes) selected from the genes set forth in Figures 34A and 34B.

In some cases, a subject method involves determining, in a lung tissue sample from a subject, an expression level (e.g., a normalized gene expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29, or 30 more genes) of the following gene set: ABCA6, ACTA2, ACTG2, ADCY5, AHNAK2, ANKRD20A8P, ANO2, ARHGEF25, ATP1A2, C16orf45, CALD1, CAMK2N1, CNN1, COL21A1, CRYAB, CSRP1, CTTNBP2, DES, DGKG, DIXDC1, DMD, DST, DTNA, DZIP1, EDIL3, FAM124B, FAM13C, FHL1, GARNL3, HPSE2, HSPA2, KALRN, LAMA4, LAMC1, LDB3, LMOD1, LPP, MAP1B, MAP7D3, MARK1, MCAM, MEIS1, MPDZ, MRVI1, MSRB3, MUSTN1, MXRA7, MYH10, MYH11, MYL9, MYLK, MYOCD, MYOM1, NEXN, NFASC, NTRK3, PALLD, PBX1, PCDHB10, PCDHB13, PCDHGC5, PDE1C, PDE5A, PDLIM3, PGM5, PGM5P2, PKD2, PLA2R1, PLCB4, PLN, PPP1R3C, PREX2, PRKAA2, PRKG1, PRUNE2, PTCHD4, PWAR5, RAB9B, SBSPON, SCRG1, SNORD116-21, SORBS1, STON1-GTF2A1L, SYNM, SYNPO2, SYTL4, TAGLN, TCEAL4, TCEAL6, TCID-7919139, TIMP2, TIMP3, TMEM47, TMOD1, TPM1, TPM2, TRPC1, TTLL7, ZC3H12B, and ZNF154.

In some cases, a subject method involves determining, in a lung tissue sample from a subject, an expression level (e.g., a normalized gene expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29, or 30 more genes) of the following gene set: ACSF2, ANKRD22, AP1M2, APOBEC3F, APOBEC3G, APOL1, APOL4, APOL6, ARHGEF38, ASPM, ATP2C2, ATP8A1, BATF2, BST2, BTN3A3, BUB1, C18orf8, C2, CCL4, CCL4L1, CCL5, CCR5, CD2, CD274, CD38, CD74, CD8A, CDK1, CDKL2, CEACAM1, CFTR, CKS2, CNDP2, CRTAM, CTLA4, CXCL10, CXCL11, CXCL9, CXCR6, DDX60, DRAM1, DUSP16, EFNA1, EPSTI1, ESRP2, ETV7, FAM26F, FHDC1, FPR3, FZD5, GBP1, GBP4, GBP5, GCH1, GPR98, GRAMD1A, GUCD1, GZMB, GZMH, HAS3, HCP5, HIST1H2AI, HIST1H2BH, HIST1H2BI, HIST1H3B, HIST1H3J, HLA-A, HLA-B, HLA-C, HLA-DMA, HLA-DOA, HLA-DPB1, HLA-E, HLA-F, HLA-G, HLA-H, HLA-J, HLA-L, HN1, IFI35, IFIH1, IFNG, IL32, IL6R, IRF1, IRF9, ITGAE, ITGAL, KCNJ15, KCTD14, KIF18A, LAP3, LARP4B, LCP2, LGALS3BP, MAP3K13, MOV10, MREG, NUSAP1, OAS2, PARP12, PARP14, PARP9, PFKFB3, PRKCQ, PSMB10, PSMB8, PSMB9, PSME1, PSME2, PTPRJ, PTTG1, PXMP4, RABGAP1L, RARRES3, RASGRP1, RBM47, RFX5, RGS16, RNF213, SAMD9L, SCTR, SDC4, SEMA4A, SFTPD, SLAMF8, SLC37A1, SMC4, SQRDL, SREBF2, STAT1, TAP1, TAP2, TAPBP, TCID-7896714, TCID-8107094, THEMIS, TLR2, TLR3, TMEM164, TNFAIP3, TOX, TRAFD1, TRIM59, TYMP, UBD, UBE2L6, USP44, WARS, ZNF385B.

### Differential diagnosis of idiopathic pulmonary fibrosis vs. interstitial lung disease other than IPF

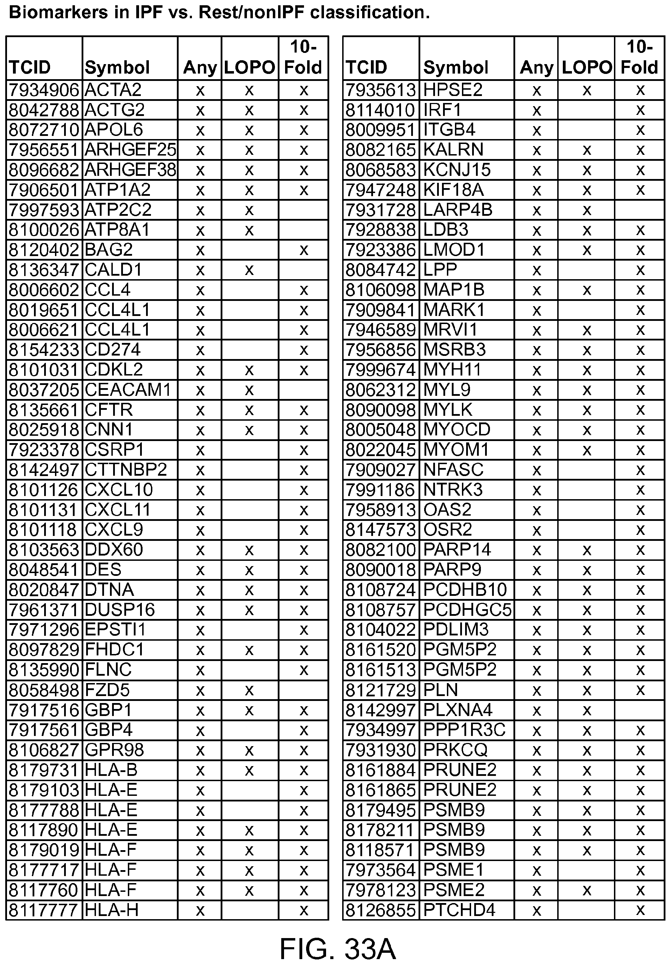

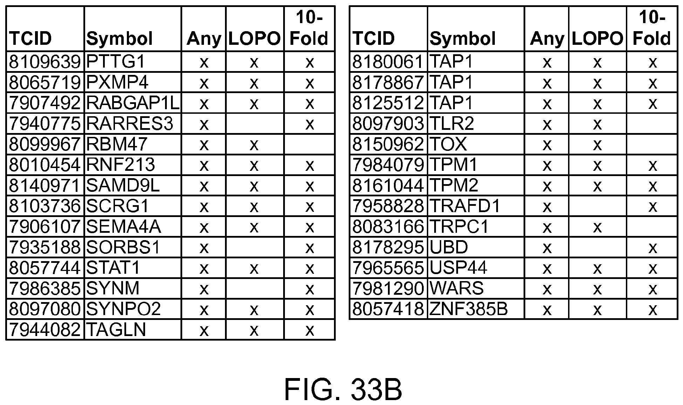

The present disclosure provides a method for differential diagnosis of IPF versus interstitial lung disease other than IPF. The methods generally involve determining, in a lung tissue sample from a subject, a normalized expression level of a gene product of a gene set forth in Figures33A-B. ILDs other than IPF are referred to as "Rest" or "Rest/non-IPF." "Rest" refers to ILDs such as HP, NSIP, and sarcoidosis.

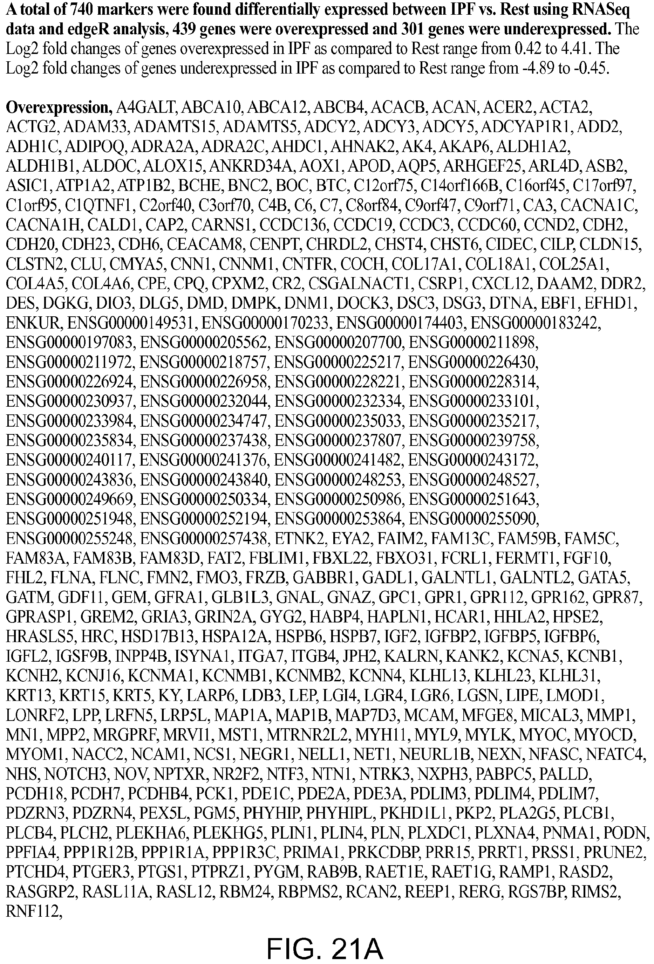

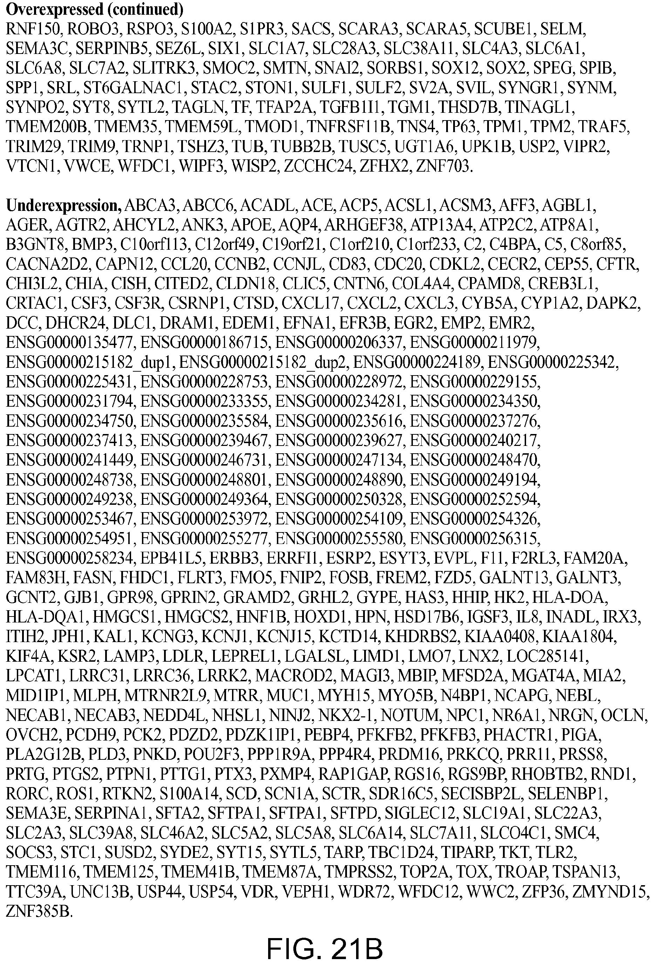

In some cases, a subject method involves determining, in a lung tissue sample from a subject, an expression level (e.g., a normalized gene expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29, or 30 more genes) set out in any one of Figures 9A-9D, 21, and 33. In some cases, a subject method involves determining, in a lung tissue sample from a subject, an expression level (e.g., a normalized gene expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29, or 30 more genes) set out in Figures 9A-D. In some cases, a subject method involves determining, in a lung tissue sample from a subject, an expression level (e.g., a normalized gene expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29, or 30 more genes) set out in Figures 21A-21B. In some cases, a subject method involves determining, in a lung tissue sample from a subject, an expression level (e.g., a normalized gene expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29, or 30 more genes) set out in Figures 33A-B.

In some cases, a subject method involves determining, in a lung tissue sample from a subject, an expression level (e.g., a normalized gene expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29, or 30 more genes) of the following gene set: ABCA6, ABLIM3, ACTA2, ACTG2, ADCY5, AHNAK2, AKAP12, ANO1, ANO2, ARHGEF25, ATP1A2, ATP1B2, BAG2, BHMT2, BMX, C16orf45, C3orf55, C3orf70, CACNA1C, CACNB2, CALD1, CAMK2N1, CCDC3, CDH6, CDR1, CLIP3, CLU, CNN1, COCH, COL21A1, COPZ2, COX7A1, CRYAB, CSRP1, CTTNBP2, DACT1, DDR2, DENND2A, DES, DIXDC1, DMD, DST, DTNA, DYNC1I1, DZIP1, EBF1, EDIL3, EFHD1, F10, FAM124B, FAM13C, FBLIM1, FEZ1, FGF10, FGF14, FGF2, FGFR1, FHL1, FHL2, FLNC, GADL1, GARNL3, GAS7, GNG12, GRIA3, GRIN2A, HOXA2, HOXA6, HOXA7, HPSE2, HRC, HRH1, HSPA2, HSPB6, HSPB7, IGFBP5, ITGB4, ITIH5, KALRN, LAMA4, LAMC1, LARP6, LDB3, LGI4, LMOD1, LPP, LTBP1, MAP1B, MAP7D3, MARK1, MCAM, MEIS1, MEOX2, MPDZ, MRGPRF, MRVI1, MSRB3, MUSTN1, MXRA7, MYH11, MYL9, MYLK, MYOCD, MYOM1, NCAM1, NEXN, NFASC, NPR2, NR2F1, NR2F2, NTRK3, OSR2, PALLD, PALMD, PARVA, PBX1, PCDH7, PCDHB10, PCDHB12, PCDHB13, PCDHB14, PCDHB18, PCDHB3, PCDHB7, PCDHGC5, PDE10A, PDE1A, PDE2A, PDE3A, PDE5A, PDE7B, PDGFRB, PDLIM3, PDZRN4, PEG3, PFKM, PGM5, PGM5P2, PHYHIPL, PKD2, PLA2R1, PLCB4, PLN, PLXNA4, PODN, PPP1R3C, PRKAA2, PRKG1, PRSS23, PRUNE2, PTCHD4, PYGM, RAB9B, RASD2, RBM24, RCAN2, RERG, RGS5, RGS7BP, RYR3, S1PR3, SBSPON, SCRG1, SCUBE1, SEMA3D, SETBP1, SGCA, SLC38A11, SLIT3, SLN, SMIM10, SNED1, SNORD116-17, SNORD116-21, SNX9, SOGA1, SORBS1, SPARCL1, SSPN, SYNM, SYNPO2, SYTL2, TAGLN, TCEAL4, TCEAL6, TIMP2, TMEM252, TMEM47, TMOD1, TPM1, TPM2, TRPC1, TTLL7, WISP2, ZAK, ZC3H12B, ZCCHC24, ZEB2, ZHX3, ZNF135, ZNF154, ZNF521.

In some cases, a subject method involves determining, in a lung tissue sample from a subject, an expression level (e.g., a normalized gene expression level) of a gene product of one or more genes (e.g., a set of 2, 3, 4, 5, 6, 7, 8, 9, 10, 11, 12, 13, 14, 15, 16, 17, 18, 19, 20, 21, 22, 23, 24, 25, 26, 27, 28, 29, or 30 more genes) of the following gene set: ACADL, ACOXL, ACSF2, AHCYL2, ANK3, AP1M2, APOL1, APOL4, APOL6, AQP4, ARAP2, ARHGEF38, ARSD, ASPM, ATP1A1, ATP2C2, ATP8A1, BATF2, BAZ1A, BMP3, BST2, BTG3, BTN3A3, BUB1, C18orf8, C2, CASP1, CASP4, CCL4, CCL4L1, CCL5, CCNA2, CCR5, CD274, CD40, CD47, CD74, CD8A, CDCA7L, CDK1, CDKL2, CEACAM1, CFTR, CKAP2, CLDN4, CLINT1, CNDP2, CNKSR1, CRTAC1, CRTAM, CSF3R, CXCL10, CXCL11, CXCL9, CXCR6, CYB5A, CYP2B6, CYP2B7P1, DAPK1, DBI, DDX60, DENND1B, DHCR24, DNASE2, DOCK8, DRAM1, DTNB, DTX3L, DUSP16, DYNLT1, EDEM1, EFNA1, EML4, ENTPD3, EPSTI1, ERAP1, ERBB3, ESRP1, ESRP2, ETV7, FAM111A, FCGR1A, FCGR1B, FDFT1, FGFR2, FHDC1, FLRT3, FNIP2, FREM2, FZD5, GALNT3, GBP1, GBP4, GBP5, GBP7, GCH1, GCNT2, GK, GNPTAB, GPR98, GRAMD1B, GRHL2, GSAP, GUCD1, GZMB, GZMH, HAS3, HCP5, HIST1H2AI, HIST1H2BI, HIST1H3B, HLA-A, HLA-B, HLA-C, HLA-DMA, HLA-DOA, HLA-DPA1, HLA-DPB1, HLA-DQA2, HLA-DRA, HLA-E, HLA-F, HLA-G, HLA-H, HLA-J, HLA-L, HMGCL, HMGCR, HMMR, HN1, HNF1B, HPS5, ICOS, IDI1, IDOl, IFI27, IFI35, IFIH1, IFIT3, IFIT5, IGSF3, IL10RB, IL32, IL6R, INADL, IRF1, IRF9, ITGAE, ITGAL, JPH1, KCNJ15, KCTD14, KIAA0247, KIAA0319L, KIAA1958, KIF18A, KLF5, L3MBTL4, LAMP3, LAP3, LARP4B, LCP2, LIMD1, LNX2, LPIN2, MAGI3, MAP3K13, MAPK13, MARK2, MB21D1, MBIP, MIA2, MICALCL, MID1IP1, MREG, MRPL14, MTMR14, MTRR, MYL1, MYO5B, N4BP1, NAV2, NEDD4L, NMI, NPC1, NUB1, NUSAP1, OAS2, OCLN, ORM1, PARP12, PARP14, PARP9, PCK2, PCLO, PDZK1IP1, PEBP4, PFKFB3, PHACTR1, PI4K2B, PLA2G12B, PLAGL2, PLCH1, PLS1, PPP1R9A, PPP2R5A, PRKCQ, PRKCZ, PRRG4, PRSS8, PSMB10, PSMB8, PSMB9, PSME1, PSME2, PTP4A1, PTPN1, PTPRJ, PTTG1, PXMP4, RAB20, RAB3IP, RABGAP1L, RANBP17, RARRES3, RBM47, RCOR1, RFC1, RFX5, RGS16, RNF19B, RNF213, S100A14, SAMD9L, SCN1A, SCTR, SDC4, SELENBP1, SEMA4A, SFTPD, SH2D4A, SHANK2, SLAMF8, SLC15A3, SLC16A13, SLC22A3, SLC37A1, SLCO4C1, SMARCA5, SMC4, SQLE, SQRDL, SREBF2, ST7, STARD10, STAT1, STX11, STX19, SYT15, TAP1, TAP2, TAPBP, TAPT1, TC2N, TCID-7895602, TCID-7896651, TCID-7896695, TCID-7951593, TCID-8113717, TDRKH, THEMIS, TKT, TLR2, TLR3, TMEM164, TMEM180, TMEM41B, TMEM87A, TMPRSS2, TMPRSS3, TNFAIP3, TOX, TRAFD1, TRIM21, TRIM24, TTLL4, UBALD2, UBD, UBE2L6, USP38, USP44, USP54, WARS, WHSC1, WWC1, ZDHHC16, ZDHHC2, ZNF385B.

### Tissue samples

A lung tissue sample for use in a subject analytical or diagnostic method can be a biopsy sample (e.g., a biopsy sample obtained by video-assisted thoracoscopic surgery; VATS); a bronchoalveolar lavage (BAL) sample; a transbronchial biopsy; a cryo-transbronchial biopsy; and the like. Lung tissue samples for analysis can be provided in a suitable preservation solution.

Tissue samples can be obtained from a patient suspected of having a lung disease, e.g., an ILD, based on clinical signs and symptoms with which the patient presents (e.g., shortness of breath (generally aggravated by exertion), dry cough), and, optionally the results of one or more of an imaging test (e.g., chest X-ray, computerized tomography (CT)), a pulmonary function test (e.g., spirometry, oximetry, exercise stress test), lung tissue analysis (e.g., histological and/or cytological analysis of samples obtained by bronchoscopy, bronchoalveolar lavage, surgical biopsy) .

The lung tissue sample can be processed in any of a variety of ways. For example, the lung tissue sample can be subjected to cell lysis. The lung tissue sample can be preserved in RNAprotect solution (a solution that inhibits RNA degradation, e.g., that inhibits nuclease digestion of RNA) and subsequently subjected to cell lysis. Components such as nucleic acids and/or proteins can be enriched or isolated from the lung tissue sample, and the enriched or isolated component can be used in a subject method. Methods of enriching for and isolating components such nucleic acids and proteins are known in the art; and any known method can be used. Methods of isolating RNA for expression analysis have been described in the art.

### In vitro methods of determining expression product levels

The general methods for determining gene expression product levels are known to the art and may include but are not limited to one or more of the following: additional cytological assays, assays for specific proteins or enzyme activities, assays for specific expression products including protein or RNA or specific RNA splice variants, in situ hybridization, whole or partial genome expression analysis, microarray hybridization assays, serial analysis of gene expression (SAGE), enzyme linked immunoabsorbance assays, mass-spectrometry, immunohistochemistry, blotting, sequencing, RNA sequencing, DNA sequencing (e.g., sequencing of cDNA obtained from RNA); Next-Gen sequencing, nanopore sequencing, pyrosequencing, or Nanostring sequencing. Gene expression product levels may be normalized to an internal standard such as total mRNA or the expression level of a particular gene including but not limited to glyceraldehyde 3 phosphate dehydrogenase, or tubulin.

In certain embodiments, a gene expression profile may be obtained by whole transcriptome shotgun sequencing ("WTSS" or "RNA-seq"; see, e.g., Ryan et al BioTechniques 45: 81-94), which makes the use of high-throughput sequencing technologies to sequence cDNA in order to about information about a sample's RNA content. In general terms, cDNA is made from RNA, the cDNA is amplified, and the amplification products are sequenced.

After amplification, the cDNA may be sequenced using any convenient method. For example, the fragments may be sequenced using Illumina's reversible terminator method, Roche's pyrosequencing method (454), Life Technologies' sequencing by ligation (the SOLiD platform) or Life Technologies' Ion Torrent platform. Examples of such methods are described in the following references: Margulies et al (Nature 2005 437: 376-80); Ronaghi et al (Analytical Biochemistry 1996 242: 84-9); Shendure (Science 2005 309: 1728); Imelfort et al (Brief Bioinform. 2009 10:609-18); Fox et al (Methods Mol Biol. 2009;553:79-108); Appleby et al (Methods Mol Biol. 2009;513:19-39) and Morozova

(Genomics. 2008 92:255-64), which are incorporated by reference for the general descriptions of the methods and the particular steps of the methods, including all starting products, reagents, and final products for each of the steps. As would be apparent, forward and reverse sequencing primer sites that compatible with a selected next generation sequencing platform can be added to the ends of the fragments during the amplification step.

In other embodiments, the products may be sequenced using nanopore sequencing (e.g. as described in Soni et al Clin Chem 53: 1996-2001 2007, or as described by Oxford Nanopore Technologies). Nanopore sequencing is a single-molecule sequencing technology whereby a single molecule of DNA is sequenced directly as it passes through a nanopore. A nanopore is a small hole, of the order of 1 nanometer in diameter. Immersion of a nanopore in a conducting fluid and application of a potential (voltage) across it results in a slight electrical current due to conduction of ions through the nanopore. The amount of current which flows is sensitive to the size and shape of the nanopore. As a DNA molecule passes through a nanopore, each nucleotide on the DNA molecule obstructs the nanopore to a different degree, changing the magnitude of the current through the nanopore in different degrees. Thus, this change in the current as the DNA molecule passes through the nanopore represents a reading of the DNA sequence. Nanopore sequencing technology as disclosed in U.S. Pat. Nos. 5,795,782, 6,015,714, 6,627,067, 7,238,485 and 7,258,838 and U.S. patent application publications US2006003171 and US20090029477.

In some embodiments, the gene expression product of the subject methods is a protein, and the amount of protein in a particular biological sample is analyzed using a classifier derived from protein data obtained from cohorts of samples. The amount of protein can be determined by one or more of the following: enzyme-linked immunosorbent assay (ELISA), mass spectrometry, blotting, or immunohistochemistry.

In some embodiments, gene expression product markers and alternative splicing markers may be determined by microarray analysis using, for example, Affymetrix arrays, cDNA microarrays, oligonucleotide microarrays, spotted microarrays, or other microarray products from Biorad, Agilent, or Eppendorf. Microarrays provide particular advantages because they may contain a large number of genes or alternative splice variants that may be assayed in a single experiment. In some cases, the microarray device may contain the entire human genome or transcriptome or a substantial fraction thereof allowing a comprehensive evaluation of gene expression patterns, genomic sequence, or alternative splicing. Markers may be found using standard molecular biology and microarray analysis techniques as described in Sambrook Molecular Cloning a Laboratory Manual 2001 and Baldi, P., and Hatfield, W. G., DNA Microarrays and Gene Expression 2002.

Microarray analysis generally begins with extracting and purifying nucleic acid from a biological sample, (e.g. a biopsy or fine needle aspirate) using methods known to the art. For expression and alternative splicing analysis it may be advantageous to extract and/or purify RNA from DNA. It may further be advantageous to extract and/or purify mRNA from other forms of RNA such as tRNA and rRNA.

Purified nucleic acid may further be labeled with a fluorescent label, radionuclide, or chemical label such as biotin, digoxigenin, or digoxin for example by reverse transcription, polymerase chain reaction (PCR), ligation, chemical reaction or other techniques. The labeling can be direct or indirect which may further require a coupling stage. The coupling stage can occur before hybridization, for example, using aminoallyl-UTP and NHS amino-reactive dyes (like cyanine dyes) or after, for example, using biotin and labelled streptavidin. In one example, modified nucleotides (e.g. at a 1 aaUTP: 4 TTP ratio) are added enzymatically at a lower rate compared to normal nucleotides, typically resulting in 1 every 60 bases (measured with a spectrophotometer). The aaDNA may then be purified with, for example, a column or a diafiltration device. The aminoallyl group is an amine group on a long linker attached to the nucleobase, which reacts with a reactive label (e.g. a fluorescent dye).

The labeled samples may then be mixed with a hybridization solution which may contain sodium dodecyl sulfate (SDS), SSC, dextran sulfate, a blocking agent (such as COT1 DNA, salmon sperm DNA, calf thymus DNA, PolyA or PolyT), Denhardt's solution, formamine, or a combination thereof.

A hybridization probe is a fragment of DNA or RNA of variable length, which is used to detect in DNA or RNA samples the presence of nucleotide sequences (the DNA target) that are complementary to the sequence in the probe. The probe thereby hybridizes to single-stranded nucleic acid (DNA or RNA) whose base sequence allows probe-target base pairing due to complementarity between the probe and target. The labeled probe is first denatured (by heating or under alkaline conditions) into single DNA strands and then hybridized to the target DNA.

To detect hybridization of the probe to its target sequence, the probe is tagged (or labeled) with a molecular marker; commonly used markers are 32P or Digoxigenin, which is nonradioactive antibody-based marker. DNA sequences or RNA transcripts that have moderate to high sequence complementarity (e.g. at least 70%, 80%, 90%, 95%, 96%, 97%, 98%, 99%, or more complementarity) to the probe are then detected by visualizing the hybridized probe via autoradiography or other imaging techniques. Detection of sequences with moderate or high complementarity depends on how stringent the hybridization conditions were applied; high stringency, such as high hybridization temperature and low salt in hybridization buffers, permits only hybridization between nucleic acid sequences that are highly similar, whereas low stringency, such as lower temperature and high salt, allows hybridization when the sequences are less similar. Hybridization probes used in DNA microarrays refer to DNA covalently attached to an inert surface, such as coated glass slides or gene chips, and to which a mobile cDNA target is hybridized.

A mix comprising target nucleic acid to be hybridized to probes on an array may be denatured by heat or chemical means and added to a port in a microarray. The holes may then be sealed and the microarray hybridized, for example, in a hybridization oven, where the microarray is mixed by rotation, or in a mixer. After an overnight hybridization, non-specific binding may be washed off (e.g. with SDS and SSC). The microarray may then be dried and scanned in a machine comprising a laser that excites the dye and a detector that measures emission by the dye. The image may be overlaid with a template grid and the intensities of the features (e.g. a feature comprising several pixels) may be quantified.

Various kits can be used for the amplification of nucleic acid and probe generation of the subject methods. Examples of kit that can be used in the present invention include but are not limited to Nugen WT-Ovation FFPE kit, cDNA amplification kit with Nugen Exon Module and Frag/Label module. The NuGEN WT-Ovation^{™}. FFPE System V2 is a whole transcriptome amplification system that enables conducting global gene expression analysis on the vast archives of small and degraded RNA derived from FFPE samples. The system is comprised of reagents and a protocol required for amplification of as little as 50 ng of total FFPE RNA. The protocol can be used for qPCR, sample archiving, fragmentation, and labeling. The amplified cDNA can be fragmented and labeled in less than two hours for GeneChip^{™}. 3' expression array analysis using NuGEN's FE-Ovation^{™}. cDNA Biotin Module V2. For analysis using Affymetrix GeneChip^{™}. Exon and Gene ST arrays, the amplified cDNA can be used with the WT-Ovation Exon Module, then fragmented and labeled using the FL-Ovation^{™}. cDNA Biotin Module V2. For analysis on Agilent arrays, the amplified cDNA can be fragmented and labeled using NuGEN's FL-Ovation^{™}. cDNA Fluorescent Module. More information on Nugen WT-Ovation FFPE kit can be obtained at www.nugeninc.com/nugen/index.cfm/products/amplification-systems/wt-ovatio- n-ffpe/.

In some embodiments, Ambion WT-expression kit can be used. Ambion WT-expression kit allows amplification of total RNA directly without a separate ribosomal RNA (rRNA) depletion step. With the Ambion^{™} WT Expression Kit, samples as small as 50 ng of total RNA can be analyzed on Affymetrix^{™}. GeneChip^{™} Human, Mouse, and Rat Exon and Gene 1.0 ST Arrays. In addition to the lower input RNA requirement and high concordance between the Affymetrix^{™} method and TaqMan^{™} real-time PCR data, the Ambion^{™}. WT Expression Kit provides a significant increase in sensitivity. For example, a greater number of probe sets detected above background can be obtained at the exon level with the Ambion^{™}. WT Expression Kit as a result of an increased signal-to-noise ratio. Ambion^{™}-expression kit may be used in combination with additional Affymetrix labeling kit.

In some embodiments, AmpTec Trinucleotide Nano mRNA Amplification kit (6299-A15) can be used in the subject methods. The ExpressArt^{™} TRinucleotide mRNA amplification Nano kit is suitable for a wide range, from 1 ng to 700 ng of input total RNA. According to the amount of input total RNA and the required yields of aRNA, it can be used for 1-round (input >300 ng total RNA) or 2-rounds (minimal input amount 1 ng total RNA), with aRNA yields in the range of >10 µg. AmpTec's proprietary TRinucleotide priming technology results in preferential amplification of mRNAs (independent of the universal eukaryotic 3'-poly(A)-sequence), combined with selection against rRNAs. More information on AmpTec Trinucleotide Nano mRNA Amplification kit can be obtained at www.amp-tec.com/products.htm. This kit can be used in combination with cDNA conversion kit and Affymetrix labeling kit.

The raw data may then be normalized, for example, by subtracting the background intensity and then dividing the intensities making either the total intensity of the features on each channel equal or the intensities of a reference gene and then the t-value for all the intensities may be calculated. More sophisticated methods, include z-ratio, loess and lowess regression and RMA (robust multichip analysis), such as for Affymetrix chips.

### DATA ANALYSIS

### (i) Comparison of Sample to Normal

Results of molecular profiling performed on a sample from a subject (test sample) may be compared to a biological sample that is known or suspected to be normal. In some embodiments, a normal sample is a sample that does not comprise or is expected to not comprise an ILD, or conditions under evaluation, or would test negative in the molecular profiling assay for the one or more ILDs under evaluation. In some embodiments, a normal sample is that which is or is expected to be free of any ILD, or a sample that would test negative for any ILD in the molecular profiling assay. The normal sample may be from a different subject from the subject being tested, or from the same subject. In some cases, the normal sample is a lung tissue sample obtained from a subject such as the subject being tested for example. The normal sample may be assayed at the same time, or at a different time from the test sample.

The results of an assay on the test sample may be compared to the results of the same assay on a sample having a known disease state (e.g., normal, affected by a selected ILD (e.g., IPF, NSIP, etc.). In some cases the results of the assay on the normal sample are from a database, or a reference. In some cases, the results of the assay on the normal sample are a known or generally accepted value or range of values by those skilled in the art. In some cases the comparison is qualitative. In other cases the comparison is quantitative. In some cases, qualitative or quantitative comparisons may involve but are not limited to one or more of the following: comparing fluorescence values, spot intensities, absorbance values, chemiluminescent signals, histograms, critical threshold values, statistical significance values, gene product expression levels, gene product expression level changes, alternative exon usage, changes in alternative exon usage, protein levels, DNA polymorphisms, copy number variations, indications of the presence or absence of one or more DNA markers or regions, or nucleic acid sequences.

### (ii) Evaluation of Results

In some embodiments, the molecular profiling results are evaluated using methods known to the art for correlating gene product expression levels or alternative exon usage with specific phenotypes such as a particular ILD, or normalcy (e.g. disease or condition free). In some cases, a specified statistical confidence level may be determined in order to provide a diagnostic confidence level. For example, it may be determined that a confidence level of greater than 90% may be a useful predictor of the presence of an ILD. In other embodiments, more or less stringent confidence levels may be chosen. For example, a confidence level of about or at least about 50%, 60%, 70%, 75%, 80%, 85%, 90%, 95%, 97.5%, 99%, 99.5%, or 99.9% may be chosen as a useful phenotypic predictor. The confidence level provided may in some cases be related to the quality of the sample, the quality of the data, the quality of the analysis, the specific methods used, and/or the number of gene expression products analyzed. The specified confidence level for providing a diagnosis may be chosen on the basis of the expected number of false positives or false negatives and/or cost. Methods for choosing parameters for achieving a specified confidence level or for identifying markers with diagnostic power include but are not limited to Receiver Operating Characteristic (ROC) curve analysis, binormal ROC, principal component analysis, partial least squares analysis, singular value decomposition, least absolute shrinkage and selection operator analysis, least angle regression, and the threshold gradient directed regularization method.

### iii) Data analysis

Raw gene expression level and alternative splicing data may in some cases be improved through the application of algorithms designed to normalize and or improve the reliability of the data. In some embodiments of the present disclosure the data analysis requires a computer or other device, machine or apparatus for application of the various algorithms described herein due to the large number of individual data points that are processed. A "machine learning algorithm" refers to a computational-based prediction methodology, also known to persons skilled in the art as a "classifier", employed for characterizing a gene expression profile. The signals corresponding to certain expression levels, which are obtained by, e.g., microarray-based hybridization assays, are typically subjected to the algorithm in order to classify the expression profile. Supervised learning generally involves "training" a classifier to recognize the distinctions among classes and then "testing" the accuracy of the classifier on an independent test set. For new, unknown samples the classifier can be used to predict the class in which the samples belong.

In some cases, the robust multi-array average (RMA) method may be used to normalize raw data. The RMA method begins by computing background-corrected intensities for each matched cell on a number of microarrays. The background corrected values are restricted to positive values as described by Irizarry et al. Biostatistics 2003 April 4 (2): 249-64. After background correction, the base-2 logarithm of each background corrected matched-cell intensity is then obtained. The back-ground corrected, log-transformed, matched intensity on each microarray is then normalized using the quantile normalization method in which for each input array and each probe expression value, the array percentile probe value is replaced with the average of all array percentile points, this method is more completely described by Bolstad et al. Bioinformatics 2003. Following quantile normalization, the normalized data may then be fit to a linear model to obtain an expression measure for each probe on each microarray. Tukey's median polish algorithm (Tukey, J. W., Exploratory Data Analysis. 1977) may then be used to determine the log-scale expression level for the normalized probe set data.

Data may further be filtered to remove data that may be considered suspect. In some embodiments, data deriving from microarray probes that have fewer than about 4, 5, 6, 7 or 8 guanosine+cytosine nucleotides may be considered to be unreliable due to their aberrant hybridization propensity or secondary structure issues. Similarly, data deriving from microarray probes that have more than about 12, 13, 14, 15, 16, 17, 18, 19, 20, 21, or 22 guanosine+cytosine nucleotides may be considered unreliable due to their aberrant hybridization propensity or secondary structure issues.

In some cases, unreliable probe sets may be selected for exclusion from data analysis by ranking probe-set reliability against a series of reference datasets. For example, RefSeq or Ensembl (EMBL) are considered very high quality reference datasets. Data from probe sets matching RefSeq or Ensembl sequences may in some cases be specifically included in microarray analysis experiments due to their expected high reliability. Similarly data from probe-sets matching less reliable reference datasets may be excluded from further analysis, or considered on a case by case basis for inclusion. In some cases, the Ensembl high throughput cDNA (HTC) and/or mRNA reference datasets may be used to determine the probe-set reliability separately or together. In other cases, probe-set reliability may be ranked. For example, probes and/or probe-sets that match perfectly to all reference datasets such as for example RefSeq, HTC, and mRNA, may be ranked as most reliable (1). Furthermore, probes and/or probe-sets that match two out of three reference datasets may be ranked as next most reliable (2), probes and/or probe-sets that match one out of three reference datasets may be ranked next (3) and probes and/or probe sets that match no reference datasets may be ranked last (4). Probes and or probe-sets may then be included or excluded from analysis based on their ranking. For example, one may choose to include data from category 1, 2, 3, and 4 probe-sets; category 1, 2, and 3 probe-sets; category 1 and 2 probe-sets; or category 1 probe-sets for further analysis. In another example, probe-sets may be ranked by the number of base pair mismatches to reference dataset entries. It is understood that there are many methods understood in the art for assessing the reliability of a given probe and/or probe-set for molecular profiling and the methods of the present disclosure encompass any of these methods and combinations thereof.

In some embodiments of the present invention, data from probe-sets may be excluded from analysis if they are not expressed or expressed at an undetectable level (not above background). A probe-set is judged to be expressed above background if for any group:

Integral from T0 to Infinity of the standard normal distribution<Significance (0.01) Where: T0=Sqr(GroupSize) (T-P)/Sqr(Pvar); GroupSize=Number of CEL files in the group, T=Average of probe scores in probe-set, P=Average of Background probes averages of GC content, and Pvar=Sum of Background probe variances/(Number of probes in probe-set) 2,

This allows including probe-sets in which the average of probe-sets in a group is greater than the average expression of background probes of similar GC content as the probe-set probes as the center of background for the probe-set and enables one to derive the probe-set dispersion from the background probe-set variance.

In some embodiments of the present disclosure, probe-sets that exhibit no, or low variance may be excluded from further analysis. Low-variance probe-sets are excluded from the analysis via a Chi-Square test. A probe-set is considered to be low-variance if its transformed variance is to the left of the 99 percent confidence interval of the Chi-Squared distribution with (N-1) degrees of freedom. (N-1)*Probe-set Variance/(Gene Probe-set Variance).about.Chi-Sq(N-1) where N is the number of input CEL files, (N-1) is the degrees of freedom for the Chi-Squared distribution, and the "probe-set variance for the gene" is the average of probe-set variances across the gene. In some embodiments of the present invention, probe-sets for a given gene or transcript cluster may be excluded from further analysis if they contain less than a minimum number of probes that pass through the previously described filter steps for GC content, reliability, variance and the like. For example in some embodiments, probe-sets for a given gene or transcript cluster may be excluded from further analysis if they contain less than about 1, 2, 3, 4, 5, 6, 7, 8, 9, 10, 11, 12, 13, 14, 15, or less than about 20 probes.

Methods of data analysis of gene expression levels or of alternative splicing may further include the use of a feature selection algorithm as provided herein. In some embodiments of the present invention, feature selection is provided by use of the LIMMA software package (Smyth, G. K. (2005). Limma: linear models for microarray data. In: Bioinformatics and Computational Biology Solutions using R and Bioconductor, R. Gentleman, V. Carey, S. Dudoit, R. Irizarry, W. Huber (eds.), Springer, New York, pages 397-420).

Methods of data analysis of gene expression levels and or of alternative splicing may further include the use of a pre-classifier algorithm. For example, an algorithm may use a cell-specific molecular fingerprint to pre-classify the samples according to their composition and then apply a correction/normalization factor. This data/information may then be fed in to a final classification algorithm which would incorporate that information to aid in the final diagnosis.

Methods of data analysis of gene expression levels and/or of alternative splicing may further include the use of a classifier algorithm as provided herein. In some embodiments of the present invention a diagonal linear discriminant analysis, k-nearest neighbor algorithm, support vector machine (SVM) algorithm, linear support vector machine, random forest algorithm, or a probabilistic model-based method or a combination thereof is provided for classification of microarray data. In some embodiments, identified markers that distinguish samples (e.g. first ILD from second ILD, normal vs. ILD) or distinguish subtypes (e.g. IPF vs. NSIP) are selected based on statistical significance of the difference in expression levels between classes of interest. In some cases, the statistical significance is adjusted by applying a Benjamin Hochberg or another correction for false discovery rate (FDR).

In some cases, the classifier algorithm may be supplemented with a meta-analysis approach such as that described by Fishel and Kaufman et al. 2007 Bioinformatics 23(13): 1599-606. In some cases, the classifier algorithm may be supplemented with a meta-analysis approach such as a repeatability analysis. In some cases, the repeatability analysis selects markers that appear in at least one predictive expression product marker set.

Methods for deriving and applying posterior probabilities to the analysis of microarray data are known in the art and have been described for example in Smyth, G. K. 2004 Stat. Appi. Genet. Mol. Biol. 3: Article 3. In some cases, the posterior probabilities may be used to rank the markers provided by the classifier algorithm. In some cases, markers may be ranked according to their posterior probabilities and those that pass a chosen threshold may be chosen as markers whose differential expression is indicative of or diagnostic for samples that are for example IPF or NSIP. Illustrative threshold values include prior probabilities of 0.7, 0.75, 0.8, 0.85, 0.9, 0.925, 0.95, 0.975, 0.98, 0.985, 0.99, 0.995 or higher.

A statistical evaluation of the results of the molecular profiling may provide a quantitative value or values indicative of one or more of the following: the likelihood of diagnostic accuracy; the likelihood of an ILD; the likelihood of a particular ILD; and the likelihood of the success of a particular therapeutic intervention. Thus a physician, who is not likely to be trained in genetics or molecular biology, need not understand the raw data. Rather, the data is presented directly to the physician in its most useful form to guide patient care. The results of the molecular profiling can be statistically evaluated using a number of methods known to the art including, but not limited to: the students T test, the two sided T test, pearson rank sum analysis, hidden markov model analysis, analysis of q-q plots, principal component analysis, one way ANOVA, two way ANOVA, LIMMA and the like.

In some embodiments of the present invention, the use of molecular profiling alone or in combination with cytological analysis may provide a classification, identification, or diagnosis that is between about 85% accurate and about 99% or about 100% accurate. In some cases, the molecular profiling process and/or cytology provide a classification, identification, diagnosis of an ILD that is about, or at least about 85%, 86%, 87%, 88%, 90%, 91%, 92%, 93%, 94%, 95%, 96%, 97%, 97.5%, 98%, 98.5%, 99%, 99.5%, 99.75%, 99.8%, 99.85%, or 99.9% accurate. In some embodiments, the molecular profiling process and/or cytology provide a classification, identification, or diagnosis of the presence of a particular ILD type (e.g. IPF; NSIP; HP) that is about, or at least about 85%, 86%, 87%, 88%, 90%, 91%, 92%, 93%, 94%, 95%, 96%, 97%, 97.5%, 98%, 98.5%, 99%, 99.5%, 99.75%, 99.8%, 99.85%, or 99.9% accurate.

In some cases, accuracy may be determined by tracking the subject over time to determine the accuracy of the original diagnosis. In other cases, accuracy may be established in a deterministic manner or using statistical methods. For example, receiver operator characteristic (ROC) analysis may be used to determine the optimal assay parameters to achieve a specific level of accuracy, specificity, positive predictive value, negative predictive value, and/or false discovery rate.

In some embodiments of the present disclosure, gene expression products and compositions of nucleotides encoding for such products which are determined to exhibit the greatest difference in expression level or the greatest difference in alternative splicing between a first ILD and a second ILD (e.g., between IPF and NSIP), or between ILD and normal may be chosen for use as molecular profiling reagents of the present disclosure. Such gene expression products may be particularly useful by providing a wider dynamic range, greater signal to noise, improved diagnostic power, lower likelihood of false positives or false negative, or a greater statistical confidence level than other methods known or used in the art.

In other embodiments of the present invention, the use of molecular profiling alone or in combination with cytological analysis may reduce the number of samples scored as non-diagnostic by about, or at least about 100%, 99%, 95%, 90%, 80%, 75%, 70%, 65%, or about 60% when compared to the use of standard cytological techniques known to the art. In some cases, the methods of the present invention may reduce the number of samples scored as intermediate or suspicious by about, or at least about 100%, 99%, 98%, 97%, 95%, 90%, 85%, 80%, 75%, 70%, 65%, or about 60%, when compared to the standard cytological methods used in the art.

In some cases the results of the molecular profiling assays, are entered into a database for access by representatives or agents of a molecular profiling business, the individual, a medical provider, or insurance provider. In some cases assay results include sample classification, identification, or diagnosis by a representative, agent or consultant of the business, such as a medical professional. In other cases, a computer or algorithmic analysis of the data is provided automatically. In some cases the molecular profiling business may bill the individual, insurance provider, medical provider, researcher, or government entity for one or more of the following: molecular profiling assays performed, consulting services, data analysis, reporting of results, or database access.

In some embodiments of the present invention, the results of the molecular profiling are presented as a report on a computer screen or as a paper record. In some cases, the report may include, but is not limited to, such information as one or more of the following: the number of genes differentially expressed, the suitability of the original sample, the number of genes showing differential alternative splicing, a diagnosis, a statistical confidence for the diagnosis, the likelihood of an ILD, and indicated therapies.

### (iv) Categorization of Samples Based on Molecular Profiling Results

The results of the molecular profiling may be classified into one of the following: ILD, a particular type of ILD, a non-ILD, or non diagnostic (providing inadequate information concerning the presence or absence of an ILD). In some cases, the results of the molecular profiling may be classified into IPF versus NSIP categories.

In some embodiments of the present invention, results are classified using a trained algorithm. Trained algorithms of the present invention include algorithms that have been developed using a reference set of known ILD and normal samples including but not limited to samples with one or more histopathologies listed in Tables 1 and 2. In some embodiments, the algorithm is further trained using one or more of the classification panels in any one of Tables 3-11 and 13-34, in any combination. In some embodiments, training comprises comparison of gene expression product levels in a first set biomarkers from a first ILD to gene expression product levels in a second set of biomarkers from a second ILD, where the first set of biomarkers includes at least one biomarkers that is not in the second set. In some embodiments, either the entire algorithm or portions of the algorithm can be trained using comparisons of expression levels of biomarker panels within a classification panel against all other biomarker panels (or all other biomarker signatures) used in the algorithm.

Algorithms suitable for categorization of samples include but are not limited to k-nearest neighbor algorithms, support vector machines, linear discriminant analysis, diagonal linear discriminant analysis, updown, naive Bayesian algorithms, neural network algorithms, hidden Markov model algorithms, genetic algorithms, or any combination thereof.

In some cases, trained algorithms of the present invention may incorporate data other than gene expression or alternative splicing data such as but not limited to DNA polymorphism data, sequencing data, scoring or diagnosis by cytologists or pathologists of the present invention, information provided by the pre-classifier algorithm of the present disclosure, or information about the medical history of the subject of the present disclosure.

When classifying a biological sample for diagnosis of ILD, there are typically two possible outcomes from a binary classifier. When a binary classifier is compared with actual true values (e.g., values from a biological sample), there are typically four possible outcomes. If the outcome from a prediction is p (where "p" is a positive classifier output, such as a particular ILD) and the actual value is also p, then it is called a true positive (TP); however if the actual value is n then it is said to be a false positive (FP). Conversely, a true negative has occurred when both the prediction outcome and the actual value are n (where "n" is a negative classifier output, such as no ILD, or absence of a particular disease tissue as described herein), and false negative is when the prediction outcome is n while the actual value is p. In one embodiment, consider a diagnostic test that seeks to determine whether a person has a certain disease. A false positive in this case occurs when the person tests positive, but actually does not have the disease. A false negative, on the other hand, occurs when the person tests negative, suggesting they are healthy, when they actually do have the disease. In some embodiments, a Receiver Operator Characteristic (ROC) curve assuming real-world prevalence of subtypes can be generated by re-sampling errors achieved on available samples in relevant proportions.

The positive predictive value (PPV), or precision rate, or post-test probability of disease, is the proportion of patients with positive test results who are correctly diagnosed. It is the most important measure of a diagnostic method as it reflects the probability that a positive test reflects the underlying condition being tested for. Its value does however depend on the prevalence of the disease, which may vary. In one example, FP (false positive); TN (true negative); TP (true positive); FN (false negative). False positive rate (.alpha.)=FP/(FP+TN)-specificity; False negative rate (.beta.)=FN/(TP+FN)-sensitivity; Power=sensitivity=1-β; Likelihood-ratio positive=sensitivity/(1-specificity); Likelihood-ratio negative=(1 -sensitivity)/specificity.

The negative predictive value is the proportion of patients with negative test results who are correctly diagnosed. PPV and NPV measurements can be derived using appropriate disease subtype prevalence estimates. An estimate of the pooled disease prevalence can be calculated from the pool of indeterminates which roughly classify into B vs M by surgery. For subtype specific estimates, in some embodiments, disease prevalence may sometimes be incalculable because there are not any available samples. In these cases, the subtype disease prevalence can be substituted by the pooled disease prevalence estimate.

In some embodiments, the level of expression products or alternative exon usage is indicate of one or the following: IPF, NSIP, or HP.

In some embodiments, the results of the expression analysis of the subject methods provide a statistical confidence level that a given diagnosis is correct. In some embodiments, such statistical confidence level is at least about, or more than about 85%, 90%, 91%, 92%, 93%, 94%, 95%, 96%, 97%, 98%, 99% 99.5%, or more.

### REPORTS

A subject method can include generating a report that provides an indication that a sample (a lung tissue sample) is an ILD sample. A subject diagnostic method can include generating a report that provides an indication as to whether an individual being tested has an ILD. A subject method can include generating a report that provides an indication as to whether an individual being tested has IPF (and not, e.g., an ILD other than IPF; e.g., the report can indicate that the individual has IPF and not NSIP).

In some embodiments, a subject method of diagnosing an ILD involves generating a report. Such a report can include information such as a likelihood that the patient has an ILD; a recommendation regarding further evaluation; a recommendation regarding therapeutic drug and/or device intervention; and the like.

For example, the methods disclosed herein can further include a step of generating or outputting a report providing the results of a subject diagnostic method, which report can be provided in the form of an electronic medium (e.g., an electronic display on a computer monitor), or in the form of a tangible medium (e.g., a report printed on paper or other tangible medium). An assessment as to the results of a subject diagnostic method (e.g., a likelihood that an individual has an ILD; a likelihood that an individual has IPF) can be referred to as a "report" or, simply, a "score." A person or entity that prepares a report ("report generator") may also perform steps such as sample gathering, sample processing, and the like. Alternatively, an entity other than the report generator can perform steps such as sample gathering, sample processing, and the like. A diagnostic assessment report can be provided to a user. A "user" can be a health professional (e.g., a clinician, a laboratory technician, a physician (e.g., a cardiologist), etc.).

A subject report can further include one or more of: 1) service provider information; 2) patient data; 3) data regarding the expression level of a given gene product or set of gene products, a score or algorithm decision; 4) follow-up evaluation recommendations; 5) therapeutic intervention recommendations; and 6) other features.

### Further evaluation

Based on the expression level of a given gene product or set of gene products, and/or based on a report (as described above), a physician or other qualified medical personnel can determine whether further evaluation of the test subject (the patient) is required. Further evaluation can include, e.g., spirometry.

### Therapeutic intervention

Based on the expression level of a given gene product or set of gene products, and/or based on a report (as described above), a physician or other qualified medical personnel can determine whether appropriate therapeutic intervention is advised.

Therapeutic intervention includes drug-based therapeutic intervention, device-based therapeutic intervention, and surgical intervention. Where a report indicates a likelihood that an individual has IPF, drug-based therapeutic intervention includes, e.g., administering to the individual an effective amount of pirfenidone, prednisone, azathioprine, or N-acetylcysteine. Surgical intervention includes, e.g., arterial bypass surgery.

### COMPUTER-IMPLEMENTED METHODS, SYSTEMS AND DEVICES

The methods of the present disclosure can be computer-implemented, such that method steps (e.g., assaying, comparing, calculating, and the like) are be automated in whole or in part. Accordingly, the present disclosure provides methods, computer systems, devices and the like in connection with computer-implemented methods of facilitating a diagnosis of an interstitial lung disease (e.g., a diagnosis of IPF, NSIP, HP, etc.), including differential diagnosis.

For example, the method steps, including obtaining values for biomarker levels, comparing normalized biomarker (gene) expression levels to a control level, calculating the likelihood of an ILD, generating a report, and the like, can be completely or partially performed by a computer program product. Values obtained can be stored electronically, e.g., in a database, and can be subjected to an algorithm executed by a programmed computer.

For example, the methods of the present disclosure can involve inputting a biomarker level (e.g., a normalized expression level of a gene product) into a computer programmed to execute an algorithm to perform the comparing and calculating step(s) described herein, and generate a report as described herein, e.g., by displaying or printing a report to an output device at a location local or remote to the computer. The output to the report can be a score (e.g., numerical score (representative of a numerical value) or a non-numerical score (e.g., non-numerical output (e.g., "IPF", "No evidence of IPF") representative of a numerical value or range of numerical values.

The present invention thus provides a computer program product including a computer readable storage medium having a computer program stored on it. The program can, when read by a computer, execute relevant calculations based on values obtained from analysis of one or more biological sample (e.g., lung tissue sample) from an individual. The computer program product has stored therein a computer program for performing the calculation(s).

The present disclosure provides systems for executing the program described above, which system generally includes: a) a central computing environment; b) an input device, operatively connected to the computing environment, to receive patient data, wherein the patient data can include, for example, biomarker level or other value obtained from an assay using a biological sample from the patient, as described above; c) an output device, connected to the computing environment, to provide information to a user (e.g., medical personnel); and d) an algorithm executed by the central computing environment (e.g., a processor), where the algorithm is executed based on the data received by the input device, and wherein the algorithm calculates a value, which value is indicative of the likelihood the subject has an ILD, as described herein.

### Computer Systems

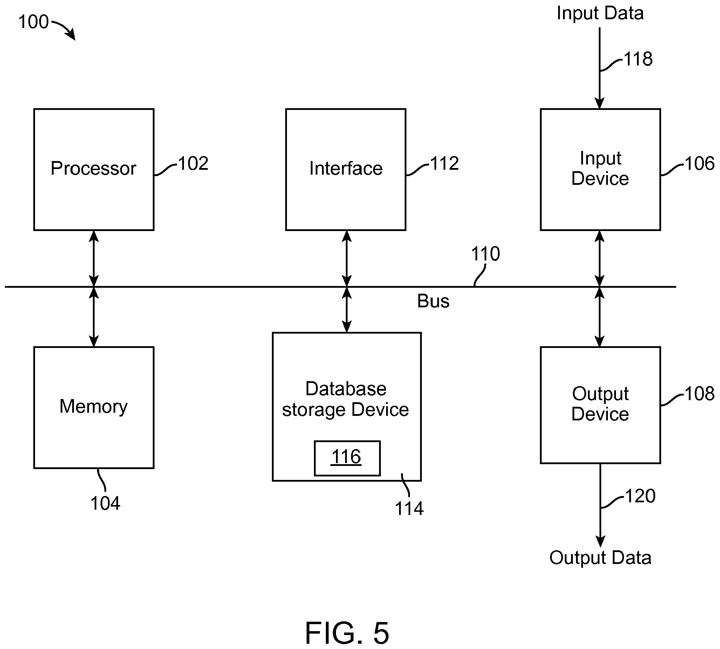

A generalized example of a computerized embodiment in which programs to facilitate execution of the methods of the present disclosure can be implemented is depicted in Figure 5, which illustrates a processing system 100 which generally comprises at least one processor 102, or processing unit or plurality of processors, memory 104, at least one input device 106 and at least one output device 108, coupled together via a bus or group of buses 110. In certain embodiments, input device 106 and output device 108 can be the same device. An interface 112 can also be provided for coupling the processing system 100 to one or more peripheral devices, for example interface 112 can be a PCI card or PC card. At least one storage device 114 which houses at least one database 116 can also be provided.

The memory 104 can be any form of memory device, for example, volatile or non-volatile memory, solid state storage devices, magnetic devices, etc. The processor 102 can comprise more than one distinct processing device, for example to handle different functions within the processing system 100. Input device 106 receives input data 118 and can comprise, for example, a keyboard, a pointer device such as a pen-like device or a mouse, audio receiving device for voice controlled activation such as a microphone, data receiver or antenna such as a modem or wireless data adaptor, data acquisition card, etc. Input data 118 can come from different sources, for example keyboard instructions in conjunction with data received via a network.

Output device 108 produces or generates output data 120 and can comprise, for example, a display device or monitor in which case output data 120 is visual, a printer in which case output data 120 is printed, a port for example a USB port, a peripheral component adaptor, a data transmitter or antenna such as a modem or wireless network adaptor, etc. Output data 120 can be distinct and derived from different output devices, for example a visual display on a monitor in conjunction with data transmitted to a network. A user can view data output, or an interpretation of the data output, on, for example, a monitor or using a printer. The storage device 114 can be any form of data or information storage means, for example, volatile or non-volatile memory, solid state storage devices, magnetic devices, etc.

In use, the processing system 100 is adapted to allow data or information to be stored in and/or retrieved from, via wired or wireless communication means, at least one database 116. The interface 112 may allow wired and/or wireless communication between the processing unit 102 and peripheral components that may serve a specialized purpose. In general, the processor 102 can receive instructions as input data 118 via input device 106 and can display processed results or other output to a user by utilizing output device 108. More than one input device 106 and/or output device 108 can be provided. The processing system 100 may be any suitable form of terminal, server, specialized hardware, or the like.

The processing system 100 may be a part of a networked communications system. Processing system 100 can connect to a network, for example the Internet or a WAN. Input data 118 and output data 120 can be communicated to other devices via the network. The transfer of information and/or data over the network can be achieved using wired communications means or wireless communications means. A server can facilitate the transfer of data between the network and one or more databases. A server and one or more databases provide an example of an information source.

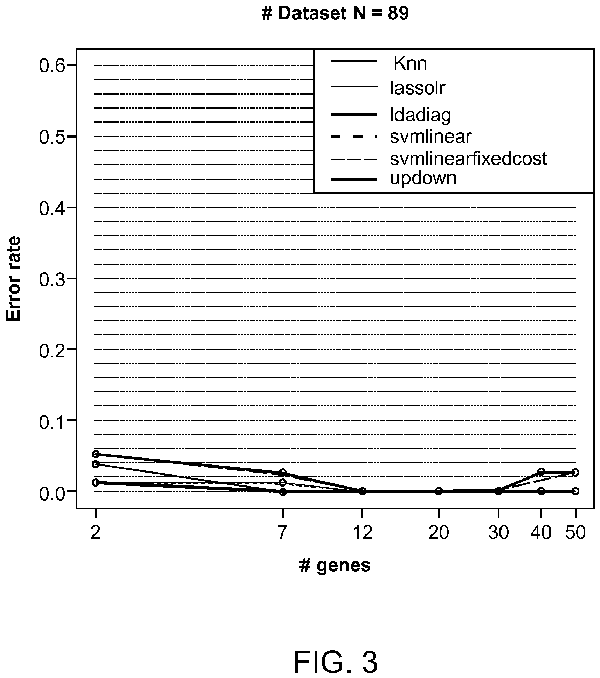

Thus, the processing computing system environment 100 illustrated in Fig. 3 may operate in a networked environment using logical connections to one or more remote computers. The remote computer may be a personal computer, a server, a router, a network PC, a peer device, or other common network node, and typically includes many or all of the elements described above.

The logical connections depicted in Fig. 5 may include a local area network (LAN) and a wide area network (WAN), but may also include other networks such as a personal area network (PAN). Such networking environments are commonplace in offices, enterprise-wide computer networks, intranets, and the Internet. For instance, when used in a LAN networking environment, the computing system environment 100 is connected to the LAN through a network interface or adapter. When used in a WAN networking environment, the computing system environment typically includes a modem or other means for establishing communications over the WAN, such as the Internet. The modem, which may be internal or external, may be connected to a system bus via a user input interface, or via another appropriate mechanism. In a networked environment, program modules depicted relative to the computing system environment 100, or portions thereof, may be stored in a remote memory storage device. It is to be appreciated that the illustrated network connections of Fig. **5** are examples and other means of establishing a communications link between multiple computers may be used.

Fig. **5** is intended to provide a brief, general description of an illustrative and/or suitable example of a computing environment in which embodiments of the methods disclosed herein may be implemented. Fig. **5** is an example of a suitable environment and is not intended to suggest any limitation as to the structure, scope of use, or functionality of an embodiment of the present invention. A particular environment should not be interpreted as having any dependency or requirement relating to any one or combination of components illustrated in an exemplary operating environment. For example, in certain instances, one or more elements of an environment may be deemed not necessary and omitted. In other instances, one or more other elements may be deemed necessary and added.

Certain embodiments may be described with reference to acts and symbolic representations of operations that are performed by one or more computing devices, such as the computing system environment 100 of Fig. **5****.** As such, it will be understood that such acts and operations, which are at times referred to as being computer-executed, include the manipulation by the processor of the computer of electrical signals representing data in a structured form. This manipulation transforms the data or maintains them at locations in the memory system of the computer, which reconfigures or otherwise alters the operation of the computer in a manner understood by those skilled in the art. The data structures in which data is maintained are physical locations of the memory that have particular properties defined by the format of the data. However, while an embodiment is being described in the foregoing context, it is not meant to be limiting as those of skill in the art will appreciate that the acts and operations described hereinafter may also be implemented in hardware.

Embodiments may be implemented with numerous other general-purpose or special-purpose computing devices and computing system environments or configurations. Examples of well-known computing systems, environments, and configurations that may be suitable for use with an embodiment include, but are not limited to, personal computers, handheld or laptop devices, personal digital assistants, multiprocessor systems, microprocessor-based systems, programmable consumer electronics, network, minicomputers, server computers, web server computers, mainframe computers, and distributed computing environments that include any of the above systems or devices.

Embodiments may be described in a general context of computer-executable instructions, such as program modules, being executed by a computer. Generally, program modules include routines, programs, objects, components, data structures, etc., that perform particular tasks or implement particular abstract data types. An embodiment may also be practiced in a distributed computing environment where tasks are performed by remote processing devices that are linked through a communications network. In a distributed computing environment, program modules may be located in both local and remote computer storage media including memory storage devices.

### Computer program products

The present disclosure provides computer program products that, when executed on a programmable computer such as that described above with reference to Fig. 5, can carry out the methods of the present disclosure. As discussed above, the subject matter described herein may be embodied in systems, apparatus, methods, and/or articles depending on the desired configuration. These various implementations may include implementation in one or more computer programs that are executable and/or interpretable on a programmable system including at least one programmable processor, which may be special or general purpose, coupled to receive data and instructions from, and to transmit data and instructions to, a storage system, at least one input device (e.g. video camera, microphone, joystick, keyboard, and/or mouse), and at least one output device (e.g. display monitor, printer, etc.).

Computer programs (also known as programs, software, software applications, applications, components, or code) include instructions for a programmable processor, and may be implemented in a high-level procedural and/or object-oriented programming language, and/or in assembly/machine language. As used herein, the term "machine-readable medium" refers to any computer program product, apparatus and/or device (e.g., magnetic discs, optical disks, memory, etc.) used to provide machine instructions and/or data to a programmable processor, including a machine-readable medium that receives machine instructions as a machine-readable signal.

It will be apparent from this description that aspects of the present disclosure may be embodied, at least in part, in software, hardware, firmware, or any combination thereof. Thus, the techniques described herein are not limited to any specific combination of hardware circuitry and/or software, or to any particular source for the instructions executed by a computer or other data processing system. Rather, these techniques may be carried out in a computer system or other data processing system in response to one or more processors, such as a microprocessor, executing sequences of instructions stored in memory or other computer-readable medium including any type of ROM, RAM, cache memory, network memory, floppy disks, hard drive disk (HDD), solid-state devices (SSD), optical disk, CD-ROM, and magnetic-optical disk, EPROMs, EEPROMs, flash memory, or any other type of media suitable for storing instructions in electronic format.

In addition, the processor(s) may be, or may include, one or more programmable general-purpose or special-purpose microprocessors, digital signal processors (DSPs), programmable controllers, application specific integrated circuits (ASICs), programmable logic devices (PLDs), trusted platform modules (TPMs), or the like, or a combination of such devices. In alternative embodiments, special-purpose hardware such as logic circuits or other hardwired circuitry may be used in combination with software instructions to implement the techniques described herein.

### ARRAYS AND KITS

The present disclosure provides arrays and kits for use in carrying out a subject evaluating method or a subject diagnostic method.

### Arrays

A subject array can comprise a plurality of nucleic acids, each of which hybridizes to a gene differentially expressed in a cell present in a tissue sample obtained from an individual being tested for an ILD.

A subject array can comprise a plurality of member nucleic acids, each of which member nucleic acids hybridizes to a different gene product, where gene products of interest are those set out in Tables 3-11 and 13-34. In some cases, two or more member nucleic acids hybridize to the same gene product; e.g., in some cases 2, 3, 4, 5, 6, 7, 8, 9, 10, or more member nucleic acids hybridize to the same gene product. A member nucleic acid can have a length of from about 5 nucleotides (nt) to about 100 nt, e.g., 5, 6, 7, 8, 9, 10, 11, 12, 13, 14, 15, 16, 17 18, 19, 20, 20-25, 25-30, 30-40, 40-50, 50-60, 60-70, 70-80, 80-90, or 90-100 nt. A nucleic acid can have one or more phosphate backbone modifications.

A subject array can include from about 10 to about 10⁵ unique member nucleic acids, or more than 10⁵ unique member nucleic acids. For example, a subject array can include from about 10 to about 102, from about 102 to about 103, from about 103 to about 104, from about 104 to about 105, or more than 105, unique member nucleic acids.

In some cases, a subject array comprises a plurality of member nucleic acids, one or more of which member nucleic acids hybridizes to a different gene product of a gene selected from: 1) THY1; 2) GALNT13; 3) CFH; 4) CRTAC1; 5) CHD3; 6) TSHZ2; 7) ENSG00000123119; 8) ISM1; 9) ENSG00000236972; 10) PDE7B; 11) SULF1; 12) FABP5; 13) FABP5; 14) PTGFRN; 15) IGFBP5; 16) CFHR1; 17) MS4A15; 18) FAM13C; 19) STC1; 20) RCAN2; 21) LG12; 22) CCND2; 23) DCLK1; 24) C22orf46; 25) IGF1; 26) CSF3R; 27) PPP4R4; 28) CEBPD; 29) NMNAT2; 30) EPB41L5; 31) ZNF385D; 32) FCN3; 33) CNTN6; 34) DGKA; 35) CSGALNACT1; 36) SYT15; 37) STX11; 38) ITSN1; 39) TMEM100; 40) EGFLAM; 41) C13orf15; 42) ENSG00000182010; 43) ERRFI1; 44) RGS16; 45) SLN; 46) ENSG00000146374; 47) TCID 8066275; 48) CSCL2; 49) ITLN1; and 50) PDE2A.

In some cases, a subject array comprises a plurality of member nucleic acids, one or more of which member nucleic acids hybridizes to a different gene product of a gene selected from: 1) ASPM; 2) BUB1; 3) PTTG1; 4) SHCBP1; 5) NUSAP1; 6) MKI67; 7) HJURP; 8) CDCA3; 9) PLK1; 10) PRR11; 11) BRCA2; 12) ORM1; 13) CCNB2; 14) SMC4; 15) HM13; 16) DMD; 17) FHL1; 18) ORM2; 19) NDUFC2-KCTD14; 20) NCAPH; 21) TTLL7; 22) DEPDC1B; 23) CNTN4; 24) PRKAA2; 25) PRKCQ; 26) CDC42BPA; 27) PARD3B; 28) SCTR; 29) CSF3R; and 30) MPDZ.

### Kits

A kit of the present disclosure can include an array, as described above; and a reagent for analyzing an expression level of a gene product.

Reagents for analyzing an expression level of a nucleic acid gene product include, e.g., reagents suitable for sequencing a nucleic acid; reagents suitable for amplifying a nucleic acid; and reagents suitable for nucleic acid hybridization.

Other optional components of the kit include: a buffer; a detectable label; components for developing a detectable label (e.g., where a nucleic acid probe includes a detectable label); etc. The various components of the kit may be present in separate containers or certain compatible components may be pre-combined into a single container, as desired.

In addition to above-mentioned components, a subject kit can include instructions for using the components of the kit to practice a subject method. The instructions for practicing a subject method are generally recorded on a suitable recording medium. For example, the instructions may be printed on a substrate, such as paper or plastic, etc. As such, the instructions may be present in the kits as a package insert, in the labeling of the container of the kit or components thereof (i.e., associated with the packaging or subpackaging) etc. In other embodiments, the instructions are present as an electronic storage data file present on a suitable computer readable storage medium, e.g. compact disc-read only memory (CD-ROM), digital versatile disk (DVD), diskette, etc. In yet other embodiments, the actual instructions are not present in the kit, but means for obtaining the instructions from a remote source, e.g. via the internet, are provided. An example of this embodiment is a kit that includes a web address where the instructions can be viewed and/or from which the instructions can be downloaded. As with the instructions, this means for obtaining the instructions is recorded on a suitable substrate.

### EXAMPLES

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Celsius, and pressure is at or near atmospheric. Standard abbreviations may be used, e.g., bp, base pair(s); kb, kilobase(s); pl, picoliter(s); s or sec, second(s); min, minute(s); h or hr, hour(s); aa, amino acid(s); kb, kilobase(s); bp, base pair(s); nt, nucleotide(s); i.m., intramuscular(ly); i.p., intraperitoneal(ly); s.c., subcutaneous(ly); and the like.

### Terms and Abbreviations

The following examples utilize terms and abbreviations briefly described here:

| | |
|---|---|
| adj.P.Value.edgeR: | False discovery rate adjusted p value of RNAseq gene expression data using edgeR analysis. |
| adj.P.Value.microarray | False discovery rate adjusted p value of RNAseq gene expression data using microarray analysis |
| adj.P.Value.npSeq: | False discovery rate adjusted p value of RNAseq gene expression data using npSeq analysis |
| BRONCH: | Broncholitis |
| CIF-NOC | Chronic Interstitial Fibrosis Not Otherwise Classified |
| edgeR: | an R package for the significance analysis of sequencing data |
| Ensembl ID: | Gene Identifier from Ensembl Genome Browser database |
| FDR: | False Discovery Rate, an adjusted p value that limits the possibility that the results are random due to the large number of genes simultaneously evaluated. |
| Gene Symbol: | Gene Identifier from HUGO Gene Nomenclature Committee |
| HP: | Hypersensitivity Pneumonitis |
| HRCT | High resolution Computed Tomography |
| IPF: | Idiopathic Pulmonary Fibrosis |
| logFC.edgeR: | Log2 fold change of RNAseq gene expression data using edgeR analysis |
| logFC.microarray: | Log2 fold change of RNAseq gene expression data using microarray analysis |
| logFC.npSeq: | Log2 fold change of RNAseq gene expression data using npSeq analysis |
| microarray: | Gene expression analysis using Affymetrix Gene 1.0 ST arrays |
| NML: | Normal Lung, usually obtained from human lung donor tissue that was ultimately never transplanted |
| npSeq: | an R package for the significance analysis of sequencing data |
| NSIP: | Non Specific Interstitial Pneumonia |
| OP: | Organizing Pneumonia |
| P.value.edgeR: | p value of RNAseq gene expression data using edgeR analysis |
| P.value.microarray: | p value of RNAseq gene expression data using microarray analysis |
| P.value.npSeqp: | value of RNAseq gene expression data using npSeq analysis |
| RB: | Respiratory Broncholitis |
| REST: | A combination of all other ILDs except the subtype it is being compared to. Usually HP and NSIP, BRONCH, CIF-NOC, OP, RB and SARC. |
| SARC: | Sarcosidosis |
| SOC: | Squamous Cell Carcinoma |
| TCID: | "TCID" or "Transcript Cluster Identifier" refers to a gene level identifier used by all Affymetrix microarrays. Each TCID is associated with a fixed reference number that identifies a set of specific probes having sequences for a specific gene. Such specific probes are present on a given array commercially available from Affymetrix. TCID numbers thus refer to a gene product(s) of a specific gene. |
| UIP: | Usual Interstitial Pneumonia; the HRCT pattern observed in IPF |
| LIMMA: | Linear Models for Microarray Data; an R package for the significance analysis of microarray data. |

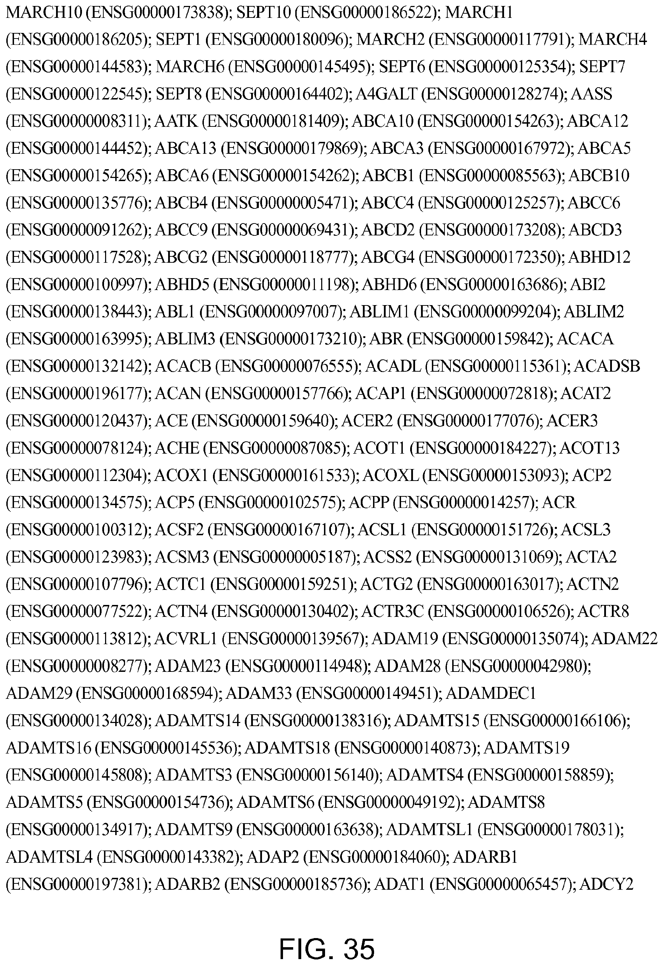

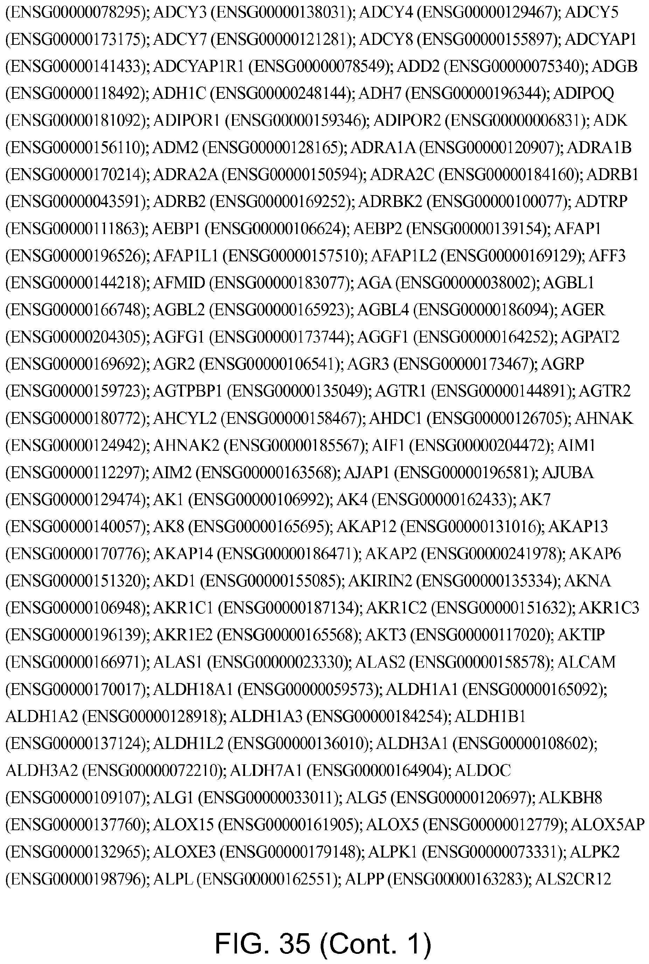

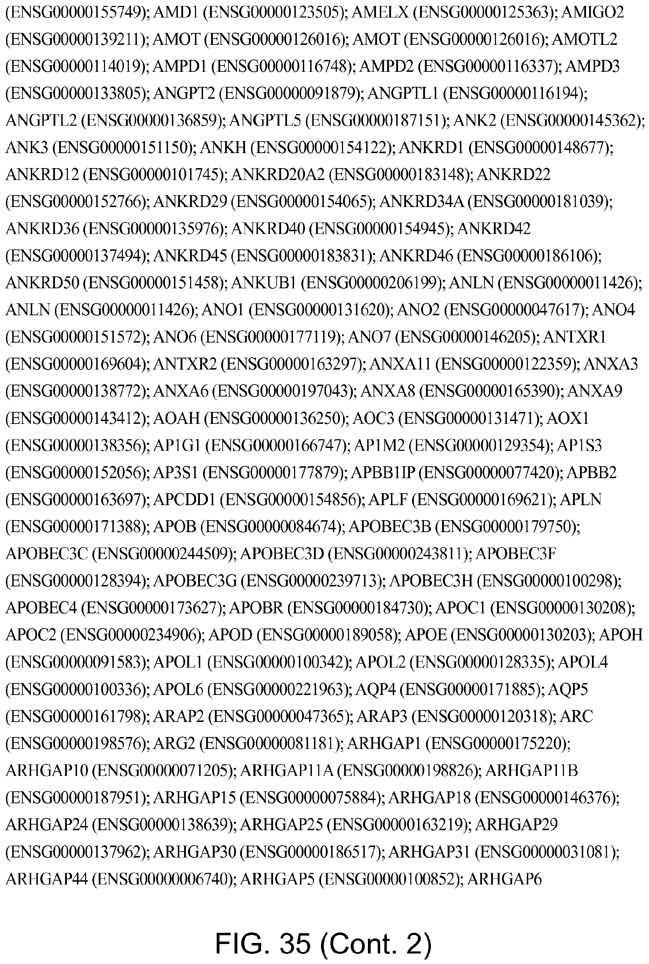

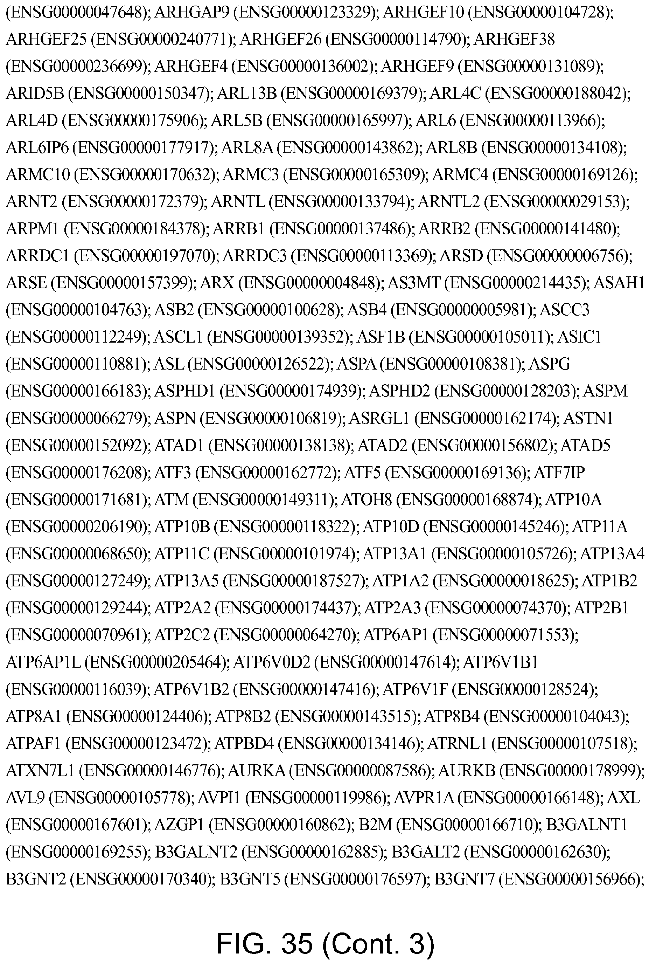

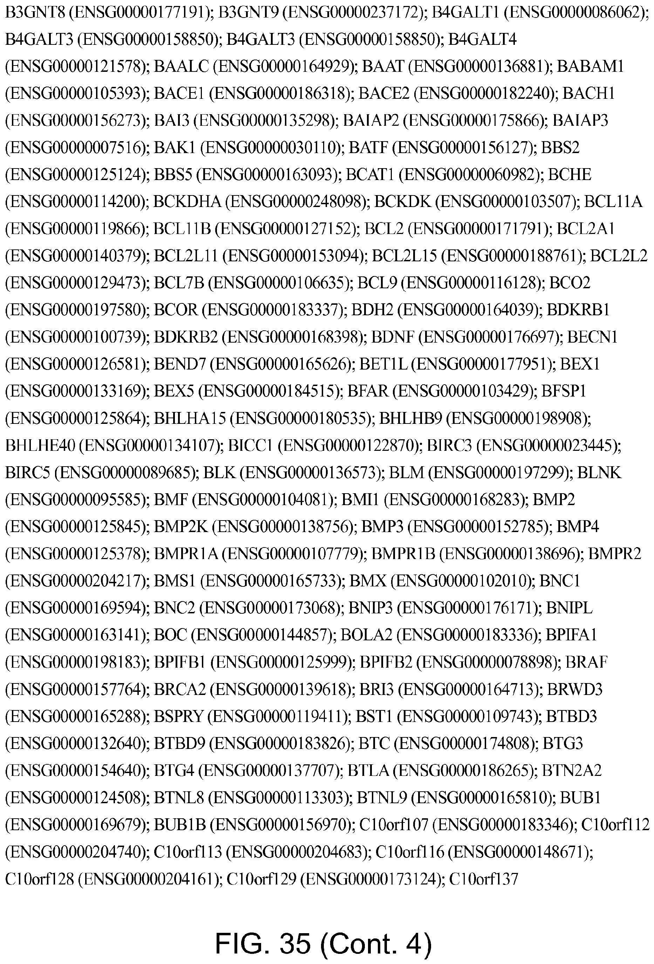

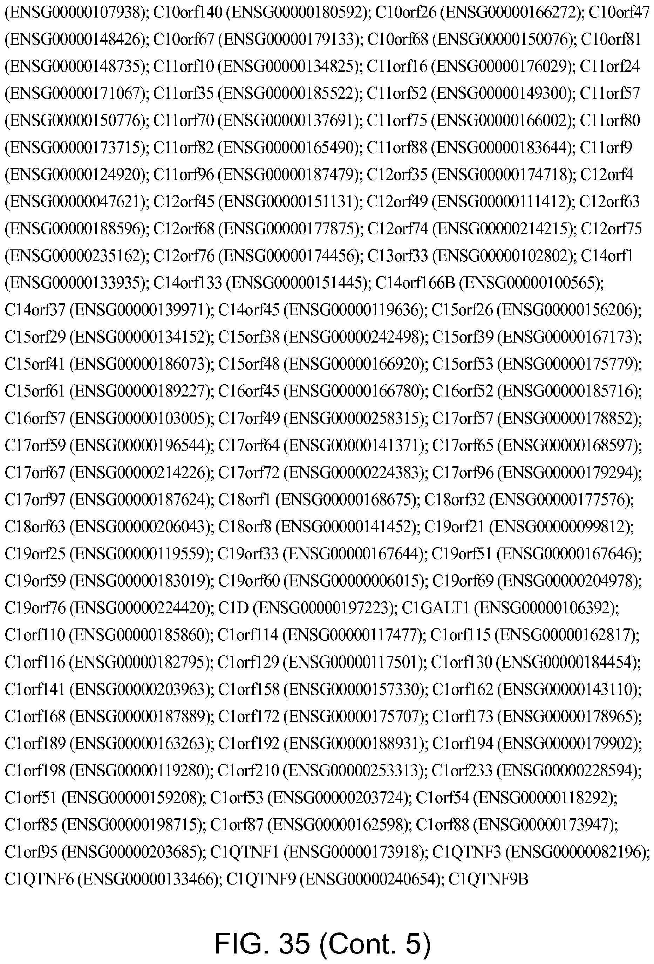

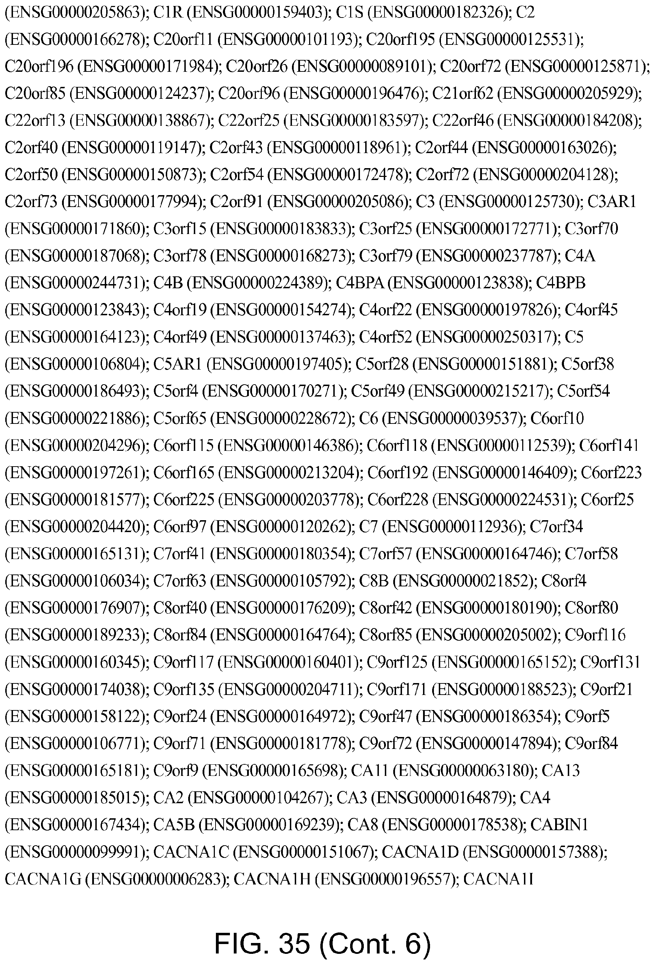

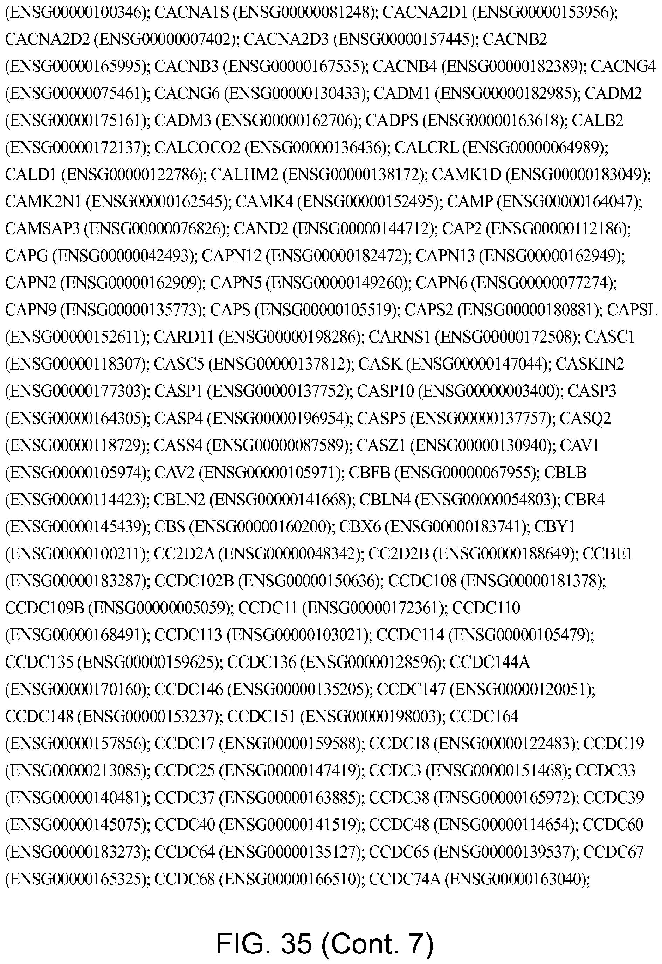

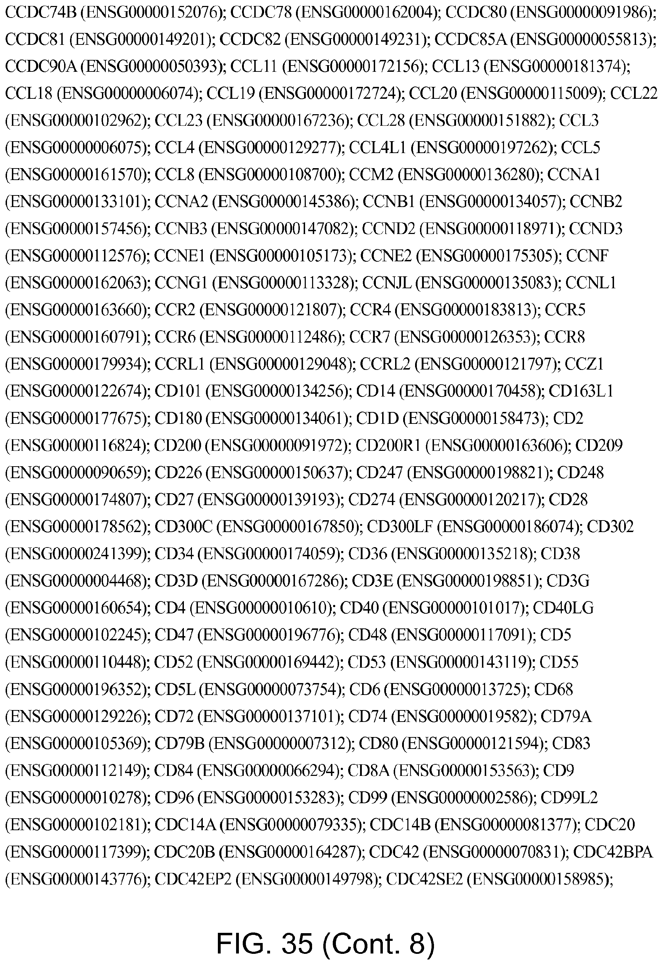

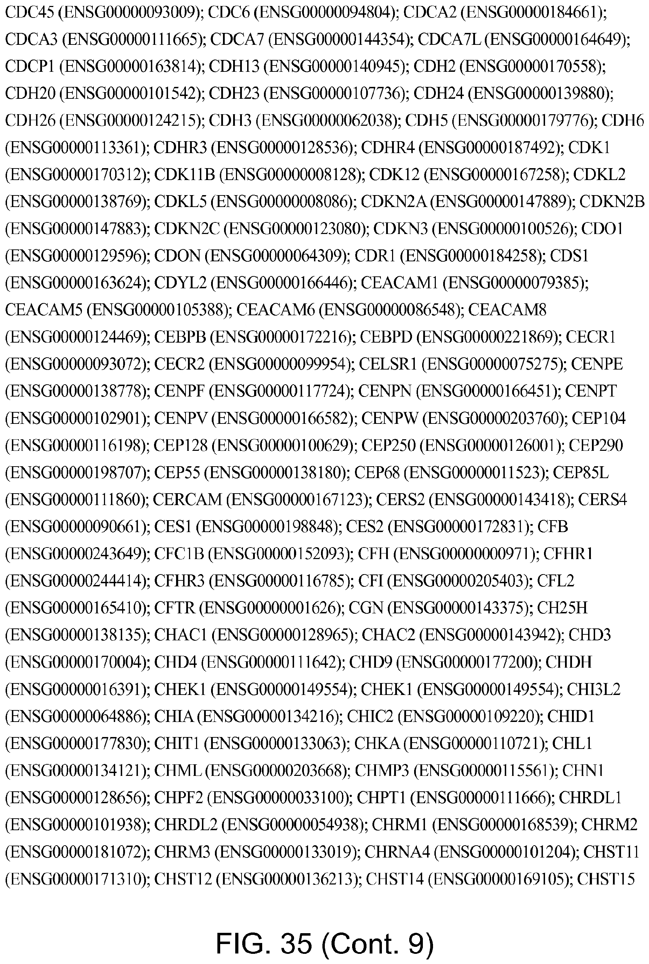

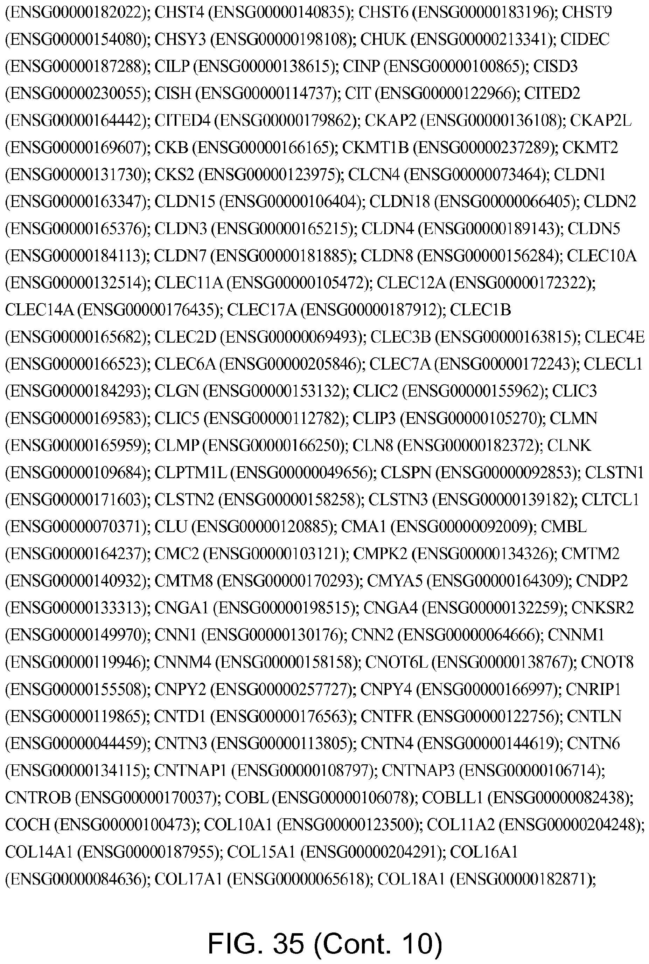

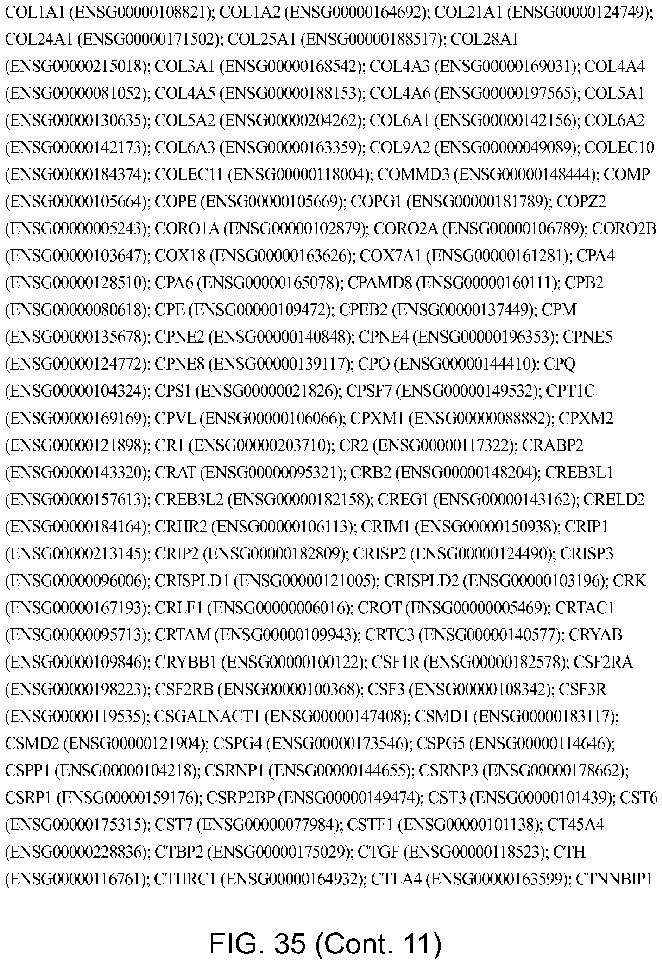

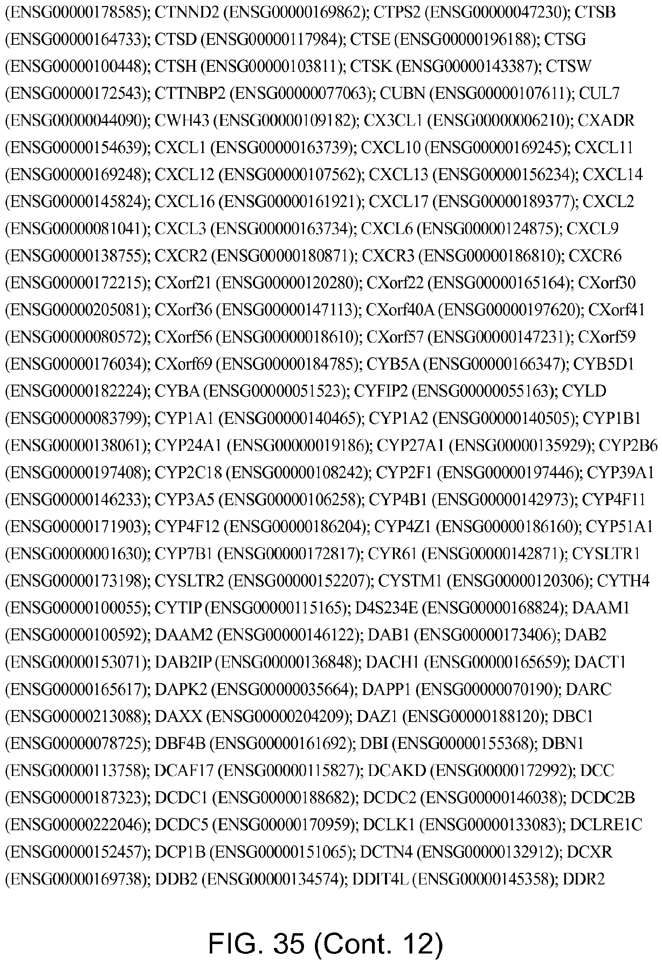

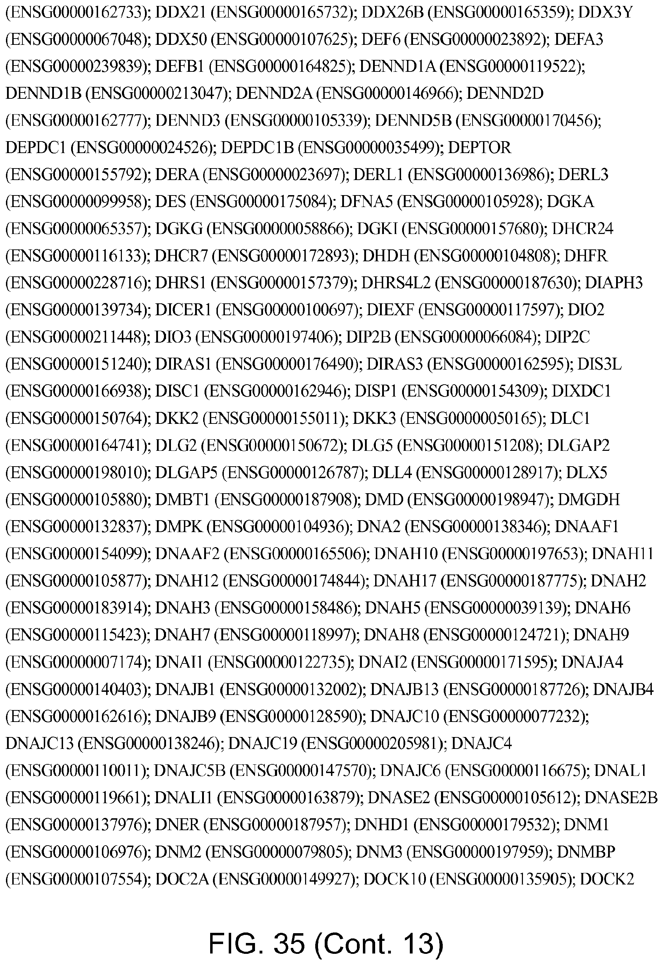

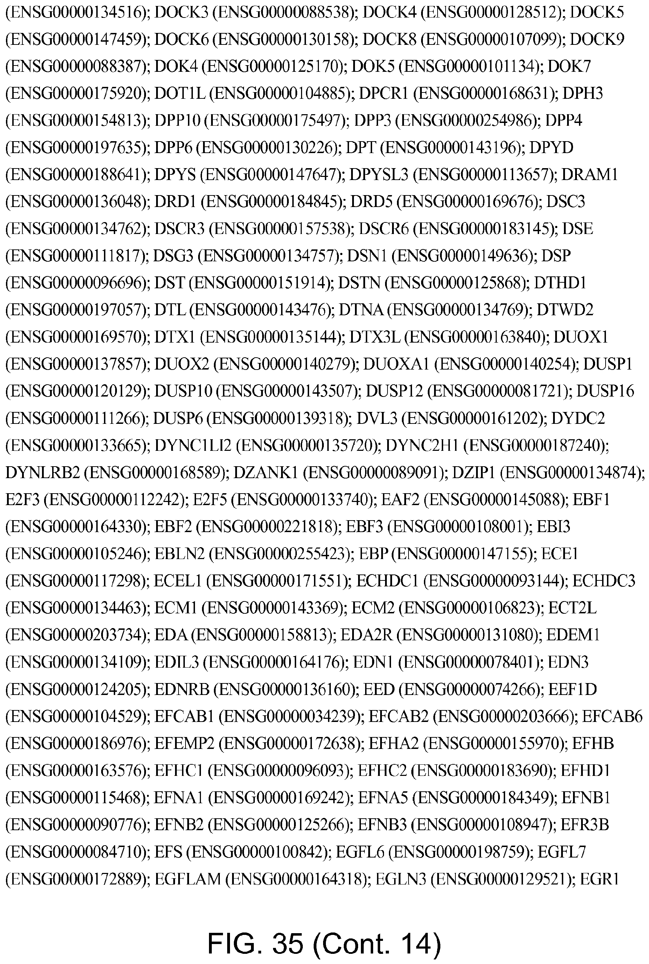

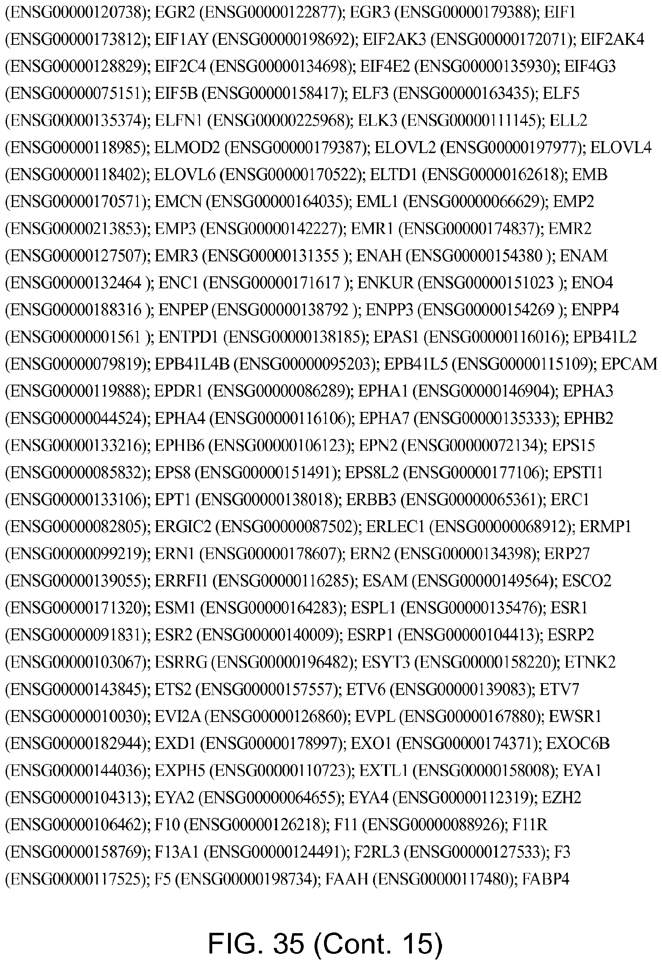

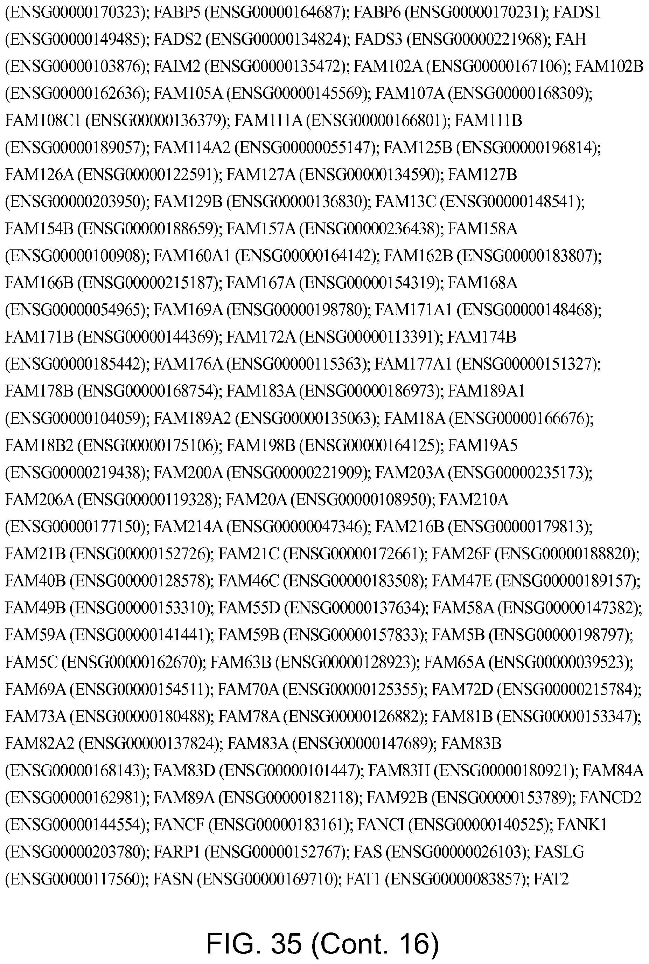

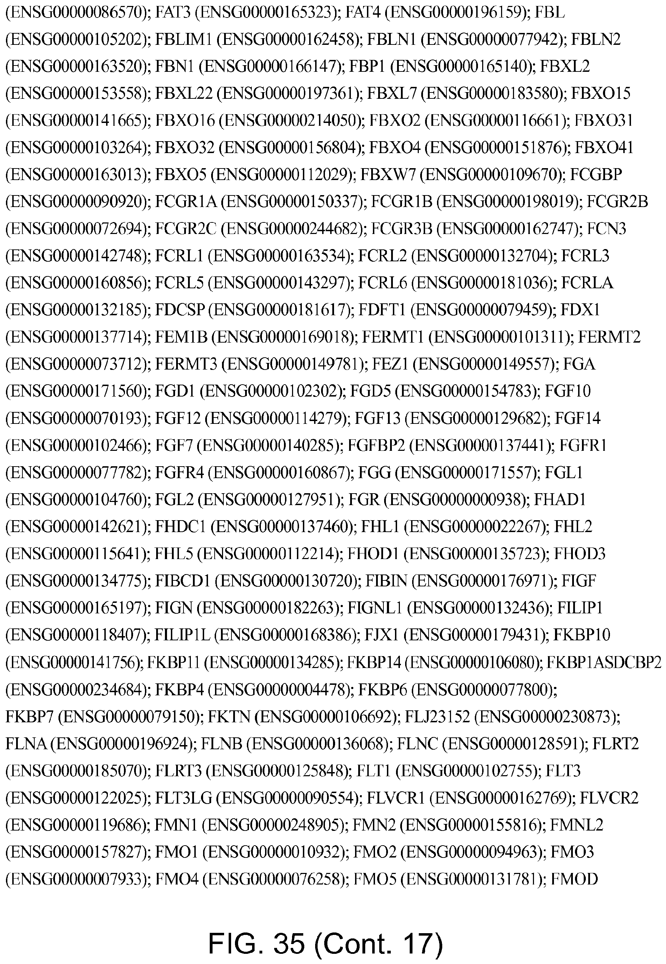

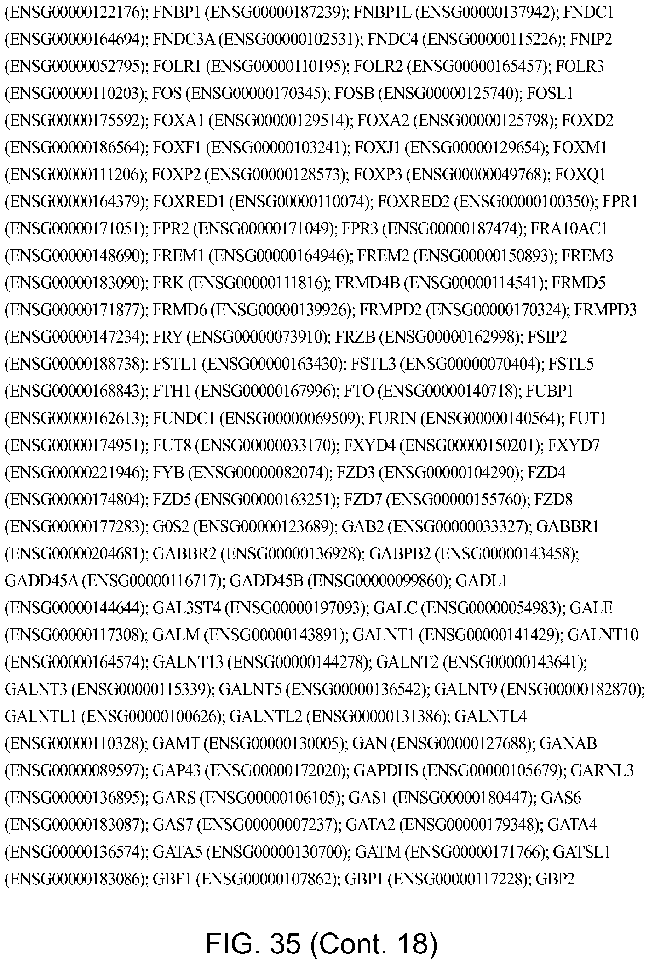

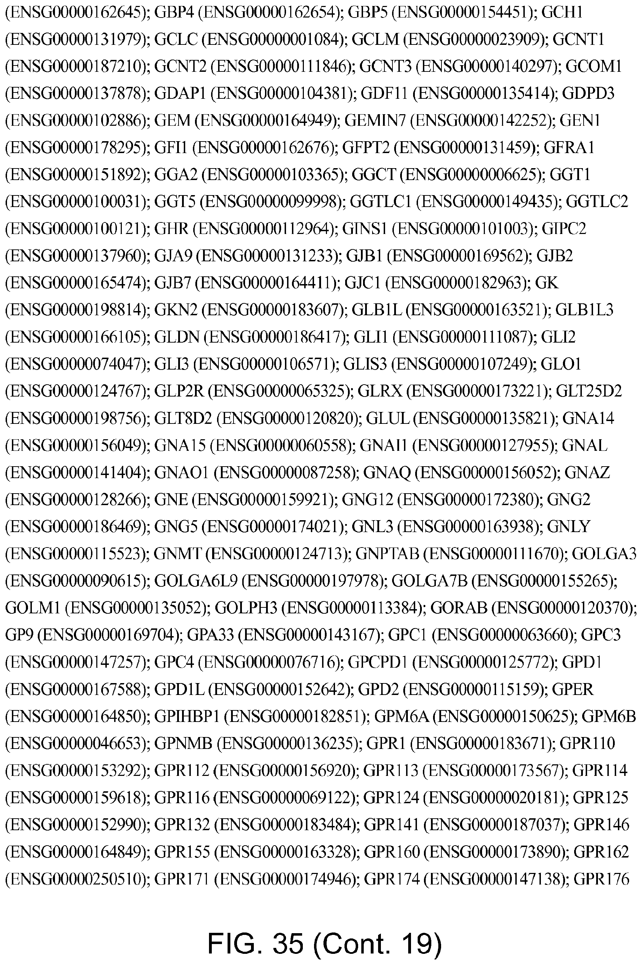

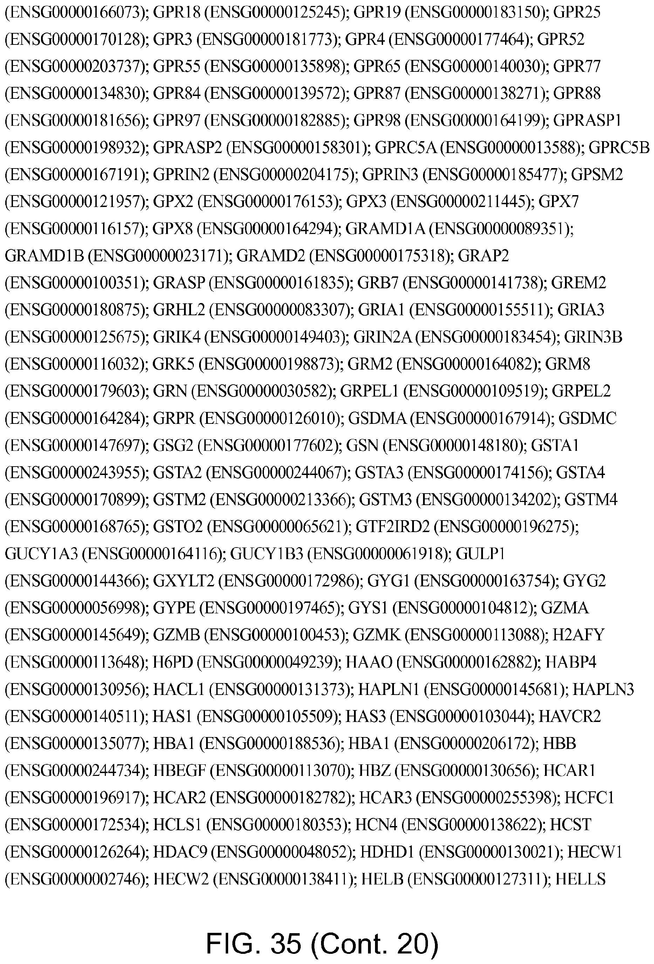

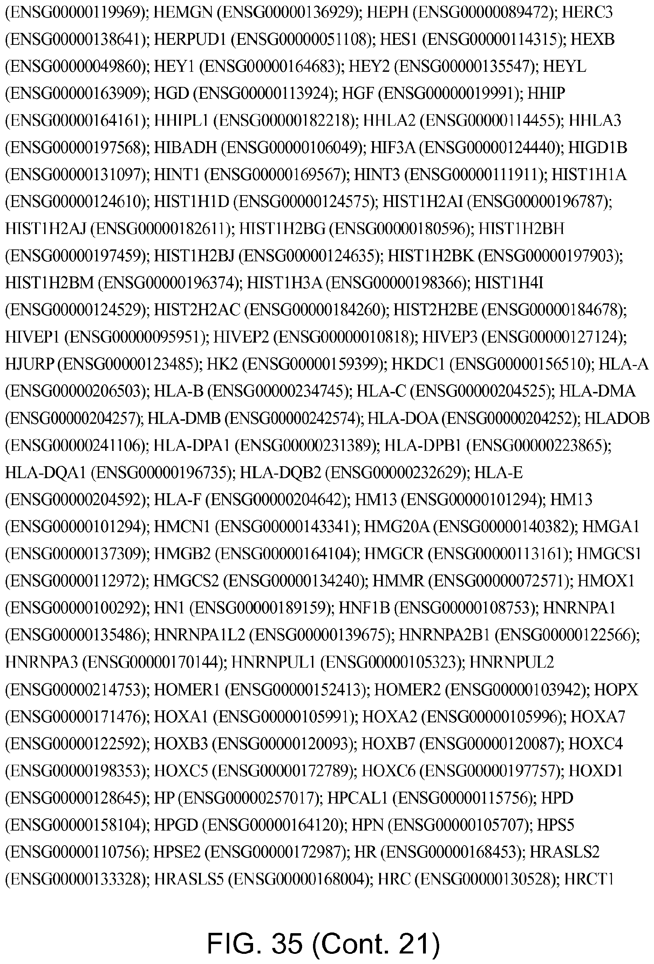

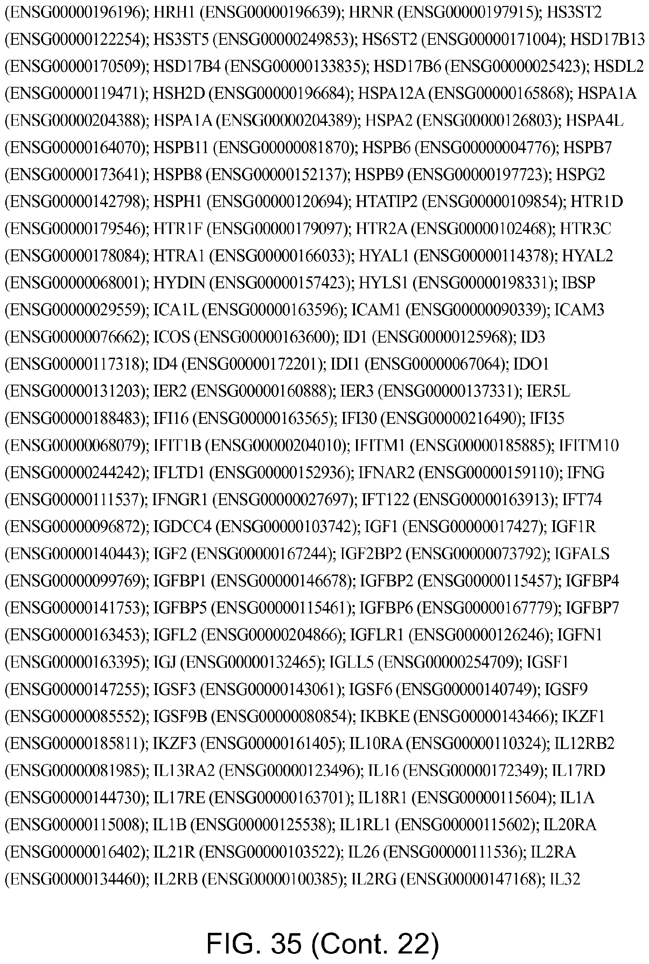

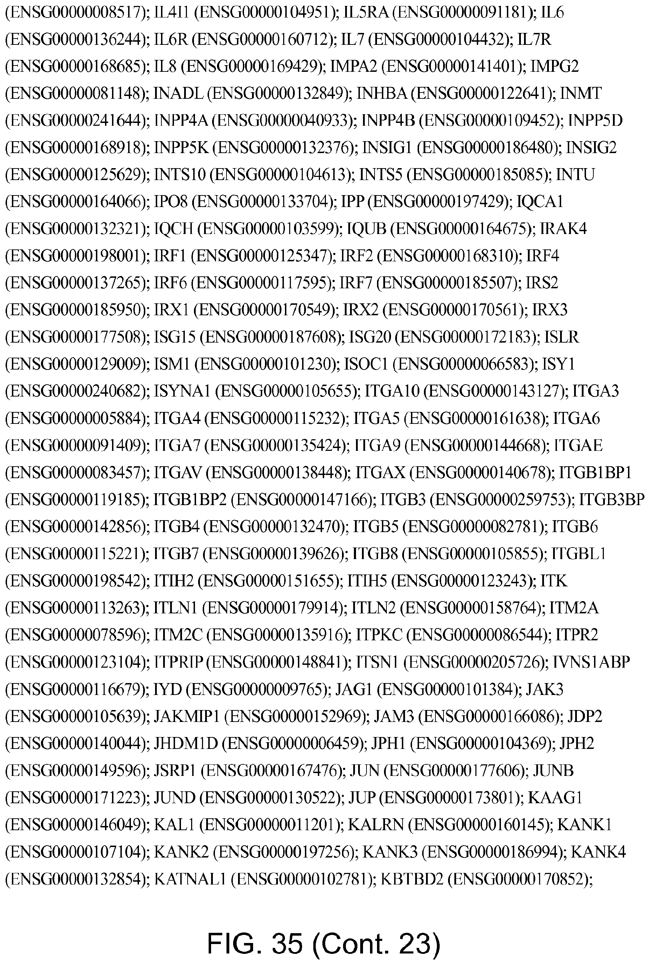

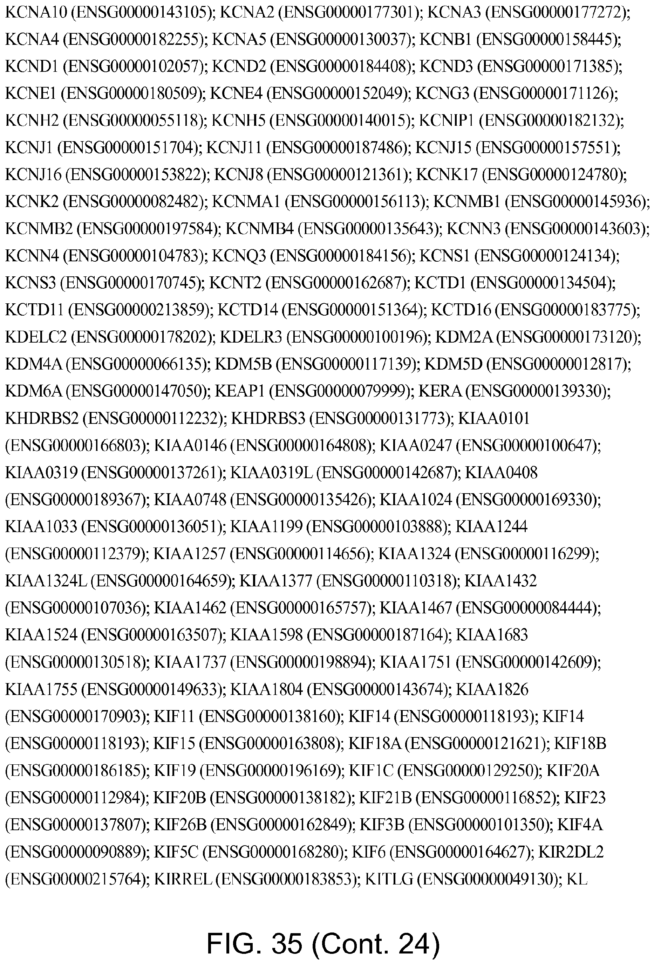

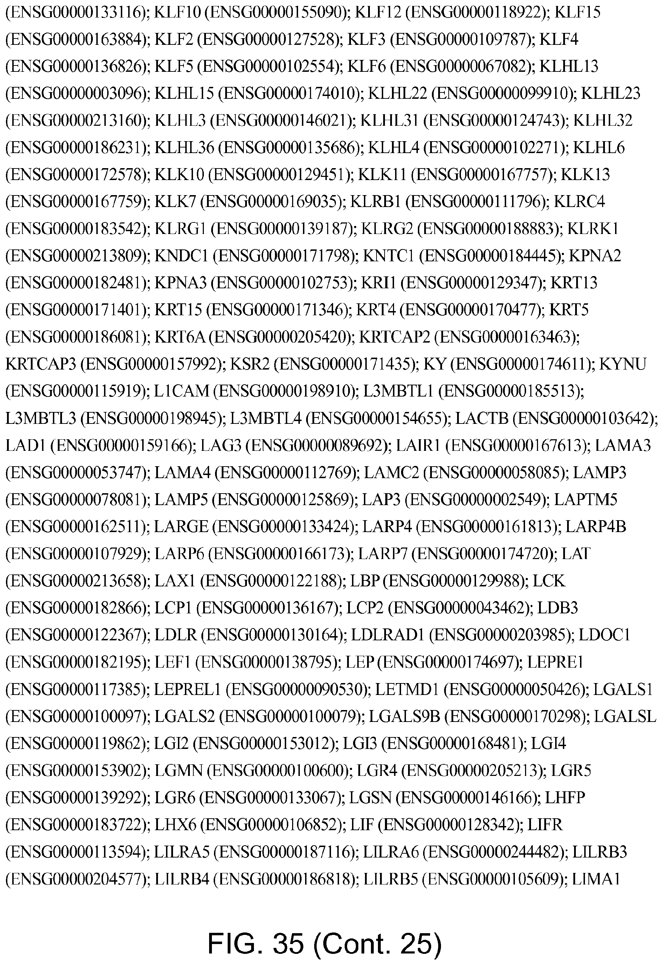

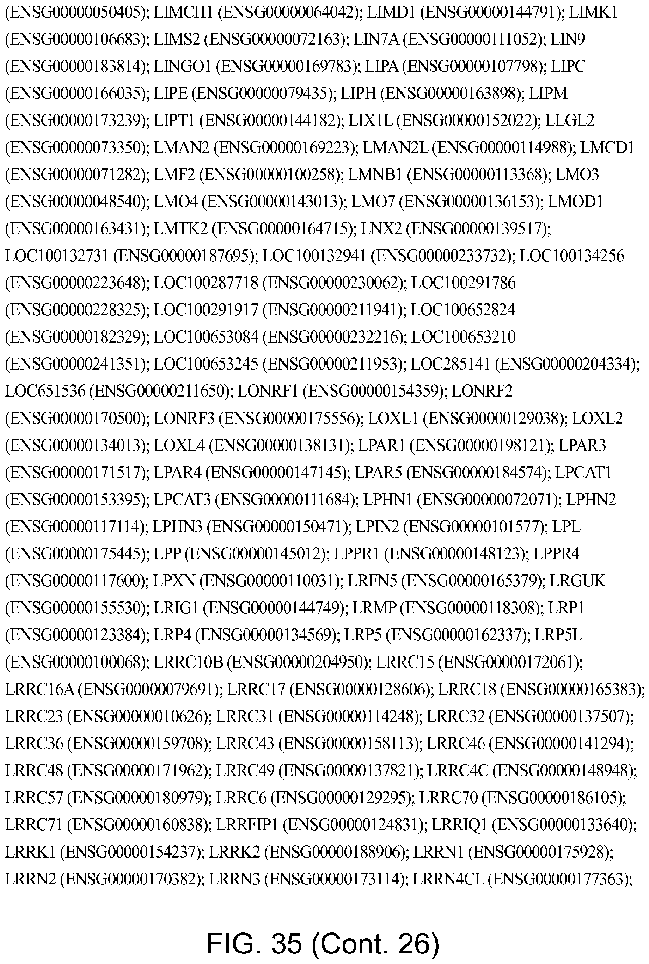

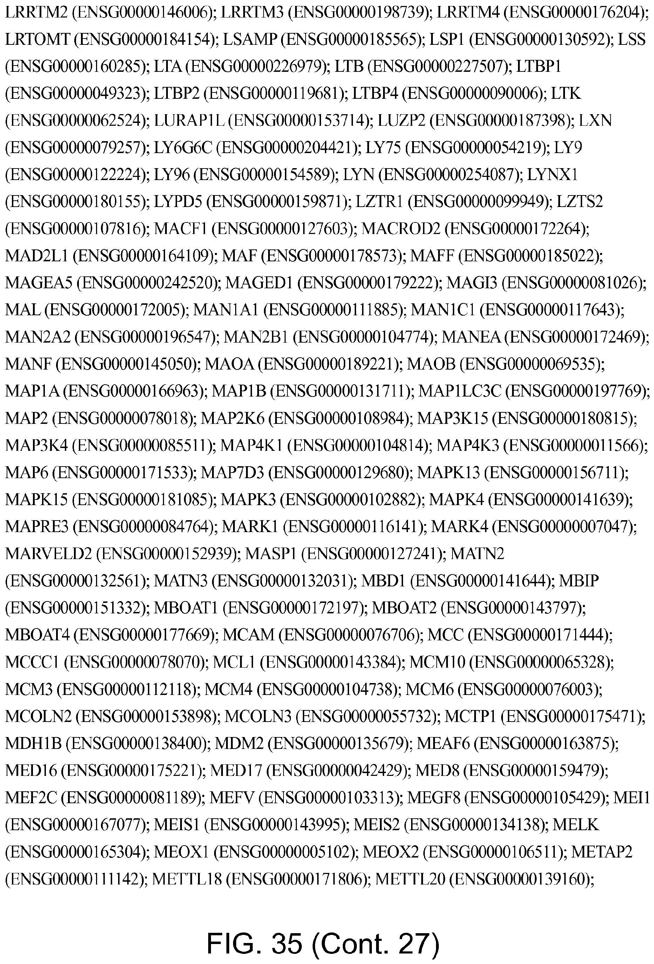

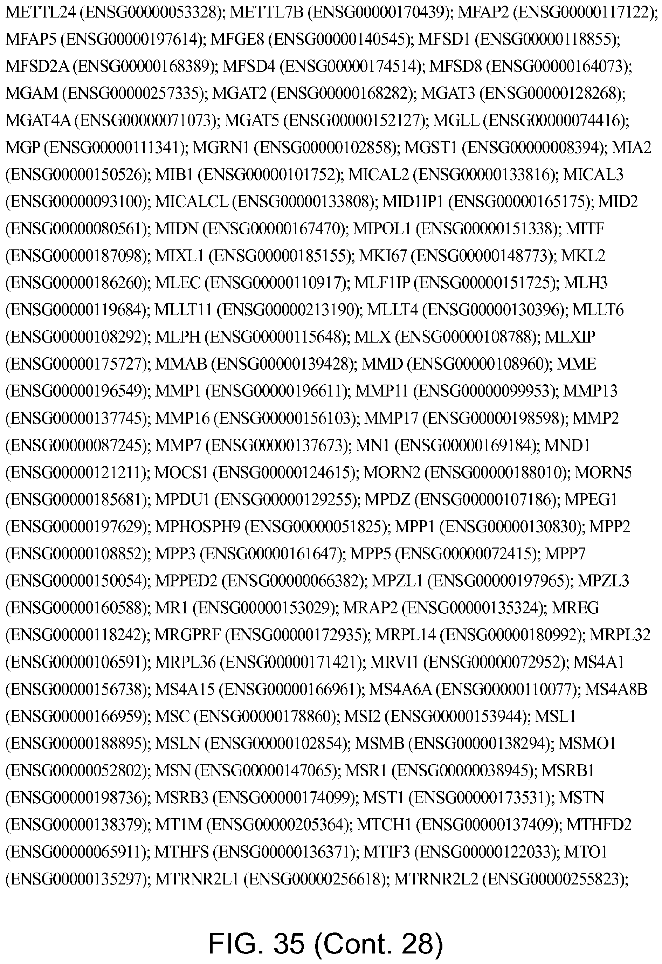

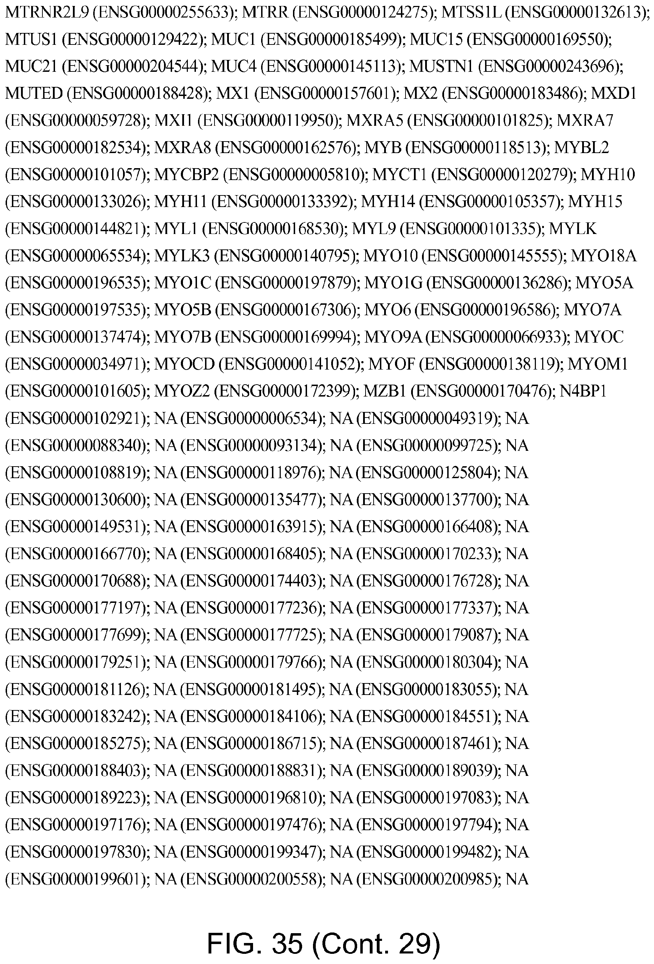

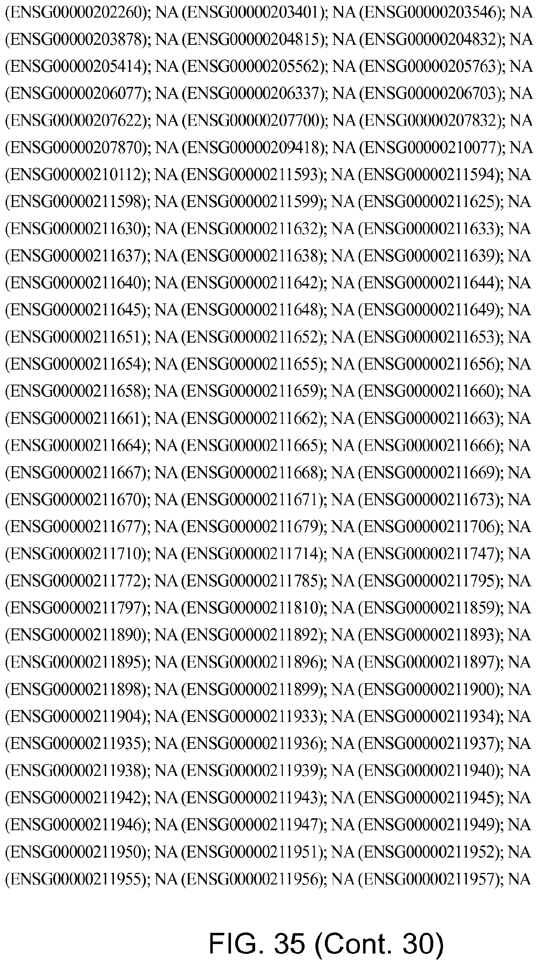

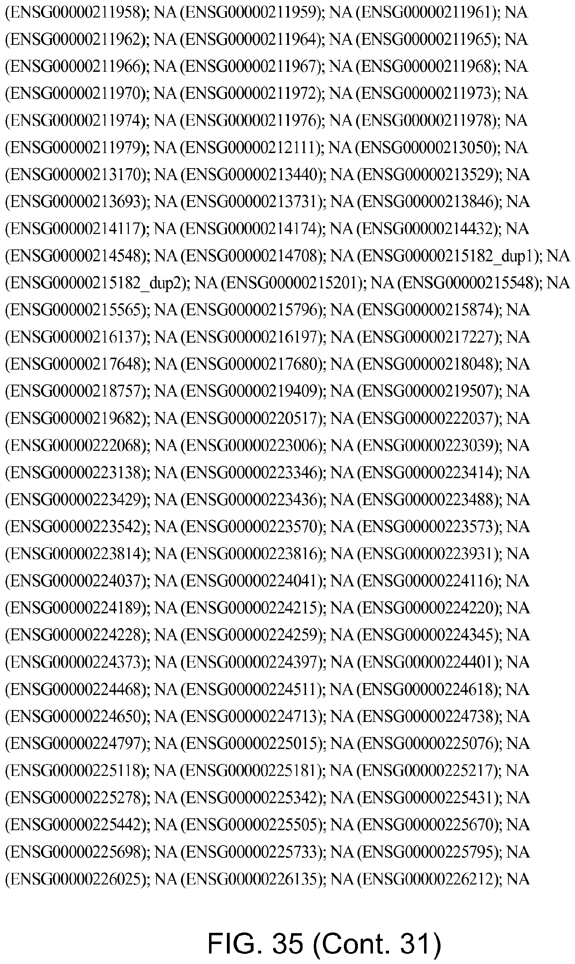

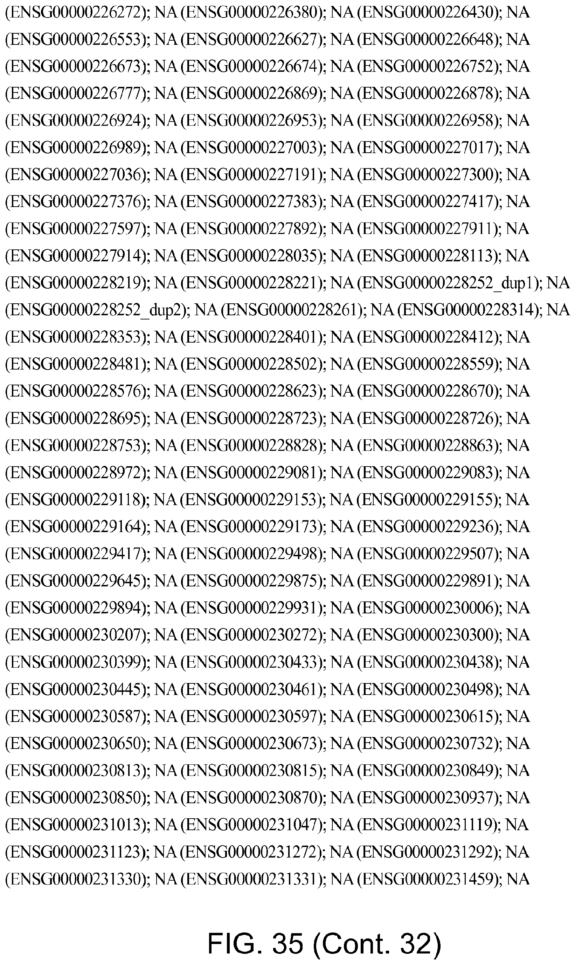

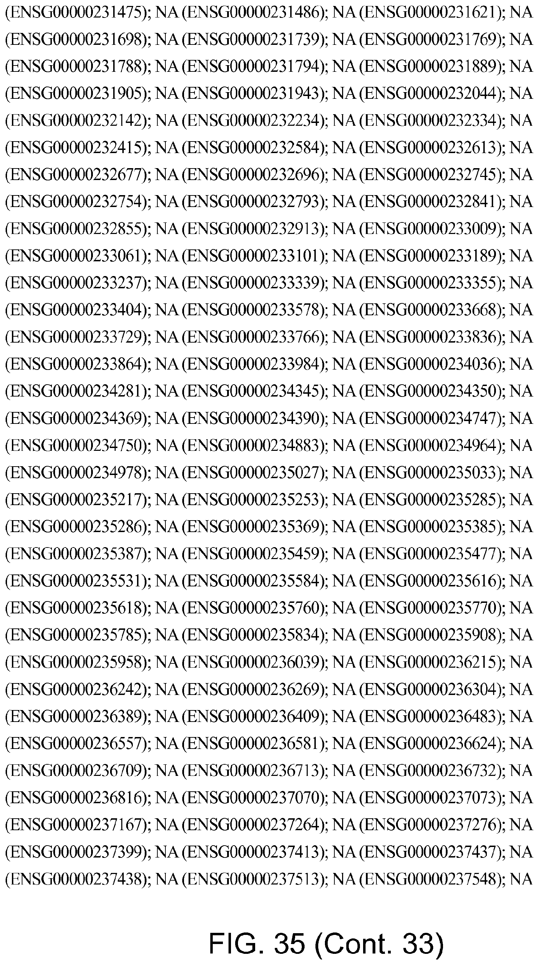

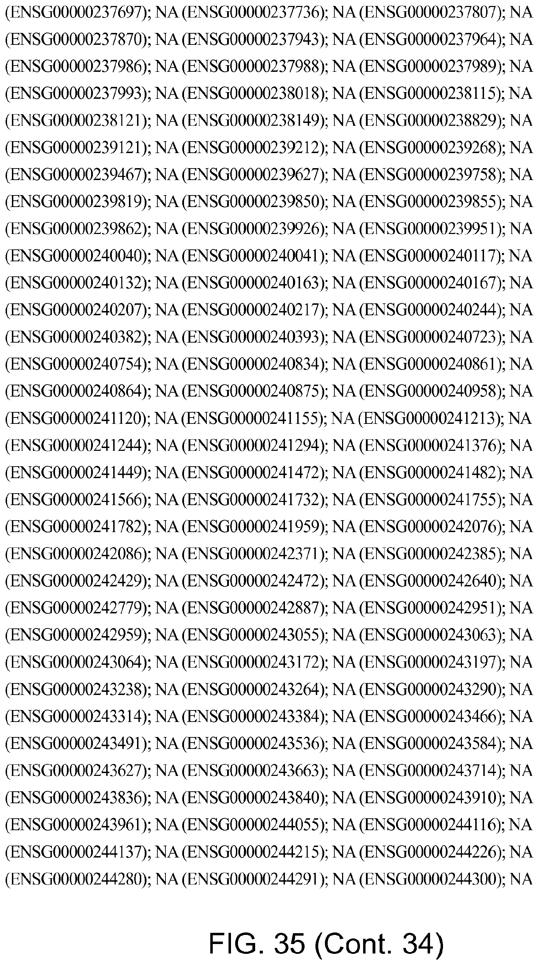

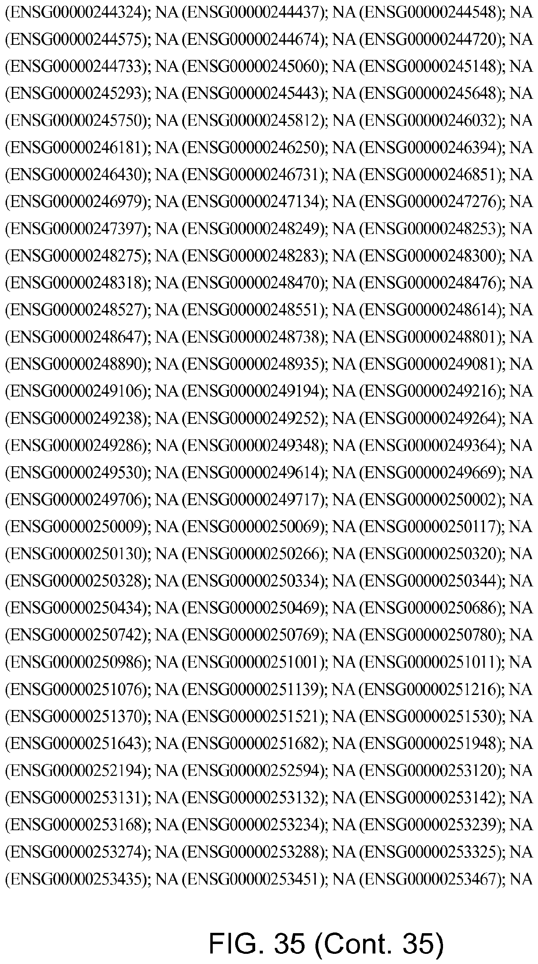

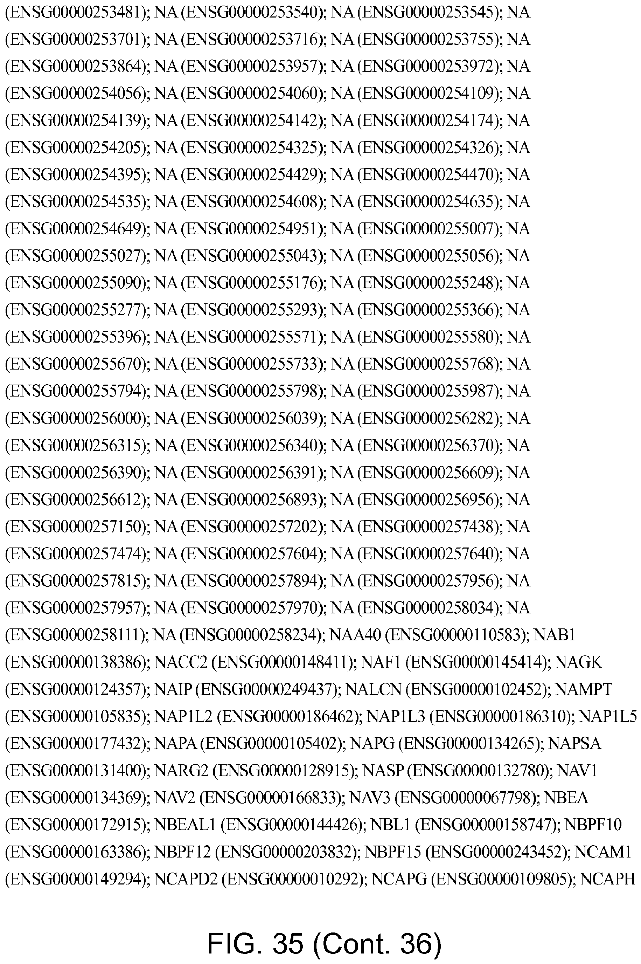

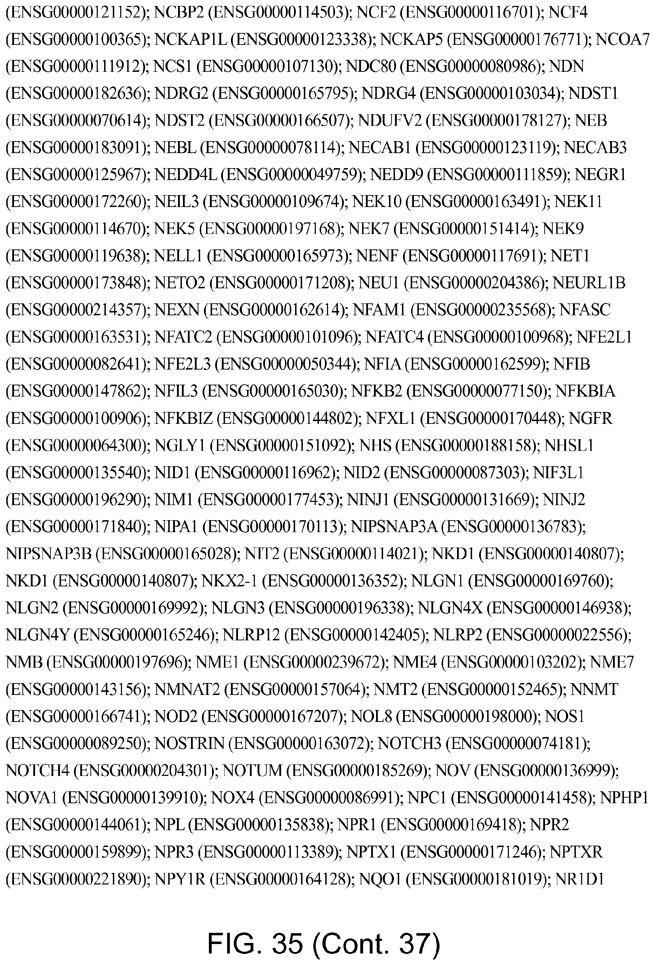

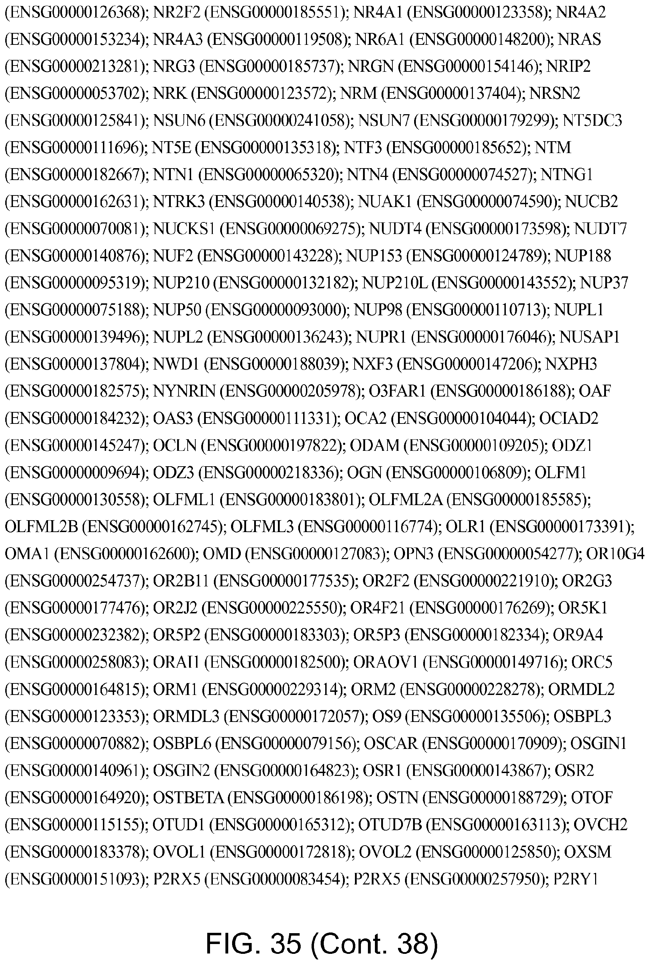

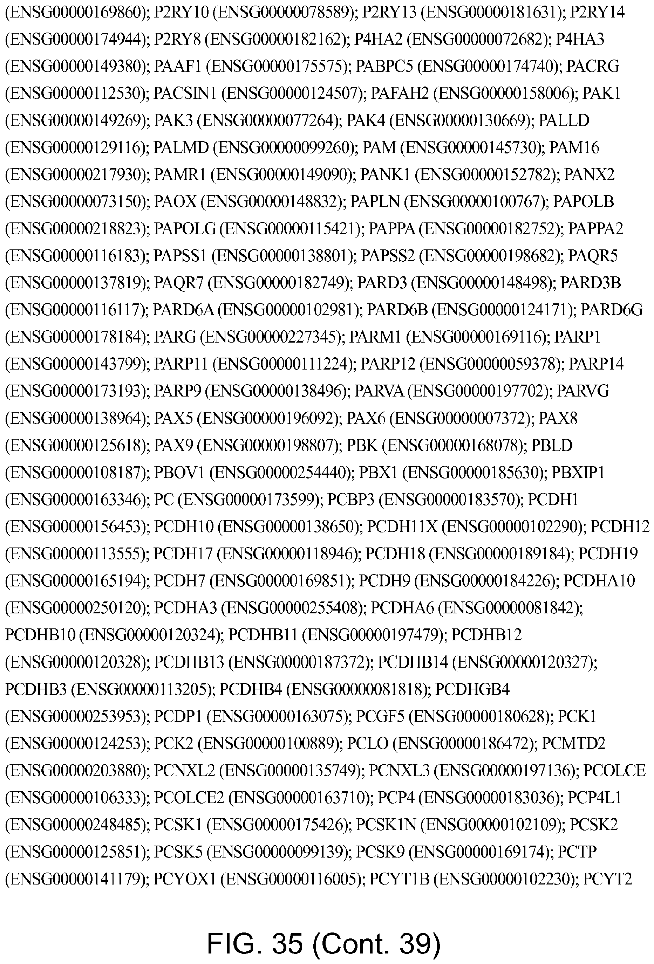

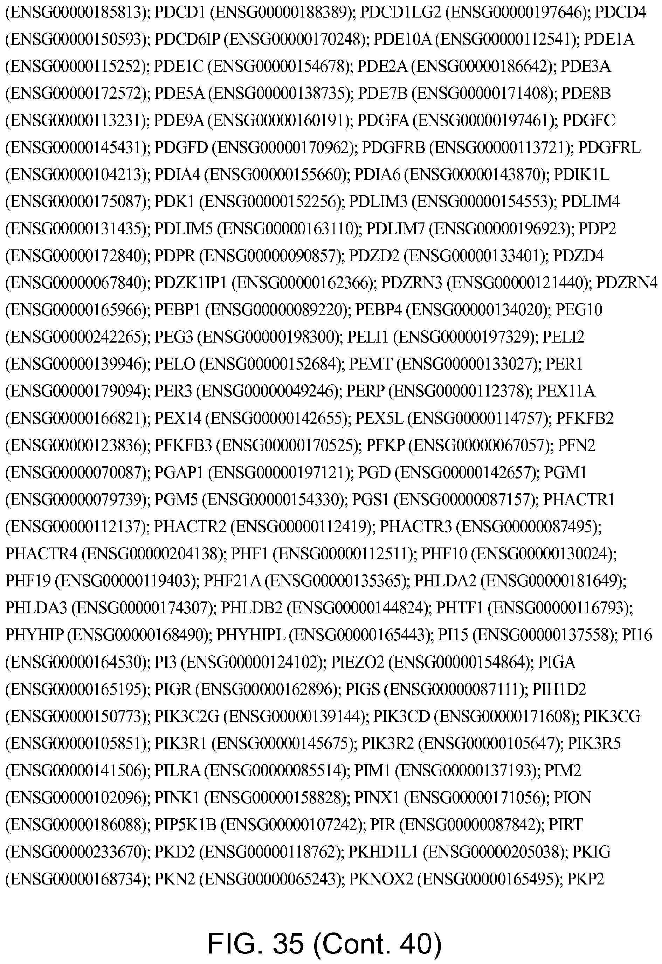

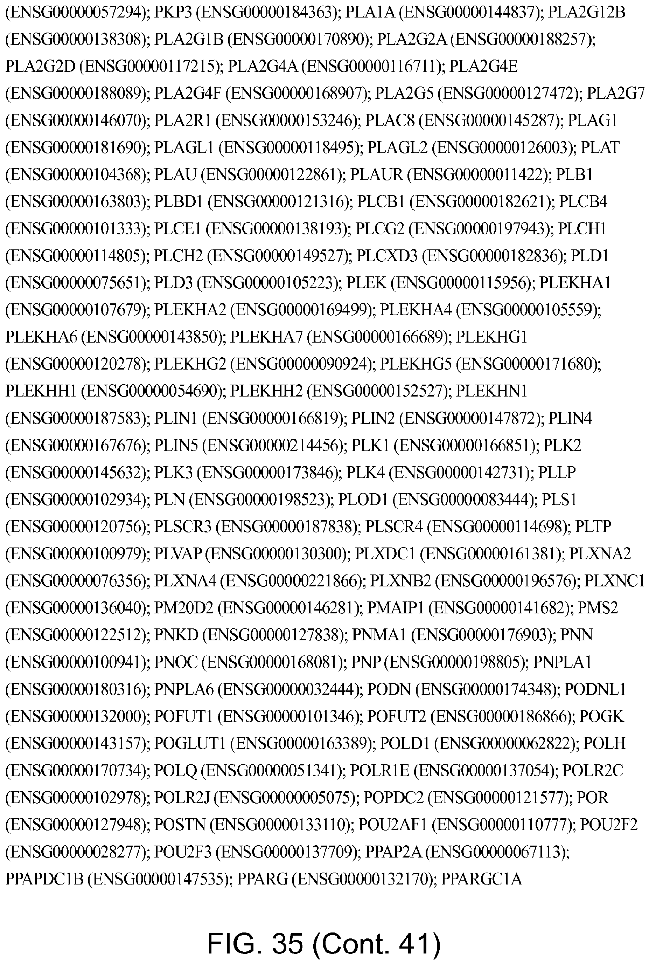

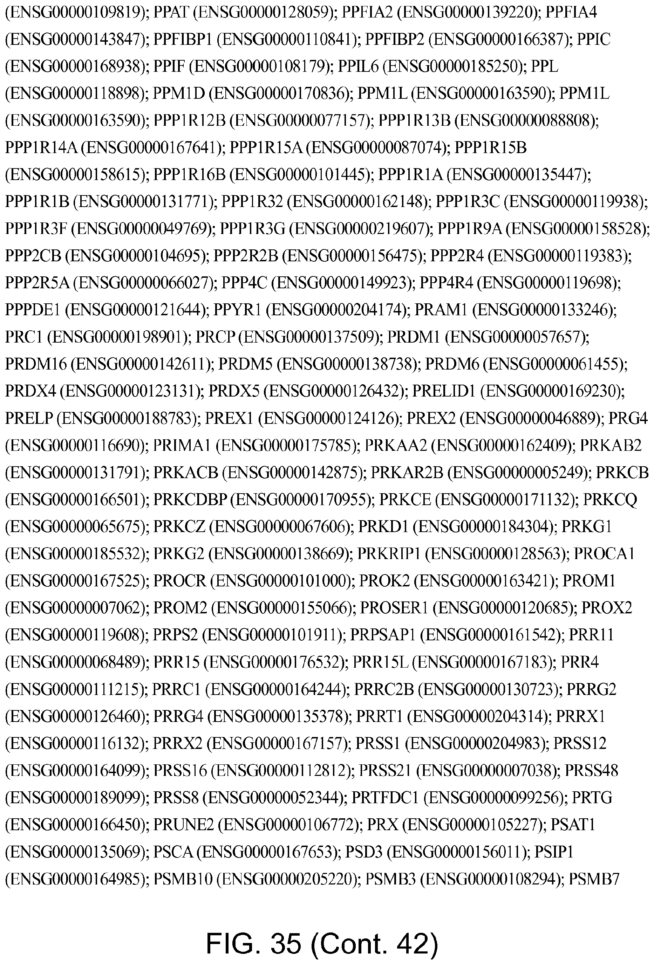

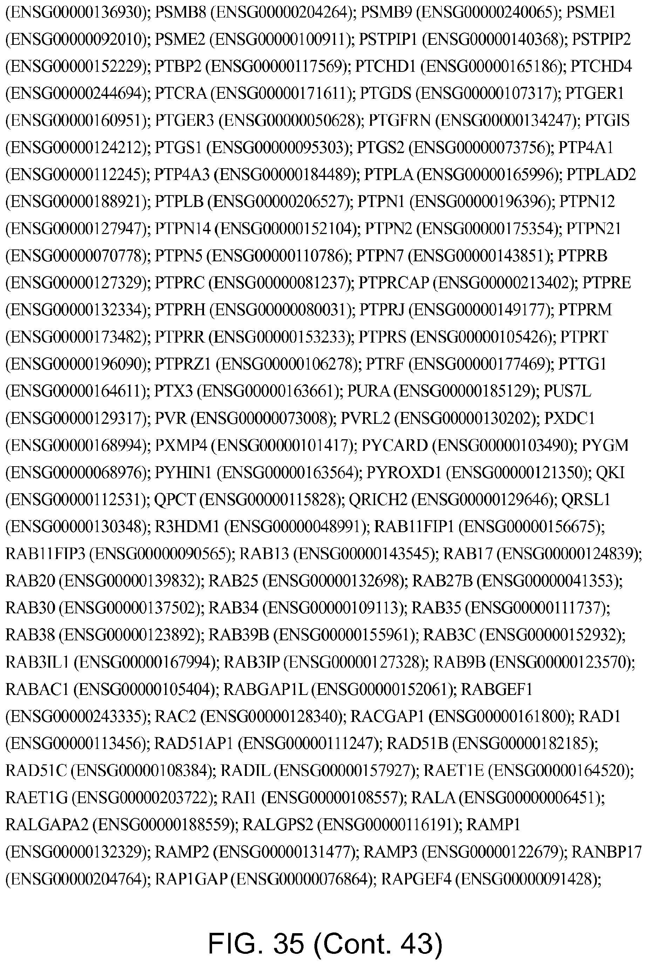

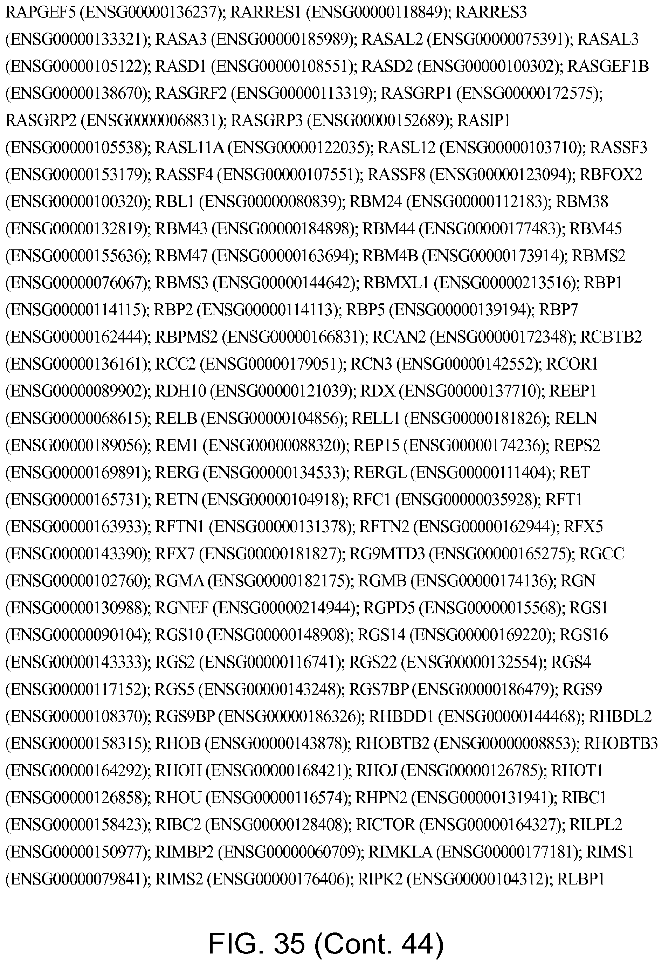

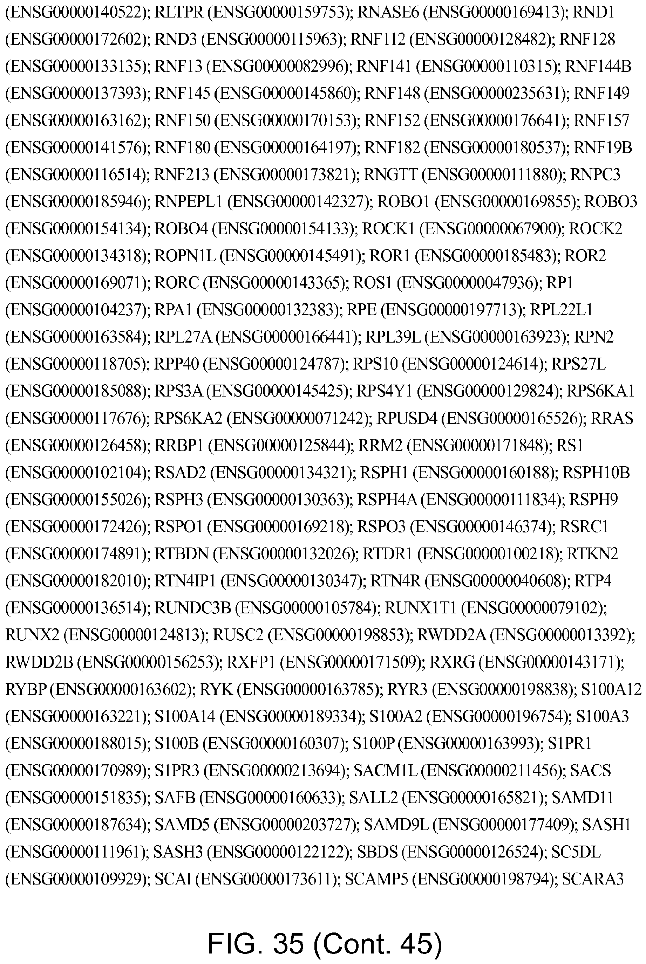

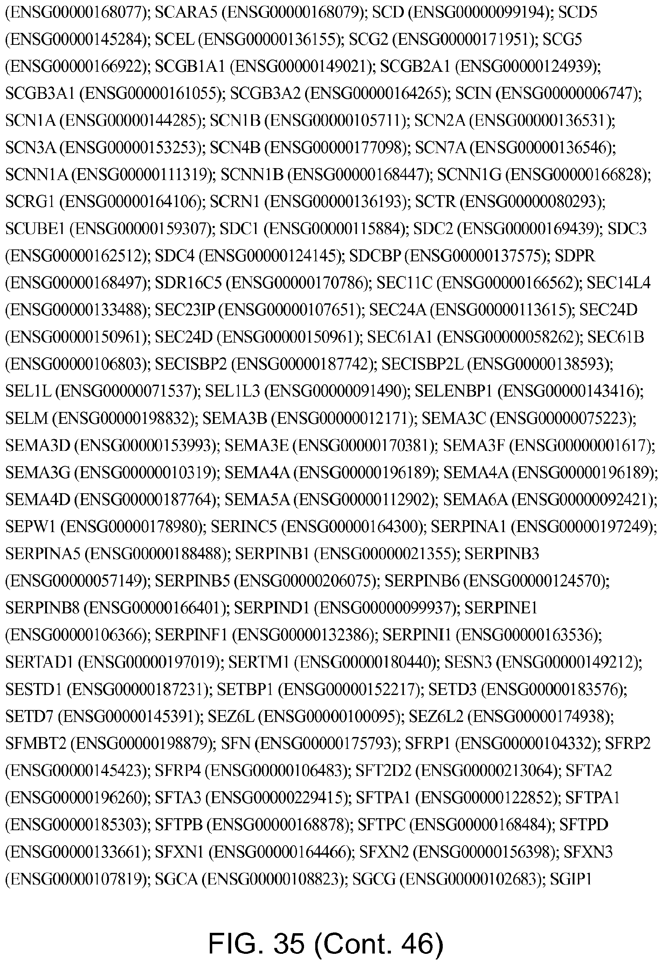

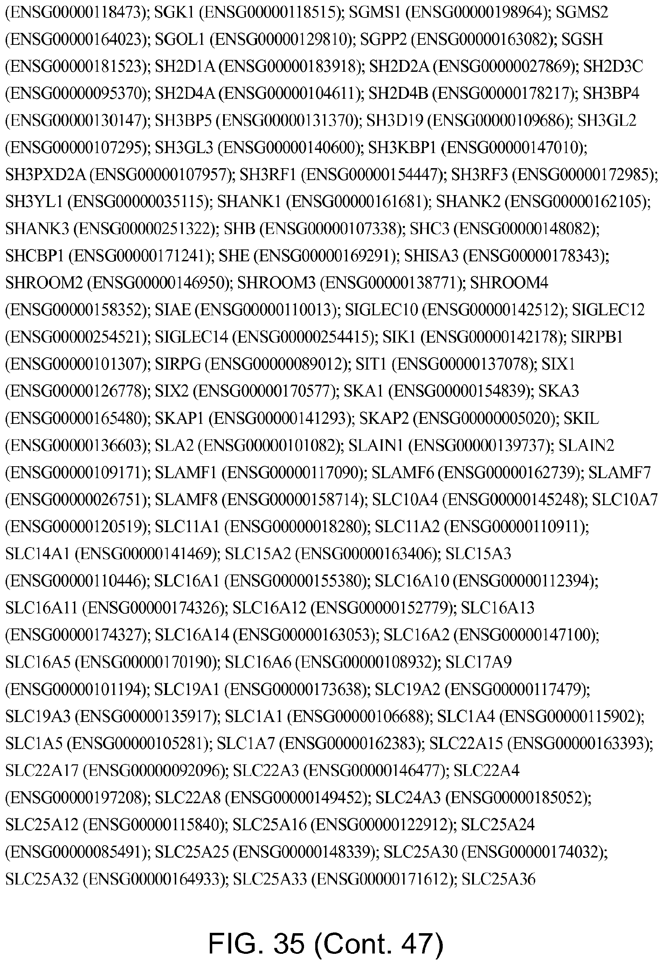

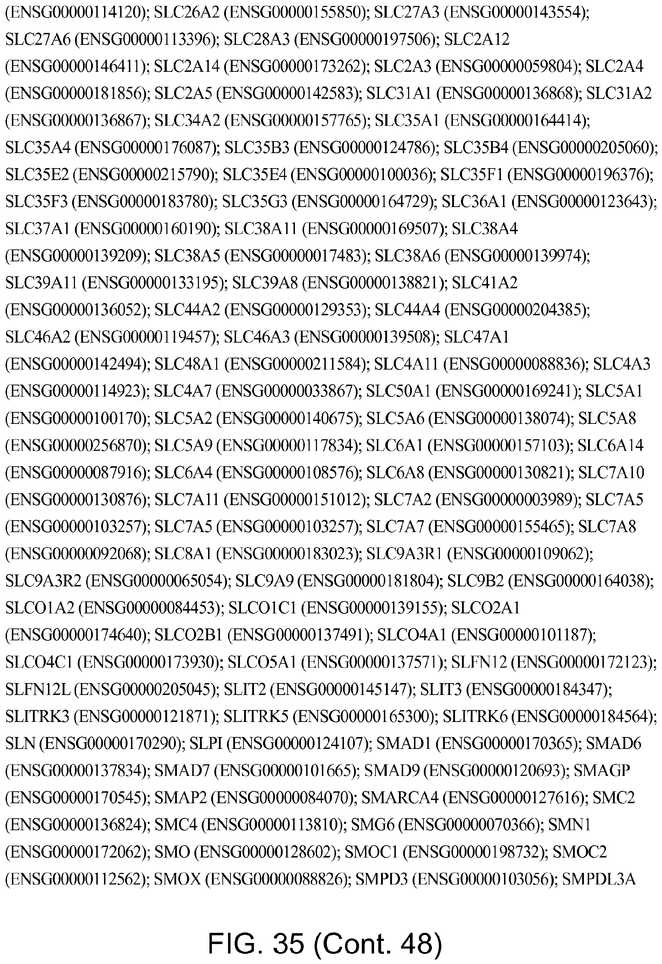

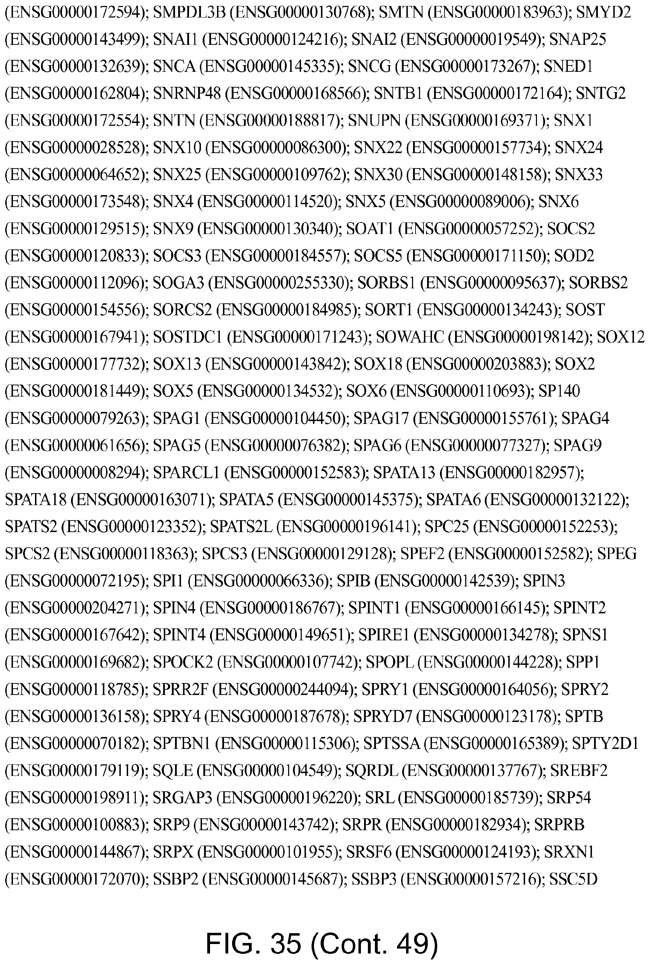

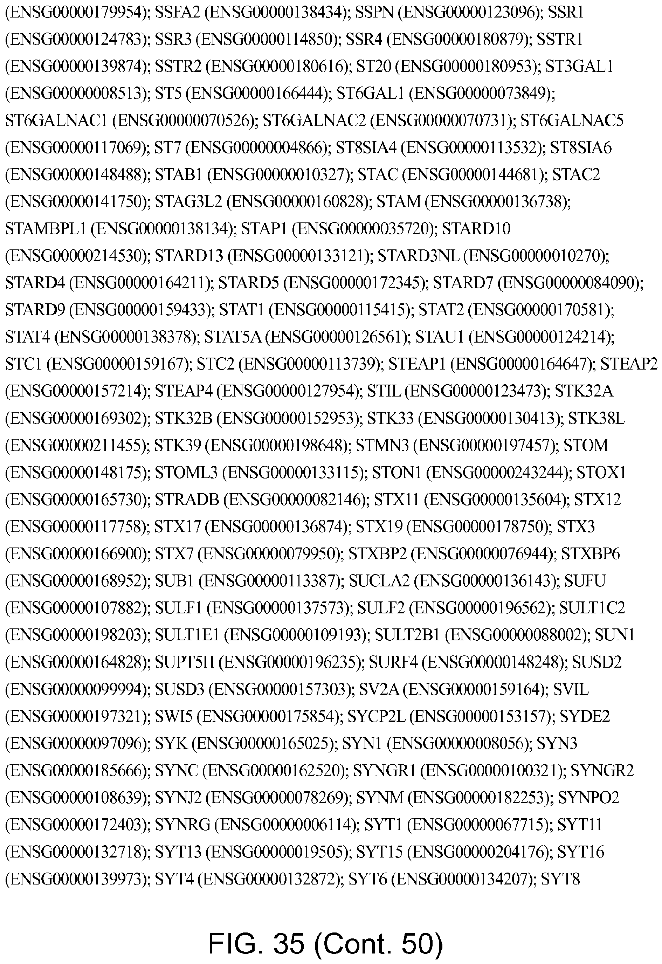

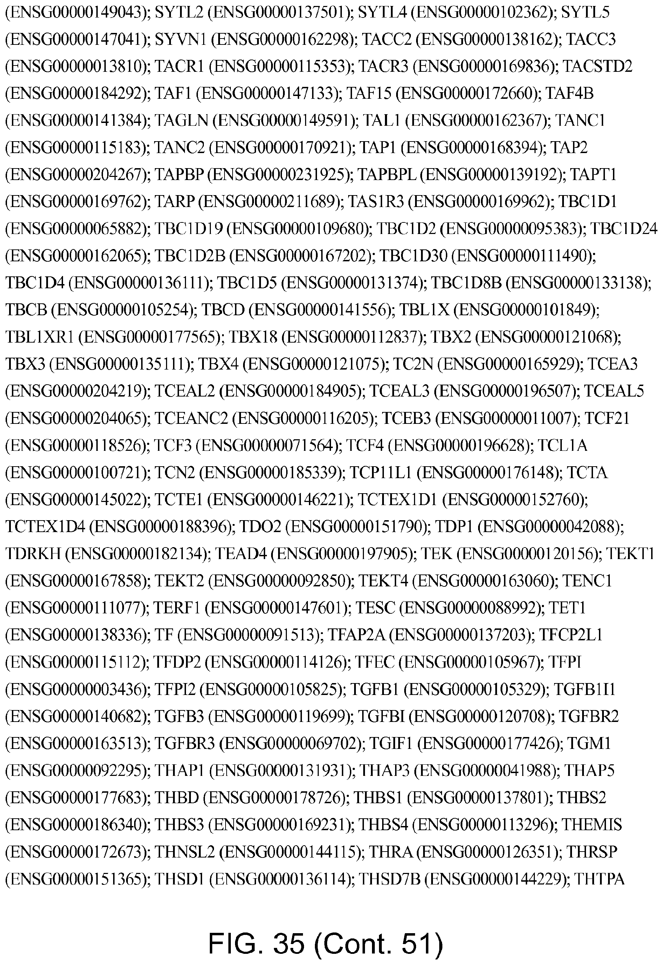

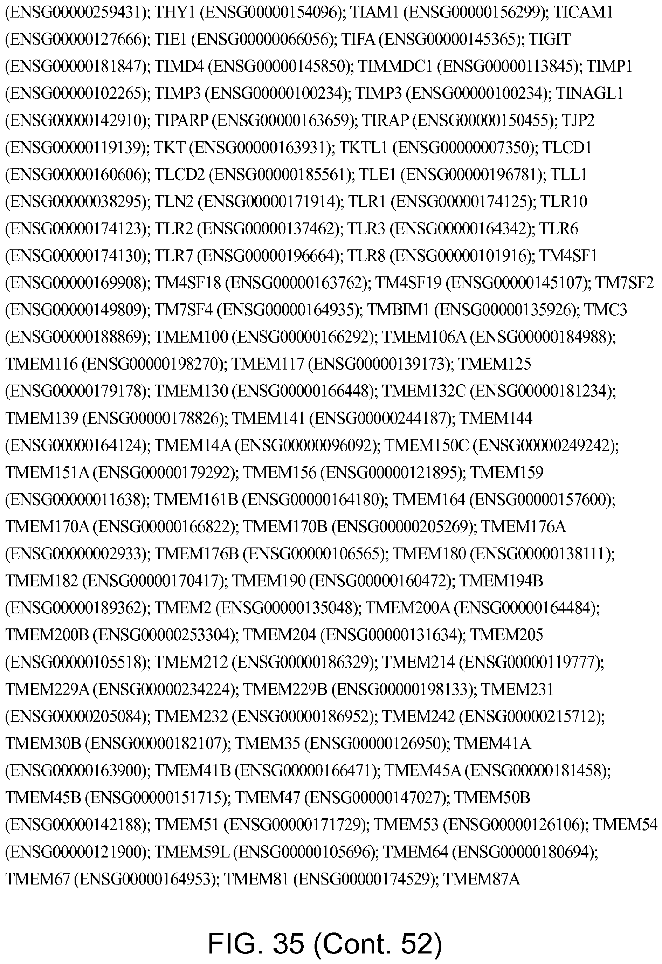

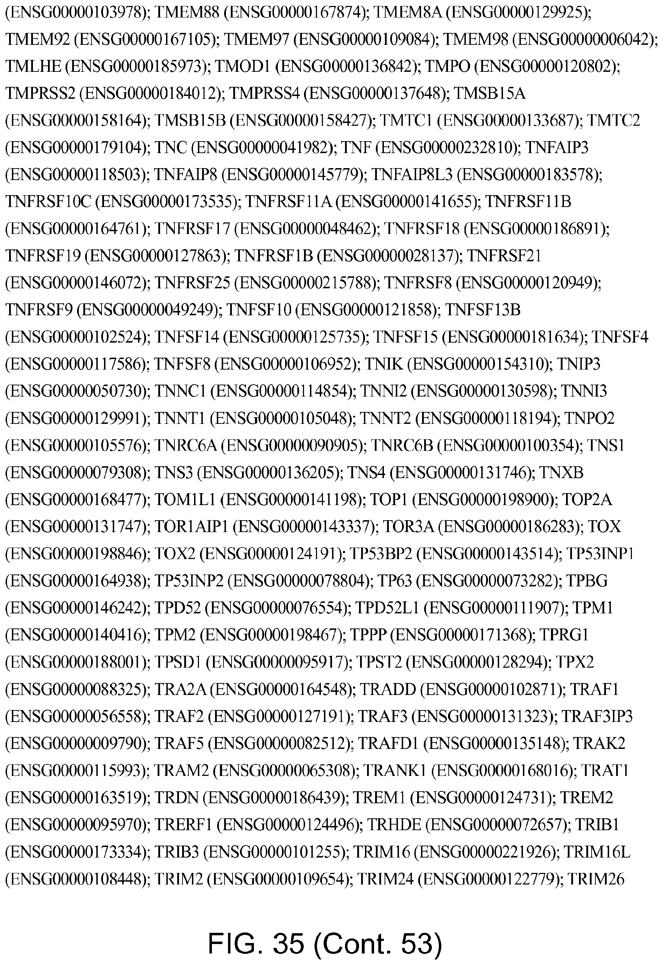

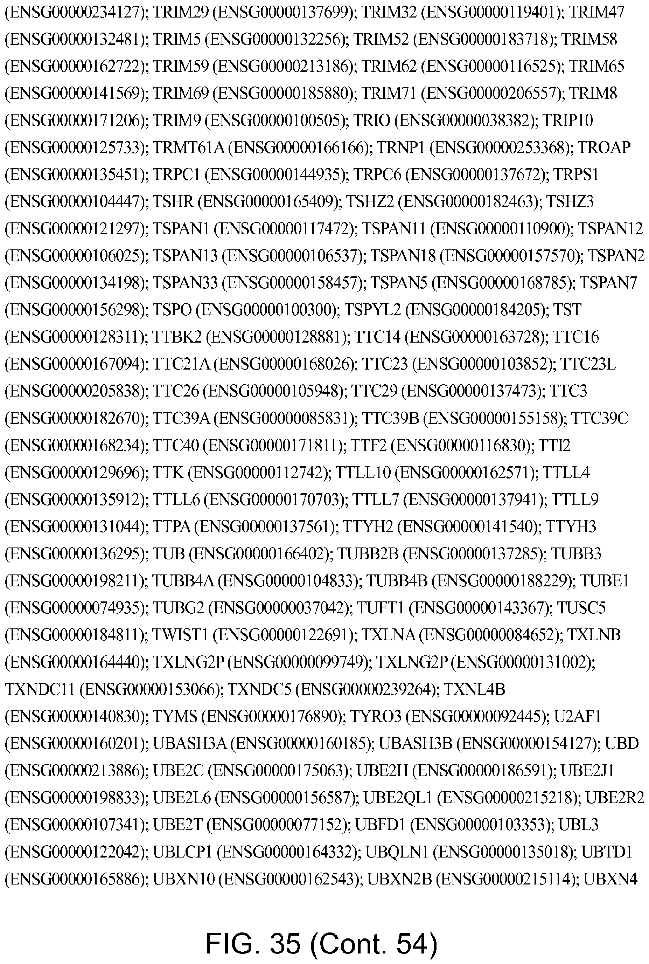

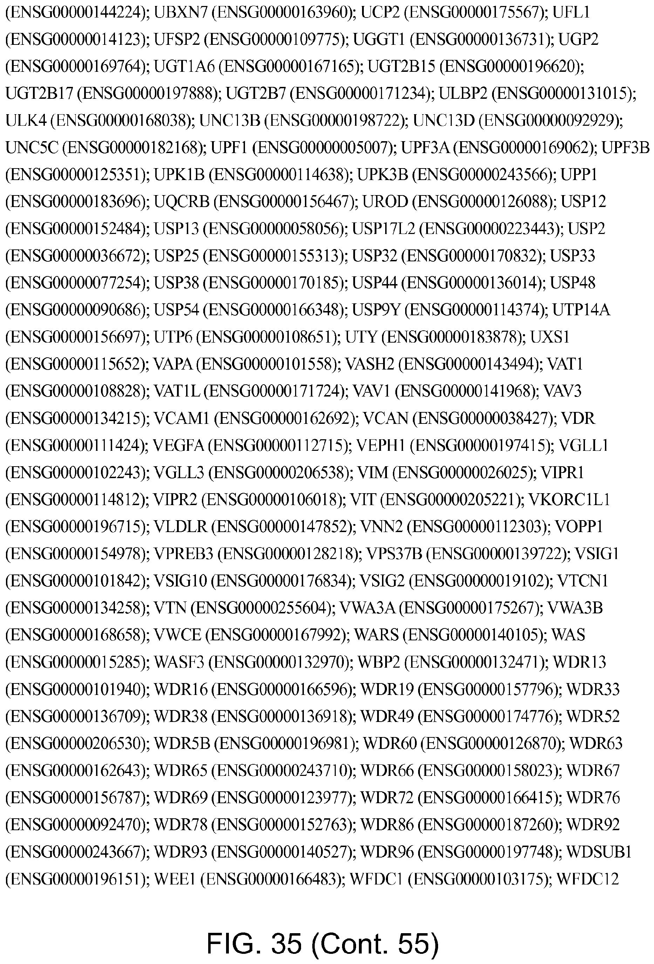

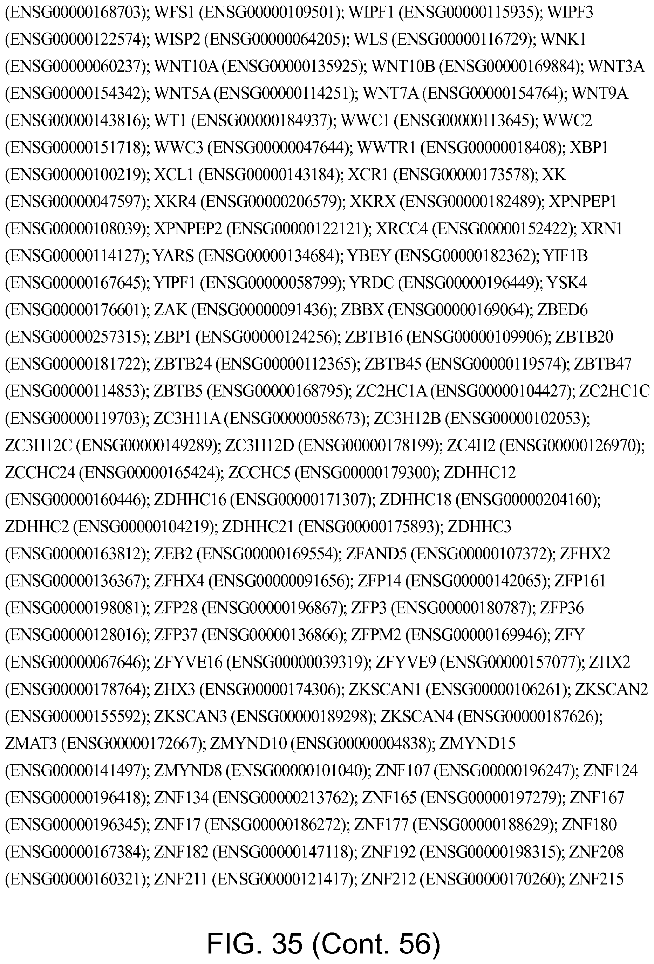

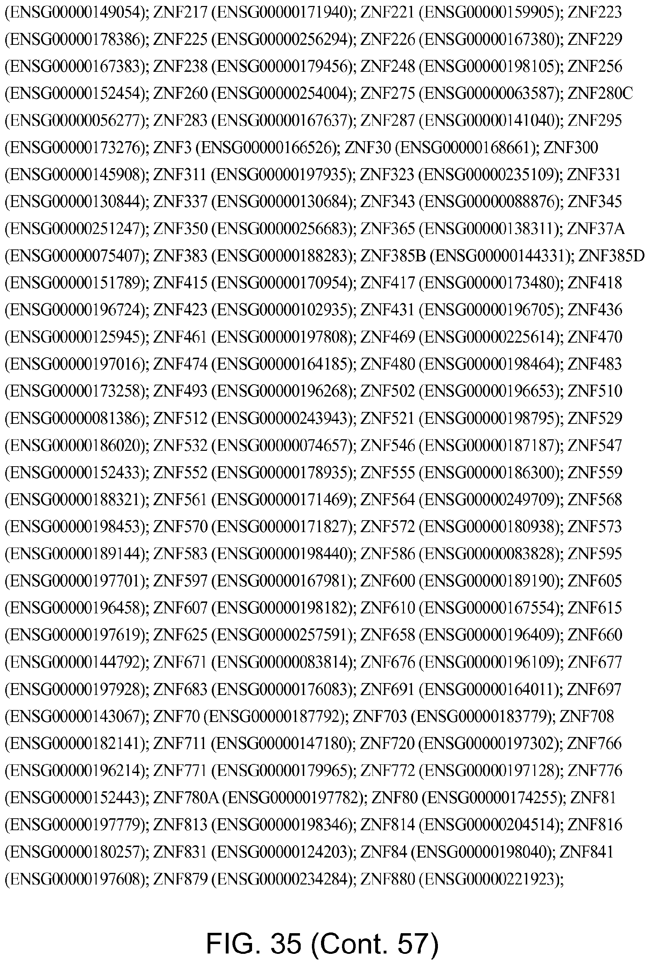

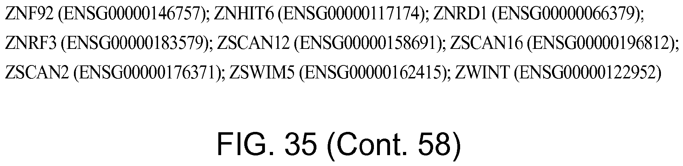

"ENSEMBL ID" refers to a gene identifier number from the Ensembl Genome Browser database. Each identifier begins with the letters ENSG to denote "Ensembl Gene". Each ENSEMBL ID number (i.e., each "gene" in the Ensembl database) refers to a gene defined by a specific start and stop position on a particular human chromosome, and therefore defines a specific locus of the human genome as defined in Figure 35. For example, the first ENSEMBL ID of Figure 35 is ENSG00000000938, which is located on human chromosome ("Chrom.") 1 ("chr1"). The locus of ENSG00000000938 begins on chromosome 1 at nucleotide position 27938575 and ends at nucleotide position 27961788, thus the locus, which includes intron sequence, can be said to be 23,213 nucleotides in length. Most genes in the database are named, while some remain unnamed (denoted with "NA").

Transcript sequences from the human genome are assigned transcript identifier numbers that begin with the letters ENST to denote "Ensembl Transcript." Each transcript that maps to a region of the human genome is therefore an expression product of that particular region. Because each locus in the genome can have one or multiple different transcripts (e.g., some genes have multiple promoter sites, encode multiple different spliceforms, etc.), each ENSG-numbered identifier can associate with more than one transcript (e.g., overlapping transcripts).

As used herein, reference to an "ENSG" ENSEMBL ID refers to gene expression product(s) produced from the corresponding locus of the human genome. For example, reference to ENSG00000000938 see (e.g., see Figure 35)) encompasses any gene products produced from human chromosome 1 from the locus starting at nucleotide position 27938575 and ending at nucleotide position 27961788. Thus, reference to ENSG00000000938 encompasses all of the transcripts listed in Table 35 (Figure 35) associated with that particular ENSG number (in this case: ENST00000374005, ENST00000399173, ENST00000545953, ENST00000374004, ENST00000469062, ENST00000374003, ENST00000457296, ENST00000475472, and/or ENST00000468038) as well as any other transcript (or encoded polypeptide) produced from the locus. All "ENSG" ENSEMBL ID numbers appearing in the application can be cross-referenced to Figure 35.

Methods for detecting gene expression products from a given locus would be readily understood by one of ordinary skill in the art and any convenient method can be used. As non-limiting examples, suitable methods to detect RNA gene expression products from a given locus include hybridization based methods (e.g., Northern blot analysis), reverse transcriptase polymerase chain reaction (RT-PCR), quantitative RT-PCR (qRT-PCR), real-time RT-PCR, microarray, RNAseq (RNA sequencing), and the like. As non-limiting examples, suitable methods to detect protein gene expression products from a given locus include antibody based methods (e.g., Western blot, immunohistochemistry, immunofluorescence, FACS (Fluorescence-activated Cell Sorting), etc.), protein sequencing methods, mass spectroscopy based methods, and the like.

### Example 1: Microarray sample cohort - biomarkers and classification of IPF

Samples are listed along with pathologic classification using expert labels. Bronchiolitis (BRONCH, n=1), chronic interstitial fibrosis, not otherwise classified (CIF-NOC, n=4), hypersensitivity pneumonitis (HP, n=4), idiopathic pulmonary fibrosis (IPF, n=21), normal lung, (NML, n=4), non-specific interstitial pneumonia (NSIP, n=8), organizing pneumonia, (OP, n=2), other (OTHER, n=4), respiratory bronchiolitis (RB, n=2), sarcoidosis (SARC, n=2), smoking related interstitial fibrosis (n=1), universal human reference RNA (n=3). All samples were obtained by video-assisted thoracoscopic surgery (VATS).

Table 1 provides a list of the samples, and the pathology label for each.

| **Table 1 Pathology Label** | **Pathology Abbreviation** |
|---|---|
| Bronchiolitis | BRONCH |
| Chronic Interstitial Fibrosis not otherwise classified | CIF-NOC |
| Chronic Interstitial Fibrosis not otherwise classified | CIF-NOC |
| Chronic Interstitial Fibrosis not otherwise classified | CIF-NOC |
| Chronic Interstitial Fibrosis not otherwise classified | CIF-NOC |
| Hypersensitivity Pneumonitis | HP |
| Hypersensitivity Pneumonitis | HP |
| Hypersensitivity Pneumonitis | HP |
| Hypersensitivity Pneumonitis | HP |
| Idiopathic Pulmonary Fibrosis | IPF |
| Idiopathic Pulmonary Fibrosis | IPF |
| Idiopathic Pulmonary Fibrosis | IPF |
| Idiopathic Pulmonary Fibrosis | IPF |
| Idiopathic Pulmonary Fibrosis | IPF |
| Idiopathic Pulmonary Fibrosis | IPF |
| Idiopathic Pulmonary Fibrosis | IPF |
| Idiopathic Pulmonary Fibrosis | IPF |
| Idiopathic Pulmonary Fibrosis | IPF |
| Idiopathic Pulmonary Fibrosis | IPF |
| Idiopathic Pulmonary Fibrosis | IPF |
| Idiopathic Pulmonary Fibrosis | IPF |
| Idiopathic Pulmonary Fibrosis | IPF |
| Idiopathic Pulmonary Fibrosis | IPF |
| Idiopathic Pulmonary Fibrosis | IPF |
| Idiopathic Pulmonary Fibrosis | IPF |
| Idiopathic Pulmonary Fibrosis | IPF |
| Idiopathic Pulmonary Fibrosis | IPF |
| Idiopathic Pulmonary Fibrosis | IPF |
| Idiopathic Pulmonary Fibrosis | IPF |
| Idiopathic Pulmonary Fibrosis | IPF |
| Normal Lung | NML |
| Normal Lung | NML |
| Normal Lung | NML |
| Normal Lung | NML |
| Normal Lung | NML |
| Normal Lung | NML |
| Normal Lung | NML |
| Normal Lung | NML |
| Normal Lung | NML |
| Normal Lung | NML |
| Non-specific interstitial pneumonia | NSIP |
| Non-specific interstitial pneumonia | NSIP |
| Non-specific interstitial pneumonia | NSIP |
| Non-specific interstitial pneumonia | NSIP |
| Non-specific interstitial pneumonia | NSIP |
| Non-specific interstitial pneumonia | NSIP |
| Non-specific interstitial pneumonia | NSIP |
| Non-specific interstitial pneumonia | NSIP |
| Organizing Pneumonia | OP |
| Organizing Pneumonia | OP |
| Other | OTHER |
| Other | OTHER |
| Other | OTHER |
| Other | OTHER |
| Respiratory Bronchiolitis | RB |
| Respiratory Bronchiolitis | RB |
| Sarcosidosis | SARC |
| Sarcosidosis | SARC |
| Smoking related interstitial fibrosis | SRIF |
| Universal Human Reference RNA | UHR |
| Universal Human Reference RNA | UHR |
| Universal Human Reference RNA | UHR |

Table 2 provides information regarding the RNAseq sample cohort. Samples are listed along with pathologic classification using expert labels. Chronic interstitial fibrosis, not otherwise classified (CIF-NOC, n=3), malignant thyroid RNA (CONTROL, n=6) hypersensitivity pneumonitis (HP, n=3), normal lung, (NML, n=5), non-specific interstitial pneumonia (NSIP, n=7), organizing pneumonia, (OP, n=1), other (OTHER, n=1), respiratory bronchiolitis (RB, n=1), squamous cell carcinoma (SQC, n=2), usual interstitial pneumonia (n=18). All samples were obtained by video-assisted thoracoscopic surgery (VATS).

| **Table 2 Pathology Label** | **Label Abbreviation** |
|---|---|
| Chronic Interstitial Fibrosis not otherwise classified | CIF-NOC |
| Chronic Interstitial Fibrosis not otherwise classified | CIF-NOC |
| Chronic Interstitial Fibrosis not otherwise classified | CIF-NOC |
| Malignant thyroid RNA | Control |
| Malignant thyroid RNA | Control |
| Malignant thyroid RNA | Control |
| Malignant thyroid RNA | Control |
| Malignant thyroid RNA | Control |
| Malignant thyroid RNA | Control |
| Hypersensitivity Pneumonitis | HP |
| Hypersensitivity Pneumonitis | HP |
| Hypersensitivity Pneumonitis | HP |
| Normal Lung | NML |
| Normal Lung | NML |
| Normal Lung | NML |
| Normal Lung | NML |
| Normal Lung | NML |
| Non-specific interstitial pneumonia | NSIP |
| Non-specific interstitial pneumonia | NSIP |
| Non-specific interstitial pneumonia | NSIP |
| Non-specific interstitial pneumonia | NSIP |
| Non-specific interstitial pneumonia | NSIP |
| Non-specific interstitial pneumonia | NSIP |
| Non-specific interstitial pneumonia | NSIP |
| Organizing Pneumonia | OP |
| Other | OTHER |
| Respiratory Bronchiolitis | RB |
| Squamous Cell Carcinoma | SQC |
| Squamous Cell Carcinoma | SQC |
| Usual interstitial pneumonia | UIP |
| Usual interstitial pneumonia | UIP |
| Usual interstitial pneumonia | UIP |
| Usual interstitial pneumonia | UIP |
| Usual interstitial pneumonia | UIP |
| Usual interstitial pneumonia | UIP |
| Usual interstitial pneumonia | UIP |
| Usual interstitial pneumonia | UIP |
| Usual interstitial pneumonia | UIP |
| Usual interstitial pneumonia | UIP |
| Usual interstitial pneumonia | UIP |
| Usual interstitial pneumonia | UIP |
| Usual interstitial pneumonia | UIP |
| Usual interstitial pneumonia | UIP |
| Usual interstitial pneumonia | UIP |
| Usual interstitial pneumonia | UIP |
| Usual interstitial pneumonia | UIP |
| Usual interstitial pneumonia | UIP |

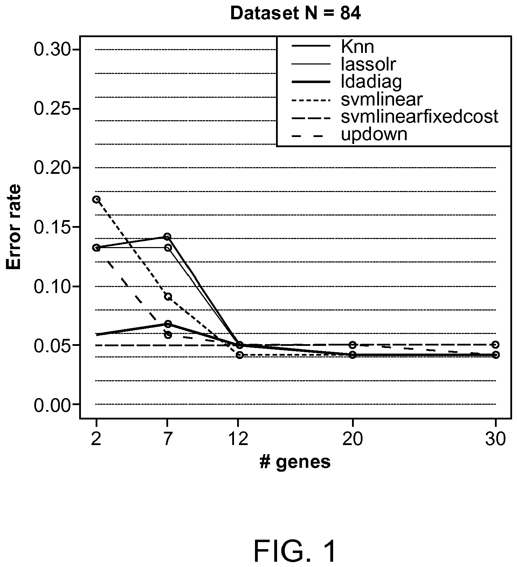

The results are depicted in Figures 1-4. The following abbreviations used in Figures 1 and 3:

knn: refers to "K nearest neighbors", which is a method for classifying a samples based on closest training examples in feature space. A sample is classified by a majority vote of its neighbors, with the object being assigned to the class most common amongst its *k* nearest neighbors (*k* is a positive integer, typically small). If *k* = 1, then the object is simply assigned to the class of its nearest neighbor.

lassolr: refers to "Lasso, a linear regression", which is a classification method that uses a variable selection approach based on Lasso, a linear regression method for estimation in linear models (see Tibshirani R.J. Regression shrinkage and selection via the LASSO. J Roy Statist Soc B. 1996;58(1):267-288), which minimizes the residual sum of squares subject to the sum of absolute value of the coefficients being less than a constant.

Ldadiag: refers to "Diagonal LDA", which is a method used in machine learning to find a linear combination of features which characterizes or separates two or more classes of samples. The 'diagonal' in DLDA indicates an assumption of independence between the genes used in training. This assumption, with proper gene selection, often offers increased performance over the more general LDA technique.

Svmlinear: refers to "Support Vector Machine with Linear Kernel", which is a supervised learning model with associated learning algorithms that analyze data and recognize patterns, used for classification. The basic SVM takes a set of input data and predicts, for each given input, which of two possible classes forms the output, making it a non-probabilistic binary linear classifier. Given a set of training examples, each marked as belonging to one of two categories, a SVM training algorithm builds a model that assigns new examples into one category or the other. A SVM model is a representation of the examples as points in space, mapped so that the examples of the separate categories are divided by a clear gap that is as wide as possible. New examples are then mapped into that same space and predicted to belong to a category based on which side of the gap they fall on. SVMs are defined by the choice of a kernel function (linear, radial or polynomial in the current implementation) and a cost penalty for misclassification which can be held constant 'fixed-cost' or varied over a grid to examine performance across a cost landscape.

Updown: refers to "Up-Down", which is a method for classifying samples based not upon the actual value of their normalized expression intensities but upon whether each gene used in training is usually "up" (i.e. up-regulated) in one class vs. the other or "down" (i.e. down-regulated). This approach to classification can be extremely robust to experimental noise and batch effects but requires genes that have large, repeatable fold changes between the two classes of samples in question.

Svmlinearfixedcost: refers to training an SVM classifier using a single cost value (e.g. 0.01) rather than "linearsvm", which involves training SVMs across a variety of cost values (e.g. -0.001,-0.01,-0.1,1, 10,100) and picking the best trade-off in performance versus complexity. This cost parameter determines the penalty for misclassification - higher penalties result in ever-more complex and potentially overfit classifiers while lower cost penalties result in simpler SVM classifiers with decreased performance in the training set, but with a the possibility of a better chance for generalizing to the test set.

The biomarkers presented here were obtained by analysis of Affymetrix Gene 1.0 ST microarrays using RNA amplified with the Nugen WT amplification system. Differential gene expression and feature selection used Limma models (Smyth 2004). The feature selection strategy can be broken down into steps 1) Select the top 50% most variable transcript cluster IDs (TCIDs, aka genes) in the data set (i.e., highest differentially expressed between two groups, such as IPF vs. NSIP) and step, 2) Analyze these TCIDs via Limma with an FDR cutoff of 10%. Classification performance was assessed with the leave-one-out method during cross-validation.

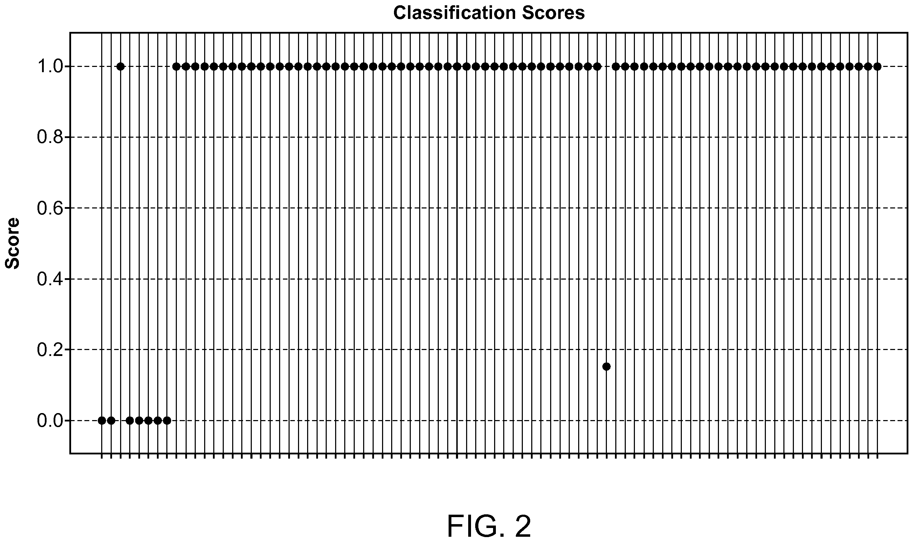

**Figure 2****.** NSIP samples score lower on the classifier while IPF samples score high. From left to right on the X-axis of Figure 2, the results for the first 8 samples correspond to NSIP samples, with the remaining results for IPF sample. Only two samples (one NSIP and one IPF) were incorrectly classified in this example.

The 30-gene set of biomarkers used in the IPF vs. NSIP classifier are set out in Table 3.

| **Table 3 Gene Symbol** | **Ensembl ID** | **TCID** | **Log FC** | **P value** | **FDR p value** |
|---|---|---|---|---|---|
| ASPM | ENSG00000066279 | 7923086 | -1.03 | 3.83E-14 | 1.28E-09 |
| BUB1 | ENSG00000169679 | 8054580 | -0.99 | 7.17E-13 | 2.72E-09 |
| PTTG1 | ENSG00000164611 | 8109639 | -1.12 | 1.49E-12 | 3.65E-09 |
| SHCBP1 | ENSG00000171241 | 8001133 | -0.82 | 1.57E-12 | 3.65E-09 |
| NUSAP1 | ENSG00000137804 | 7982889 | -0.88 | 7.51E-12 | 1.19E-08 |
| MKI67 | ENSG00000148773 | 7937020 | -0.92 | 1.13E-11 | 1.57E-08 |
| HJURP | ENSG00000123485 | 8059838 | -0.55 | 1.71E-11 | 2.27E-08 |
| CDCA3 | ENSG00000111665 | 7960702 | -0.55 | 4.03E-11 | 4.47E-08 |
| PLK1 | ENSG00000166851 | 7994109 | -0.61 | 8.94E-11 | 8.53E-08 |
| PRR11 | ENSG00000068489 | 8008784 | -0.67 | 4.01E-10 | 2.90E-07 |
| BRCA2 | ENSG00000139618 | 7968484 | -0.67 | 1.07E-09 | 6.84E-07 |
| ORM1 | ENSG00000229314 | 8157446 | -1.49 | 2.09E-09 | 1.24E-06 |
| CCNB2 | ENSG00000157456 | 7983969 | -0.65 | 1.81E-08 | 6.84E-06 |
| SMC4 | ENSG00000113810 | 8083709 | -0.51 | 2.76E-08 | 9.10E-06 |
| HM13 | ENSG00000101294 | 8061542 | -0.37 | 5.02E-08 | 1.41E-05 |
| DMD | ENSG00000198947 | 8171921 | 0.73 | 6.76E-08 | 1.72E-05 |
| FHL1 | ENSG00000022267 | 8170119 | 0.70 | 4.33E-07 | 6.83E-05 |
| ORM2 | ENSG00000228278 | 8157450 | -0.80 | 1.54E-06 | 1.79E-04 |
| NDUFC2-KCTD 14 | ENSG00000151364 | 7950641 | -0.68 | 1.71E-06 | 1.91E-04 |
| NCAPH | ENSG00000121152 | 8043602 | -0.44 | 2.52E-06 | 2.50E-04 |
| TTLL7 | ENSG00000137941 | 7917199 | 0.67 | 1.09E-05 | 7.44E-04 |
| DEPDC1B | ENSG00000035499 | 8112260 | -0.46 | 1.24E-05 | 8.06E-04 |
| CNTN4 | ENSG00000144619 | 8077323 | 0.52 | 1.29E-04 | 3.90E-03 |
| PRKAA2 | ENSG00000162409 | 7901720 | 0.64 | 1.52E-04 | 4.39E-03 |
| PRKCQ | ENSG00000065675 | 7931930 | -0.50 | 2.00E-04 | 5.24E-03 |
| CDC42BPA | ENSG00000143776 | 7924773 | 0.28 | 8.24E-04 | 1.30E-02 |
| PARD3B | ENSG00000116117 | 8047709 | 0.22 | 2.55E-03 | 2.56E-02 |
| SCTR | ENSG00000080293 | 8054846 | -0.43 | 3.53E-03 | 3.12E-02 |
| CSF3R | ENSG00000119535 | 7914950 | -0.34 | 7.16E-03 | 4.84E-02 |
| MPDZ | ENSG00000107186 | 8160088 | 0.27 | 1.24E-02 | 6.84E-02 |

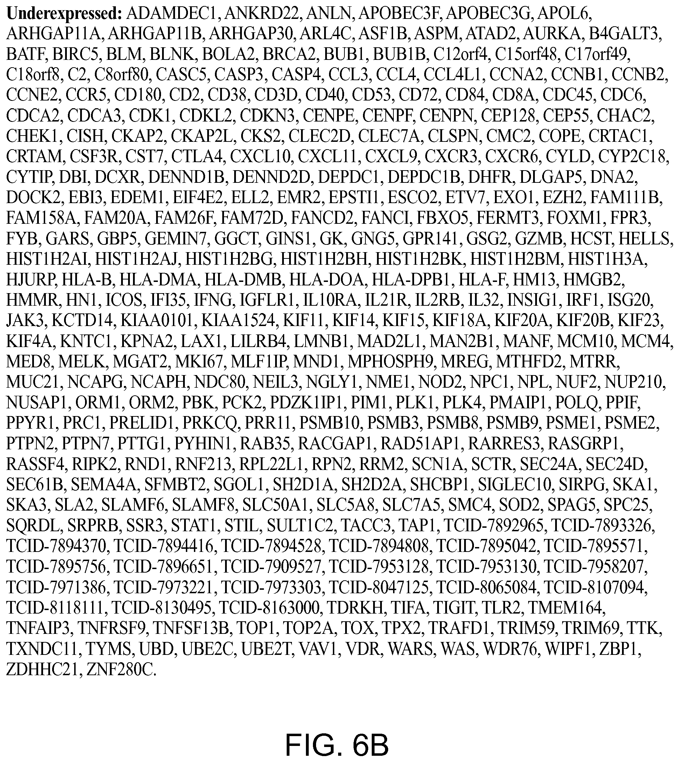

A comprehensive list of markers based on significance (FDR p<0.05) is provided in **Figures 6A** **and** **6B****.** A total of 495 markers were found to be significant with this analysis. Genes are listed here as either overexpressed in IPF or under-expressed. The genes listed in Figures 6A and 6B are differentially expressed between IPF and NSIP.

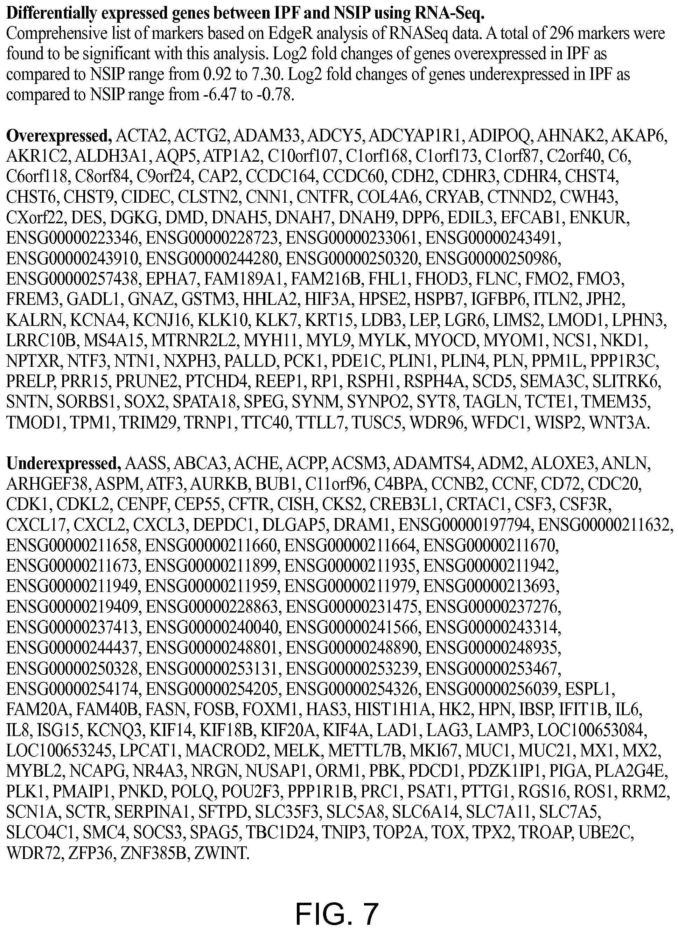

**Figure 7** provides genes differentially expressed between IFP and NSIP using RNA-Seq. Figure 7 provides a comprehensive list of markers based on EdgeR analysis of RNASeq data. A total of 296 markers were found to be significant with this analysis. Genes are have been ranked by FDR p-value.

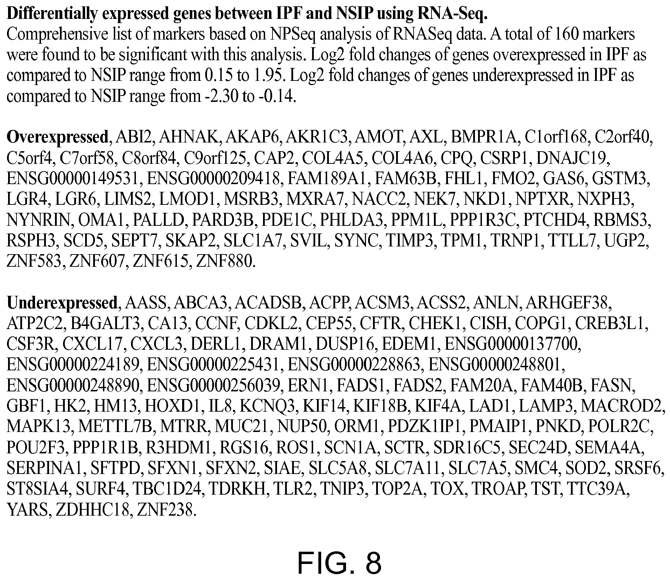

**Figure 8** provides genes differentially expressed between IFP and NSIP using RNA-Seq. Comprehensive list of markers based on NPSeq analysis of RNASeq dat. A total of 160 markers were found to be significant with this analysis. Genes are have been ranked by FDR p-value.

The biomarkers presented here were obtained by analysis of Affymetrix Gene ST microarrays using RNA amplified with the Nugen WT amplification system. Differential gene expression and feature selection used Limma models (cite Smyth). The feature selection strategy can be broken down into steps 1) Select the top 50% most variable transcript cluster IDs (TCIDs, aka genes) in the data set (ie highest differentially expressed between two groups, such as IPF vs. NSIP) and step, 2) Analyze these TCIDs via Limma with an FDR cutoff of 10%. Classification performance was assessed with the leave-one-out method during cross-validation.

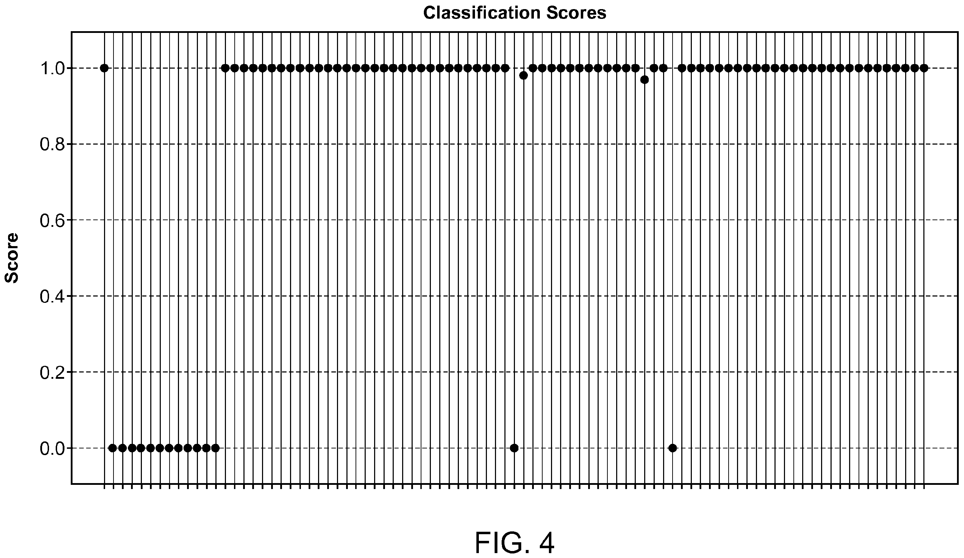

Table 4 provides a list of biomarkers used in the IPF versus normal classifier. The data presented in **Figures 3** **and** **4** show results using the 50-gene biomarker signature set forth in Table 4.

**Figure 3****.** Classification error rate of IPF vs. Normal using a preferred 50-gene signature.

**Figure 4****. Classification** of IPF vs. Normal using a 50-gene signature. From left to right on the X-axis of Figure 4, the results for the first 13 samples correspond to normal samples, with the remaining results for IPF samples.

Normal lung samples score low on the classifier while IPF samples score high. Only three samples (one Normal and two IPF) were incorrectly classified in this example.

| **Table 4 Gene Symbol** | **Ensembl ID** | **TCID** | **Log FC** | **p value** | **FDR p value** |
|---|---|---|---|---|---|
| THY1 | ENSG00000154096 | 7952268 | 1.80 | 3.25E-20 | 1.08E-16 |
| GALNT13 | ENSG00000144278 | 8045776 | -1.28 | 3.59E-18 | 6.29E-15 |
| CFH | ENSG00000000971 | 7908459 | 0.94 | 3.88E-18 | 6.47E-15 |
| CRTAC1 | ENSG00000095713 | 7935535 | -1.14 | 5.62E-17 | 7.49E-14 |
| CHD3 | ENSG00000170004 | 8004699 | 0.45 | 8.73E-17 | 1.08E-13 |
| TSHZ2 | ENSG00000182463 | 8063437 | 1.22 | 1.39E-16 | 1.66E-13 |
| NA | ENSG00000123119 | 8147244 | -1.79 | 2.01E-16 | 2.23E-13 |
| ISM1 | ENSG00000101230 | 8061013 | 1.22 | 1.95E-15 | 1.58E-12 |
| NA | ENSG00000236972 | 7971998 | -0.65 | 2.59E-15 | 1.96E-12 |
| PDE7B | ENSG00000171408 | 8122222 | 1.26 | 4.76E-15 | 3.30E-12 |
| SULF1 | ENSG00000137573 | 8146863 | 1.49 | 5.66E-15 | 3.84E-12 |
| FABP5 | ENSG00000164687 | 7948420 | -0.75 | 1.04E-14 | 6.77E-12 |
| FABP5 | ENSG00000164687 | 8147049 | -0.74 | 1.49E-14 | 8.57E-12 |
| PTGFRN | ENSG00000134247 | 7904293 | 0.75 | 1.40E-13 | 6.76E-11 |
| IGFBP5 | ENSG00000115461 | 8058857 | 1.18 | 8.20E-13 | 3.03E-10 |
| CFHR1 | ENSG00000244414 | 7908488 | 0.73 | 1.79E-12 | 5.90E-10 |
| MS4A15 | ENSG00000166961 | 7940333 | -1.13 | 3.22E-12 | 1.00E-09 |
| FAM13C | ENSG00000148541 | 7933733 | 1.07 | 2.39E-11 | 5.00E-09 |
| STC1 | ENSG00000159167 | 8149825 | -1.00 | 1.23E-10 | 1.91E-08 |
| RCAN2 | ENSG00000172348 | 8126760 | 0.74 | 2.24E-10 | 3.09E-08 |
| LGI2 | ENSG00000153012 | 8099685 | 0.80 | 1.13E-09 | 1.14E-07 |
| CCND2 | ENSG00000118971 | 7953200 | 0.49 | 4.00E-09 | 3.13E-07 |
| DCLK1 | ENSG00000133083 | 7970954 | 0.88 | 9.45E-09 | 6.27E-07 |
| C22orf46 | ENSG00000184208 | 8073470 | 0.32 | 1.32E-08 | 8.19E-07 |
| IGF1 | ENSG00000017427 | 7965873 | 1.04 | 3.77E-08 | 1.97E-06 |
| CSF3R | ENSG00000119535 | 7914950 | -0.64 | 5.85E-08 | 2.78E-06 |
| PPP4R4 | ENSG00000119698 | 7976451 | -0.58 | 1.85E-07 | 7.36E-06 |
| CEBPD | ENSG00000221869 | 8150592 | -0.42 | 4.41E-07 | 1.53E-05 |
| NMNAT2 | ENSG00000157064 | 7922756 | 0.68 | 1.63E-06 | 4.48E-05 |
| EPB41L5 | ENSG00000115109 | 8044882 | -0.62 | 2.62E-06 | 6.67E-05 |
| ZNF385D | ENSG00000151789 | 8085774 | 1.04 | 3.68E-06 | 8.68E-05 |
| FCN3 | ENSG00000142748 | 7914075 | -1.41 | 5.96E-06 | 1.30E-04 |
| CNTN6 | ENSG00000134115 | 8077299 | -0.57 | 5.22E-05 | 7.77E-04 |
| DGKA | ENSG00000065357 | 7956046 | 0.44 | 8.10E-05 | 1.12E-03 |
| CSGALNACT 1 | ENSG00000147408 | 8149574 | 0.47 | 0.00010316 | 1.36E-03 |
| SYT15 | NA | 7933298 | -0.34 | 0.000125693 | 1.59E-03 |
| STX11 | ENSG00000135604 | 8122457 | -0.44 | 0.000194671 | 2.25E-03 |
| ITSN1 | ENSG00000205726 | 8068305 | 0.23 | 0.001160814 | 9.50E-03 |
| TMEM100 | ENSG00000166292 | 8016841 | -0.89 | 0.001540623 | 1.20E-02 |
| EGFLAM | ENSG00000164318 | 8105013 | 0.25 | 0.001609943 | 1.24E-02 |
| C13orf15 | ENSG00000102760 | 7968789 | -0.43 | 0.004402873 | 2.75E-02 |
| NA | ENSG00000182010 | 7933855 | -0.95 | 0.00804948 | 4.40E-02 |
| ERRFI1 | ENSG00000116285 | 7912157 | -0.46 | 0.008595702 | 4.63E-02 |
| RGS16 | ENSG00000143333 | 7922717 | -0.31 | 0.009073453 | 4.82E-02 |
| SLN | ENSG00000170290 | 7951479 | 1.04 | 0.011401833 | 5.77E-02 |
| NA | ENSG00000146374 | 8121916 | 0.56 | 0.011522324 | 5.82E-02 |
| NA | NA | 8066275 | -0.38 | 0.029116378 | 1.15E-01 |
| CXCL2 | ENSG00000081041 | 8100994 | -0.55 | 0.037662664 | 1.39E-01 |
| ITLN1 | ENSG00000179914 | 7921690 | 0.93 | 0.21456173 | 4.45E-01 |
| PDE2A | ENSG00000186642 | 7950162 | 0.10 | 0.377332151 | 6.18E-01 |

**Figures 9A-9D** provide genes differentially expressed in IPF versus "Rest" using microarray data. "Rest" in Figures 9A-D refers to a combination of ILDs other than IPF; for example, in Figures 9A-D, "Rest" refers to ILDs such as HP, NSIP, and sarcoidosis.

In Figures 9A-D, markers were ranked based on significance (p<0.05) after correction for false discovery rate (FDR). A total of 1342 markers were found to be significant with this analysis. Genes are that are overexpressed in IPF and genes that are underexpressed in IPF are listed.

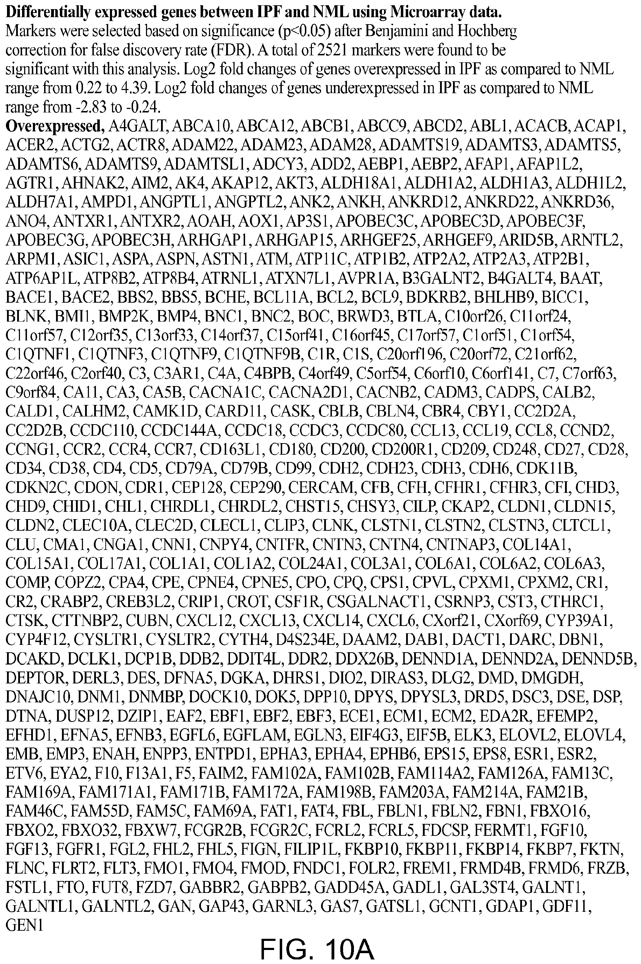

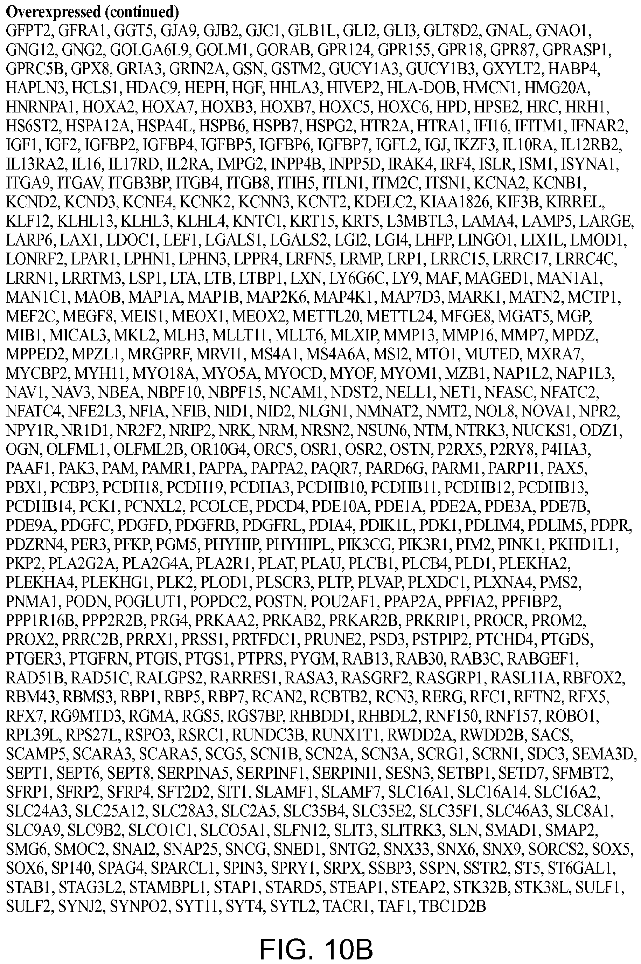

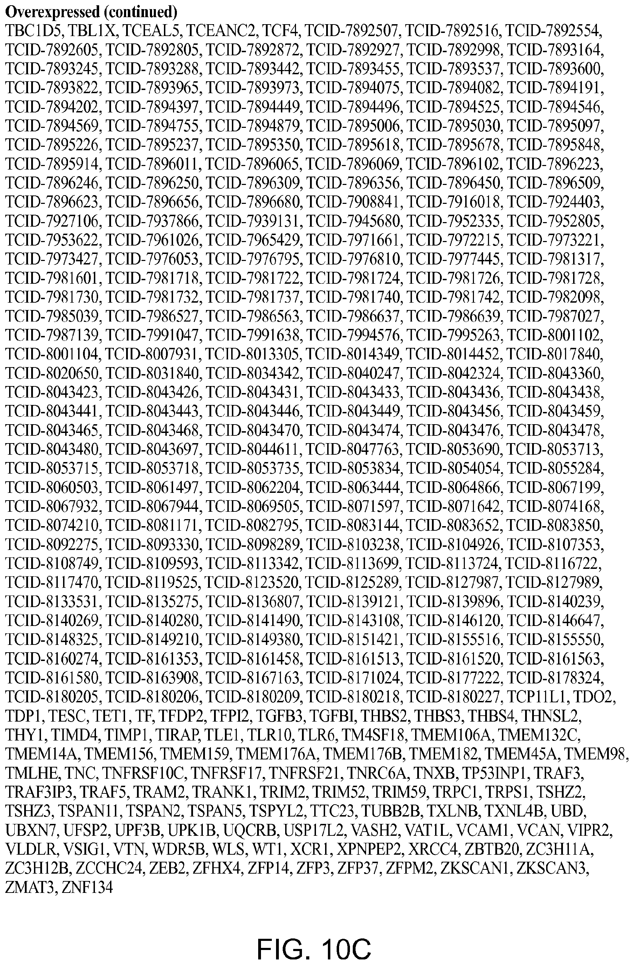

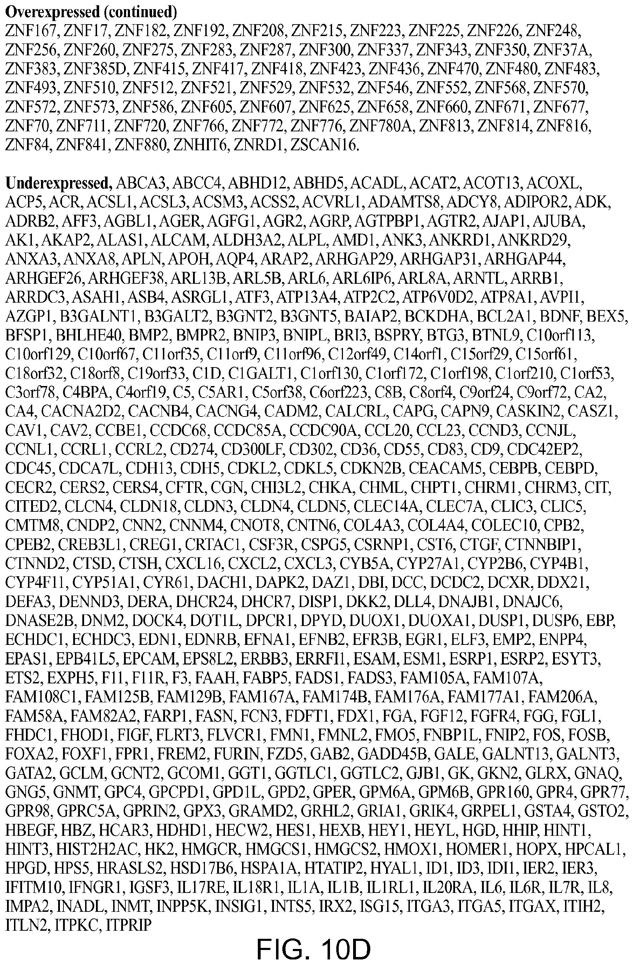

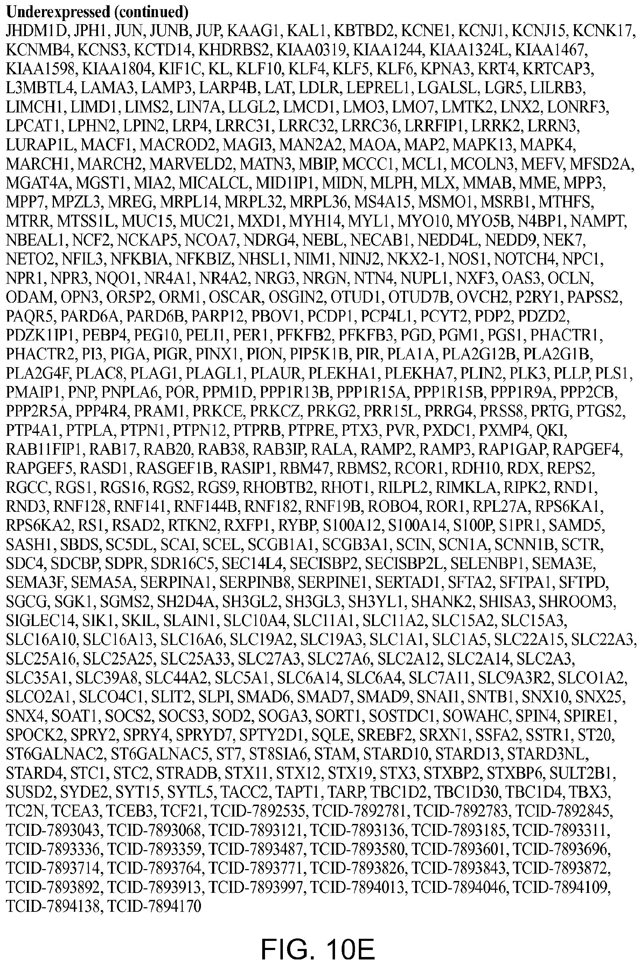

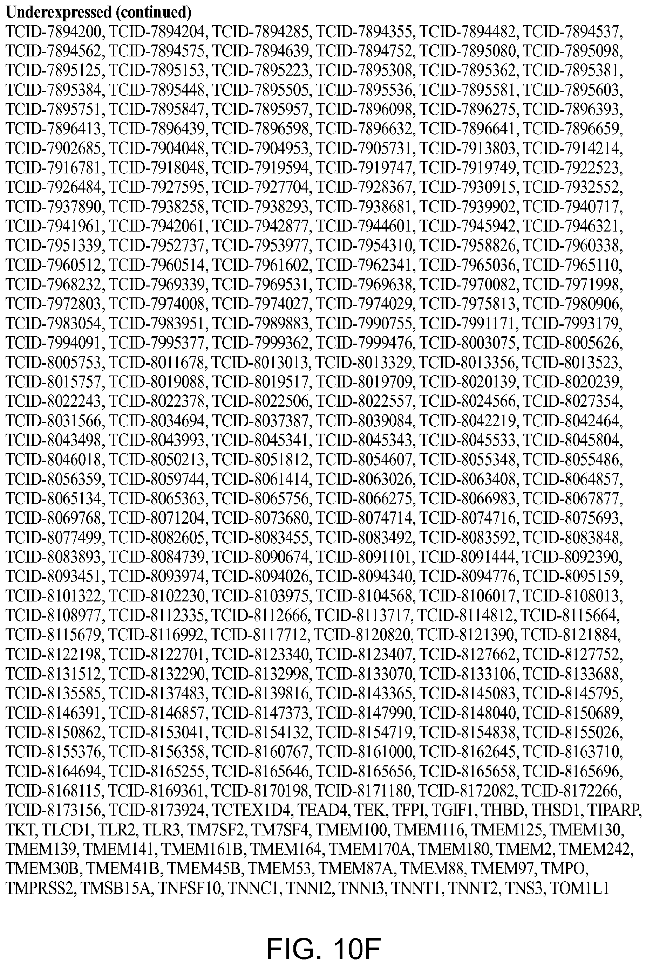

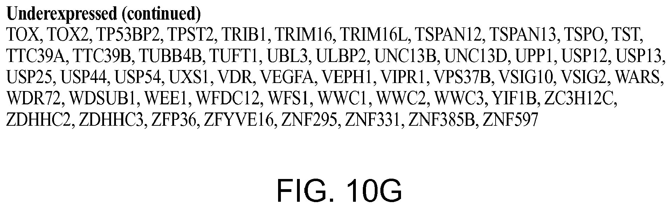

**Figures 10A-10G** provide genes differentially expressed in IPF versus normal, using microarray data. In Figures 10A-G, markers were ranked based on significance (p<0.05) after Benjamini and Hochberg correction for FDR. A total of 2521 markers were found to be significant with this analysis. Genes are listed here include those that are overexpressed in IPF, and those that are under-expressed in IPF.

**Figure 11A-11F** provide genes differentially expressed in NSIP versus normal using microarray data. In Figures 11A-F, markers were ranked based on significance (p<0.05) after Benjamini and Hochberg correction for FDR. A total of 2253 markers were found to be significant with this analysis. Genes listed include those that are overexpressed in IPF, and those that are under-expressed in IPF.

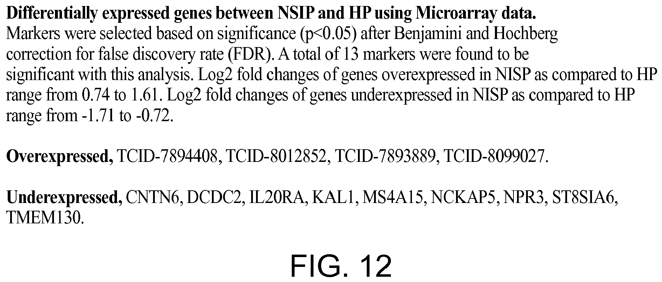

**Figure 12** provides genes differentially expressed in NSIP versus HP using microarray data. In Figure 12, markers were ranked based on significance (p<0.05) after Benjamini and Hochberg correction for false discovery rate (FDR). A total of 13 markers were found to be significant with this analysis. Genes listed include those that are overexpressed in NSIP, and those that are under-expressed in NSIP.

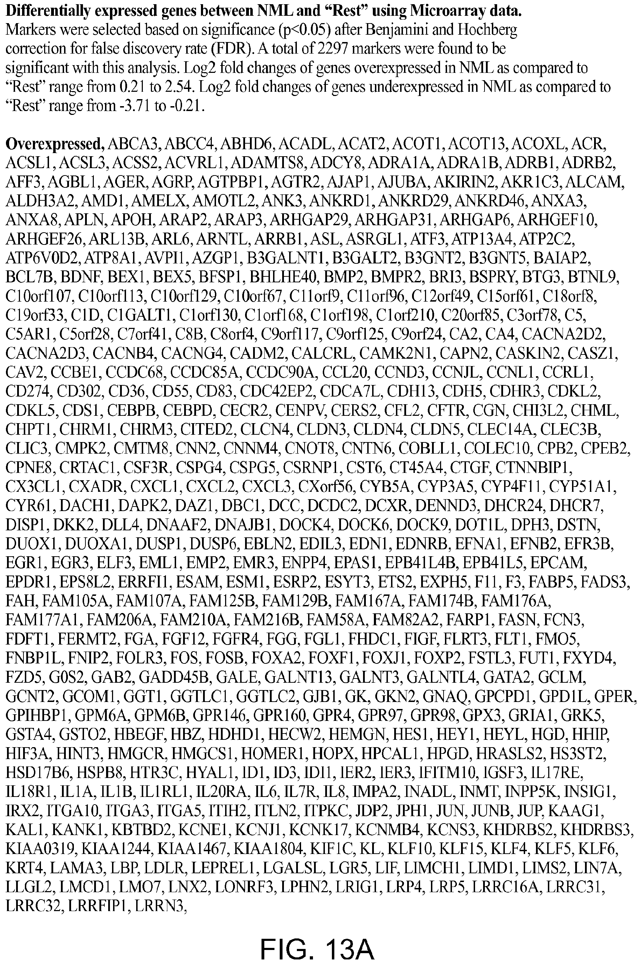

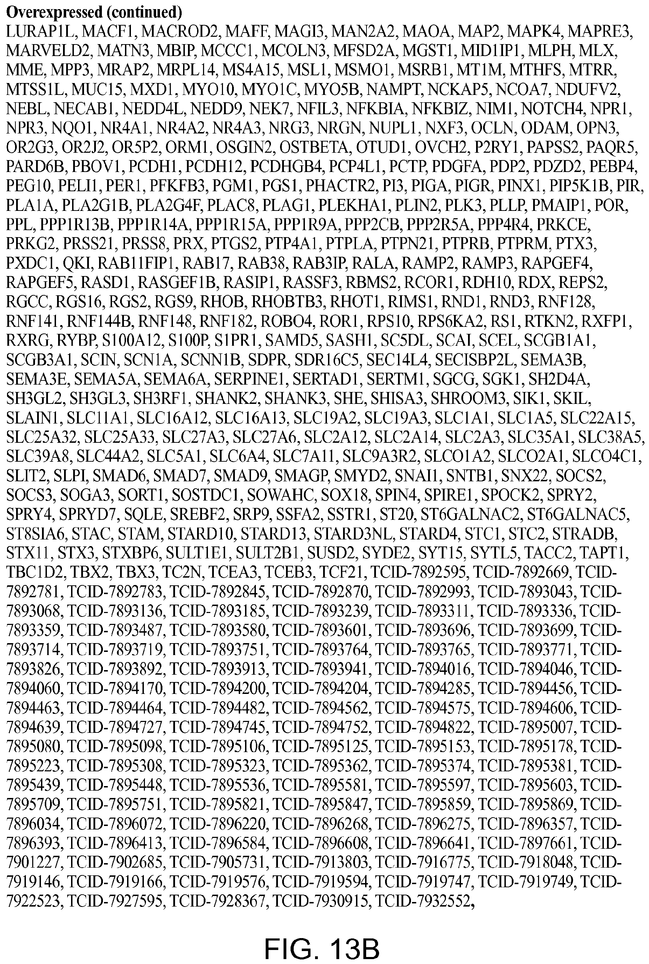

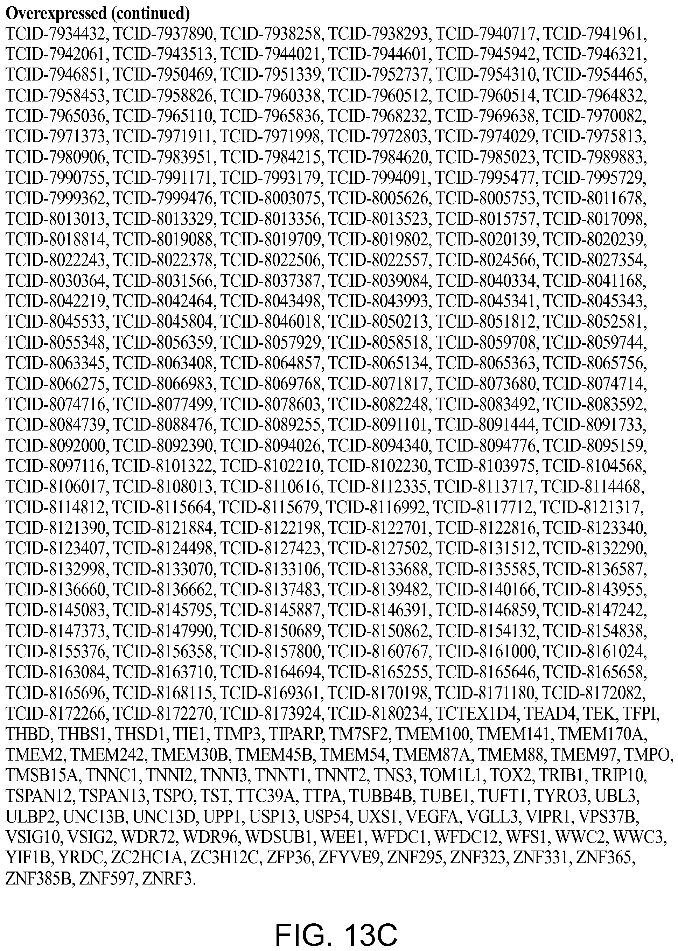

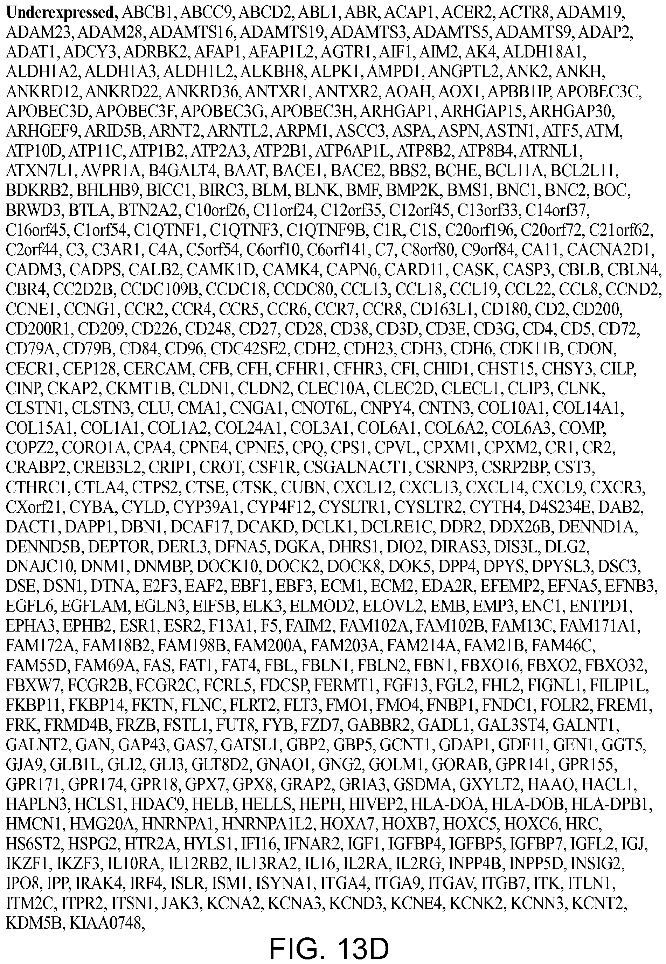

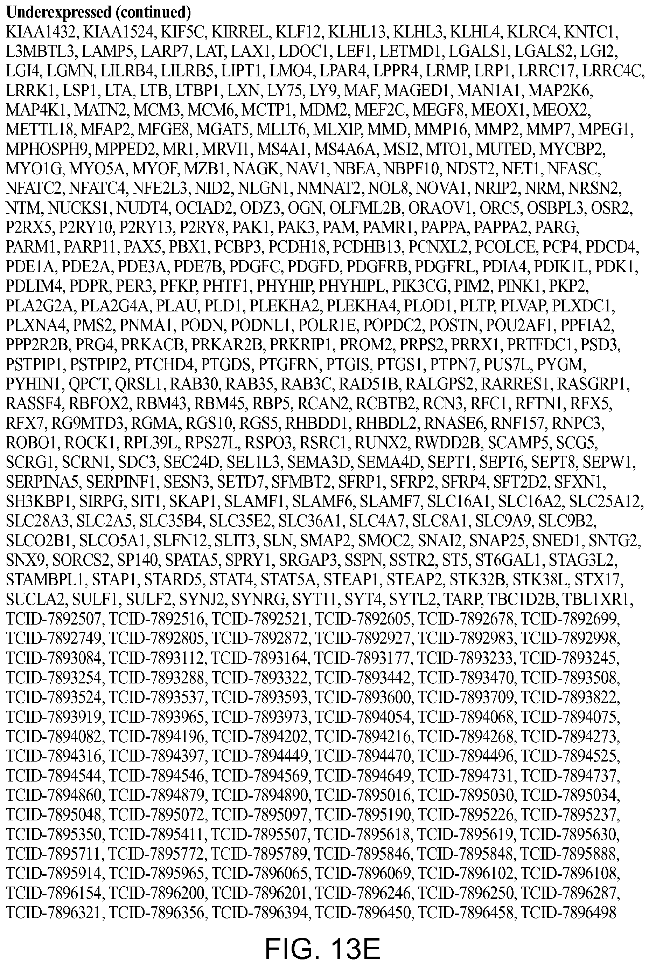

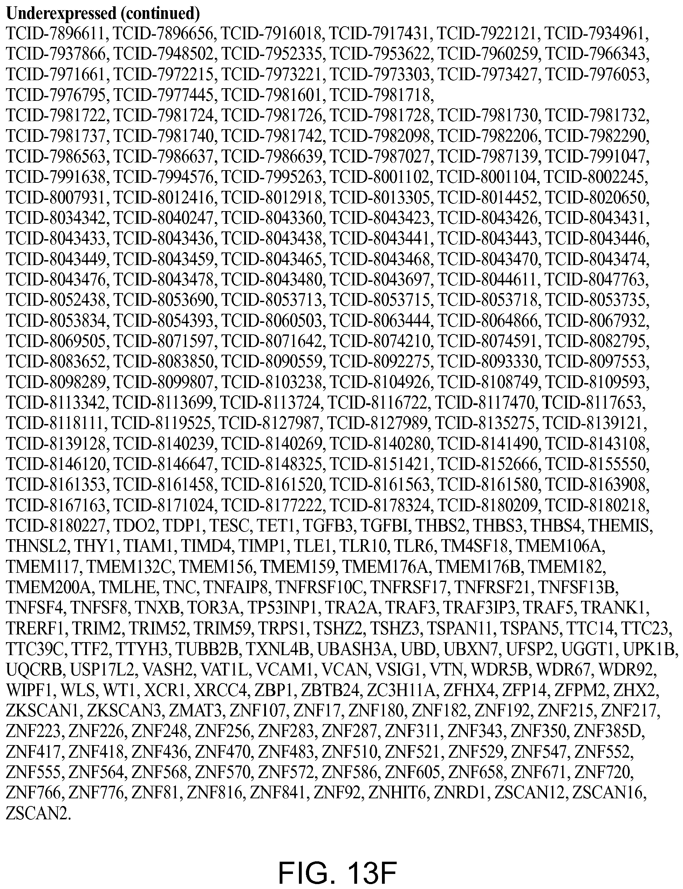

**Figures 13A-13F** provide genes differentially expressed in normal (NML) versus "Rest" using microarray data. In Figures 13A-F, markers were ranked based on significance (p<0.05) after Benjamini and Hochberg correction for FDR. A total of 2297 markers were found to be significant with this analysis. Genes listed include those that are overexpressed in NML (log FC column), and those that are under-expressed in NML.

**Figure 14** provides genes differentially expressed in NP versus NML using microarray data. In Figure 14, markers were selected based on significance (p<0.05) after Benjamini and Hochberg correction for FDR. A total of 311 markers were found to be significant with this analysis. Genes listed include those that are overexpressed in HP, and those that are under-expressed in HP.

Table 5 summarizes the numbers of significant genes using RNA-Seq and microarray data.

| **Table 5** | **Number of significant genes detected per analytical approach** | | |
|---|---|---|---|
| **Comparison** | **EdgeR RNA-Seq** | **NPSeq RNA Seq** | **LIMMA Microarray** |
| IPF vs. NSIP | 296 | 160 | 495 |
| IPF vs. Rest | 740 | 934 | 1342 |
| IPF vs. NML | 1223 | 1687 | 2521 |
| IPF vs. HP | 43 | 0 | 24 |
| NSIP vs. NML | 1628 | 1654 | 2253 |
| NSIP vs. HP | 214 | 0 | 13 |
| NML vs. Rest | 896 | 1436 | 2297 |
| HP vs. Rest | 8 | 0 | 0 |
| HP vs. NML | 876 | 0 | 311 |

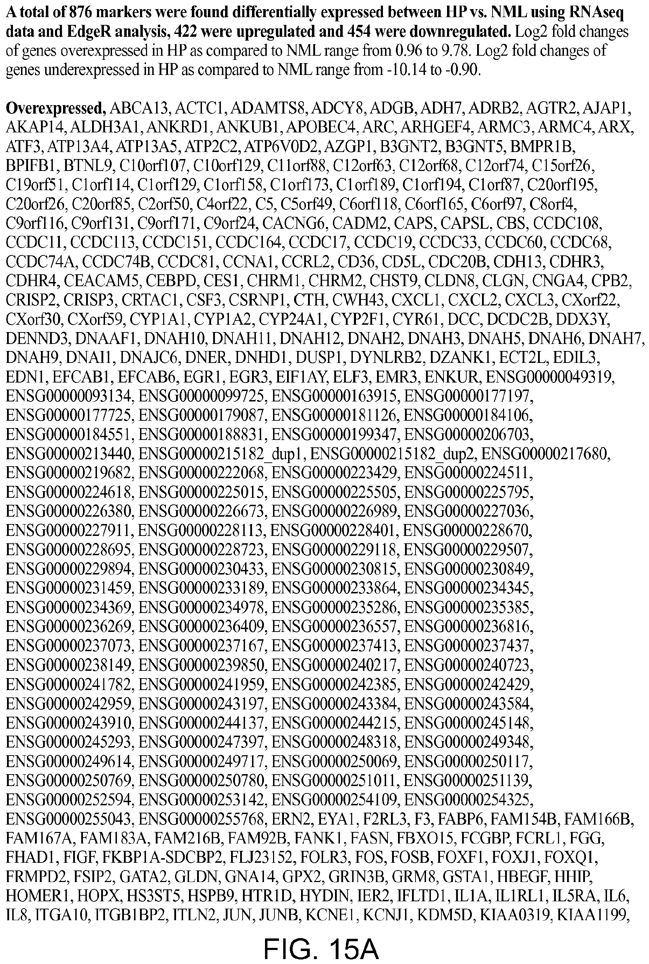

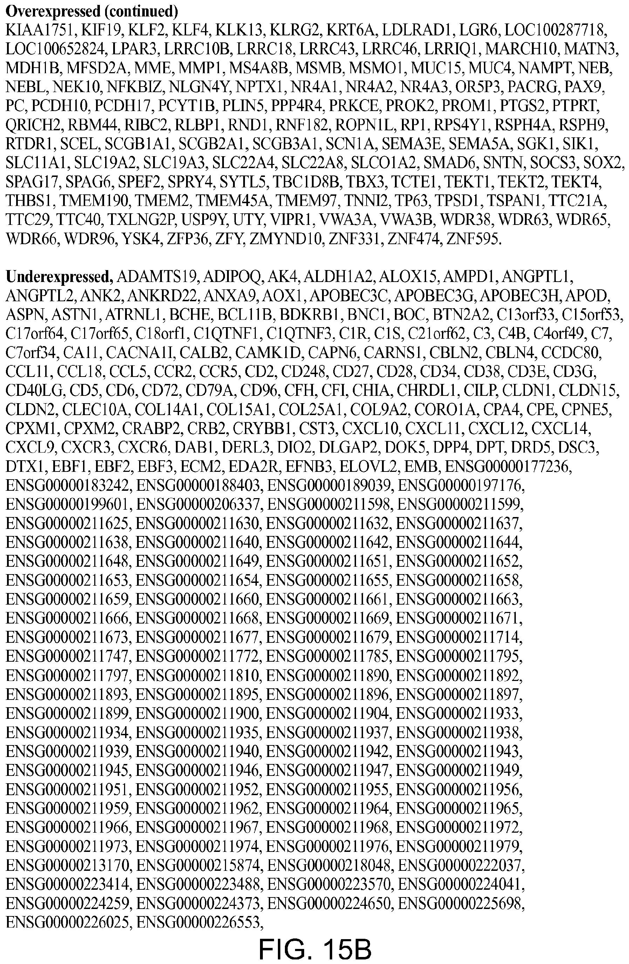

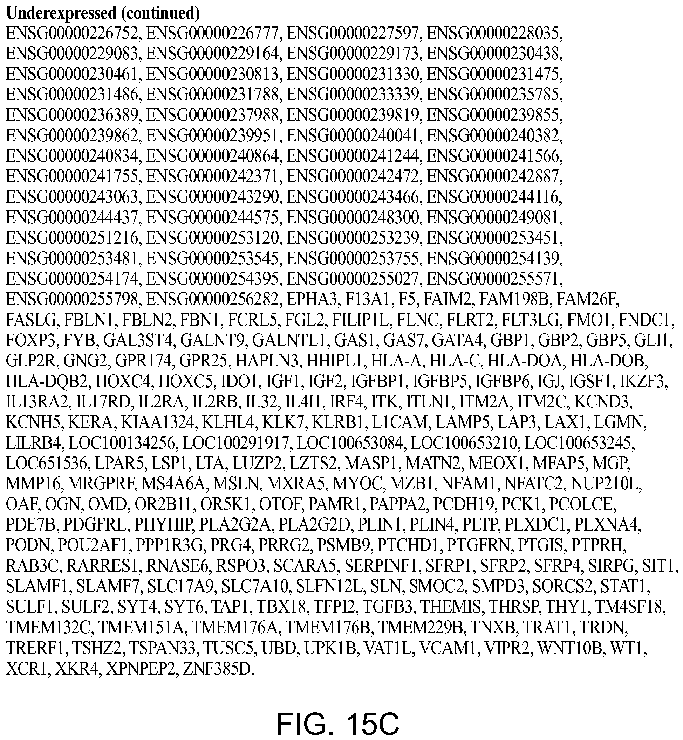

Results of additional gene expression analyses are provided in Figures 15A-27E.

### Example 2 - Data analysis and algorithms

### Sample collection

ILD samples were collected by video assisted thoracoscopic surgery (VATS), while normal lung (NML) was collected from normal adjacent tissue left over after resection during or after lung transplantation. Both were placed on dry iced and stored at -80C until used.

### RNA isolation, amplification, and microarray hybridization

RNA from VATS samples was extracted using the AllPrep micro kit (Qiagen). The quantity of RNA was determined using a Quant-IT RNA kit (Invitrogen, Carlsbad, CA) and RNA quality determined using the Bioanalyzer Picochip system (Agilent Technologies, Santa Clara, CA) to generate a RNA integrity number (RIN). Fifteen nanograms of total RNA were amplified using the NuGEN (San Carlos, CA) WTA Ovation amplification system (WTA FFPE Ovation), resulting in 5.0 µg of biotin-labeled cDNA for hybridization to the microarray. This was followed by washing, staining and scanning on a GeneChip Fluidics 450 / Scanner 3000 7G system and/or Gene Chip system (Affymetrix, Santa Clara, CA) following manufacturer's protocols. Amplified samples were hybridized to Human Gene 1.0 ST microarrays (Affymetrix) using standard processes.

### Post-hybridization, quality control and normalization

The R/Bioconductor *oligo* package (version 1.22.0) was used to process, normalize and summarize output (post-hybridization) microarray data (.CEL) files through background correction, quantile normalization, and robust multichip average (RMA). This automated processing produces a probeset-level intensity matrix and a gene-level intensity matrix. Post-hybridization quality control included percent detection above background (pDET or DABG, >= 0.21) and a housekeeping to anti-genomic signal AUC for control probes (HAAUC, >= 0.88). Accurate classification may be encumbered by a variety of technical factors including failed or suboptimal hybridization. Post-Hybridization QC metrics are correlated with Pre-Hybridization QC variables to identify the technical factors that may obscure or bias signal intensity.

### Composition of the training set

The training set for the preliminary IPF vs. NSIP or IPF vs. Normal classifiers used the samples labeled as such listed in **Table 1.** Training labels for ILD subtypes were defined based on expert histopathology diagnosis for patients with surgically resected biopsy (VATS).

### Classifier training and feature selection

Each ILD classifier is a binary classifier attempting to classify individual samples as either showing evidence of IPF or an alternative class: either normal lung tissue or lung tissue with an NSIP pathology pattern present.

Classifier performance was evaluated using nested cross-validation (as detailed below) with a sequential procedure for feature selection used inside each cross-validation loop to identify markers differentiating individual subtypes. *Limma* analyses of subtype-specific expression were applied to these data sets (Smyth, G. K. (2005). Limma: linear models for microarray data. In: 'Bioinformatics and Computational Biology Solutions using R and Bioconductor'. R. Gentleman, V. Carey, S. Dudoit, R. Irizarry, W. Huber (eds), Springer, New York, pages 397-420). Top markers from *limma* analyses

(ranked in order of ascending false discovery rate) were included in the initial feature set. These markers were evaluated using multiple classification methods including a support vector machine using a linear kernel function (SVM linear) (C.Cortes and V.Vapnik. Support vector networks. Machine Learning, 20:273--297, 1995), k-nearest neighbor (KNN), Linear Discriminant Analysis (LDA DIAG) and Up/DOWN classifiers (updown). All classification was run in R using publicly available software packages, and cross-validation parameters were edited locally (i.e., 30-fold leave-one-out cross validation).

### PERFORMANCE ASSESSMENT AND MODEL SELECTION

For each target classification task (e.g., IPF vs. Normal; or IPF vs. NSIP), performance was assessed on training data using a leave-one-out (LOO) cross-validation strategy. This performance data was used to generate a ROC curve for review. To ensure validity of performance estimates, all components associated with model building (both feature selection and classifier training) were included in the inner loop of the cross-validation procedure. Cross-validation was performed on the entire training set. Using this methodology for performance evaluation, a comparison of classifiers resulted in selection of the linear SVM with 30 (IPF vs. NSIP) or 50 (IPF vs. NML) transcript clusters chosen by *limma* as described above. Each of these selected classifiers was then tested on a set of explant normal, IPF and NSIP samples resulting in performance similar to that seen in training under LOO CV. Table 6 lists the software used during development of ILD classifier.

| **Table 6 Software used:** | **R Package** | **Build** |
|---|---|---|
| Used to normalize array data | oligo | 1.22.0 |
| Provided array probe and probe set annotation | pd.hugene.1.0.st.v1 | 3.8.0 |
| Used for SVM classification (linear and fixed cost) | e1071 | 1.6.1 |
| Used for gene/feature selection | limma | 3.14.1 |
| Used for LDA classification | MASS | 7.3-22 |
| Used for ROC curve visualization | ROCR | 1.0-4 |
| Used for diagonal LDA classifier | sfsmisc | 1.0-23 |
| Used for KNN classification | class | 7.3-4 |
| Used for LASSO classification | lasso | 1.9-3 |
| Used for Up/Down classification | Internal software measuring the sum of the absolute value of gene fold changes. | |

### Example 3 - Biomarkers useful in the diagnosis of IPF and other lung diseases

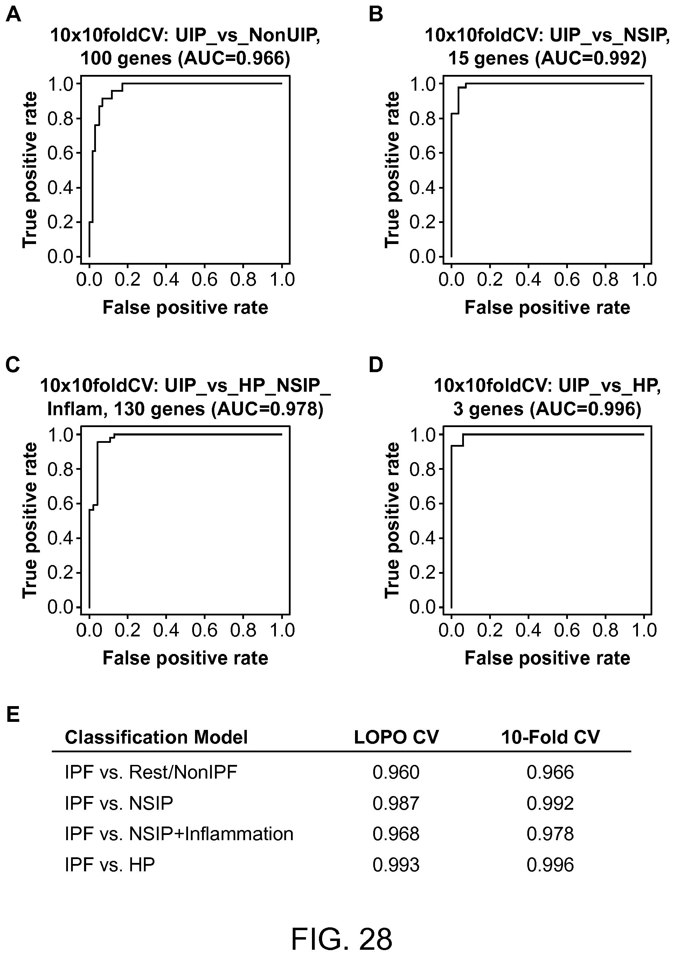

Machine learning methods used to develop clinically and commercially useful classifiers are known in the art. However, training of these classifiers and subsequent validation using an independent cohort of samples can prove challenging. In this Example, differentially expressed biomarkers shown to accurately predict IPF status of VATS lung tissue biopsies during cross-validation (Figure 28), regardless of the comparator group examined, are described.

Figure 28. High AUCs demonstrate high accuracy of ILD classifiers. Eight distinct classifiers were trained and evaluated using Leave-One-Patient-Out (LOPO) or 10-Fold cross-validation. The area under the curve (AUC) of the receiver operating characteristic (ROC) exceeds 0.96 in each analysis regardless of the method used indicating that highly accurate results can be expected in commercial application.

Table 7 presents the VATS lung tissue sample cohort studied by microarray. Samples are listed according to pathologic classification using expert labels. Samples were obtained by video-assisted thoracoscopic surgery (VATS), or bronchoscopy.

**Table 7**

| **Pathology Label** | **Pathology Abbreviation** | **Total No. of samples** |
|---|---|---|
| Bronchiolitis | BRONCH | 1 |
| Chronic Interstitial Fibrosis not otherwise classified | CIF-NOC | 8 |
| Hypersensitivity pneumonitis | HP | 16 |
| Idiopathic Pulmonary Fibrosis | IPF | 46 |
| Other | OTHER | 6 |
| Non-specific interstitial pneumonia | NSIP | 26 |
| Organizing pneumonia | OP | 2 |
| Respiratory Bronchiolitis | RB | 4 |
| Sarcoidosis | SARC | 2 |
| Smoking related interstitial fibrosis | SRIF | 1 |
| **Total** | | **112** |

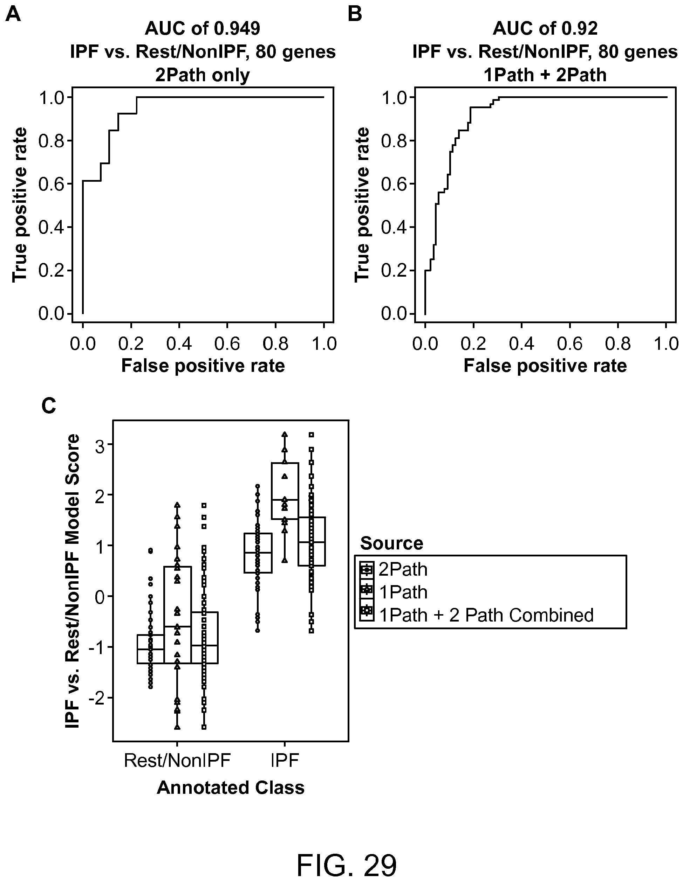

These classifiers were developed using IPF training labels generated only when at least two pathologists agreed on the IPF diagnosis (2Path). The 2Path classifiers were applied to an independent cohort of samples with training labels by a single pathologist (1Path) and show that while classification is possible in some fraction of these samples, a large proportion are misclassified (Figure 29).

Figure 29. Classifier comparisons. Classifiers trained with samples that have concordant labels by two pathologists (2Path, Panel A) outperform classifiers trained with labels from a single pathologist (1Path), or a combination of both sample cohorts (Panel B). Classifiers scores are more widely distributed in 1Path, or combined classifiers (Panel C).

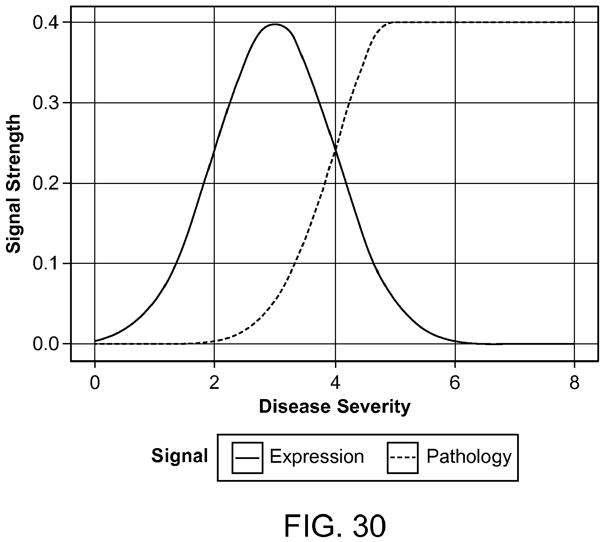

Furthermore, when both cohorts (2Path and 1Path) are combined the resulting classifiers are less accurate and decision boundaries are difficult to draw. These data highlight the importance of obtaining MDT (multidisciplinary team; a team of medical experts) gold standard labels during classifier training. One hypothesis that may explain the observed data is that the biomarkers discovered in the development of this invention are so sensitive, that samples that do not yet show signs of IPF to a pathologist are already showing the molecular signature of the disease (Figure 30).

Figure 30. IPF expression signal hypothesis. Graphical representation of a hypothesis that may explain the observed data of a strong IPF molecular signature detectable in samples not yet showing severe IPF gross pathology. While false positive results are not uncommon during classifier development, the observed data suggest that a detectable signal is present prior to full-blown IPF disease.

The molecular signatures discovered here are developed to train additional classifiers aimed at evaluating not only lung VATS tissue biopsies, but also transbronchial biopsies (TBB), bronchoalveolar lavage (BAL), and cryobiopsy samples. Each one of these specimen types (VATS, TBB, BAL or cryobiopsy) samples a distinct cellular compartment and it can be expected that within a single patient each one of these specimens will produce its own disease signature, which is generally only partially overlapping with other specimen types. The new classifiers utilize a combination of VATS and TBB, or VATS and BAL, TBB and BAL, TBB and cryobiopsy, VATS and cryobiopsy, or either specimen type alone (or in any combination of one, two, three or four) in order arrive at the molecular signature that gives classification with specificity of >95%, PPV, >95%, and sensitivity >70%. Additional classifiers that utilize further combinations of specimens from the same patient including, but not limited to blood plasma, blood serum, saliva, and sputum are also included. Furthermore, while the best classifiers may be trained by using a combination of VATS, TBB, BAL, blood serum, blood plasma, saliva and/or sputum from the same patient, the methods may also be used to develop classifiers using any combination of these specimen types from different patients (e.g., VATS from patient 1, blood serum from patient 2, saliva from patient 3, etc.). Additionally, the methods can be used to develop classifiers where 2 or more specimen types from a single patient are evaluated simultaneously in order to accurately detect, predict or diagnose disease (e.g., BAL and saliva from patient 1, or VATS and TBB from patient 1, or TBB and blood plasma from patient 1, or any combination thereof). These methods can also be used in the prognosis of disease severity and in the evaluation of treatment response prior to, during, or after drug, respiratory rehabilitation, or other treatments. For example, the methods can be used to identify patients that are candidates for treatment with specific drug targets, or to exclude patients that are not candidates for treatment with specific drug targets.

The methods have identified molecular signatures for all major ILD subtypes (Table 27) and these methods maybe applied narrowly and differently for each specific subtype, or more broadly to a combination or group of subtypes. For example, inflammation is a hallmark of HP, NSIP, Sarcoidosis, and Organizing Pneumonia (OP), and as a group (ILD inflammation) these subtypes can be accurately distinguished from IPF (Figures 28, Table 8, and listed below); and IPF can be distinguished from these subtypes and classified separately from ILD inflammation. While the methods aim at the classification of ILD disease and may be used to identify specific ILD subtypes, training of classifiers expected to perform in a clinical setting with high accuracy, sensitivity, specificity, and reproducibility, does require utilizing samples slightly outside the ILD disease spectrum. For example classifier training may include samples from patients, with COPD, pulmonary hypertension, connective tissue disease, lung nodules, etc., as symptoms of these may mimic ILD and it is important to be able to train a classifier that can distinguish ILDs from these other conditions.

The molecular signatures identified in this invention point to genes involved in muscle physiology as important biomarkers of IPF. Muscle genes are upregulated in IPF and not in any other ILD subtype, similarly inflammation genes are significantly downregulated in IPF, while they are greatly upregulated in HP, NSIP, OP, and sarcoidosis. While the methods are not limited by gene ontology, molecular function, or specific pathway, it is also understood that any biomarker in the muscle or inflammation categories maybe used.

### IPF vs. NSIP classification biomarkers

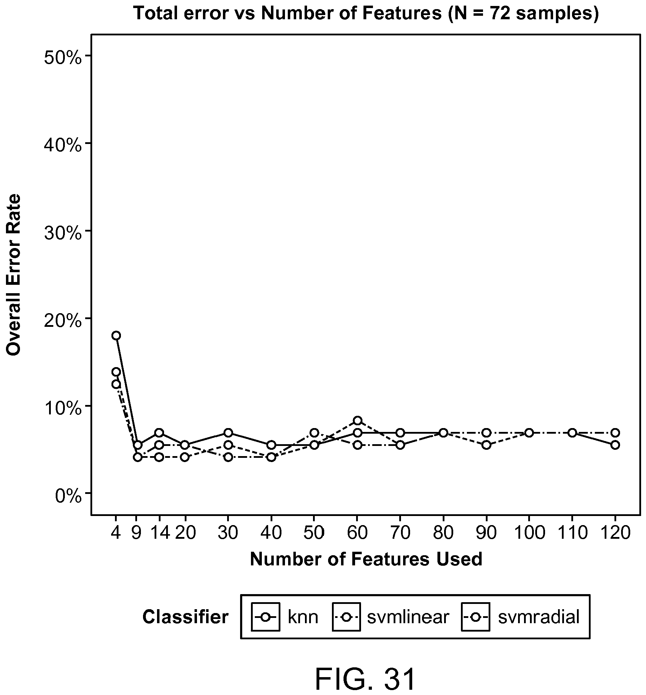

Figure 31 depicts the classification error rate of IPF vs. NSIP using a 9-gene signature. During leave-one patient out (LOPO) cross-validation three distinct classifiers (KNN, SVMlinear, and SVMradial) show similar performance with less than 10% overall classification.

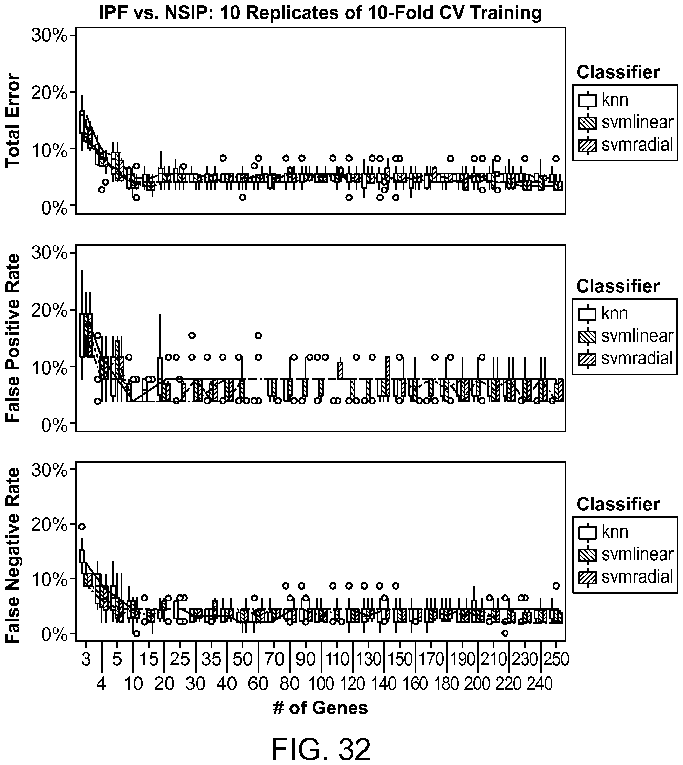

Figure 32 depicts the classification error rate of IPF vs. NSIP using a 15-gene signature. During 10-fold cross-validation three distinct classifiers (KNN, SVMlinear, and SVMradial) show similar performance with less than 10% overall errors.

Table 8 provides exemplary biomarkers used in IPF vs. NSIP classification. As indicated by an "x" in Table 8, some biomarkers were useful for classification using any number of classification methods and leave-one-patient-out (LOPO) cross-validation, and/or a 10-fold cross-validation (10-Fold), and/or any other classification and cross-validation method (Any).

**Table 8**

| **TCID** | **Symbol** | **Any** | **LOPO** | **10-Fold** |
|---|---|---|---|---|
| 8101031 | CDKL2 | x | x | x |
| 8037205 | CEACAM1 | x | | x |
| 8127234 | DST | x | x | x |
| 8112980 | EDIL3 | x | x | x |
| 8179019 | HLA-F | x | x | x |
| 8177717 | HLA-F | x | x | x |
| 8117760 | HLA-F | x | x | x |
| 7947248 | KIF18A | x | | x |
| 7923386 | LMOD1 | x | x | |
| 7956856 | MSRB3 | x | x | x |
| 7999674 | MYH 11 | x | x | |
| 8090098 | MYLK | x | | x |
| 7934997 | PPP1R3C | x | | x |
| 8126853 | PTCHD4 | x | | x |
| 8109639 | PTTG1 | x | | x |
| 8172022 | TMEM47 | x | | x |
| 7917199 | TTLL7 | x | | x |

A set of 250 differentially expressed (overexpressed; underexpressed) biomarkers which satisfied analytical criteria indicative of usefulness in IPF vs. NSIP classification using Microarray data is listed below. Log2 fold changes of genes overexpressed in IPF (n=101) range from 0.22 to 1.37. Log2 fold changes of genes under-expressed in IPF (n=149) range from -2.20 to -0.20. The FDR p-value range for all 250 genes in this analysis is 2.21E-11 to 2.46E-06. If HUGO gene symbols were not available, the Affymetrix transcript cluster identifier (TCID) was given to specify the gene.

**Overexpressed:** ACTA2, ACTG2, AHNAK, AHNAK2, AKT3, ANO2, AOC3, ARHGEF25, ATP1A2, CALD1, CAMK2N1, CD109, CDH13, CNKSR2, CNN1, COL21A1, COPZ2, COX7A1, CRIM1, CRYAB, CSRP1, DENND2A, DES, DGKG, DIXDC1, DMD, DNAJB4, DPYSL3, DST, DTNA, EDIL3, EOGT, EPB41L2, FGF14, FHL1, FMO2, FMO3, GARNL3, HOXA-AS2, HOXA4, HOXA6, HPSE2, LAMA4, LARP6, LHFP, LMCD1, LMOD1, LRRFIP1, MAP1B, MARK1, MCAM, MFAP4, MPDZ, MSRB3, MXRA7, MYH10, MYH11, MYL9, MYLK, MYOCD, NEXN, PALLD, PCDHB10, PCDHGC5, PDE1C, PDE5A, PDLIM3, PEAK1, PGM5, PGM5P2, PHLDB2, PKD2, PKIG, PLCB4, PLCE1, PLN, PPP1R3C, PREX2, PRKAA2, PRKAB2, PRKG1, PRUNE2, PTCHD4, PTPRB, REEP1, RERG, SBF2, SORBS1, SYNM, SYNPO2, TAGLN, TIMP2, TIMP3, TMEM47, TMOD1, TPM1, TPM2, TRPC1, TTLL7, UACA, ZC3H12B.

**Underexpressed:** ABCA3, ACADSB, ACOXL, ADAR, APOL6, ARHGEF38, ARPC5L, ASPM, ATP11A, ATP2C2, ATP8A1, BUB1, C18orf8, C2, CASC5, CCL4, CCL4L1, CCNA2, CCNB1, CCNB2, CCR5, CD38, CDC45, CDK1, CDKL2, CEACAM1, CFTR, CISH, CLINT1, CNDP2, CRTAC1, CSF3R, CXCL10, CXCL11, CXCL9, CXCR6, DGKD, DLGAP5, DRAM1, DUSP16, EDEM1, EFNA1, EPSTI1, ESRP2, ETV7, EZH2, FHDC1, FOXM1, FZD5, GALNT3, GBP1, GBP5, GPR98, GRAMD1A, GUCD1, HAS3, HERC6, HIST1H2BI, HIST1H2BM, HIST1H3E, HIST1H3J, HLA-A, HLA-B, HLA-C, HLA-DOA, HLA-F, HLA-H, HMMR, IFI35, IRF1, IRF9, KCNJ15, KCNQ3, KCTD14, KIF11, KIF14, KIF18A, KIF4A, LARP4B, LGALS3BP, LNX2, MAP3K13, MAPK13, MARK2, METTL7B, MKI67, MTUS1, NPC1, NUSAP1, OAS2, ORM1, ORM2, PARP14, PARP9, PLAGL2, PRKCQ, PSMB10, PSMB8, PSMB9, PSME1, PSME2, PTTG1, PXMP4, RABGAP1L, RAD51AP1, RAP1GAP, RBM47, RCOR1, RFX5, RGS16, RNF145, RNF213, SCTR, SDC4, SDR16C5, SEMA4A, SFTPD, SLC16A13, SLC22A3, SMC4, SNX30, SQSTM1, SREBF2, STAT1, STIL, TAP1, TAPBP, TCID-7896714, TCF19, TKT, TLR2, TMED6, TMEM164, TMEM41B, TOP1, TOP2A, TOX, TRAFD1, TRIM26, TYMP, UBD, UBE2S, USP44, VDR, WARS, WWC1, YARS, ZDHHC16, ZNF385B.

### IPF vs. Rest/non-IPF biomarkers

Figures 33A-B provide biomarkers useful for IPF vs. Rest/non-IPF classification. As indicated by an x on the table some biomarkers were useful for classification using any number of classification methods and leave-one-patient-out (LOPO) cross-validation, and/or a 10-fold cross-validation (10-Fold), and/or any other classification and cross-validation method (Any).

A set of 500 differentially expressed (overexpressed; underexpressed) biomarkers which satisfied analytical criteria indicative of usefulness in IPF vs. Rest/NonIPF classification using microarray data are listed below. Log2 fold changes of genes overexpressed in IPF (n=202) range from 0.17 to 1.41. Log2 fold changes of genes under-expressed in IPF (n=298) range from -2.08 to -0.15. The FDR p-value range for all 500 genes in this analysis is 1.47E-12 to 9.87E-06. If HUGO gene symbols were not available, the Affymetrix transcript cluster identifier (TCID) was given to specify the gene.

**Overexpressed:** ABCA6, ABLIM3, ACTA2, ACTG2, ADCY5, AHNAK2, AKAP12, ANO1, ANO2, ARHGEF25, ATP1A2, ATP1B2, BAG2, BHMT2, BMX, C16orf45, C3orf55, C3orf70, CACNA1C, CACNB2, CALD1, CAMK2N1, CCDC3, CDH6, CDR1, CLIP3, CLU, CNN1, COCH, COL21A1, COPZ2, COX7A1, CRYAB, CSRP1, CTTNBP2, DACT1, DDR2, DENND2A, DES, DIXDC1, DMD, DST, DTNA, DYNC1I1, DZIP1, EBF1, EDIL3, EFHD1, F10, FAM124B, FAM13C, FBLIM1, FEZ1, FGF10, FGF14, FGF2, FGFR1, FHL1, FHL2, FLNC, GADL1, GARNL3, GAS7, GNG12, GRIA3, GRIN2A, HOXA2, HOXA6, HOXA7, HPSE2, HRC, HRH1, HSPA2, HSPB6, HSPB7, IGFBP5, ITGB4, ITIH5, KALRN, LAMA4, LAMC1, LARP6, LDB3, LGI4, LMOD1, LPP, LTBP1, MAP1B, MAP7D3, MARK1, MCAM, MEIS1, MEOX2, MPDZ, MRGPRF, MRVI1, MSRB3, MUSTN1, MXRA7, MYH11, MYL9, MYLK, MYOCD, MYOM1, NCAM1, NEXN, NFASC, NPR2, NR2F1, NR2F2, NTRK3, OSR2, PALLD, PALMD, PARVA, PBX1, PCDH7, PCDHB10, PCDHB12, PCDHB13, PCDHB14, PCDHB18, PCDHB3, PCDHB7, PCDHGC5, PDE10A, PDE1A, PDE2A, PDE3A, PDE5A, PDE7B, PDGFRB, PDLIM3, PDZRN4, PEG3, PFKM, PGM5, PGM5P2, PHYHIPL, PKD2, PLA2R1, PLCB4, PLN, PLXNA4, PODN, PPP1R3C, PRKAA2, PRKG1, PRSS23, PRUNE2, PTCHD4, PYGM, RAB9B, RASD2, RBM24, RCAN2, RERG, RGS5, RGS7BP, RYR3, S1PR3, SBSPON, SCRG1, SCUBE1, SEMA3D, SETBP1, SGCA, SLC38A11, SLIT3, SLN, SMIM10, SNED1, SNORD116-17, SNORD116-21, SNX9, SOGA1, SORBS1, SPARCL1, SSPN, SYNM, SYNPO2, SYTL2, TAGLN, TCEAL4, TCEAL6, TIMP2, TMEM252, TMEM47, TMOD1, TPM1, TPM2, TRPC1, TTLL7, WISP2, ZAK, ZC3H12B, ZCCHC24, ZEB2, ZHX3, ZNF135, ZNF154, ZNF521.

**Underexpressed:** ACADL, ACOXL, ACSF2, AHCYL2, ANK3, AP1M2, APOL1, APOL4, APOL6, AQP4, ARAP2, ARHGEF38, ARSD, ASPM, ATP1A1, ATP2C2, ATP8A1, BATF2, BAZ1A, BMP3, BST2, BTG3, BTN3A3, BUB1, C18orf8, C2, CASP1, CASP4, CCL4, CCL4L1, CCL5, CCNA2, CCR5, CD274, CD40, CD47, CD74, CD8A, CDCA7L, CDK1, CDKL2, CEACAM1, CFTR, CKAP2, CLDN4, CLINT1, CNDP2, CNKSR1, CRTAC1, CRTAM, CSF3R, CXCL10, CXCL11, CXCL9, CXCR6, CYB5A, CYP2B6, CYP2B7P1, DAPK1, DBI, DDX60, DENND1B, DHCR24, DNASE2, DOCK8, DRAM1, DTNB, DTX3L, DUSP16, DYNLT1, EDEM1, EFNA1, EML4, ENTPD3, EPSTI1, ERAP1, ERBB3, ESRP1, ESRP2, ETV7, FAM111A, FCGR1A, FCGR1B, FDFT1, FGFR2, FHDC1, FLRT3, FNIP2, FREM2, FZD5, GALNT3, GBP1, GBP4, GBP5, GBP7, GCH1, GCNT2, GK, GNPTAB, GPR98, GRAMD1B, GRHL2, GSAP, GUCD1, GZMB, GZMH, HAS3, HCP5, HIST1H2AI, HIST1H2BI, HIST1H3B, HLA-A, HLA-B, HLA-C, HLA-DMA, HLA-DOA, HLA-DPA1, HLA-DPB1, HLA-DQA2, HLA-DRA, HLA-E, HLA-F, HLA-G, HLA-H, HLA-J, HLA-L, HMGCL, HMGCR, HMMR, HN1, HNF1B, HPS5, ICOS, IDI1, IDO1, IFI27, IFI35, IFIH1, IFIT3, IFIT5, IGSF3, IL10RB, IL32, IL6R, INADL, IRF1, IRF9, ITGAE, ITGAL, JPH1, KCNJ15, KCTD14, KIAA0247, KIAA0319L, KIAA1958, KIF18A, KLF5, L3MBTL4, LAMP3, LAP3, LARP4B, LCP2, LIMD1, LNX2, LPIN2, MAGI3, MAP3K13, MAPK13, MARK2, MB21D1, MBIP, MIA2, MICALCL, MID1IP1, MREG, MRPL14, MTMR14, MTRR, MYL1, MYOSB, N4BP1, NAV2, NEDD4L, NMI, NPC1, NUB1, NUSAP1, OAS2, OCLN, ORM1, PARP12, PARP14, PARP9, PCK2, PCLO, PDZK1IP1, PEBP4, PFKFB3, PHACTR1, PI4K2B, PLA2G12B, PLAGL2, PLCH1, PLS1, PPP1R9A, PPP2R5A, PRKCQ, PRKCZ, PRRG4, PRSS8, PSMB10, PSMB8, PSMB9, PSME1, PSME2, PTP4A1, PTPN1, PTPRJ, PTTG1, PXMP4, RAB20, RAB3IP, RABGAP1L, RANBP17, RARRES3, RBM47, RCOR1, RFC1, RFX5, RGS16, RNF19B, RNF213, S100A14, SAMD9L, SCN1A, SCTR, SDC4, SELENBP1, SEMA4A, SFTPD, SH2D4A, SHANK2, SLAMF8, SLC15A3, SLC16A13, SLC22A3, SLC37A1, SLCO4C1, SMARCA5, SMC4, SQLE, SQRDL, SREBF2, ST7, STARD10, STAT1, STX11, STX19, SYT15, TAP1, TAP2, TAPBP, TAPT1, TC2N, TCID-7895602, TCID-7896651, TCID-7896695, TCID-7951593, TCID-8113717, TDRKH, THEMIS, TKT, TLR2, TLR3, TMEM164, TMEM180, TMEM41B, TMEM87A, TMPRSS2, TMPRSS3, TNFAIP3, TOX, TRAFD1, TRIM21, TRIM24, TTLL4, UBALD2, UBD, UBE2L6, USP38, USP44, USP54, WARS, WHSC1, WWC1, ZDHHC16, ZDHHC2, ZNF385B.

### IPF vs. HP biomarkers

Table 9 provides biomarkers useful in IPF vs. HP classification. As indicated by an x on the table some biomarkers were useful for classification using any number of classification methods and leave-one-patient-out (LOPO) cross-validation, and/or a 10-fold cross-validation (10-Fold), and/or any other classification and cross-validation method (Any).

**Table 9**

| **TCID** | **Symbol** | **Any** | **LOPO** | **10-Fold** |
|---|---|---|---|---|
| 8179019 | HLA-F | x | x | x |
| 8177717 | HLA-F | x | x | x |
| 8117760 | HLA-F | x | x | x |
| 8079377 | CXCR6 | x | x | |

A set of 250 differentially expressed (overexpressed; underexpressed) biomarkers which satisfied analytical criteria indicative of usefulness in IPF vs. HP classification using microarray data are listed below. Log2 fold changes of genes overexpressed in IPF (n=43) range from 0.23 to 1.21. Log2 fold changes of genes under-expressed in IPF (n=207) range from -2.71 to -0.21. The FDR p-value range for all 250 genes in this analysis is 1.62E-13 to 2.02E-04. If HUGO gene symbols were not available, the Affymetrix transcript cluster identifier (TCID) was given to specify the gene.

**Overexpressed:** ACTA2, ACTG2, ARHGEF25, CACNA1C, CALD1, CNN1, COL21A1, DST, EDIL3, GPC6, HERC2P2, HSPA2, KALRN, LINC00328, LMOD1, MAP1B, MEIS1, MEOX2, MYH11, MYLK, MYOCD, NBPF3, NEXN, OSMR, PAPPA, PCDHB18, PDE3A, PLXNA4, PRUNE2, PTCHD4, PTGFRN, SCRG1, SLC25A27, SLIT3, SOGA1, SORBS1, SYNPO2, TAGLN, TCID-7906969, TPM1, TPM2, VCAN, ZNF154.

**Underexpressed:** ADAMDEC1, ANKRD22, APOBEC3C, APOBEC3F, APOBEC3G, APOBEC3H, APOL1, APOL3, APOL4, APOL6, ASCL2, BATF, BATF2, BST2, BTN2A2, BTN3A1, BTN3A2, BTN3A3, C17orf49, CAMK4, CASP1, CASP4, CCL4, CCL4L1, CCL5, CCR5, CD2, CD226, CD244, CD247, CD274, CD3D, CD3E, CD3G, CD40, CD40LG, CD5, CD6, CD72, CD74, CD8A, CD96, CIITA, CLEC6A, CMTM1, CORO1A, CRTAM, CST7, CTLA4, CXCL10, CXCL11, CXCL9, CXCR3, CXCR6, DDX60, DNASE2, EBI3, EIF4E3, EPSTI1, ETV7, FAM26F, FAM78A, FASLG, FGL2, FYB, GATA3, GBP1, GBP2, GBP3, GBP4, GBP5, GBP7, GCH1, GPR171, GPR174, GPR68, GRAMD1B, GZMA, GZMB, GZMH, HCP5, HCST, HIST1H2BI, HLA-A, HLA-B, HLA-C, HLA-DMA, HLA-DMB, HLA-DOA, HLA-DPA1, HLA-DPB1, HLA-DPB2, HLA-E, HLA-F, HLA-G, HLA-H, HLA-J, HLA-L, ICOS, IDO1, IFI27, IFI27L2, IFI35, IFNG, IL12RB2, IL23A, IL26, IL27RA, IL2RB, IL2RG, IL32, IL6R, IRF1, IRF9, ITGA4, ITGAE, ITGAL, ITGB2, ITGB7, ITK, JAKMIP1, KIAA1324, KIF18A, KLRC2, KLRK1, L3MBTL4, LAP3, LAT, LCK, LCP2, LGALS2, LGR5, MIA2, NAGK, NFATC2, PAQR8, PARP12, PARP14, PARP9, PDCD1LG2, PLA2G12B, PLXNC1, PRKCQ, PSMB10, PSMB8, PSMB9, PSME1, PSME2, PTPN22, PTPRJ, PTTG1, PYCARD, PYHIN1, RABGAP1L, RARRES3, RASGRP1, RFX5, RGS10, RNF167, RNF19B, RNF213, RTP4, RUNX3, SAMD9L, SEMA4D, SEPT1, SH2D1A, SH2D2A, SKAP1, SLA2, SLAMF8, SLC35D2, STAT1, STAT4, TAP1, TAP2, TAPBP, TARP, TCID-7896691, TCID-7896701, TCID-7896702, TCID-7896719, TCID-7896720, TCID-7917530, TCID-7948502, TCID-8117739, TCID-8163000, TDRKH, THEMIS, TLR3, TLR8, TNF, TRAFD1, TRAT1, TRAV8-3, TRDV2, TRERF1, TUBA4A, UBASH3A, UBD, UBE2L6, VAMP5, WARS, XCL1, ZAP70, ZNF683, ZNF831.

### IPF vs. ILD Inflammation biomarkers

Figures 34A and 34B provide biomarkers useful in classification of IPF vs. ILD inflammation. As indicated by an "x" on the table presented in Figures 34A and 34B, some biomarkers were useful for classification using any number of classification methods and leave-one-patient-out (LOPO) cross-validation, and/or a 10-fold cross-validation (10-Fold), and/or any other classification and cross-validation method (Any).

A set of 250 differentially expressed (overexpressed; underexpressed) biomarkers which satisfied analytical criteria indicative of usefulness in classification of IPF vs. ILD Inflammation using Microarray data are shown below. Log2 fold changes of genes overexpressed in IPF (n=100) range from 0.20 to 1.30. Log2 fold changes of genes under-expressed in IPF (n=150) range from -2.46 to -0.18. The FDR p-value range for all 250 genes in this analysis is 3.48E-18 to 4.05E-07. If HUGO gene symbols were not available, the Affymetrix transcript cluster identifier (TCID) was given to specify the gene.

**Overexpressed:** ABCA6, ACTA2, ACTG2, ADCY5, AHNAK2, ANKRD20A8P, ANO2, ARHGEF25, ATP1A2, C16orf45, CALD1, CAMK2N1, CNN1, COL21A1, CRYAB, CSRP1, CTTNBP2, DES, DGKG, DIXDC1, DMD, DST, DTNA, DZIP1, EDIL3, FAM124B, FAM13C, FHL1, GARNL3, HPSE2, HSPA2, KALRN, LAMA4, LAMC1, LDB3, LMOD1, LPP, MAP1B, MAP7D3, MARK1, MCAM, MEIS1, MPDZ, MRVI1, MSRB3, MUSTN1, MXRA7, MYH10, MYH11, MYL9, MYLK, MYOCD, MYOM1, NEXN, NFASC, NTRK3, PALLD, PBX1, PCDHB10, PCDHB13, PCDHGC5, PDE1C, PDE5A, PDLIM3, PGM5, PGM5P2, PKD2, PLA2R1, PLCB4, PLN, PPP1R3C, PREX2, PRKAA2, PRKG1, PRUNE2, PTCHD4, PWAR5, RAB9B, SBSPON, SCRG1, SNORD116-21, SORBS1, STON1-GTF2A1L, SYNM, SYNPO2, SYTL4, TAGLN, TCEAL4, TCEAL6, TCID-7919139, TIMP2, TIMP3, TMEM47, TMOD1, TPM1, TPM2, TRPC1, TTLL7, ZC3H12B, ZNF154.

**Underexpressed:** ACSF2, ANKRD22, AP1M2, APOBEC3F, APOBEC3G, APOL1, APOL4, APOL6, ARHGEF38, ASPM, ATP2C2, ATP8A1, BATF2, BST2, BTN3A3, BUB1, C18orf8, C2, CCL4, CCL4L1, CCL5, CCR5, CD2, CD274, CD38, CD74, CD8A, CDK1, CDKL2, CEACAM1, CFTR, CKS2, CNDP2, CRTAM, CTLA4, CXCL10, CXCL11, CXCL9, CXCR6, DDX60, DRAM1, DUSP16, EFNA1, EPSTI1, ESRP2, ETV7, FAM26F, FHDC1, FPR3, FZD5, GBP1, GBP4, GBP5, GCH1, GPR98, GRAMD1A, GUCD1, GZMB, GZMH, HAS3, HCP5, HIST1H2AI, HIST1H2BH, HIST1H2BI, HIST1H3B, HIST1H3J, HLA-A, HLA-B, HLA-C, HLA-DMA, HLA-DOA, HLA-DPB1, HLA-E, HLA-F, HLA-G, HLA-H, HLA-J, HLA-L, HN1, IFI35, IFIH1, IFNG, IL32, IL6R, IRF1, IRF9, ITGAE, ITGAL, KCNJ15, KCTD14, KIF18A, LAP3, LARP4B, LCP2, LGALS3BP, MAP3K13, MOV10, MREG, NUSAP1, OAS2, PARP12, PARP14, PARP9, PFKFB3, PRKCQ, PSMB10, PSMB8, PSMB9, PSME1, PSME2, PTPRJ, PTTG1, PXMP4, RABGAP1L, RARRES3, RASGRP1, RBM47, RFX5, RGS16, RNF213, SAMD9L, SCTR, SDC4, SEMA4A, SFTPD, SLAMF8, SLC37A1, SMC4, SQRDL, SREBF2, STAT1, TAP1, TAP2, TAPBP, TCID-7896714, TCID-8107094, THEMIS, TLR2, TLR3, TMEM164, TNFAIP3, TOX, TRAFD1, TRIM59, TYMP, UBD, UBE2L6, USP44, WARS, ZNF385B.

**Table 10** presents a summary of exemplary biomarkers useful in classification of ILD subtypes using microarray data.

**Table 10**

| | **Number of significant biomarkers detected per analytical approach** | | | **Listed in:** |
|---|---|---|---|---|
| **Analysis** | **LOPO Classifier** | **10-Fold Classifier** | **Combined Biomarkers** | |
| IPF vs. NSIP | 9 | 15 | 17 | Table 8 |
| IPF vs. NonIPF/Rest | 80 | 100 | 111 | Figures 33A-B |
| IPF vs. HP | 4 | 3 | 4 | Table 9 |
| IPF vs. Inflammation | 50 | 130 | 130 | Figures 34A and 34B |

While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

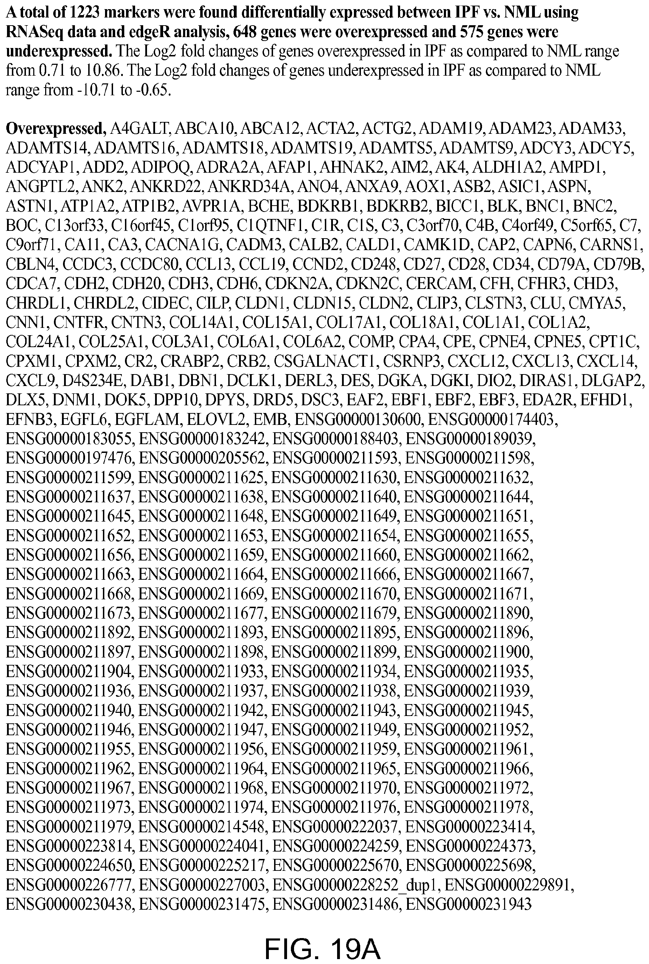

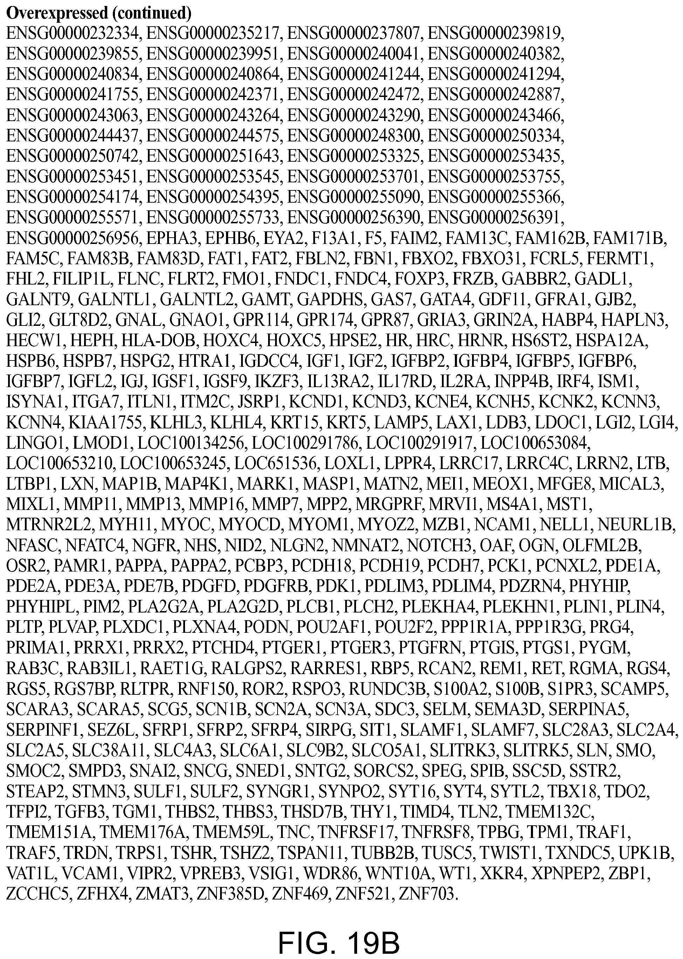

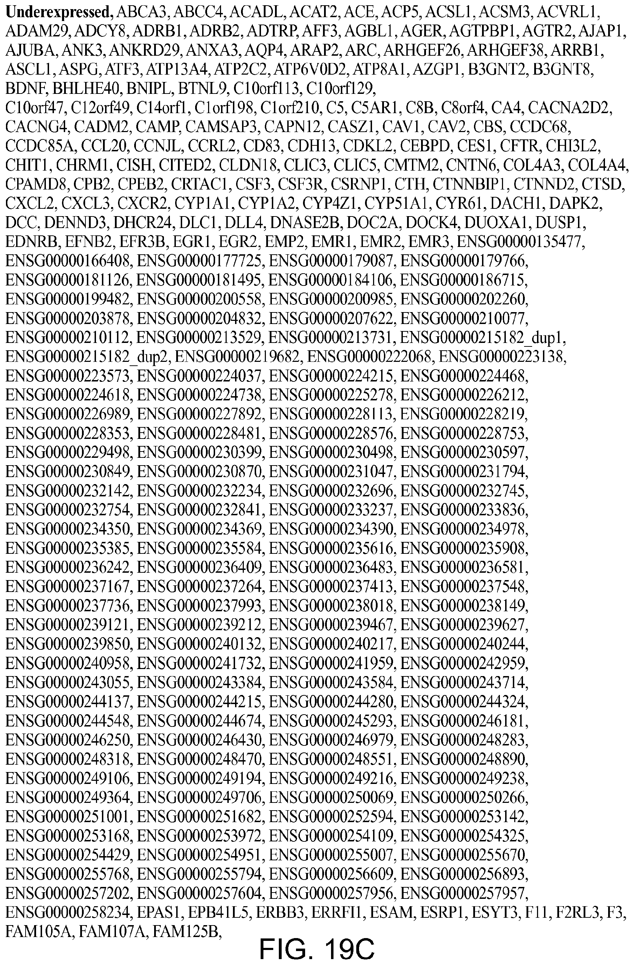

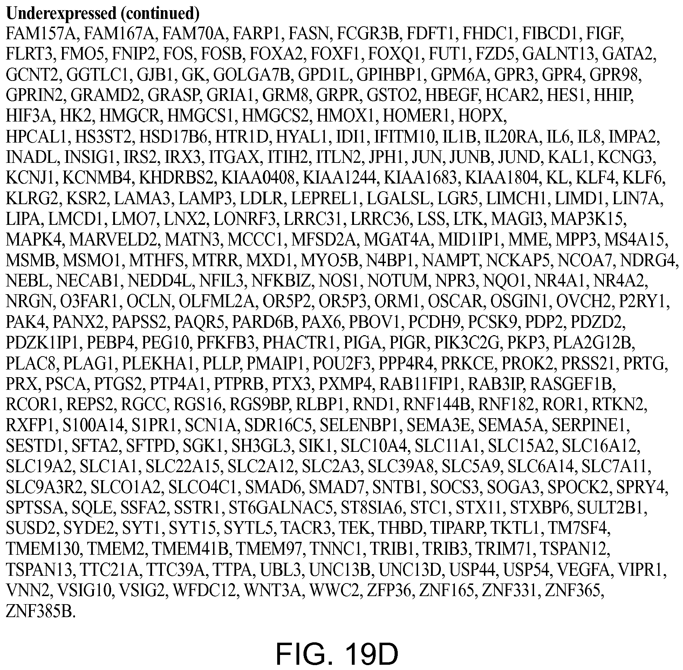

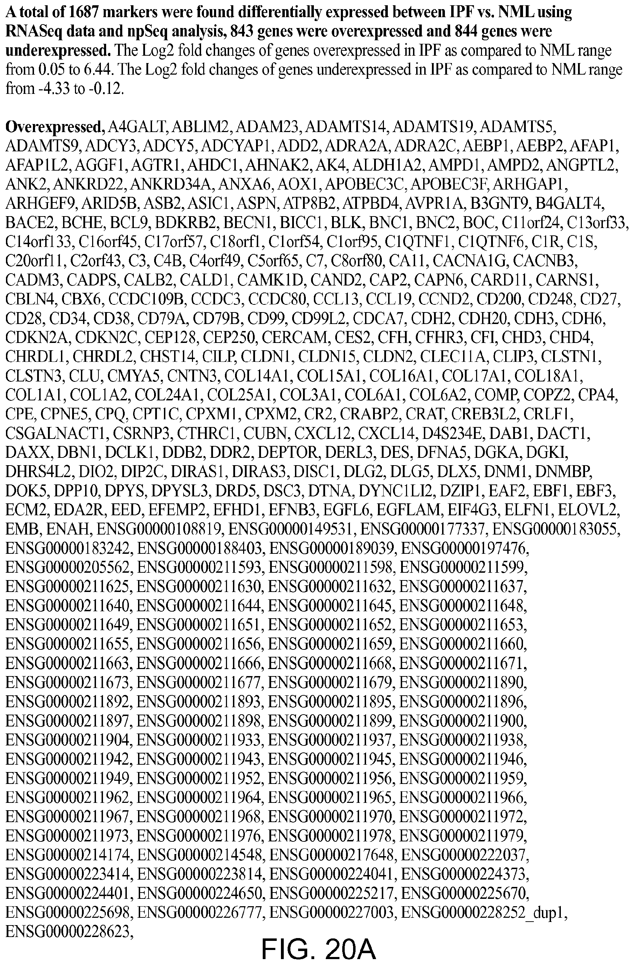

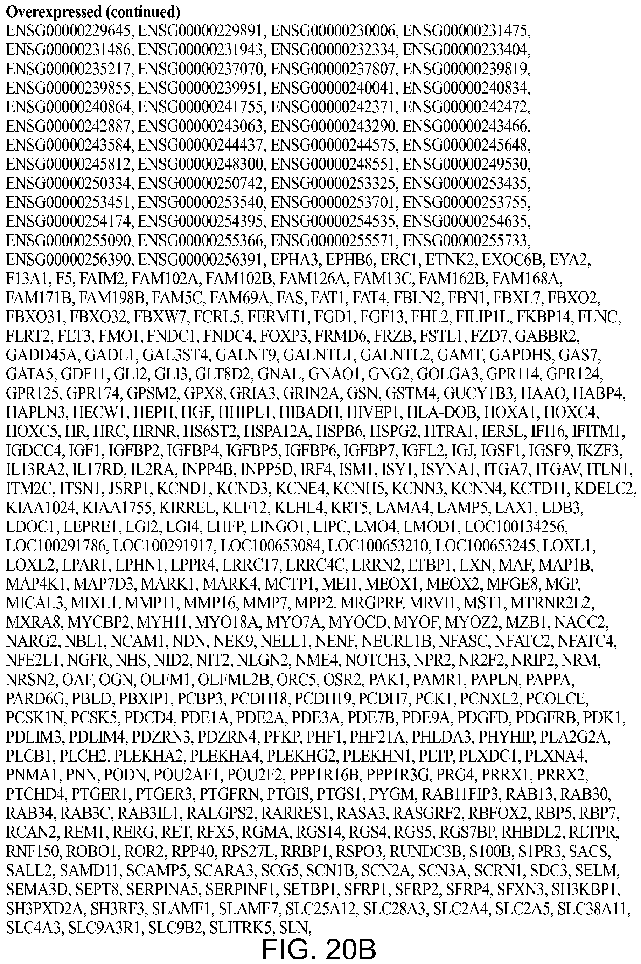

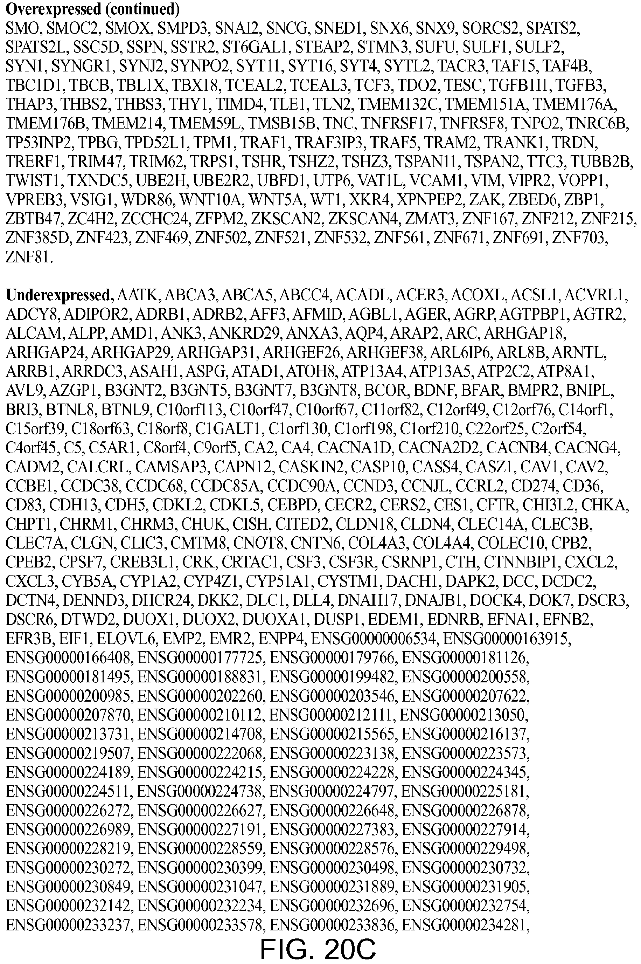

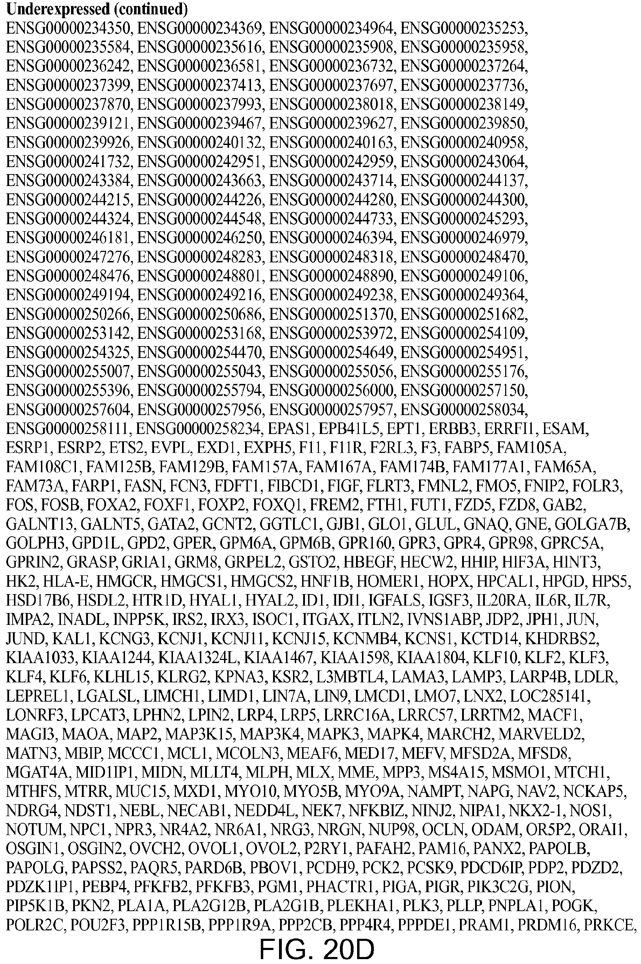

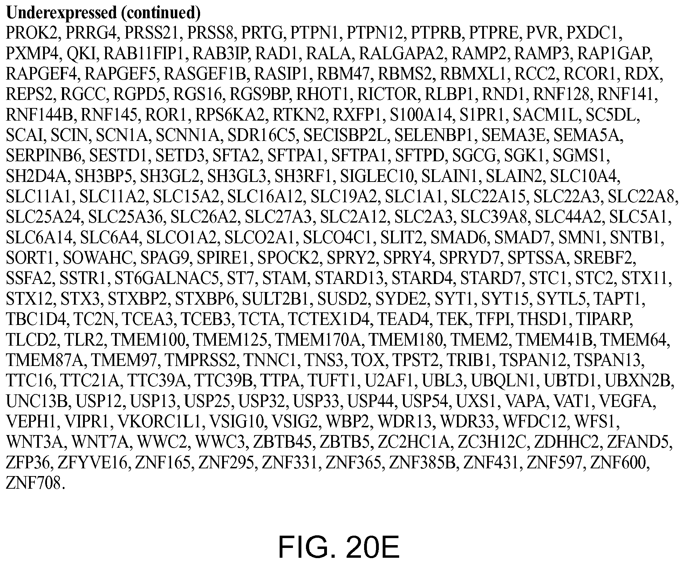

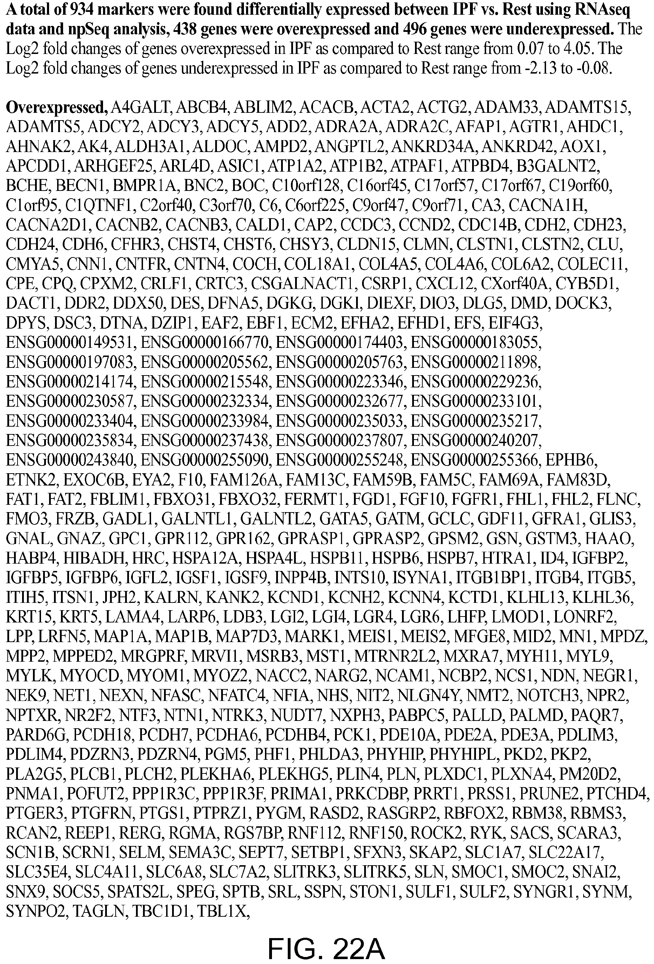

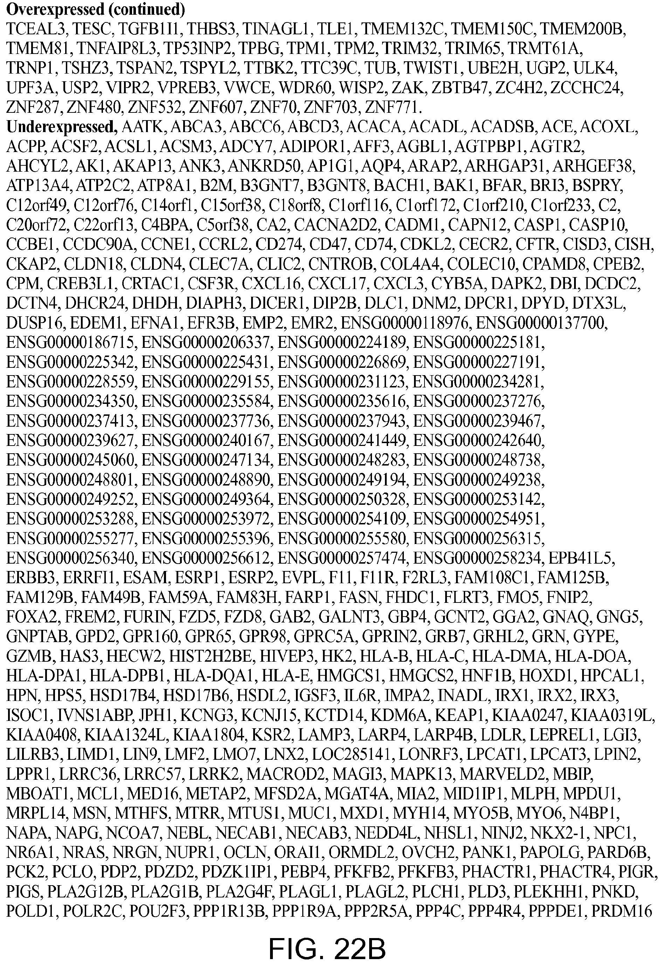

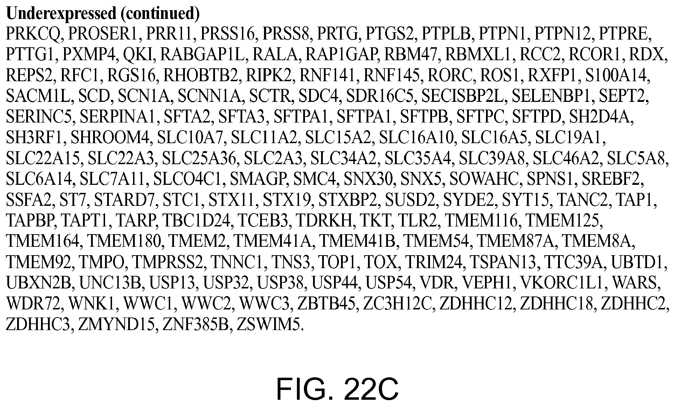

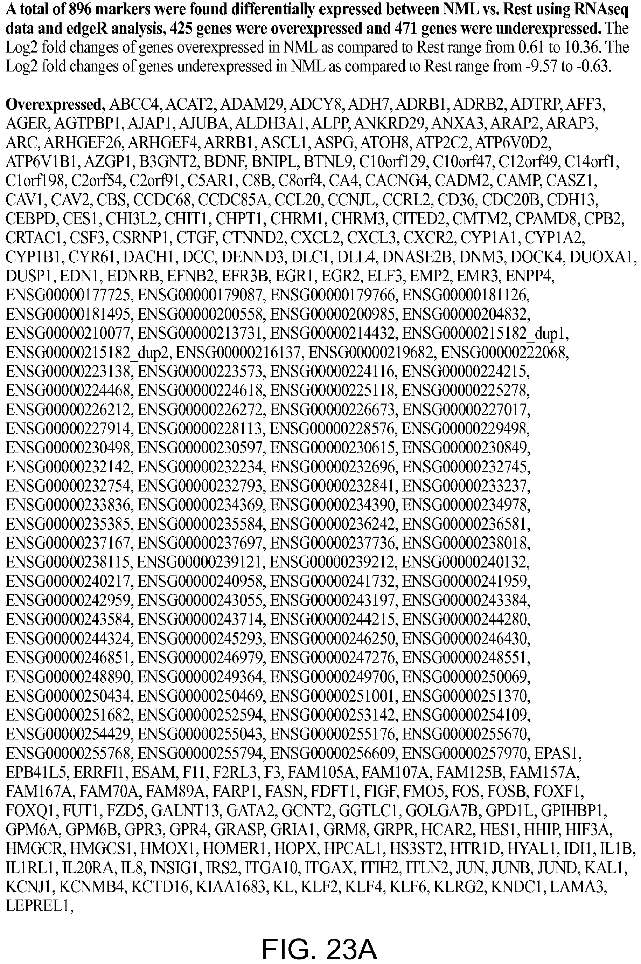

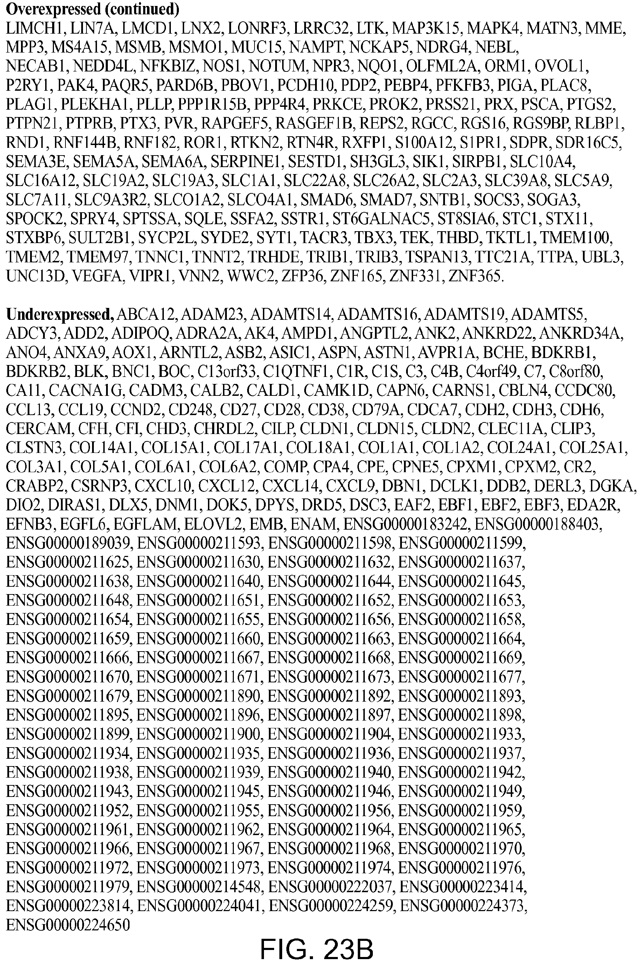

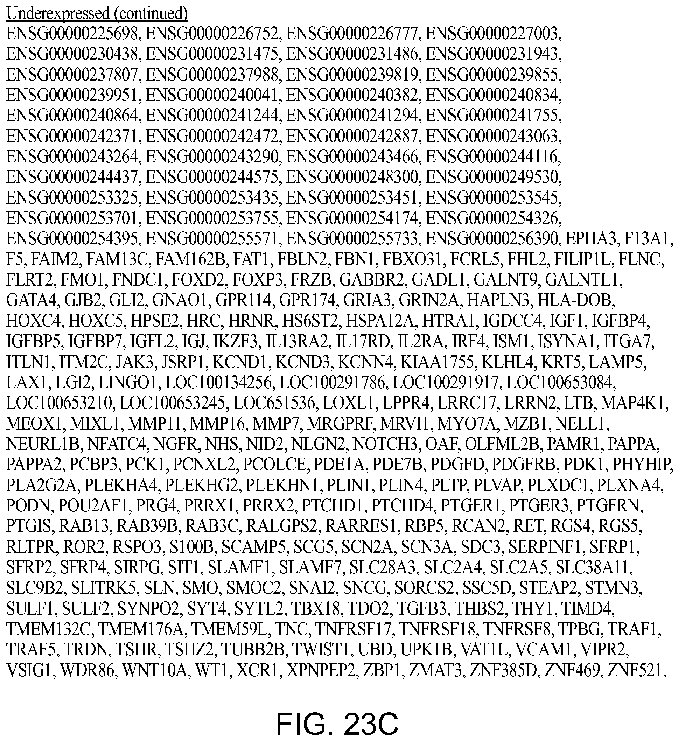

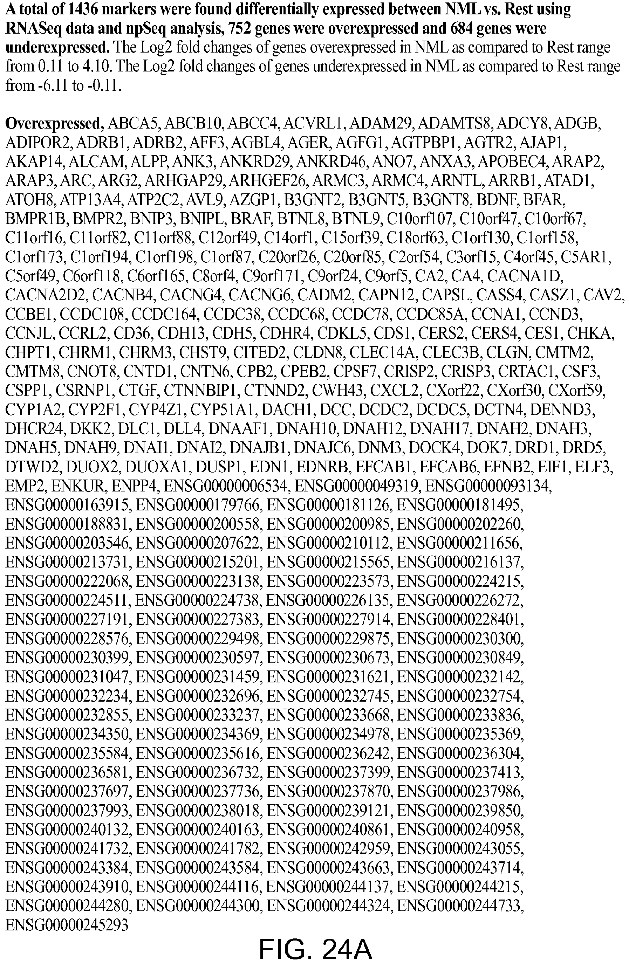

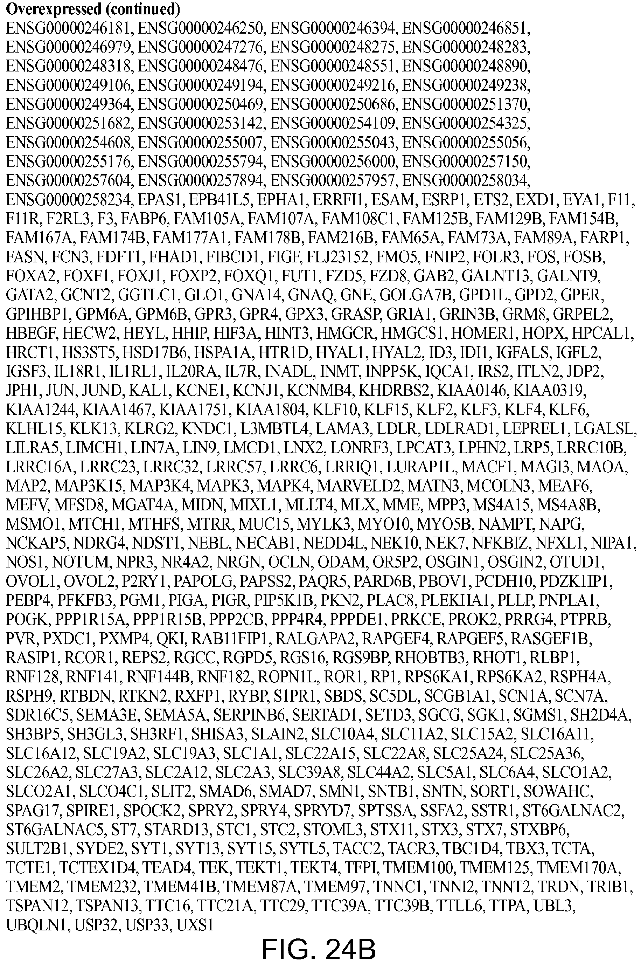

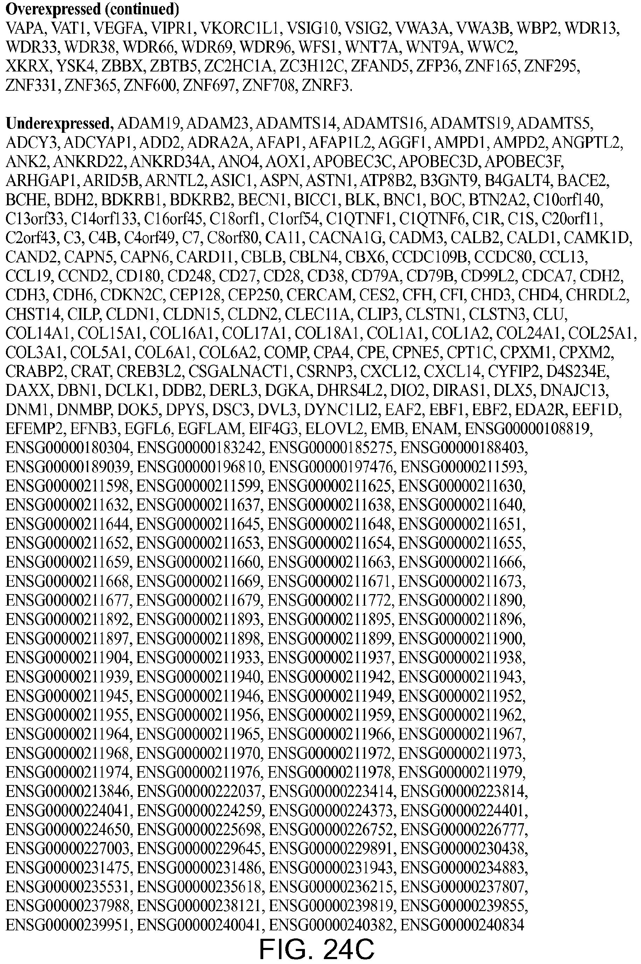

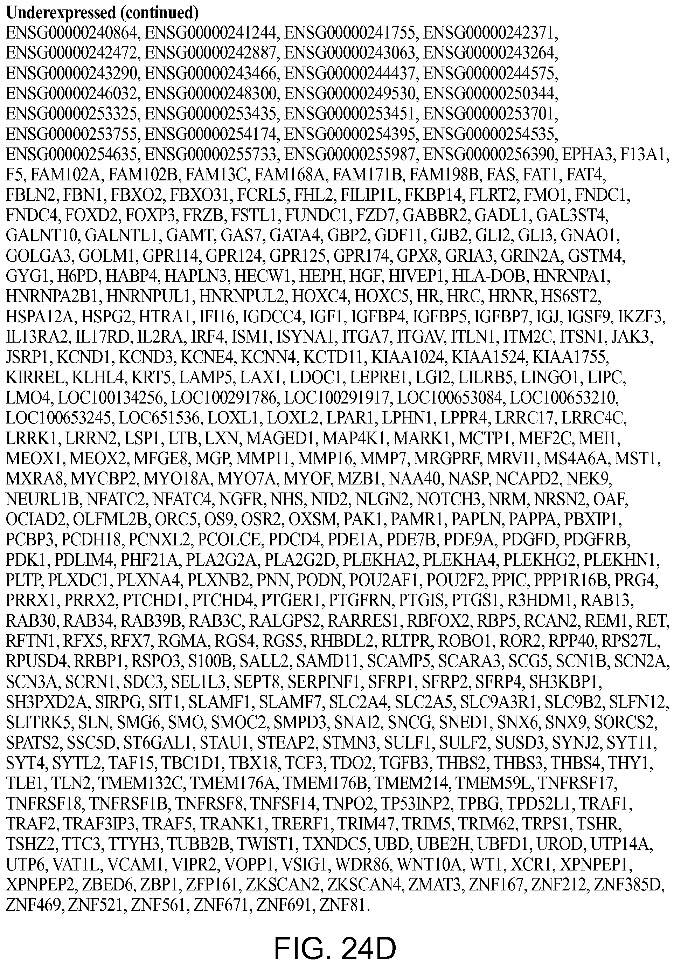

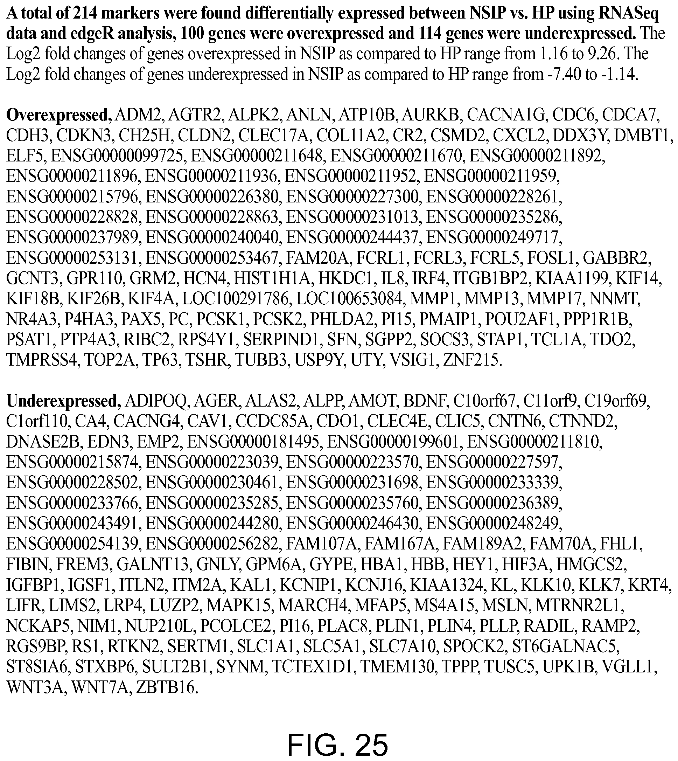

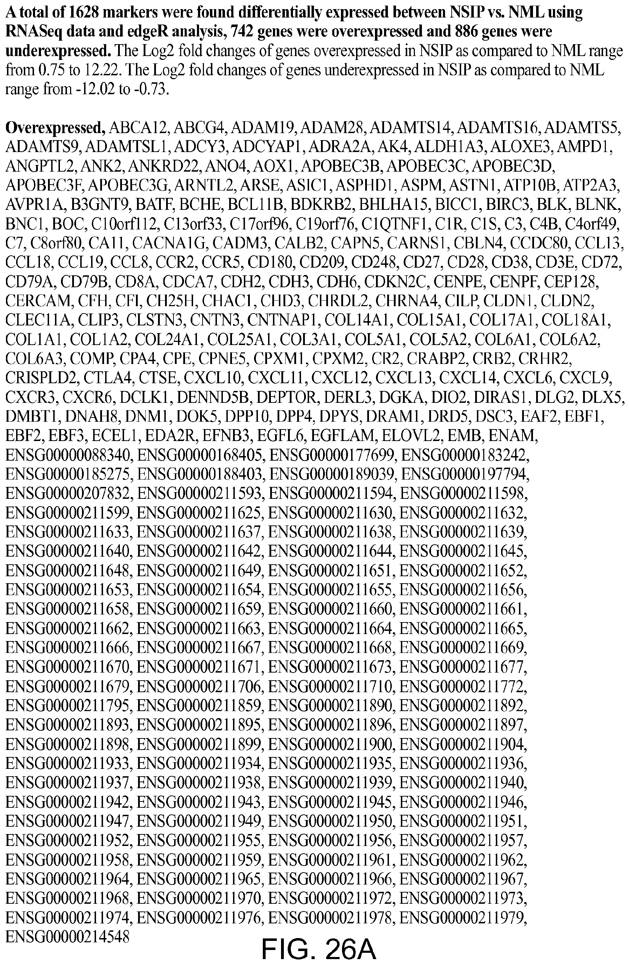

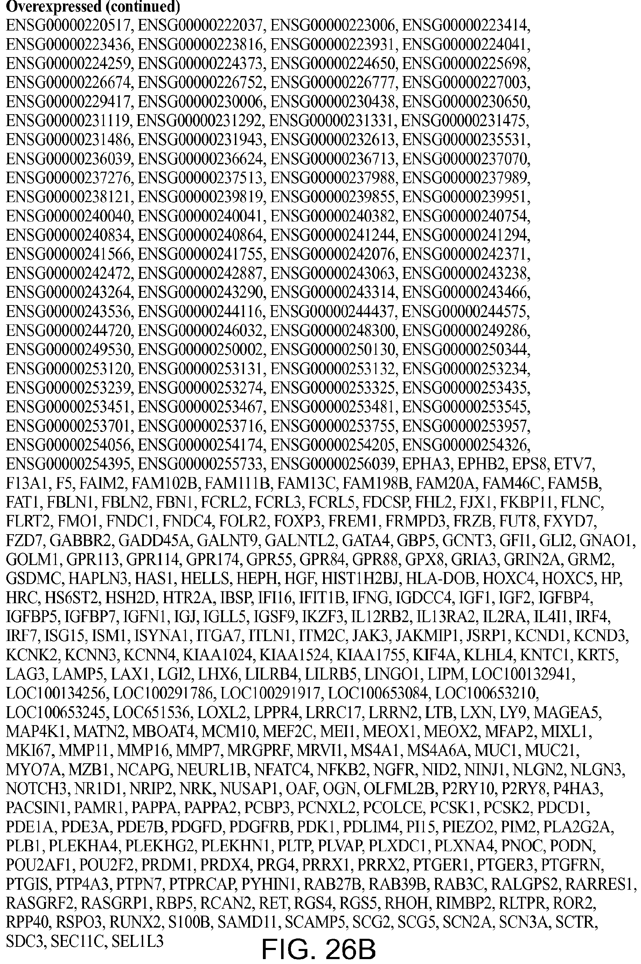

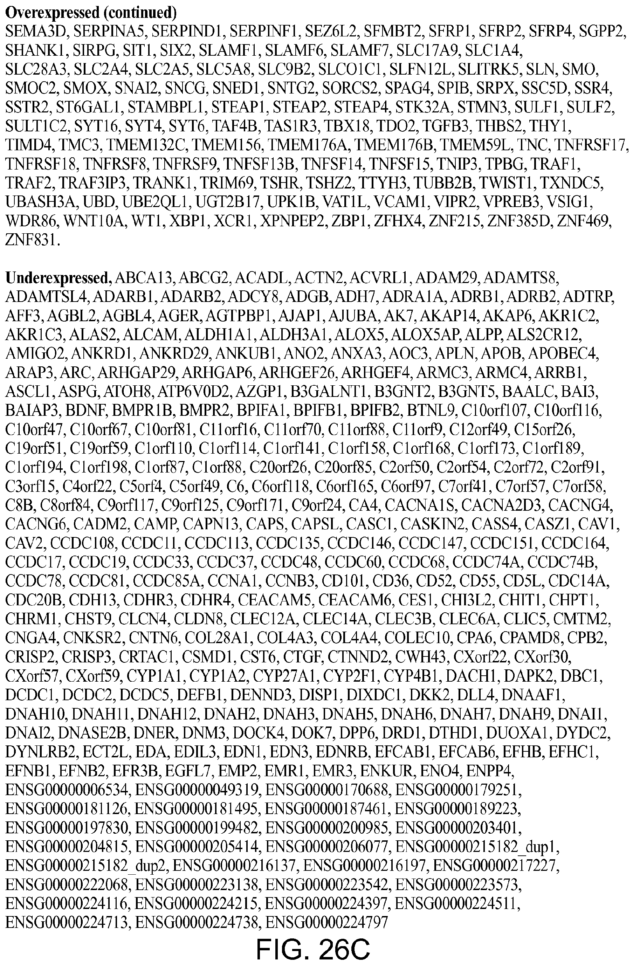

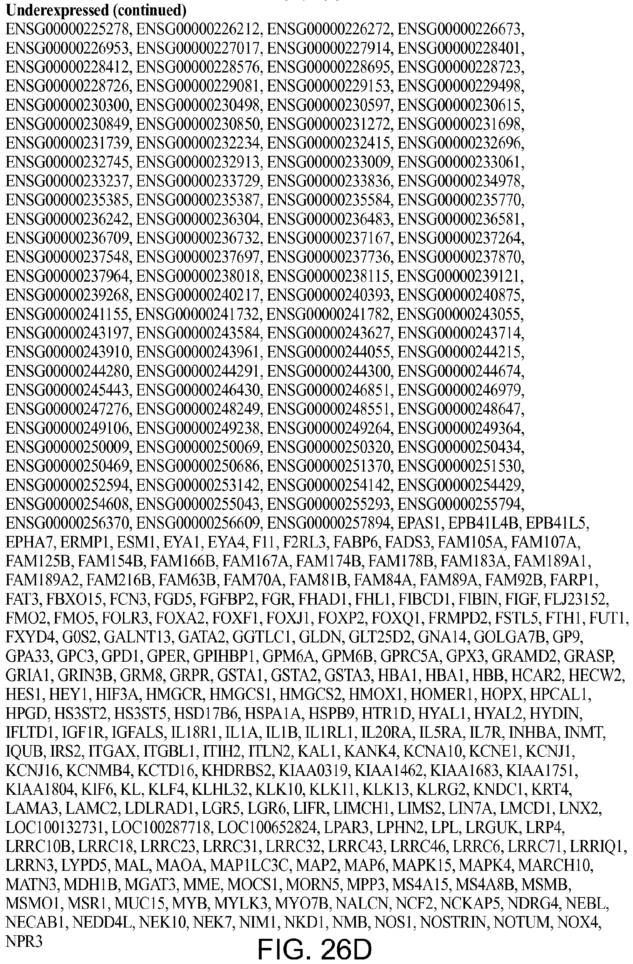

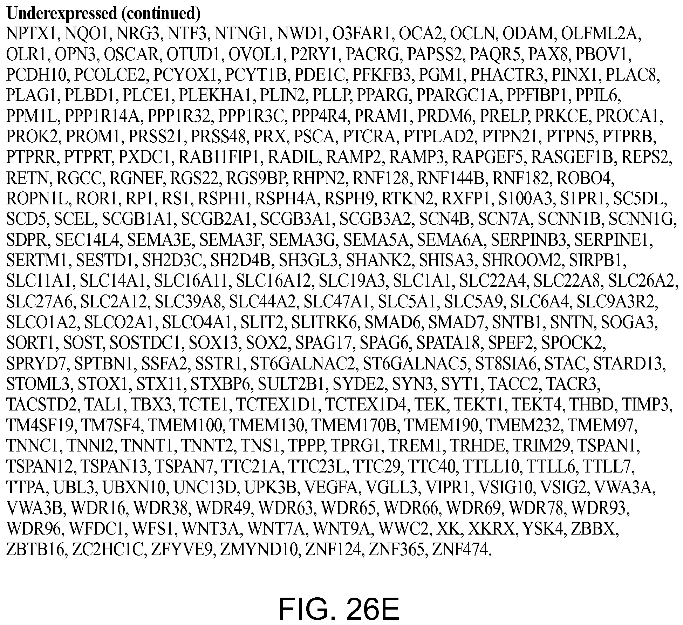

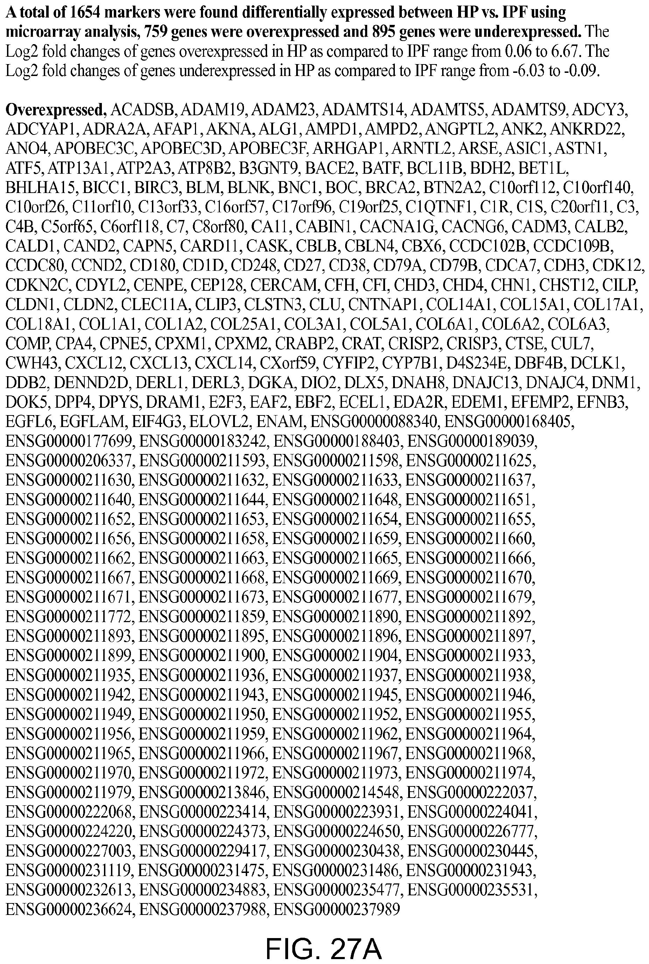

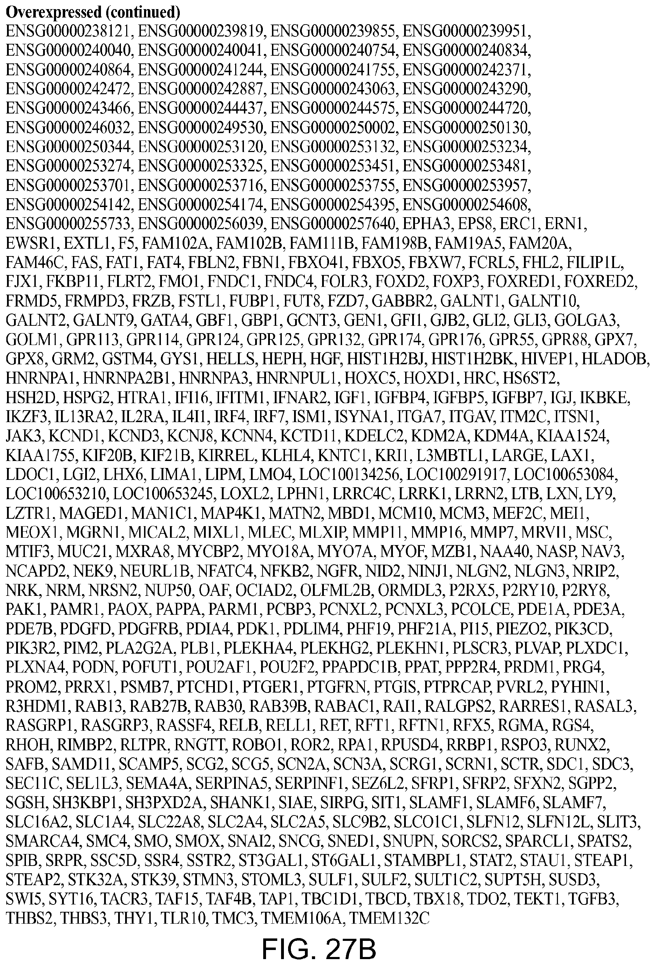

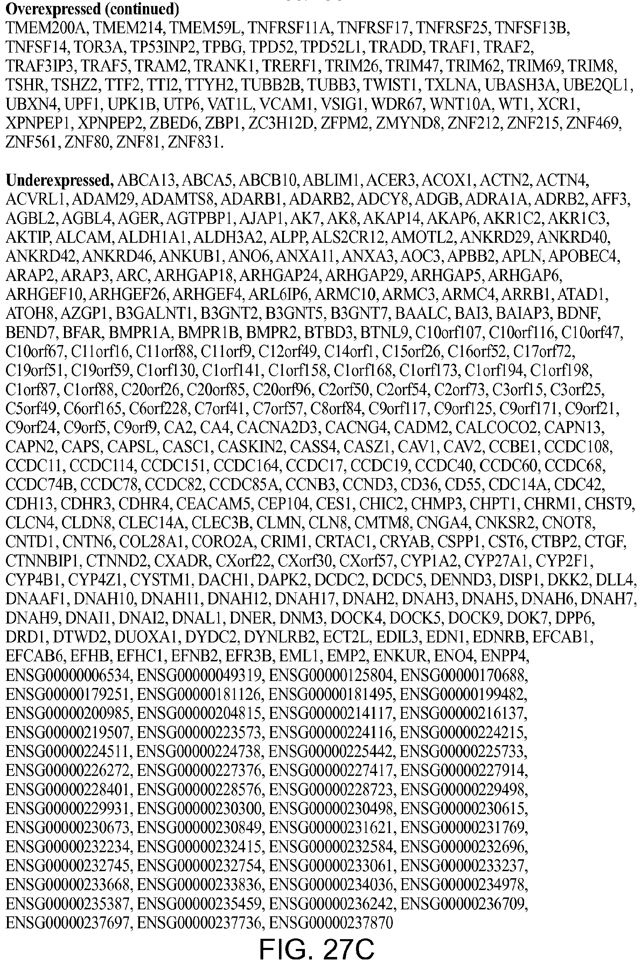

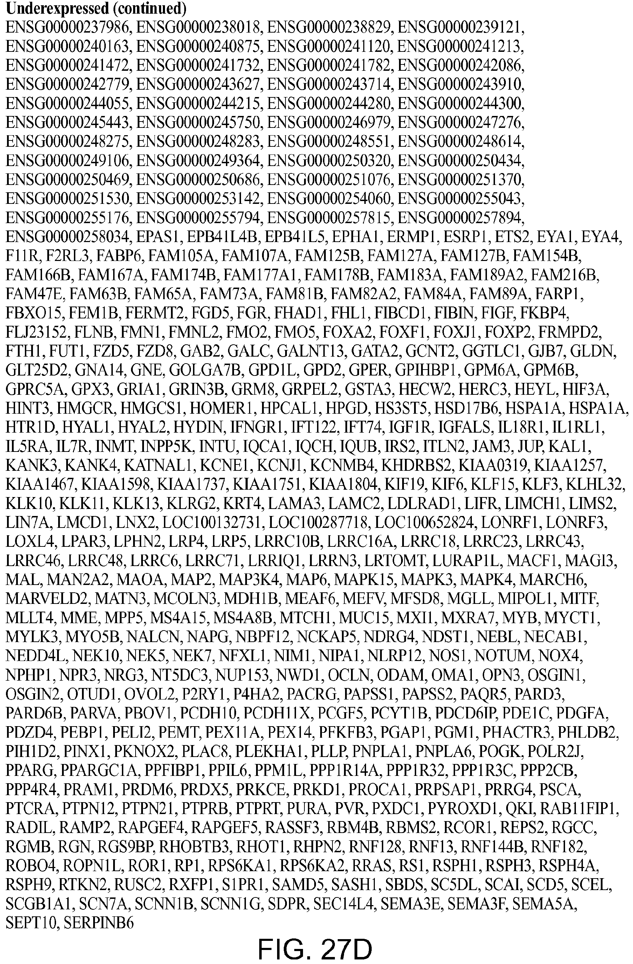

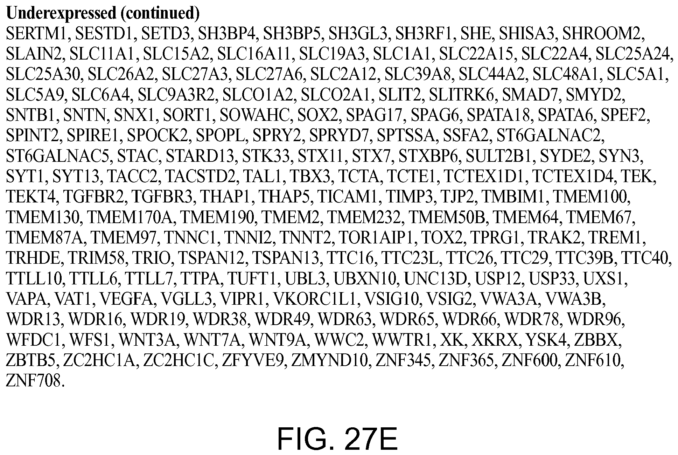

The invention discloses the following items 1 to 58.
1. A method for evaluating a lung tissue, the method comprising:
   a) determining an expression level of a gene product of one or more genes set forth in any of Figures 6A-27E, 33A-B, or 34A-B, or in any of Tables 3, 5, 8, and 9 in a lung tissue sample obtained from a patient, generating an expression level value; and
   b) classifying the lung tissue sample as an interstitial lung disease (ILD) tissue sample by comparing the expression level value to a reference expression level value.
2. A method of diagnosing an interstitial lung disease (ILD) in a patient, the method comprising:
   a) determining an expression level of a gene product of one or more genes set forth in any one of Figures 6A-27E, 33A-B, or 34A-B, or in any of Tables 3, 5, 8, and 9 in a lung tissue sample obtained from a patient suspected of having an ILD; and
   b) providing a diagnosis of an ILD based on comparison of the expression level value to a reference expression level value.
3. A method of diagnosing an interstitial lung disease (ILD) in patient, the method comprising:
   a) assaying, in a tissue sample obtained from a patient suspected of having an ILD, an expression level of a gene product of one or more genes set forth in any one of Figures 6A-27E, 33A-B, or 34A-B, or in any of Tables 3, 5, 8, and 9, generating an expression level value;
   b) identifying the patient as having an ILD when the expression level value differs significantly from a reference gene expression level value; and
   c) outputting a report indicating that the patient has an ILD based on said identifying to facilitate a treatment decision by a clinician.
4. A method of diagnosing an interstitial lung disease (ILD), the method comprising:
   a) assaying, in a tissue sample obtained from a patient suspected of having an ILD, an expression level of a gene product of one or more genes set forth in any one of Figures 6A-27E, 33A-B, or 34A-B, or in any of Tables 3, 5, 8, and 9, generating an expression level value; and
   b) inputting the expression level value into a computer programmed to execute an algorithm that compares the expression level value to expression level value for a reference expression level value and determines whether the expression level value differs significantly from a reference expression level value, said inputting generating a result from execution of the algorithm; and
   c) generating a report providing the result.
5. A method of diagnosing idiopathic pulmonary fibrosis (IPF) in a patient, the method comprising:
   a) assaying, in a tissue sample obtained from a patient suspected of having an interstitial lung disease, an expression level of a gene product of one or more genes set forth in any one of Figures 9A-9D, 10A-10G, 19A-22C, and 33A-B, or Table 4, generating an expression level value;
   b) identifying the patient as having IPF when the expression level value differs significantly from a reference expression level value.
6. The method of any one of items 1-4, wherein the ILD is idiopathic pulmonary fibrosis, sarcoidosis, Hamman-Rich syndrome, antisynthetase syndrome, silicosis, asbestosis, berylliosis, hypersensitivity pneumonitis, or non-specific interstitial pneumonia.
7. The method of any one of items 1-4, wherein the ILD is associated with a connective tissue disease selected from systemic sclerosis, polymyositis, systemic lupus erythematosus, or rheumatoid arthritis.
8. The method of any one of items 1-4, wherein the ILD is drug induced.
9. The method of any one of items 1-4, wherein the ILD results from a viral infection, a bacterial infection, or tuberculosis.
10. The method of any one of items 1-5, wherein the diagnosis provides for differentiation between idiopathic pulmonary fibrosis (IPF) and an ILD other than IPF.
11. The method of any one of items 1-5, wherein said one or more genes is selected from 1) ASPM; 2) BUB1; 3) PTTG1; 4) SHCBP1; 5) NUSAP1; 6) MKI67; 7) HJURP; 8) CDCA3; 9) PLK1; 10) PRR11; 11) BRCA2; 12) ORM1 ; 13) CCNB2; 14) SMC4; 15) HM13; 16) DMD; 17) FHL1; 18) ORM2; 19) NDUFC2-KCTD14; 20) NCAPH; 21) TTLL7; 22) DEPDC1B; 23) CNTN4; 24) PRKAA2; 25) PRKCQ; 26) CDC42BPA; 27) PARD3B; 28) SCTR; 29) CSF3R; and 30) MPDZ.
12. The method of any one of items 1-5, further comprising the step of creating a report summarizing said diagnosis.
13. The method of any one of items 1-5, further comprising providing a recommendation for treatment of the ILD.
14. The method of any one of items 1-5, wherein said assaying comprises assaying an expression level of a gene product selected from 1) ASPM; 2) BUB1; 3) PTTG1; 4) SHCBP1; 5) NUSAP1; 6) MKI67; 7) HJURP; 8) CDCA3; 9) PLK1; 10) PRR11; 11) BRCA2; 12) ORM1; 13) CCNB2; 14) SMC4; 15) HM13; 16) DMD; 17) FHL1; 18) ORM2; 19) NDUFC2-KCTD14; 20) NCAPH; 21) TTLL7; 22) DEPDC1B; 23) CNTN4; 24) PRKAA2; 25) PRKCQ; 26) CDC42BPA; 27) PARD3B; 28) SCTR; 29) CSF3R; and 30) MPDZ.
15. The method of item 4, wherein said inputting provides for differential diagnosis of idiopathic pulmonary fibrosis versus non-specific interstitial pneumonia.
16. The method of item 4, wherein said assaying comprises assaying an expression level of a gene product selected from 1) ASPM; 2) BUB I; 3) PTTG1 ; 4) SHCBP1; 5) NUSAP1; 6) MKI67; 7) HJURP; 8) CDCA3; 9) PLK1; 10) PRR11; 11) BRCA2; 12) ORM1; 13) CCNB2; 14) SMC4; 15) HM13; 16) DMD; 17) FHL1; 18) ORM2; 19) NDUFC2-KCID14; 20) NCAPH; 21) TTLL7; 22) DEPDC1B; 23) CNTN4; 24) PRKAA2; 25) PRKCQ; 26) CDC42BPA; 27) PARD3B; 28) SCTR; 29) CSF3R; and 30) MPDZ.
17. The method of item 4, wherein said inputting provides for differential diagnosis of idiopathic pulmonary fibrosis versus hypersensitivity pneumonia.
18. The method of item 4, wherein said inputting provides for a diagnosis of hypersensitivity pneumonia.
19. The method of item 4, wherein the report includes a treatment recommendation based on the result.
20. The method of any one of items 1-5, wherein the gene product is mRNA.
21. The method of any one of items 1-5, wherein the normalized expression level is obtained by comparing the expression level of a gene product to gene expression data for at least two different sets of biomarkers, the gene expression data for each set of biomarkers comprising one or more reference gene expression levels correlated with the presence of one or more tissue types, wherein said expression level is compared to gene expression data for said at least two sets of biomarkers sequentially.
22. The method of item 21, wherein said sequential comparison ends with comparing said expression level to gene expression data for a final set of biomarkers by analyzing said expression level using a main classifier, said main classifier obtained from gene expression data from one or more sets of biomarkers.
23. The method of any one of items 1-5, wherein said assaying comprises determining a level of mRNA using microarray, serial analysis of gene expression, blotting, reverse transcription-polymerase chain reaction, sequencing, or quantitative polymerase chain reaction.
24. The method of any one of items 1-5, wherein said normalized expression level is normalized relative to the expression level of an RNA transcript of at least one reference gene.
25. The method of any one of items 1-5, wherein said one or more genes is selected from:
   a) ABCA6, ABLIM3, ACTA2, ACTG2, ADCY5, AHNAK2, AKAP12, ANO1, ANO2, ARHGEF25, ATP1A2, ATP1B2, BAG2, BHMT2, BMX, Cl6orf45, C3orf55, C3orf70, CACNA1C, CACNB2, CALD1, CAMK2N1, CCDC3, CDH6, CDR1, CLIP3, CLU, CNN1, COCH, COL21A1, COPZ2, COX7A1, CRYAB, CSRP1, CTTNBP2, DACT1, DDR2, DENND2A, DES, DIXDC1, DMD, DST, DTNA, DYNC1I1, DZIP1, EBF1, EDIL3, EFHD1, Fl0, FAM124B, FAM13C, FBLIMI, FEZ1, FGF10, FGF14, FGF2, FGFR1, FHL1, FHL2, FLNC, GADL1, GARNL3, GAS7, GNG12, GRIA3, GRIN2A, HOXA2, HOXA6, HOXA7, HPSE2, HRC, HRH1, HSPA2, HSPB6, HSPB7, IGFBP5, ITGB4, ITIH5, KALRN, LAMA4, LAMC1, LARP6, LDB3, LGI4, LMOD1, LPP, LTBP1, MAP1B, MAP7D3, MARK1, MCAM, MEIS1, MEOX2, MPDZ, MRGPRF, MRVI1, MSRB3, MUSTN1, MXRA7, MYH11, MYL9, MYLK, MYOCD, MYOM1, NCAM1, NEXN, NFASC, NPR2, NR2F1, NR2F2, NTRK3, OSR2, PALLD, PALMD, PARVA, PBX1, PCDH7, PCDHB10, PCDHB12, PCDHB13, PCDHB14, PCDHB18, PCDHB3, PCDHB7, PCDHGC5, PDE10A, PDE1A, PDE2A, PDE3A, PDE5A, PDE7B, PDGFRB, PDLIM3, PDZRN4, PEG3, PFKM, PGM5, PGM5P2, PHYHIPL, PKD2, PLA2R1, PLCB4, PLN, PLXNA4, PODN, PPP1R3C, PRKAA2, PRKG1 , PRSS23, PRUNE2, PTCHD4, PYGM, RAB9B, RASD2, RBM24, RCAN2, RERG, RGS5, RGS7BP, RYR3, S1PR3, SBSPON, SCRG1, SCUBE1, SEMA3D, SETBP1, SGCA, SLC38A11, SLIT3, SLN, SMIM10, SNED1, SNORD116-17, SNORD116-21, SNX9, SOGA1, SORBS1, SPARCL1, SSPN, SYNM, SYNPO2, SYTL2, TAGLN, TCEAL4, TCEAL6, TIMP2, TMEM252, TMEM47, TMOD1, TPM1, TPM2, TRPC1, TTLL7, WISP2, ZAK, ZC3H12B, ZCCHC24, ZEB2, ZHX3, ZNF135, ZNF154, and ZNF521;
   b) ACADL, ACOXL, ACSF2, AHCYL2, ANK3, AP1M2, APOL1, APOL4, APOL6, AQP4, ARAP2, ARHGEF38, ARSD, ASPM, ATP1A1, ATP2C2, ATP8A1, BATF2, BAZ1A, BMP3, BST2, BTG3, BTN3A3, BUB1, C18orf8, C2, CASP1, CASP4, CCL4, CCL4L1, CCL5, CCNA2, CCR5, CD274, CD40, CD47, CD74, CD8A, CDCA7L, CDK1, CDKL2, CEACAM1, CHR, CKAP2, CLDN4, CLINT1, CNDP2, CNKSR1, CRTAC1, CRTAM, CSF3R, CXCL10, CXCL11, CXCL9, CXCR6, CYB5A, CYP2B6, CYP2B7P1, DAPK1, DBI, DDX60, DENND1B, DHCR24, DNASE2, DOCK8, DRAM1, DTNB, DTX3L, DUSP16, DYNLT1 , EDEM1, EFNA1, EML4, ENTPD3, EPSTI1, ERAP1, ERBB3, ESRP1, ESRP2, ETV7, FAM111A, FCGR1A, FCGR1B, FDFT1, FGFR2, FHDC1, FLRT3, FNIP2, FREM2, FZD5, GALNT3, GBPI, GBP4, GBP5, GBP7, GCH1, GCNT2, GK, GNPTAB, GPR98, GRAMD1B, GRHL2, GSAP, GUCD1, GZMB, GZMH, HAS3, HCP5, HIST1H2AI, HIST1H2BI, HIST1H3B, HLA-A, HLA-B, HLA-C, HLA-DMA, HLA-DOA, HLA-DPA1, HLA-DPB1, HLA-DQA2, HLA-DRA, HLA-E, HLA-F, HLA-G, HLA-H, HLA-J, HLA-L, HMGCL, HMGCR, HMMR, HN1, HNF1B, HPS5, ICOS, IDI1, IDO1, IFI27, IFI35, IFIH1, IFIT3, IFIT5, IGSF3, IL10RB, IL32, IL6R, INADL, IRF1, IRF9, ITGAE, ITGAL, JPH1, KCNJ15, KCTD14, KIAA0247, KIAA0319L, KIAA1958, KIF18A, KLFS, L3MBTL4, LAMP3, LAP3, LARP4B, LCP2, LIMD1, LNX2, LPIN2, MAGI3, MAP3K13, MAPK13, MARK2, MB21D1, MBIP, MIA2, MICALCL, MID1IPI, MREG, MRPL14, MTMR14, MTRR, MYL1, MYOSB, N4BP1, NAV2, NEDD4L, NMI, NPC1, NUB1, NUSAP1, OAS2, OCLN, ORM1 , PARP12, PARP14, PARP9, PCK2, PCLO, PDZK1IP1, PEBP4, PFKFB3, PHACTR1, PI4K2B, PLA2G12B, PLAGL2, PLCH1, PLS1, PPP1R9A, PPP2R5A, PRKCQ, PRKCZ, PRRG4, PRSS8, PSMB10, PSMB8, PSMB9, PSME1, PSME2, PTP4A1, PTPN1, PTPRJ, PTTG1, PXMP4, RAB20, RAB31P, RABGAP1L, RANBP17, RARRES3, RBM47, RCOR1, RFC1, RFX5, RGS16, RNF19B, RNF213, S100A14, SAMD9L, SCN1A, SCTR, SDC4, SELENBP1, SEMA4A, SFTPD, SH2D4A, SHANK2, SLAMF8, SLC15A3, SLC16A13, SLC22A3, SLC37A1, SLCO4C1, SMARCAS, SMC4, SQLE, SQRDL, SREBF2, ST7, STARD10, STAT1, STX11, STX19, SYT15, TAP1, TAP2, TAPBP, TAPT1, TC2N, TCID-7895602, TCID-7896651, TCID-7896695, TCID-7951593, TCID-8113717, TDRKH, THEMIS, TKT, TLR2, TLR3, TMEM164, TMEM180, TMEM41B, TMEM87A, TMPRSS2, TMPRSS3, TNFAIP3, TOX, TRAFD1, TRIM21, TRIM24, TTLL4, UBALD2, UBD, UBE2L6, USP38, USP44, USP54, WARS, WHSC1, WWC1, ZDHHCI6, ZDHHC2, and ZNF385B; or
   c) a gene set forth in any one of Figures 9A-9D, 21A-21B, and 33A-B.
26. The method of any one of items 1-25, wherein the gene is set forth in Figures 6A-6B.
27. The method of any one of items 1-26, wherein the gene is set forth in Figure 7.
28. The method of any one of items 1-27, wherein the gene is set forth in Figure 8.
29. The method of any one of items 1-28, wherein the gene is set forth in Figures 9A-9D.
30. The method of any one of items 1-29, wherein the gene is set forth in Figures 10A-10G.
31. The method of any one of items 1-30, wherein the gene is set forth in Figures 11A-11F.
32. The method of any one of items 1-31, wherein the gene is set forth in Figure 12.
33. The method of any one of items 1-32, wherein the gene is set forth in Figures 13A-13F.
34. The method of any one of items 1-33, wherein the gene is set forth in Figure 14.
35. The method of any one of items 1-34, wherein the gene is set forth in Figures 15A-15C.
36. The method of any one of items 1-35, wherein the gene is set forth in Figure 16.
37. The method of any one of items 1-36, wherein the gene is set forth in Figure 17.
38. The method of any one of items 1-37, wherein the gene is set forth in Figure 18.
39. The method of any one of items 1-38, wherein the gene is set forth in Figures 19A-19D.
40. The method of any one of items 1-39, wherein the gene is set forth in Figures 20A-20E.
41. The method of any one of items 1-40, wherein the gene is set forth in Figures 21A-21B.
42. The method of any one of items 1-41, wherein the gene is set forth in Figures 22A-22C.
43. The method of any one of items 1-42, wherein the gene is set forth in Figures 23A-23C.
44. The method of any one of items 1-43, wherein the gene is set forth in Figures 24A-24D.
45. The method of any one of items 1-44, wherein the gene is set forth in Figure 25.
46. The method of any one of items 1-45, wherein the gene is set forth in Figures 26A-26E.
47. The method of any one of items 1-46, wherein the gene is set forth in Figures 27A-27E.
48. The method of any one of items 1-47, wherein the gene is set forth in Figures 33A-B.
49. The method of any one of items 1-48, wherein the gene is set forth in Figures 34A and 34B.
50. The method of any one of items 1-49, wherein the gene is set forth in Table 3.
51. The method of any one of items 1-50, wherein the gene is set forth in Table 5.
52. The method of any one of items 1-51, wherein the gene is set forth in Table 8.
53. The method of any one of items 1-52, wherein the gene is set forth in Table 9.
54. A method of modifying therapy of a patient, the method comprising:
   diagnosing an interstitial lung disease (ILD) in the patient, according to the method of any one of items 2-25; and
   modifying therapy in the patient according to said diagnosing.
55. A method of treating an interstitial lung disease (ILD) in an individual, the method comprising:
   diagnosing an interstitial lung disease (ILD) in the patient, according to the method of any one of items 2-25; and
   treating the individual for the LLD.
56. The method of item 55, wherein, if said diagnosing step indicates that the individual has idiopathic pulmonary fibrosis, said treating step comprises administering to the individual an effective amount of pirfenidone, prednisone, azathioprine, or N-acetylcysteine.
57. An array comprising a plurality of nucleic acids, each of which hybridizes to a gene differentially expressed in a cell present in a tissue sample obtained from an individual being tested for an interstitial lung disease.
58. A kit for analyzing a lung tissue sample, the kit comprising:
   an array according to item 57; and
   a reagent for analyzing an expression level of a gene product.

## Claims

1. A method of diagnosing idiopathic pulmonary fibrosis (IPF) in a patient, the method comprising:
a) assaying, in a tissue sample obtained from a patient suspected of having an interstitial lung disease, an expression level of a gene product of one or more genes selected from the group consisting of FAM13C, PDE7B, SULF1, ERRFI1 or any other gene set forth in Table 4, generating an expression level value;
b) identifying the patient as having IPF when the expression level value differs significantly from a reference expression level value.

2. The method of claim 1, wherein the diagnosing provides for differentiation between idiopathic pulmonary fibrosis (IPF) and an interstitial lung disease (ILD) other than IPF.

3. The method of claim 2, wherein the ILD other than IPF is sarcoidosis, Hamman-Rich syndrome, anti synthetase syndrome, silicosis, asbestosis, berylliosis, hypersensitivity pneumonitis (HP), or non-specific interstitial pneumonia (NSIP).

4. The method of claim 2, wherein the ILD other than IPF is associated with a connective tissue disease selected from systemic sclerosis, polymyositis, systemic lupus erythematosus, or rheumatoid arthritis.

5. The method of claim 2, wherein the ILD other than IPF is drug induced.

6. The method of claim 2, wherein the ILD other than IPF results from a viral infection, a bacterial infection, or tuberculosis.

7. The method of claim 2, wherein the ILD other than IPF is an ILD inflammation selected from hypersensitivity pneumonitis (HP), non-specific interstitial pneumonia (NSIP), sarcoidosis and/or organizing pneumonia.

8. The method of any one of the preceding claims, further comprising creating a report summarizing said diagnosis.

9. The method of any one of the preceding claims, further comprising providing a recommendation for treatment of the IPF.

10. The method of any one of the preceding claims, wherein the gene product is mRNA.

11. The method of any one of the preceding claims, wherein said assaying comprises determining a level of mRNA using microarray, serial analysis of gene expression, blotting, reverse transcription-polymerase chain reaction, sequencing, or quantitative polymerase chain reaction.

12. The method of any one of the preceding claims, wherein said expression level is normalized relative to the expression level of an RNA transcript of at least one reference gene.

13. The method of any one of the preceding claims, wherein the tissue sample is a lung tissue sample.

14. The method of claim 13, wherein the lung tissue sample is a biopsy sample, a bronchoalveolar lavage (BAL) sample, a transbronchial biopsy, or a cryo-transbronchial biopsy.

15. The method of any one of the preceding claims, wherein the one or more genes comprise at least two genes, at least three genes, or at least four genes.
